# EUROPEAN PATENT APPLICATION

(11) **EP 3 644 385 A1**
(43) Date of publication of application: **29.04.2020**
(21) Application number: 19204511.0
(22) Date of filing: 22.10.2019
(51) Int. Cl.: H01L 51/50, C09K 11/06, C07D 401/10, C07D 413/10, C07D 417/10, C07D 491/048, C07D 495/04, C07F 7/08

(54) **CONDENSED CYCLIC COMPOUND, COMPOSITION INCLUDING THE SAME AND ORGANIC LIGHT-EMITTING DEVICE INCLUDING THE SAME**

(30) Priority: 25.10.2018 KR 20180128325
(71) Applicant: Samsung Electronics Co., Ltd., Gyeonggi-do 16677 (KR); Samsung SDI Co., Ltd., Gyeonggi-do (KR)
(72) Inventor: JUNG, Yongsik, 16678 Gyeonggi-do (KR); KWON, Eunsuk, 16678 Gyeonggi-do (KR); KIM, Sangmo, 16678 Gyeonggi-do (KR); SON, Jhunmo, 16678 Gyeonggi-do (KR); LEE, Dongseon, 16678 Gyeonggi-do (KR); JEON, Soonok, 16678 Gyeonggi-do (KR); CHUNG, Yeonsook, 16678 Gyeonggi-do (KR); KIM, Jongsoo, 16678 Gyeonggi-do (KR)
(74) Representative: Elkington and Fife LLP

(57) **Abstract**

A condensed cyclic compound represented by Formula 1 or Formula 2: 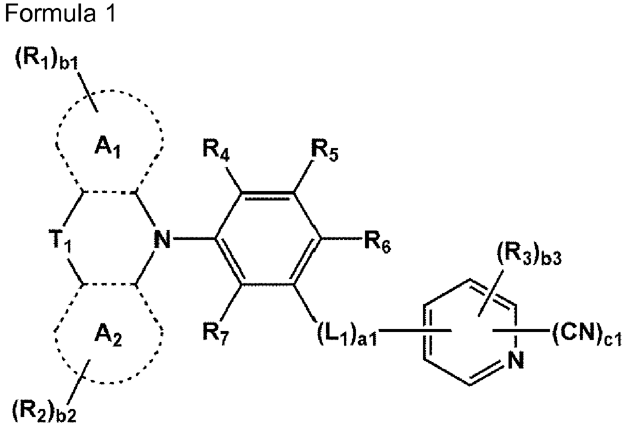 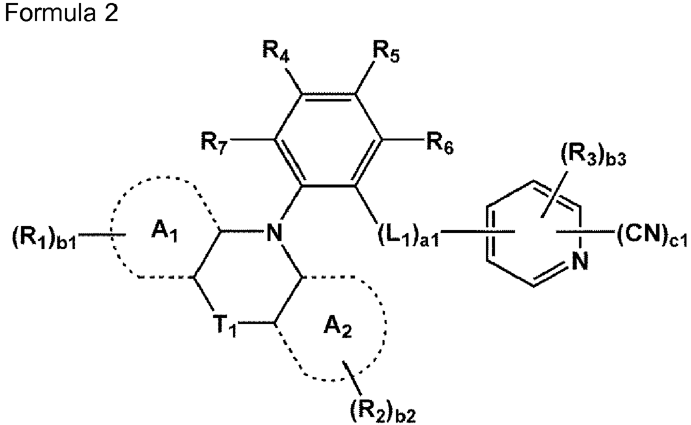 wherein ring A₁, ring A₂, T₁, L₁, a1, R₁ to R₉, b1 to b3, and c1 in Formulae 1 and 2 are the same as described in the specification.

## Description

### FIELD OF THE INVENTION

The present disclosure relates to condensed cyclic compounds, compositions containing the condensed cyclic compounds, and organic light-emitting devices including the compounds or the compositions.

### BACKGROUND OF THE INVENTION

Organic light-emitting devices (OLEDs) are self-emissive devices that have wide viewing angles, high contrast ratios, and short response times, and that show excellent characteristics in terms of luminance, driving voltage, and response speed.

In an example, an organic light-emitting device includes an anode, a cathode, and an organic layer disposed between the anode and the cathode, wherein the organic layer includes an emission layer. A hole transport region may be disposed between the anode and the emission layer, and an electron transport region may be disposed between the emission layer and the cathode. Holes provided from the anode may move toward the emission layer through the hole transport region, and electrons provided from the cathode may move toward the emission layer through the electron transport region. The holes and the electrons, which are carriers, recombine in the emission layer to produce excitons. These excitons transition from an excited state to a ground state, thereby generating light.

Various types of organic light emitting devices are known. However, there still remains a need in OLEDs having low driving voltage, high efficiency, high brightness, and long lifespan.

### SUMMARY OF THE INVENTION

Provided are a condensed cyclic compound, a composition containing the same, and an organic light-emitting device using the same.

Additional aspects will be set forth in part in the description which follows and, in part, will be apparent from the description, or may be learned by practice of the presented embodiments.

According to an aspect of an embodiment, condensed cyclic compounds are represented by Formula 1 or Formula 2: 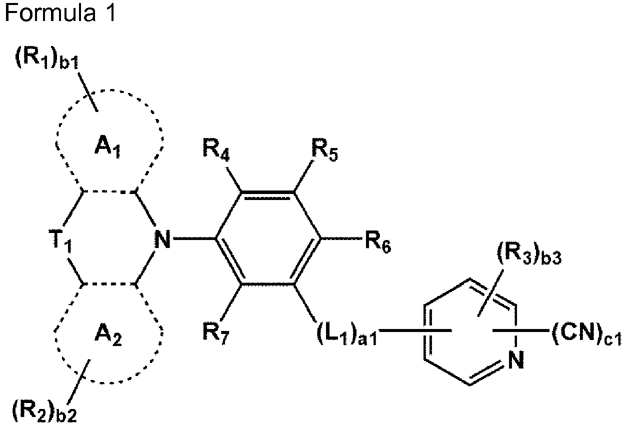 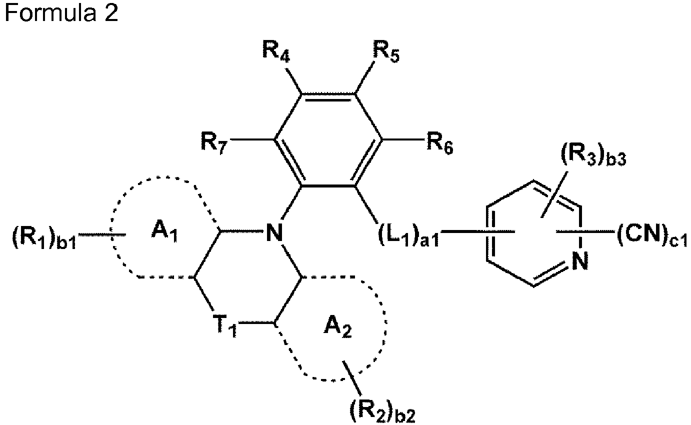 wherein, in Formulae 1 and 2,
ring A₁ and ring A₂ are each independently a C₅-C₃₀ carbocyclic group or a C₁-C₃₀ heterocyclic group,
T₁ is a single bond, O, S, N(R₈), C(R₈)(R₉), or Si(R₈)(R₉),
L₁ is a single bond, a C₆-C₆₀ arylene group unsubstituted or substituted with at least one R₁₀ₐ, a C₁-C₆₀ heteroarylene group unsubstituted or substituted with at least one R₁₀ₐ, a divalent non-aromatic condensed polycyclic group unsubstituted or substituted with at least one R₁₀ₐ, or a divalent non-aromatic condensed heteropolycyclic group unsubstituted or substituted with at least one R₁₀ₐ,
a1 is an integer from 1 to 5, wherein when a1 is two or more, two or more groups L₁ may be identical to or different from each other,
R₁ to R₉ may each independently be selected from hydrogen, deuterium, -F, -CI, -Br, -I, a hydroxyl group, a cyano group (CN), a nitro group, an amino group, an amidino group, a hydrazine group, a hydrazone group, a carboxylic acid group or a salt thereof, a sulfonic acid group or a salt thereof, a phosphoric acid group or a salt thereof, a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₇-C₆₀ arylalkyl group, a substituted or unsubstituted C₁-C₆₀ heteroaryl group, a substituted or unsubstituted C₁-C₆₀ heteroaryloxy group, a substituted or unsubstituted C₁-C₆₀ heteroarylthio group, a substituted or unsubstituted C₂-C₆₀ heteroarylalkyl group, a substituted or unsubstituted monovalent non-aromatic condensed polycyclic group, a substituted or unsubstituted monovalent non-aromatic condensed heteropolycyclic group, -Si(Q₁)(Q₂)(Q₃), -N(Q₄)(Q₅), -B(Q₆)(Q₇), -S(Q₁), and -C(=O)(Q₁),
b1 and b2 may each independently be an integer from 0 to 10,
b3 may be an integer from 0 to 3,
c1 may be an integer from 1 to 4,
R₁₀ₐ may be the same as described in connection with R₄,
at least one substituent selected from the substituted C₁-C₆₀ alkyl group, the substituted C₂-C₆₀ alkenyl group, the substituted C₂-C₆₀ alkynyl group, the substituted C₁-C₆₀ alkoxy group, the substituted C₃-C₁₀ cycloalkyl group, the substituted C₁-C₁₀ heterocycloalkyl group, the substituted C₃-C₁₀ cycloalkenyl group, the substituted C₁-C₁₀ heterocycloalkenyl group, the substituted C₆-C₆₀ aryl group, the substituted C₆-C₆₀ aryloxy group, the substituted C₆-C₆₀ arylthio group, the substituted C₇-C₆₀ arylalkyl group, the substituted C₁-C₆₀ heteroaryl group, the substituted C₁-C₆₀ heteroaryloxy group, the substituted C₁-C₆₀ heteroarylthio group, the substituted C₂-C₆₀ heteroarylalkyl group, the substituted monovalent non-aromatic condensed polycyclic group, and the substituted monovalent non-aromatic condensed heteropolycyclic group may be selected from:
   deuterium, -CD₃, -CD₂H, -CDH₂, -F, -CI, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, and a C₁-C₆₀ alkoxy group;
   a C₁-C₆₀ alkyl group, a C₂-C₆₀ alkenyl group, a C₂-C₆₀ alkynyl group, and a C₁-C₆₀ alkoxy group, each substituted with at least one selected from deuterium,-CD₃, -CD₂H, -CDH₂, -F, -CI, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₇-C₆₀ arylalkyl group, a C₁-C₆₀ heteroaryl group, a C₁-C₆₀ heteroaryloxy group, a C₁-C₆₀ heteroarylthio group, a C₂-C₆₀ heteroarylalkyl group, a monovalent non-aromatic condensed polycyclic group, a monovalent non-aromatic condensed heteropolycyclic group, -Si(Q₁₁)(Q₁₂)(Q₁₃), -N(Q₁₄)(Q₁₅), and -B(Q₁₆)(Q₁₇);
   a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₇-C₆₀ arylalkyl group, a C₁-C₆₀ heteroaryl group, a C₁-C₆₀ heteroaryloxy group, a C₁-C₆₀ heteroarylthio group, a C₂-C₆₀ heteroarylalkyl group, a monovalent non-aromatic condensed polycyclic group, and a monovalent non-aromatic condensed heteropolycyclic group;
   a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₇-C₆₀ arylalkyl group, a C₁-C₆₀ heteroaryl group, a C₁-C₆₀ heteroaryloxy group, a C₁-C₆₀ heteroarylthio group, a C₂-C₆₀ heteroarylalkyl group, a monovalent non-aromatic condensed polycyclic group, and a monovalent non-aromatic condensed heteropolycyclic group, each substituted with at least one selected from deuterium, -CD₃, -CD₂H, -CDH₂, -F, -CI, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₇-C₆₀ arylalkyl group, a C₁-C₆₀ heteroaryl group, a C₁-C₆₀ heteroaryloxy group, a C₁-C₆₀ heteroarylthio group, a C₂-C₆₀ heteroarylalkyl group, a monovalent non-aromatic condensed polycyclic group, a monovalent non-aromatic condensed heteropolycyclic group, -Si(Q₂₁)(Q₂₂)(Q₂₃), -N(Q₂₄)(Q₂₅), and-B(Q₂₆)(Q₂₇); and
   -Si(Q₃₁)(Q₃₂)(Q₃₃), -N(Q₃₄)(Q₃₅), and -B(Q₃₆)(Q₃₇), and
Q₁ to Q₇, Q₁₁ to Q₁₇, Q₂₁ to Q₂₇, and Q₃₁ to Q₃₇ may each independently be selected from hydrogen, deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₁-C₆₀ heteroaryl group, a monovalent non-aromatic condensed polycyclic group, and a monovalent non-aromatic condensed heteropolycyclic group.

Another aspect provides a composition including:
a first compound and a second compound, wherein
the first compound is the condensed cyclic compound,
the second compound is different from the first compound,
the second compound includes at least one selected from a carbazole group, a dibenzofuran group, a dibenzothiophene group, an indenocarbazole group, an indolocarbazole group, a benzofurocarbazole group, a benzothienocarbazole group, an acridine group, a dihydroacridine group, and a triindolobenzene group, and does not include an electron withdrawing group,
the electron withdrawing group may be selected from
   -F, -CFH₂, -CF₂H, -CF₃, -CN, and -NO₂;
a C₁-C₆₀ alkyl group substituted with at least one selected from -F, -CFH₂,-CF₂H, -CF₃, -CN, and -NO₂;
a C₁-C₆₀ heteroaryl group and a monovalent non-aromatic condensed polycyclic heterocyclic group, each including *=N-*' as a ring-forming moiety; and
a C₁-C₆₀ heteroaryl group and a monovalent non-aromatic condensed polycyclic heterocyclic group, each of which includes *=N-*' as a ring-forming moiety and is substituted with at least one selected from deuterium, -F, -CFH₂, -CF₂H, -CF₃, -CI, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₇-C₆₀ arylalkyl group, a substituted or unsubstituted C₁-C₆₀ heteroaryl group, a substituted or unsubstituted C₁-C₆₀ heteroaryloxy group, a substituted or unsubstituted C₁-C₆₀ heteroarylthio group, a substituted or unsubstituted C₂-C₆₀ heteroarylalkyl group, a substituted or unsubstituted monovalent non-aromatic condensed polycyclic group, and a substituted or unsubstituted monovalent non-aromatic condensed heteropolycyclic group.

Another aspect provides an organic light-emitting device including:
a first electrode;
a second electrode; and
an organic layer including an emission layer disposed between the first electrode and the second electrode, wherein the organic layer includes at least one condensed cyclic compound.

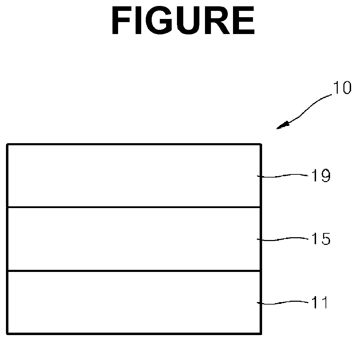

### BRIEF DESCRIPTION OF THE DRAWING

These and/or other aspects will become apparent and more readily appreciated from the following description of the embodiments, taken in conjunction with
the FIGURE which shows a schematic cross-sectional view of an organic light-emitting device according to an embodiment.

### DETAILED DESCRIPTION OF THE EMBODIMENTS

Reference will now be made in detail to embodiments, examples of which are illustrated in the accompanying drawings, wherein like reference numerals refer to like elements throughout. In this regard, the present embodiments may have different forms and should not be construed as being limited to the descriptions set forth herein. Accordingly, the embodiments are merely described below, by referring to the figures, to explain aspects. As used herein, the term "and/or" includes any and all combinations of one or more of the associated listed items. Expressions such as "at least one of," when preceding a list of elements, modify the entire list of elements and do not modify the individual elements of the list.

Reference will now be made in detail to embodiments, examples of which are illustrated in the accompanying drawings, wherein like reference numerals refer to like elements throughout. In this regard, the present embodiments may have different forms and should not be construed as being limited to the descriptions set forth herein. Accordingly, the embodiments are merely described below, by referring to the figures, to explain aspects of the present description. As used herein, the term "and/or" includes any and all combinations of one or more of the associated listed items. Expressions such as "at least one of," when preceding a list of elements, modify the entire list of elements and do not modify the individual elements of the list.

It will be understood that when an element is referred to as being "on" another element, it can be directly in contact with the other element or intervening elements may be present therebetween. In contrast, when an element is referred to as being "directly on" another element, there are no intervening elements present.

It will be understood that, although the terms first, second, third etc. may be used herein to describe various elements, components, regions, layers, and/or sections, these elements, components, regions, layers, and/or sections should not be limited by these terms. These terms are only used to distinguish one element, component, region, layer, or section from another element, component, region, layer, or section. Thus, a first element, component, region, layer, or section discussed below could be termed a second element, component, region, layer, or section without departing from the teachings of the present embodiments.

The terminology used herein is for the purpose of describing particular embodiments only and is not intended to be limiting. As used herein, the singular forms "a," "an," and "the" are intended to include the plural forms as well, unless the context clearly indicates otherwise.

The term "or" means "and/or." It will be further understood that the terms "comprises" and/or "comprising," or "includes" and/or "including" when used in this specification, specify the presence of stated features, regions, integers, steps, operations, elements, and/or components, but do not preclude the presence or addition of one or more other features, regions, integers, steps, operations, elements, components, and/or groups thereof.

Unless otherwise defined, all terms (including technical and scientific terms) used herein have the same meaning as commonly understood by one of ordinary skill in the art to which this general inventive concept belongs. It will be further understood that terms, such as those defined in commonly used dictionaries, should be interpreted as having a meaning that is consistent with their meaning in the context of the relevant art and the present disclosure, and will not be interpreted in an idealized or overly formal sense unless expressly so defined herein.

Exemplary embodiments are described herein with reference to cross section illustrations that are schematic illustrations of idealized embodiments. As such, variations from the shapes of the illustrations as a result, for example, of manufacturing techniques and/or tolerances, are to be expected. Thus, embodiments described herein should not be construed as limited to the particular shapes of regions as illustrated herein but are to include deviations in shapes that result, for example, from manufacturing. For example, a region illustrated or described as flat may, typically, have rough and/or nonlinear features. Moreover, sharp angles that are illustrated may be rounded. Thus, the regions illustrated in the figures are schematic in nature and their shapes are not intended to illustrate the precise shape of a region and are not intended to limit the scope of the present claims.

"About" or "approximately" as used herein is inclusive of the stated value and means within an acceptable range of deviation for the particular value as determined by one of ordinary skill in the art, considering the measurement in question and the error associated with measurement of the particular quantity (*i.e.,* the limitations of the measurement system). For example, "about" can mean within one or more standard deviations, or within ± 30%, 20%, 10%, 5% of the stated value.

The condensed cyclic compound may be represented by Formula 1 or Formula 2:

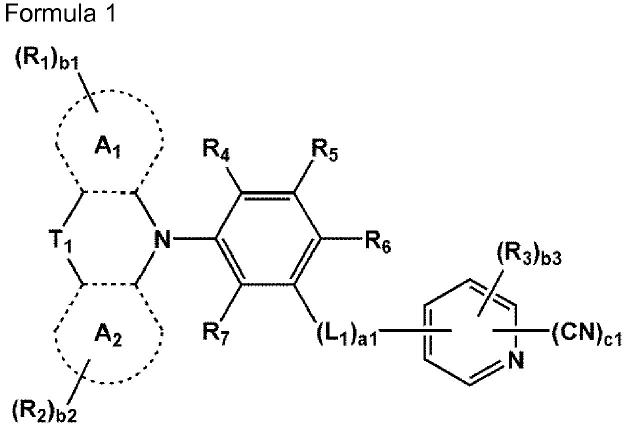

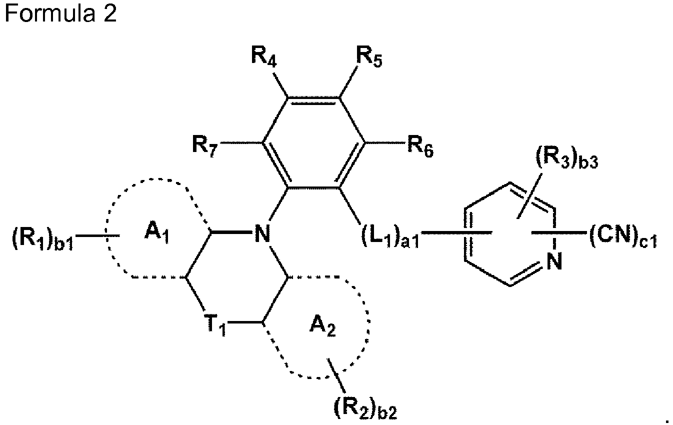

Ring A₁ and ring A₂ in Formula 1 and Formula 2 may each independently be a C₅-C₃₀ carbocyclic group or a C₁-C₃₀ heterocyclic group.

For example, ring A₁ and ring A₂ may each independently be a benzene group, a naphthalene group, a phenanthrene group, an anthracene group, a cyclopentene group, a pyrrole group, a furan group, a thiophene group, a silole group, an indene group, an indole group, a benzofuran group, a benzothiophene group, a benzosilole group, a fluorene group, a carbazole group, a dibenzofuran group, a dibenzothiophene group, a dibenzosilole group, a benzocarbazole group, an indenocarbazole group, an indolocarbazole group, a benzofurocarbazole group, a benzothienocarbazole group, an acridine group, a dihydroacridine group, or a triindolobenzene group.

In an embodiment, ring A₁ and ring A₂ may be a benzene group, but embodiments of the present disclosure are not limited thereto.

T₁ in Formula 1 and 2 may be a single bond, O, S, N(R₈), C(R₈)(R₉), or Si(R₈)(R₉).

In an embodiment, T₁ may be a single bond, but embodiments of the present disclosure are not limited thereto.

For example, a group represented by 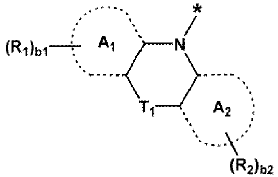 in Formulae 1 and 2 may be a group represented by one of Formulae 3-1 to 3-7:

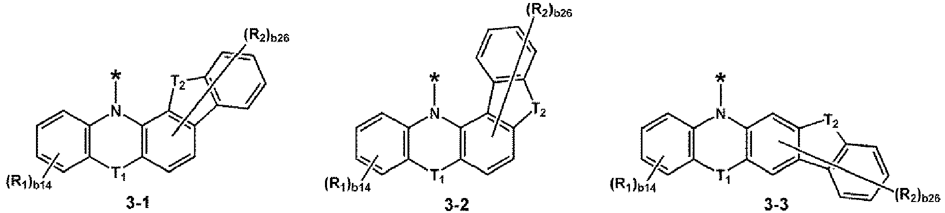

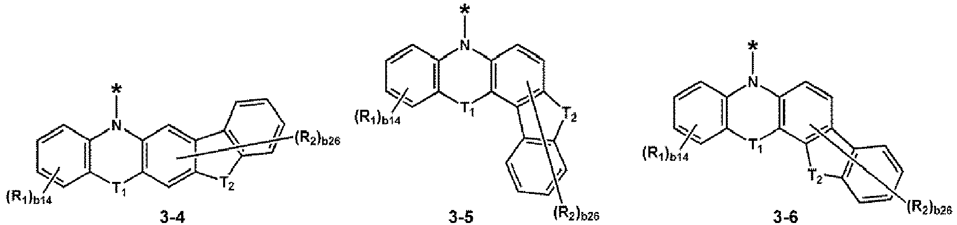

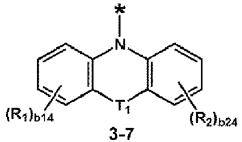

In Formulae 3-1 to 3-7,
T₁, R₁, and R₂ are the same as described herein
T₂ is C(R₁₀)(R₁₁), Si(R₁₀)(R₁₁), N(R₁₀), O, or S,
R₁₀ and R₁₁ are the same as described in connection with R₂,
b14 and b24 may each independently be an integer from 0 to 4,
b26 may be an integer from 0 to 6, and
* indicates a binding site to a neighboring atom.

L₁ in Formulae 1 and 2 may be a single bond, a C₆-C₆₀ arylene group unsubstituted or substituted with at least one R₁₀ₐ, a C₁-C₆₀ heteroarylene group unsubstituted or substituted with at least one R₁₀ₐ, a divalent non-aromatic condensed polycyclic group unsubstituted or substituted with at least one R₁₀ₐ, or a divalent non-aromatic condensed heteropolycyclic group unsubstituted or substituted with at least one R₁₀ₐ. R₁₀ₐ may be the same as described in connection with R₄.

For example, L₁ may be selected from
a single bond; and
a cyclopentylene group, a cyclohexylene group, a cyclopentenylene group, a cyclohexenylene group, a cycloheptenylene group, a phenylene group, a biphenylene group, teraphenylene group, a pentalenylene group, an indenylene group, a naphthylene group, an azulenylene group, a heptalenylene group, an indacenylene group, an acenaphthylene group, a fluorenylene group, a spiro-bifluorenylene group, a phenalenylene group, a phenanthrenylene group, an anthracenylene group, a fluoranthenylene group, a triphenylenylene group, a pyrenylene group, a chrysenylene group, a naphthacenylene group, a picenylene group, a perylenylene group, a pentaphenylene group, a hexacenylene group, a pyrrolylene group, an imidazolylene group, a pyrazolylene group, a pyridinylene group, a pyrazinylene group, a pyrimidinylene group, a pyridazinylene group, an isoindolylene group, an indolylene group, an indazolylene group, a purinylene group, a quinolinylene group, an isoquinolinylene group, a benzoquinolinylene group, a phthalazinylene group, a naphthyridinylene group, a quinoxalinylene group, a quinazolinylene group, a cinnolinylene group, a carbazolylene group, a phenanthridinylene group, an acridinylene group, a phenanthrolinylene group, a phenazinylene group, a benzoxazolylene group, a benzimidazolylene group, a furanylene group, a benzofuranylene group, a thiophenylene group, a benzothiophenylene group, a thiazolylene group, an isothiazolylene group, a benzothiazolylene group, an isoxazolylene group, an oxazolylene group, a triazolylene group, a tetrazolylene group, an oxadiazolylene group, a triazinylene group, a dibenzofuranylene group, a dibenzothiophenylene group, a benzocarbazolylene group, a dibenzocarbazolylene group, imidazopyridimidinylene group, an imidazopyridinylene group, a pyridoindolylene group, a benzofuropyridinylene group, a benzothienopyridinylene group, a pyrimidoindolylene group, benzofuropyrimidinylene group, a benzothienopyrimidinylene group, a phenoxazinylene group, a pyridobenzoxazinylene group, and a pyridobenzothiazinylene group, each unsubstituted or substituted with at least one R₁₀ₐ.

In an embodiment, L₁ in Formulae 1 and 2 may be a single bond; or a group represented by one of Formulae 4-1 to 4-38:

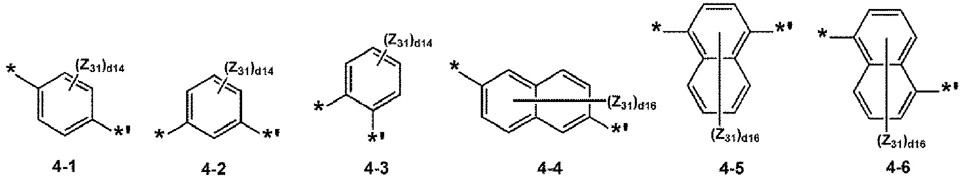

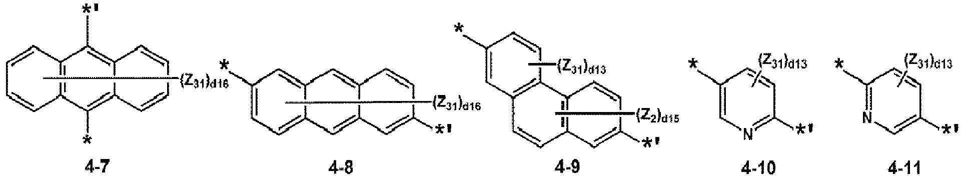

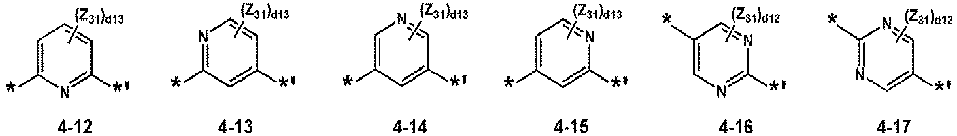

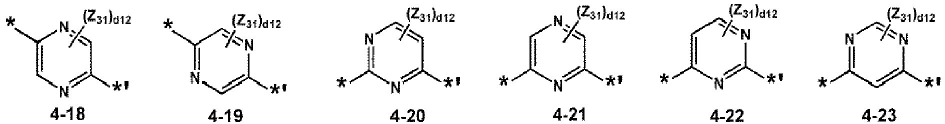

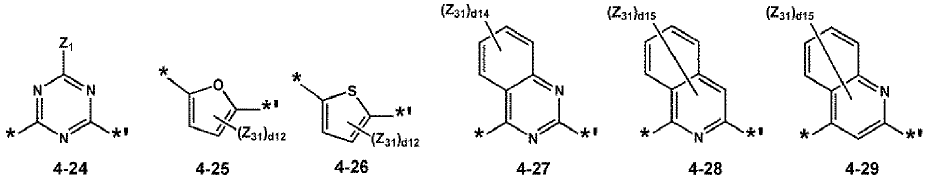

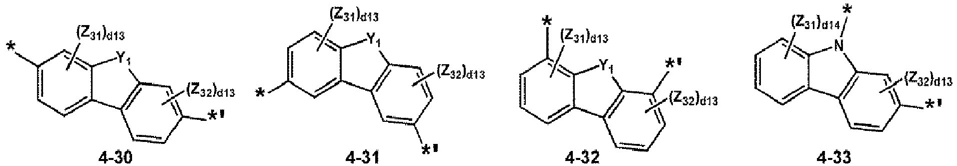

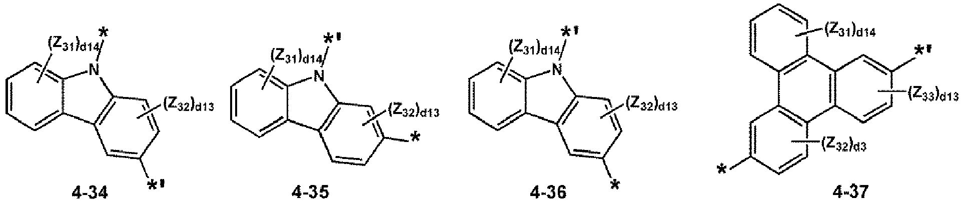

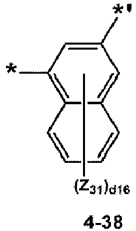

In Formulae 4-1 to 4-38,
Y₁ may be O, S, C(Z₃₄)(Z₃₅), N(Z₃₄), or Si(Z₃₄)(Z₃₅),
Z₃₁ to Z₃₅ are the same as described in connection with R₄,
d16 may be an integer from 0 to 6,
d15 may be an integer from 0 to 5,
d14 may be an integer from 0 to 4,
d13 may be an integer from 0 to 3,
d12 may be an integer from 0 to 2,
* and *' each indicate a binding site to a neighboring atom.

In an embodiment, L₁ in Formulae 1 and 2 may be a single bond; or a group represented by one of Formulae 5-1 to 5-9:

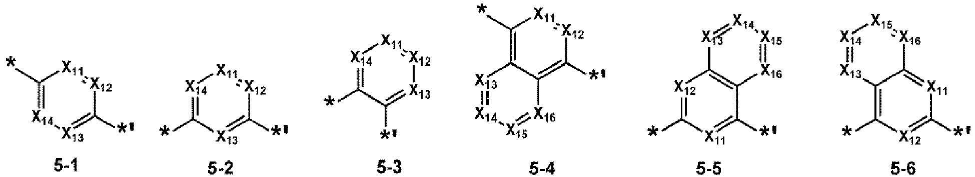

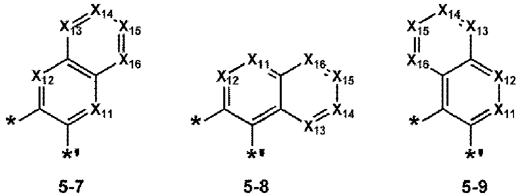

In Formulae 5-1 to 5-9,
X₁₁ may be C(Z₄₁) or N, X₁₂ may be C(Z₄₂) or N, X₁₃ may be C(Z₄₃) or N, X₁₄ may be C(Z₄₄) or N, X₁₅ may be C(Z₄₅) or N, and X₁₆ may be C(Z₄₆) or N,
Z₄₁ to Z₄₆ are the same as described in connection with R₄,
* and *' each indicate a binding site to a neighboring atom.

For example, in Formulae 5-1 to 5-3, X₁₁ may be C(Z₄₁), X₁₂ may be C(Z₄₂), X₁₃ may be C(Z₄₃), and X₁₄ may be C(Z₄₄).

In an embodiment, at least one, two or three selected from X₁₁ to X₁₄ of Formulae 5-1 to 5-3 may be N.

In an embodiment, in Formulae 5-4 to 5-9, X₁₁ may be C(Z₄₁), X₁₂ may be C(Z₄₂), X₁₃ may be C(Z₄₃), X₁₄ may be C(Z₄₄), X₁₅ may be C(Z₄₅), and X₁₆ may be C(Z₄₆).

In one or more embodiments, L₁ in Formulae 1 and 2 may be a single bond; or a group represented by one of Formulae 5-2 and 5-3, but embodiments of the present disclosure are not limited thereto.

a1 in Formula 1 and 2 indicates the number of groups L₁, and may be an integer from 1 to 5. When a1 is two or more, two or more of groups L₁ may be identical to or different from each other. For example, a1 may be 1, 2, or 3. In an embodiment, a1 may be 1 or 2, but embodiments of the present disclosure are not limited thereto.

In an embodiment, in Formulae 1 and 2,
i) L₁ may be a single bond, or
ii) L₁ may not be a single bond and a1 may be 1, 2, or 3 (for example, 1 or 2).

R₁ to R₉ may each independently be selected from hydrogen, deuterium, -F, -Cl, -Br, -I, a hydroxyl group, a cyano group (CN), a nitro group, an amino group, an amidino group, a hydrazine group, a hydrazone group, a carboxylic acid group or a salt thereof, a sulfonic acid group or a salt thereof, a phosphoric acid group or a salt thereof, a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₇-C₆₀ arylalkyl group, a substituted or unsubstituted C₁-C₆₀ heteroaryl group, a substituted or unsubstituted C₁-C₆₀ heteroaryloxy group, a substituted or unsubstituted C₁-C₆₀ heteroarylthio group, a substituted or unsubstituted C₂-C₆₀ heteroarylalkyl group, a substituted or unsubstituted monovalent non-aromatic condensed polycyclic group, a substituted or unsubstituted monovalent non-aromatic condensed heteropolycyclic group,-Si(Q₁)(Q₂)(Q₃), -N(Q₄)(Q₅), -B(Q₆)(Q₇), -S(Q₁), and -C(=O)(Q₁). Q₁ to Q₇ are the same as described herein.

In an embodiment, R₁ to R₉ may each independently be selected from:
hydrogen, deuterium, -F, a hydroxyl group, a cyano group, and a nitro group;
a C₁-C₂₀ alkyl group and a C₁-C₂₀ alkoxy group, each unsubstituted or substituted with at least one selected from deuterium, -F, a hydroxyl group, a cyano group, and a nitro group;
a cyclopentyl group, a cyclohexyl group, a cyclopentenyl group, a cyclohexenyl group, a cycloheptenyl group, a phenyl group, a biphenyl group, a terphenyl group, a pentalenyl group, an indenyl group, a naphthyl group, an azulenyl group, a heptalenyl group, an indacenyl group, an acenaphthyl group, a fluorenyl group, a spiro-bifluorenyl group, a phenalenyl group, a phenanthrenyl group, an anthracenyl group, a fluoranthenyl group, a triphenylenyl group, a pyrenyl group, a chrysenyl group, a naphthacenyl group, a picenyl group, a perylenyl group, a pentaphenyl group, a hexacenyl group, a pyrrolyl group, an imidazolyl group, a pyrazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a carbazolyl group, a phenanthridinyl group, an acridinyl group, a phenanthrolinyl group, a phenazinyl group, a benzoxazolyl group, a benzimidazolyl group, a furanyl group, a benzofuranyl group, a thiophenyl group, a benzothiophenyl group, a thiazolyl group, an isothiazolyl group, a benzothiazolyl group, an isoxazolyl group, an oxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyridimidinyl group, an imidazopyridinyl group, a pyridoindolyl group, a benzofuropyridinyl group, a benzothienopyridinyl group, a pyrimidoindolyl group, a benzofuropyrimidinyl group, a benzothienopyrimidinyl group, a phenoxazinyl group, a pyridobenzoxazinyl group, and a pyridobenzothiazinyl group, each unsubstituted or substituted with at least one selected from deuterium, -F, a hydroxyl group, a cyano group, a nitro group, a C₁-C₂₀ alkyl group, a C₁-C₂₀ alkoxy group, a phenyl group, a biphenyl group, and a terphenyl group; and
-N(Q₄)(Q₅), -S(Q₁), and -C(=O)(Q₁), and
Q₁, Q₄, and Q₅ may each independently be selected from hydrogen, deuterium, a C₁-C₁₀ alkyl group, a phenyl group, a naphthyl group, a fluorenyl group, a carbazolyl group, a dibenzofuranyl group, a dibenzothiophenyl group, and a biphenyl group.

In one or more embodiments, R₁ to R₉ may each independently be selected from:
hydrogen, deuterium, -F, a cyano group, and a nitro group;
a C₁-C₁₀ alkyl group and a C₁-C₁₀ alkoxy group, each unsubstituted or substituted with at least one selected from deuterium, -F, a cyano group, and a nitro group;
a phenyl group, a biphenyl group, a terphenyl group, a naphthyl group, a fluorenyl group, a carbazolyl group, a dibenzofuranyl group, and a dibenzothiophenyl group, each unsubstituted or substituted with at least one selected from deuterium, -F, a cyano group, a nitro group, a C₁-C₂₀ alkyl group, a C₁-C₁₀ alkoxy group, a phenyl group, a biphenyl group, and a terphenyl group; and
-N(Q₄)(Q₅), -S(Q₁), and -C(=O)(Q₁),
Q₁, Q₄, and Q₅ may each independently be selected from hydrogen, deuterium, a C₁-C₁₀ alkyl group, a phenyl group, a naphthyl group, a fluorenyl group, a carbazolyl group, a dibenzofuranyl group, a dibenzothiophenyl group, and a biphenyl group, but embodiments of the present disclosure are not limited thereto.

b1 to b3 in Formulae 1 and 2 indicate the numbers of R₁ to R₃, respectively, and b1 and b2 may each independently be an integer from 0 to 10, and b3 may be an integer from 0 to 3. When b1 is two or more, two or more of groups R₁ may be identical to or different from each other, and when b2 is two or more, two or more of groups R₂ may be identical to or different from each other, and when b3 is two or more, two or more of groups R₃ may be identical to or different from each other.

For example, b1 and b2 may each independently be 0, 1, or 2, and b3 may be 0 or 1, but embodiments of the present disclosure are not limited thereto.

For example, a group represented by 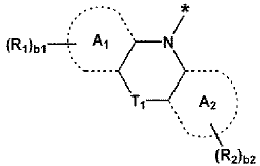 in Formulae 1 and 2 may be a group represented by Formula 3(1):

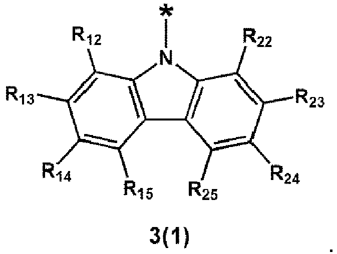

Regarding Formula 3(1), R₁₂ to R₁₅ are each the same as described in connection with R₁, and R₂₂ to R₂₅ are each the same as described in connection with R₂, and * indicates a binding site to a neighboring atom.

For example, R₁₂ to R₁₅ and R₂₂ to R₂₅ of Formula 3(1) may each be hydrogen.

In an embodiment, at least one selected from R₁₄ and R₂₄ in Formula 3(1) may not be hydrogen.

In an embodiment, at least one selected from R₁₄ and R₂₄ in Formula 3(1) may each independently be a cyano group, a C₁-C₁₀ alkyl group, a phenyl group, a (C₁-C₁₀ alkyl)phenyl group, a di(C₁-C₁₀ alkyl)phenyl group, a biphenyl group, a di(phenyl)phenyl group, a (cyano)phenyl group, or a di(cyano) phenyl group.

c1 in Formulae 1 and 2 indicates the number of cyano groups, and may be an integer from 1 to 4. For example, c1 may be 1 or 2, but embodiments of the present disclosure are not limited thereto.

In an embodiment, a group represented by 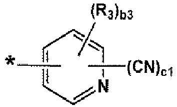 in Formulae 1 and 2 may be a group represented by one of Formulae 6-1 to 6-3:

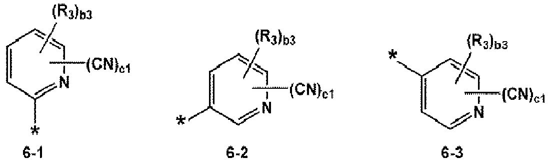

R₃, b3, and c1 in Formulae 6-1 to 6-3 are the same as described herein, and * indicates a binding site to a neighboring atom.

In one or more embodiments, a group represented by 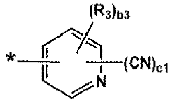 in Formulae 1 and 2 may be a group represented by one of Formulae 7-1 to 7-39:

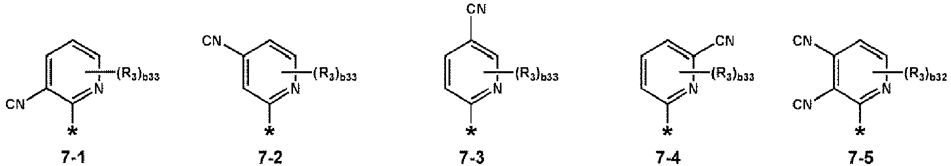

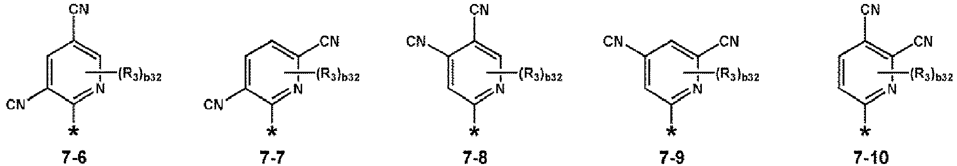

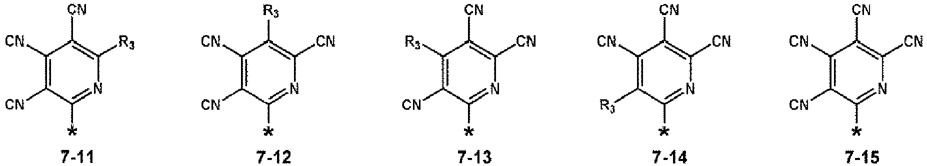

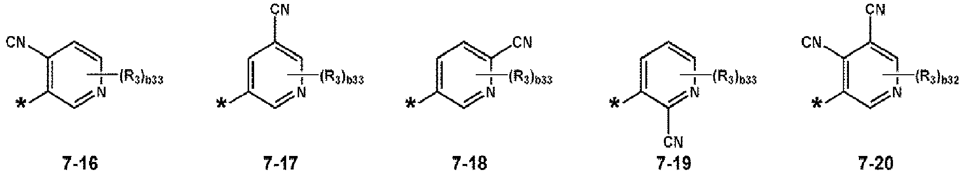

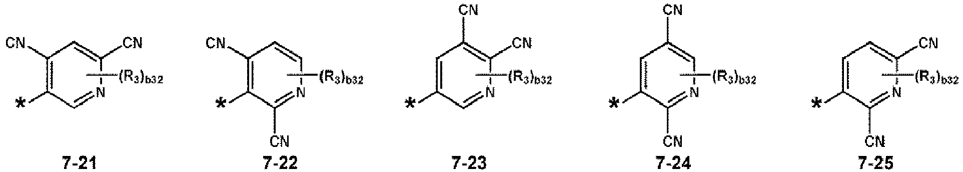

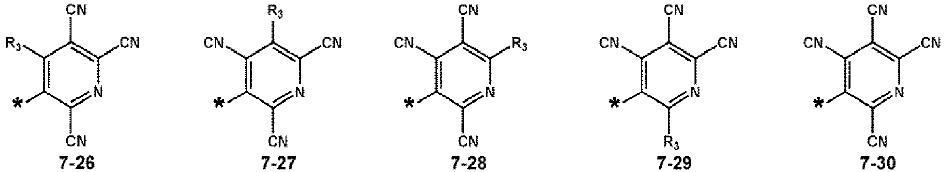

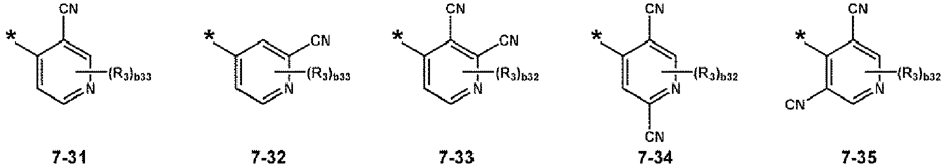

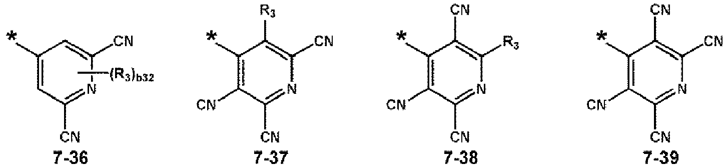

In Formulae 7-1 to 7-39,
R₃ may be understood by referring to the description above and R₃ is not a cyano group,
b33 may be an integer from 0 to 3,
b32 may be an integer from 0 to 2,
* indicates a binding site to a neighboring atom.

In one or more embodiments, the number of cyano groups included in each of Formulae 1 and 2 may be 1, 2, or 3. In other words, the number of cyano groups included in the condensed cyclic compound represented by Formula 1 or Formula 2 may be 1, 2, or 3.

For example, the condensed cyclic compound may be selected from Compounds 1 to 1000, but embodiments of the present disclosure are not limited thereto:

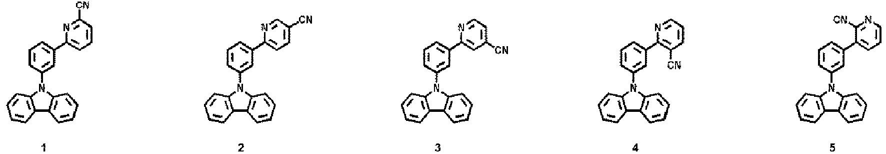

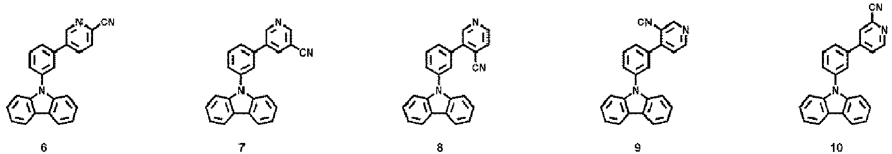

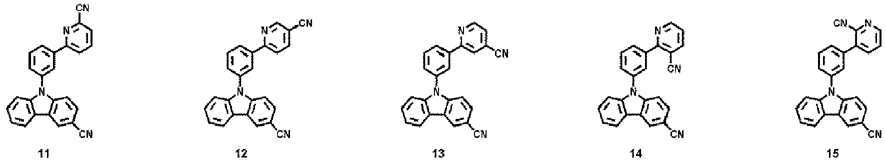

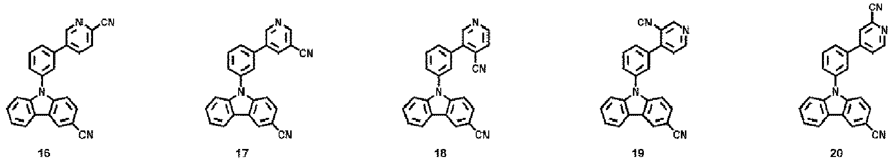

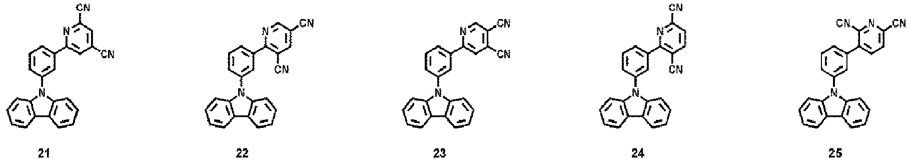

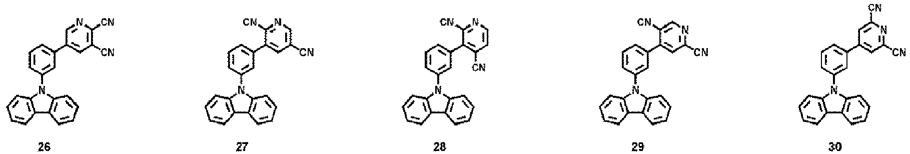

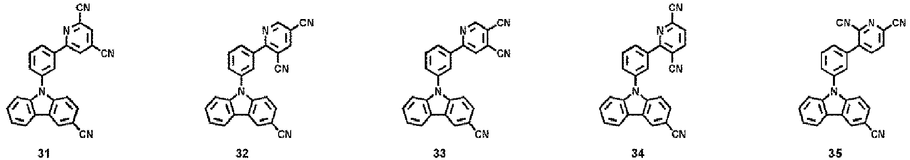

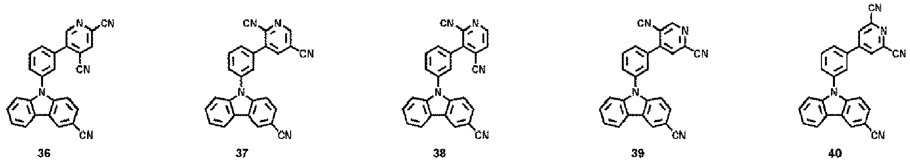

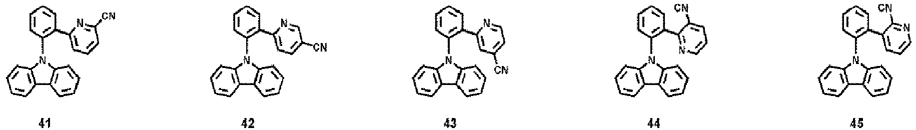

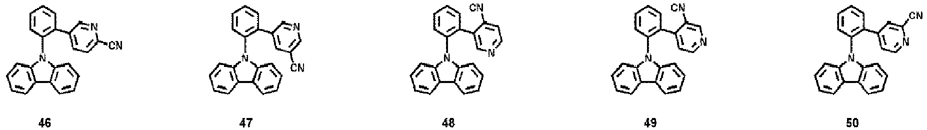

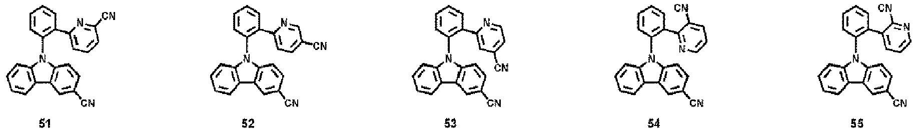

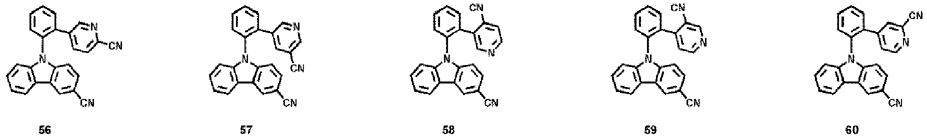

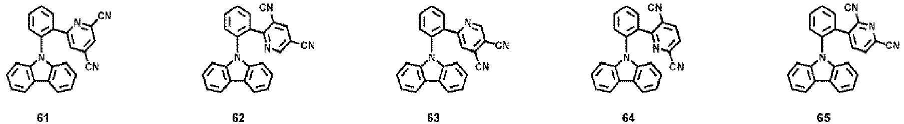

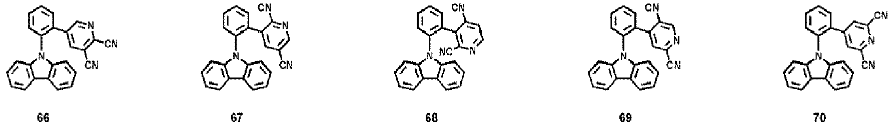

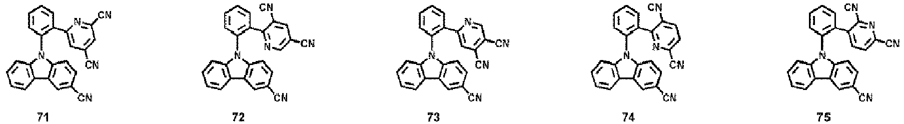

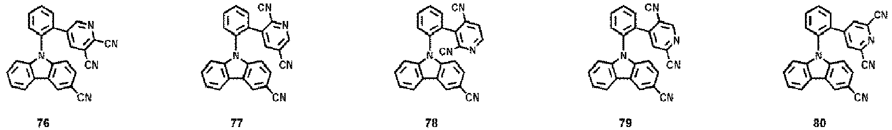

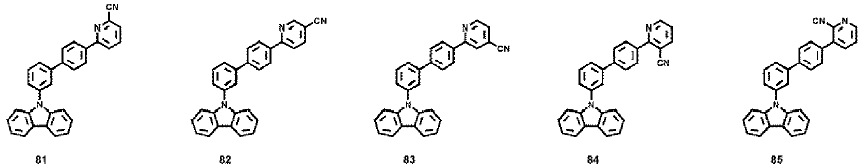

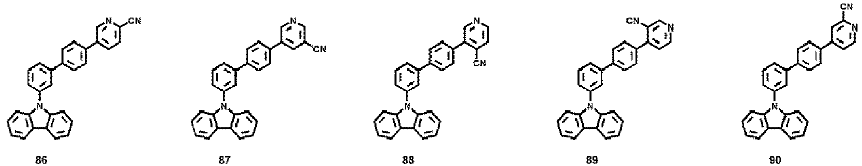

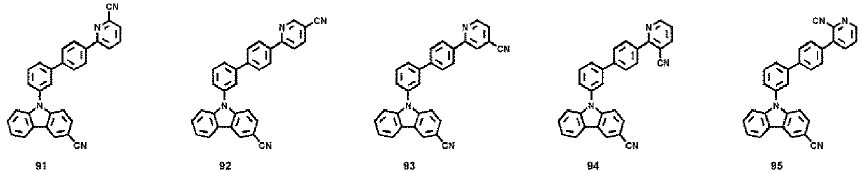

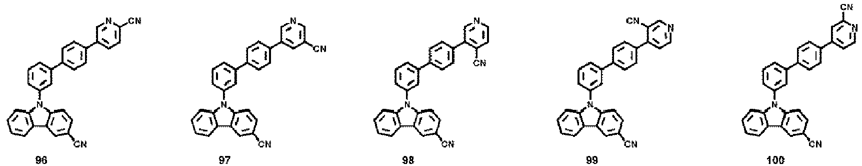

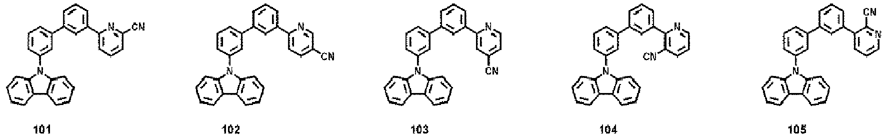

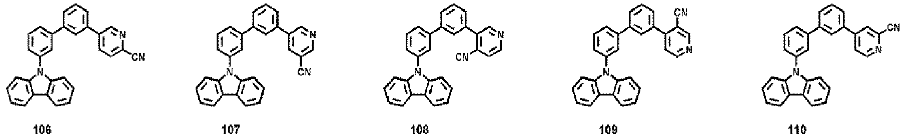

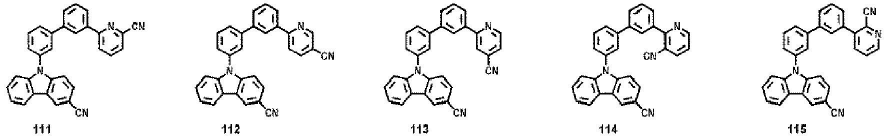

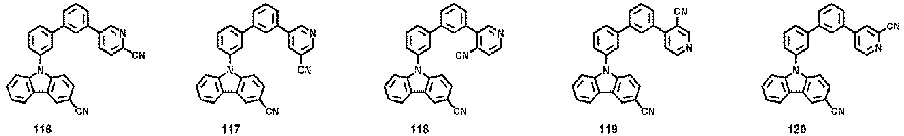

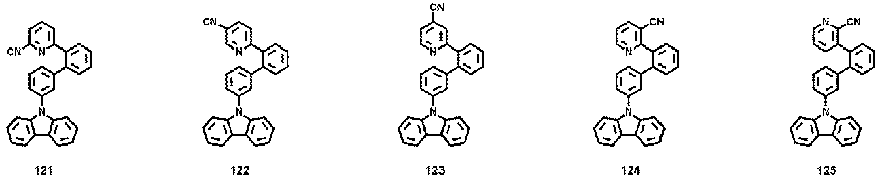

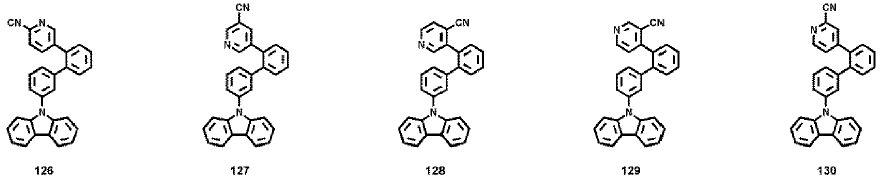

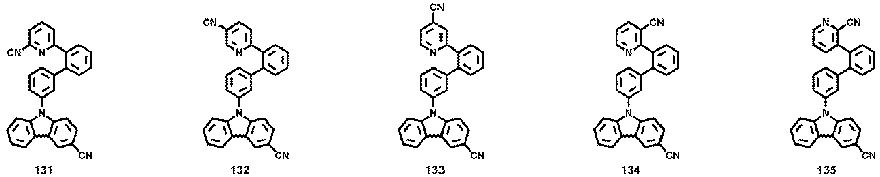

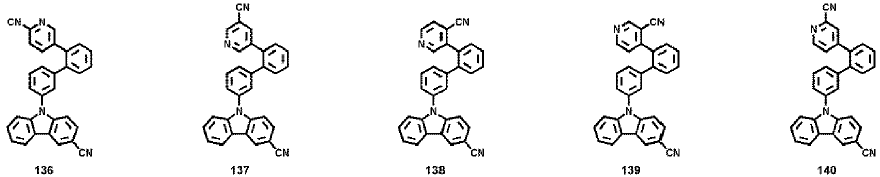

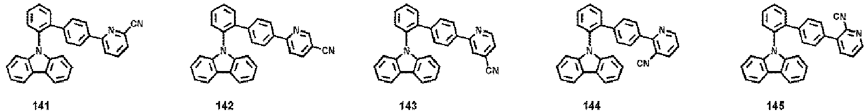

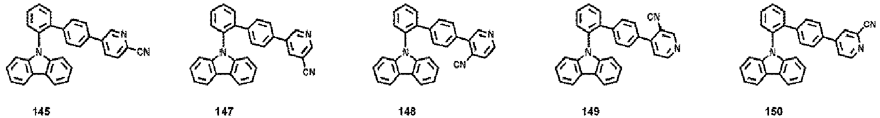

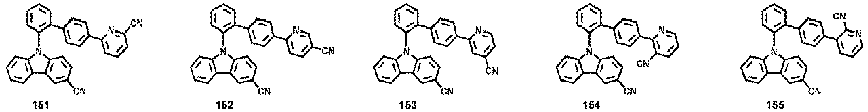

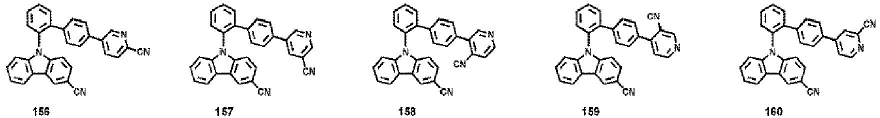

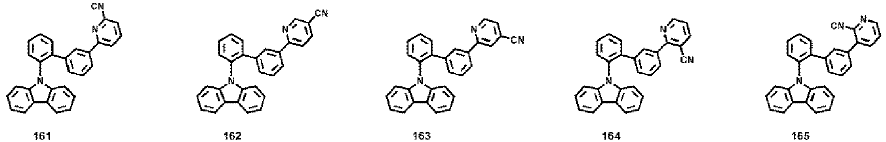

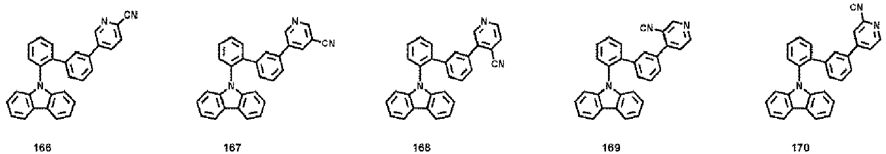

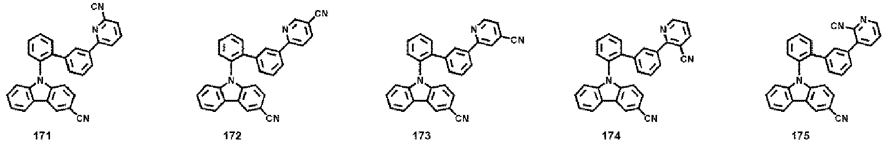

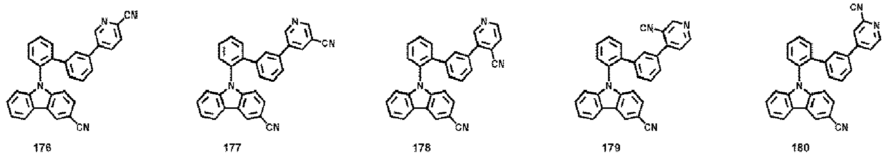

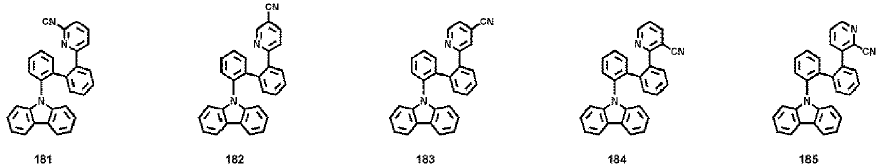

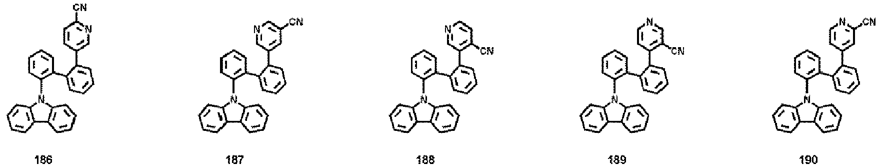

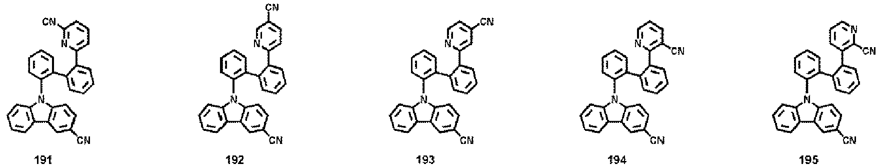

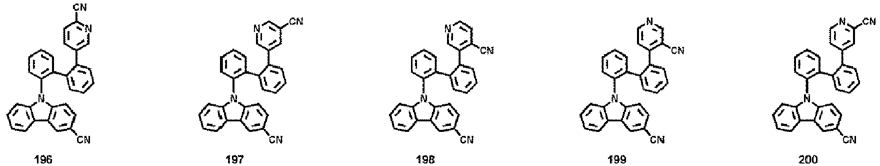

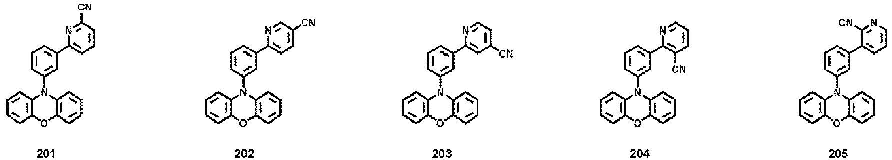

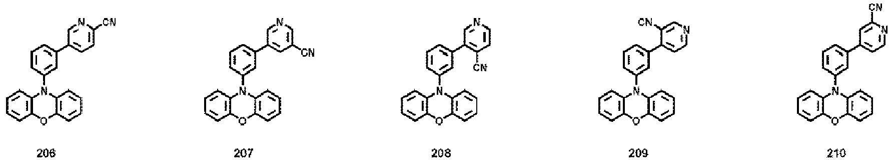

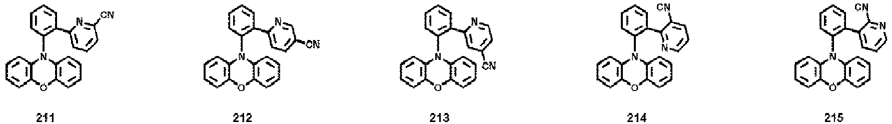

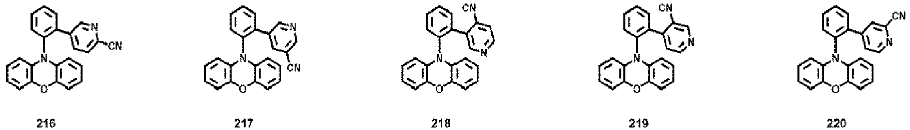

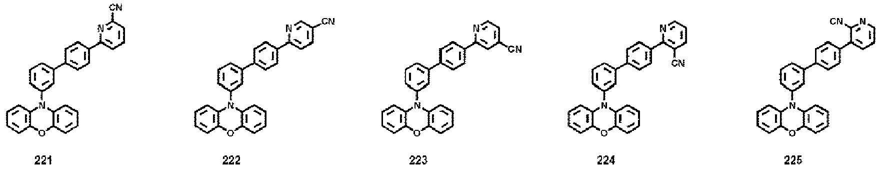

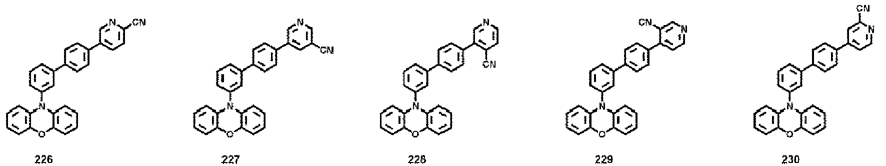

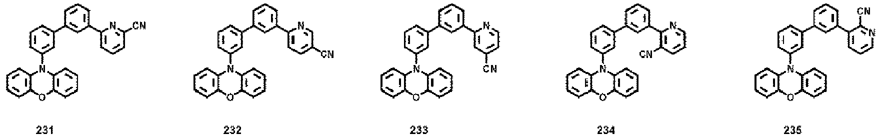

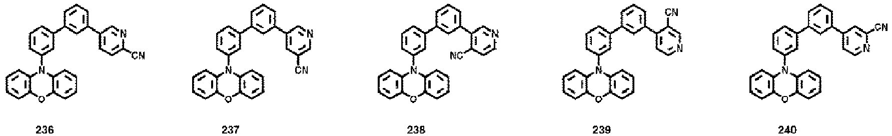

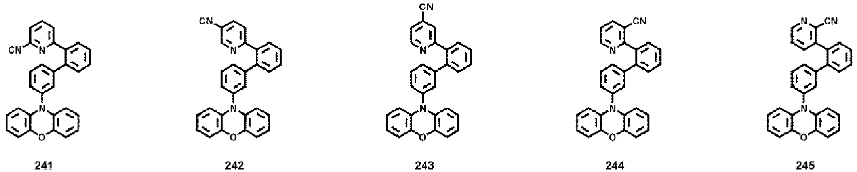

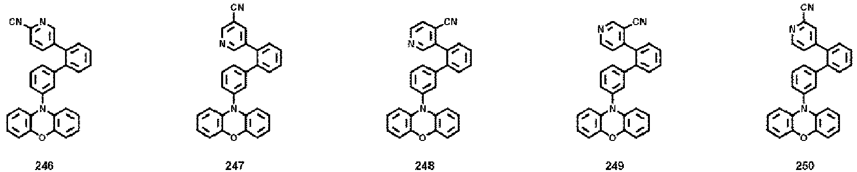

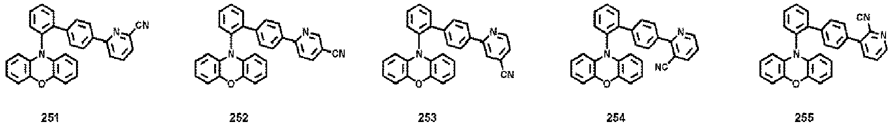

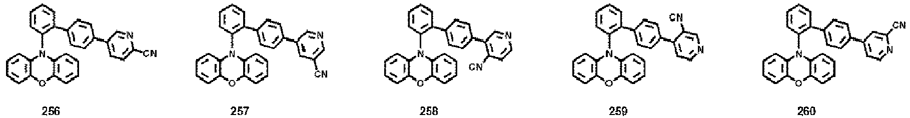

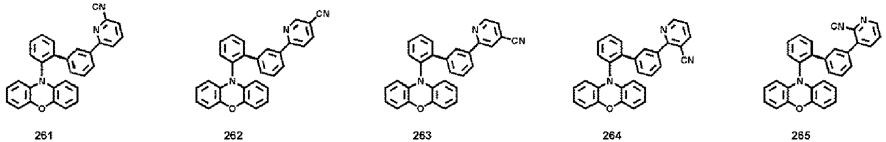

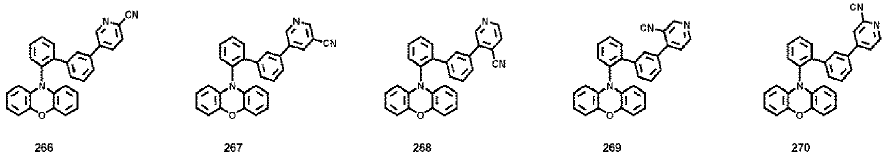

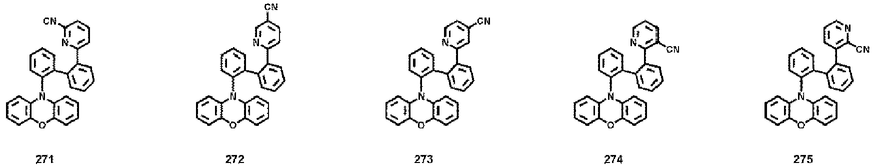

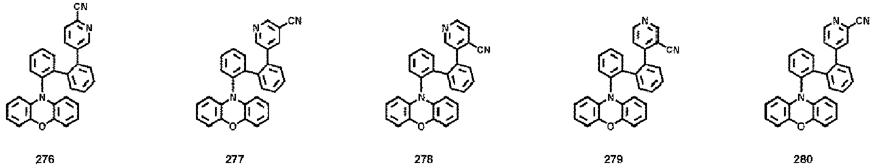

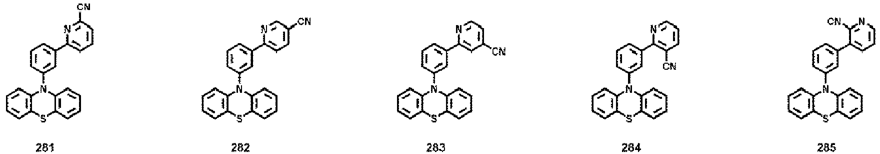

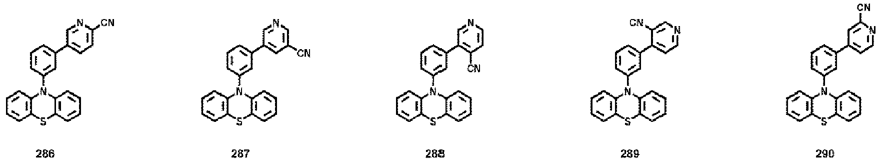

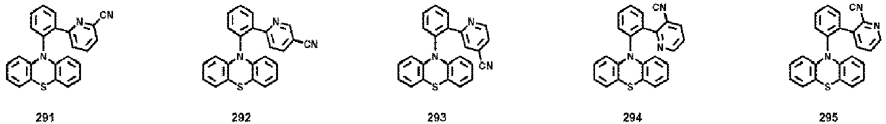

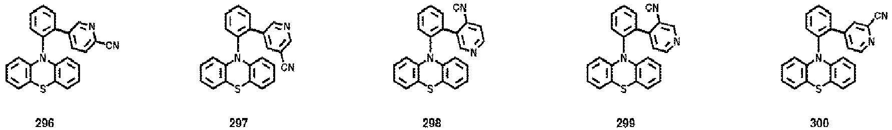

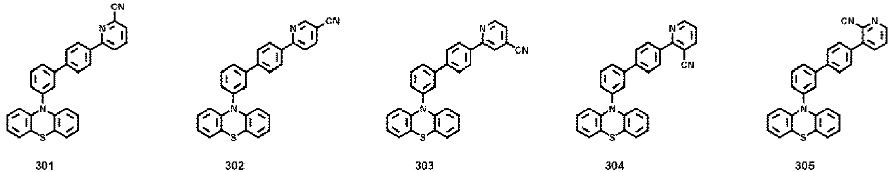

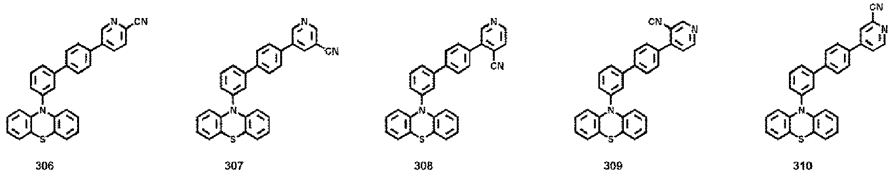

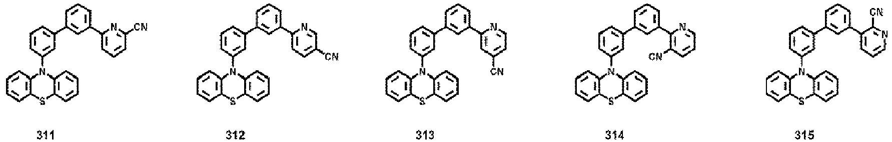

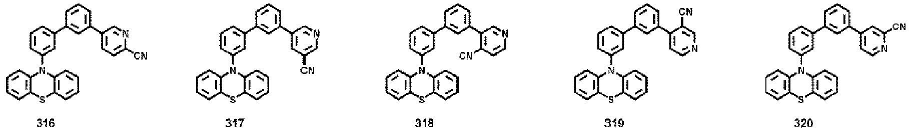

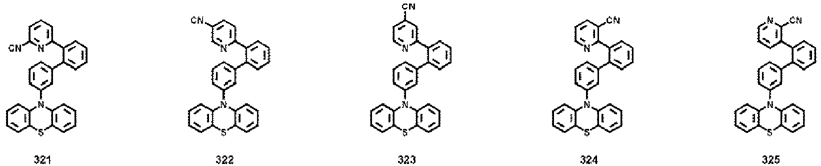

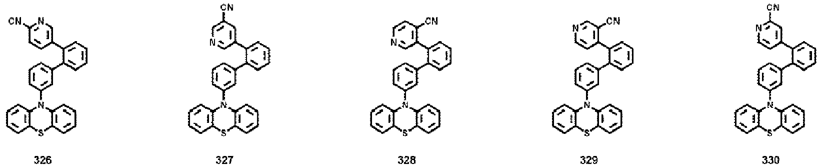

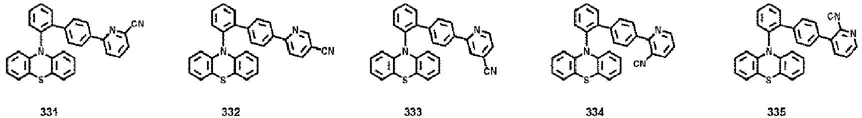

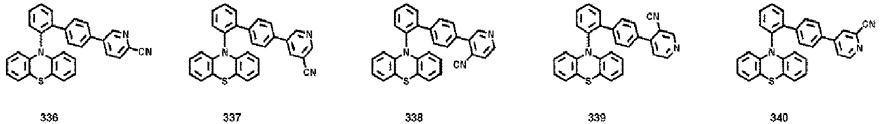

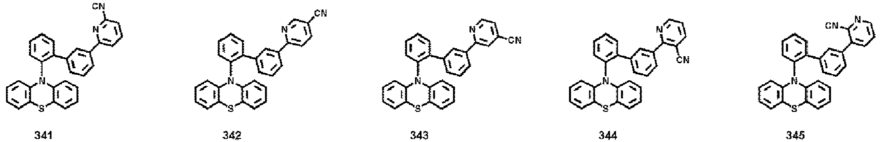

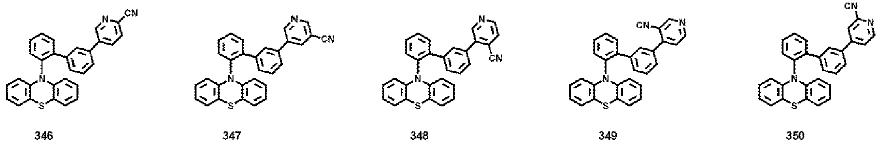

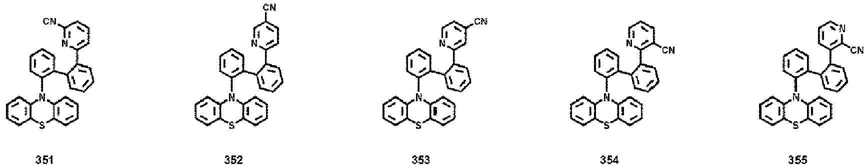

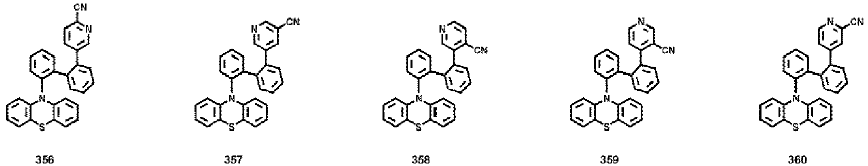

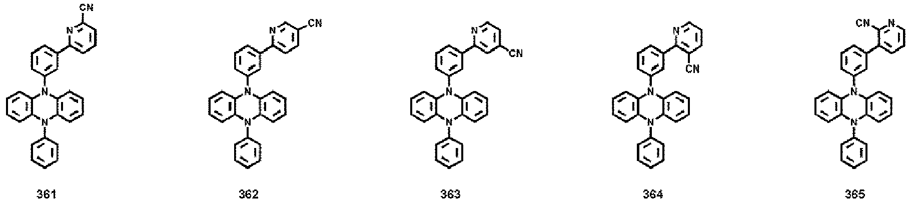

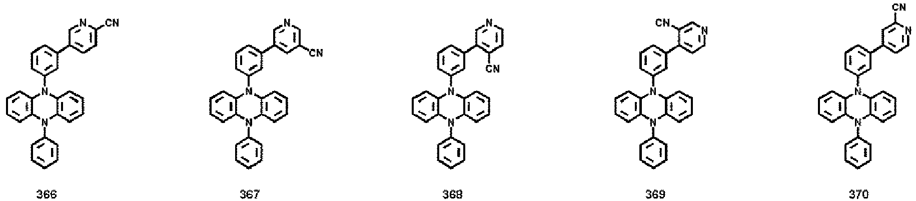

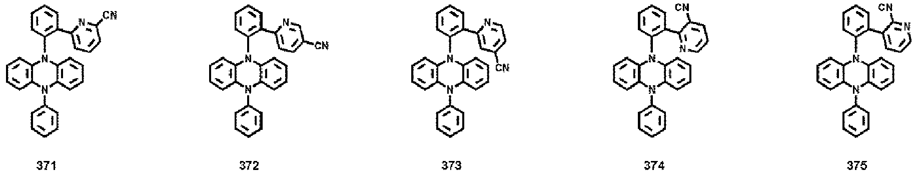

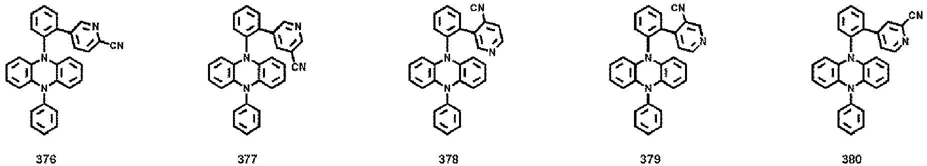

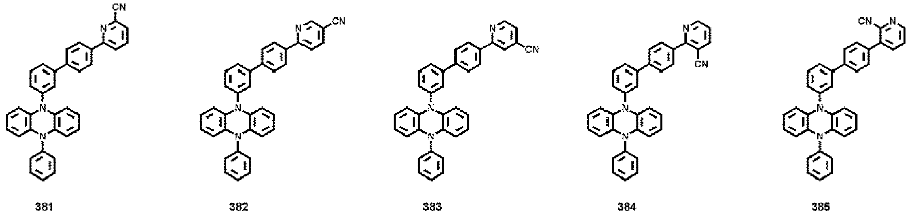

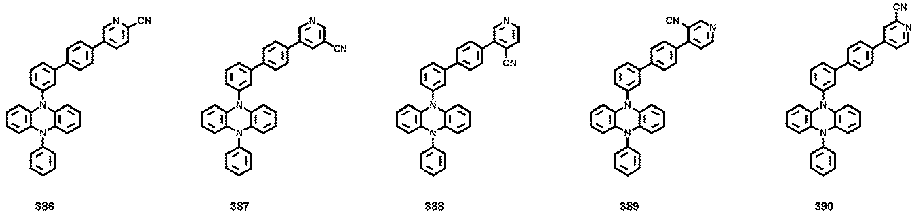

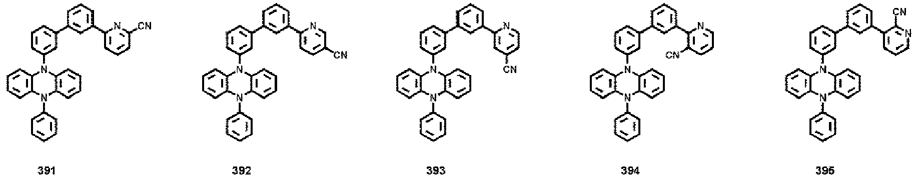

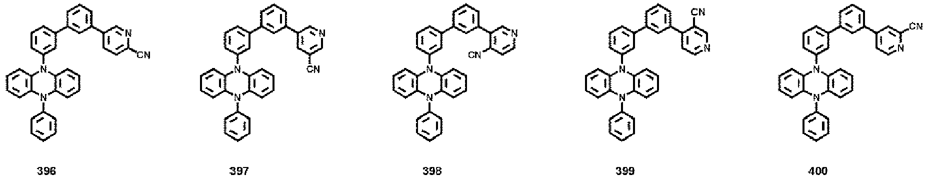

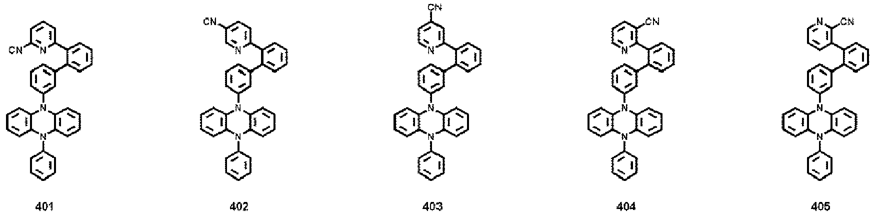

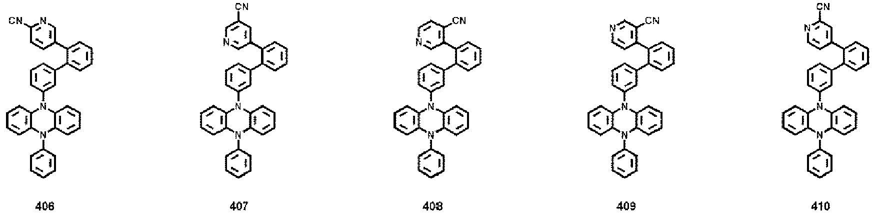

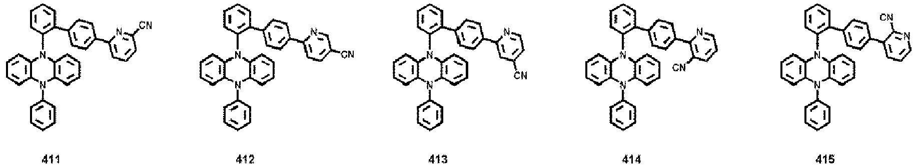

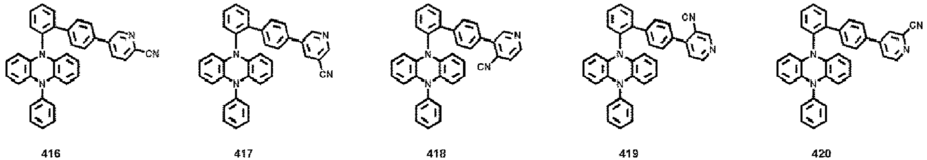

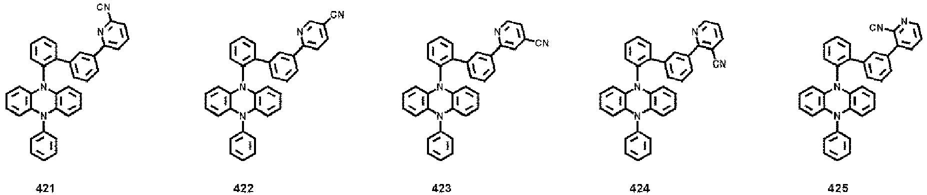

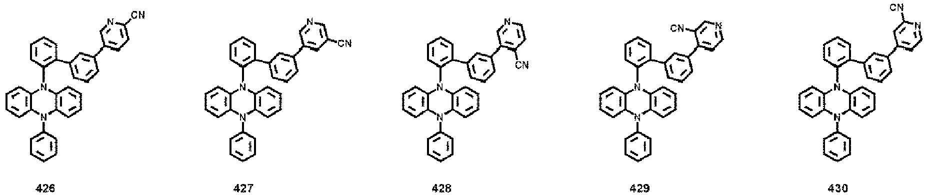

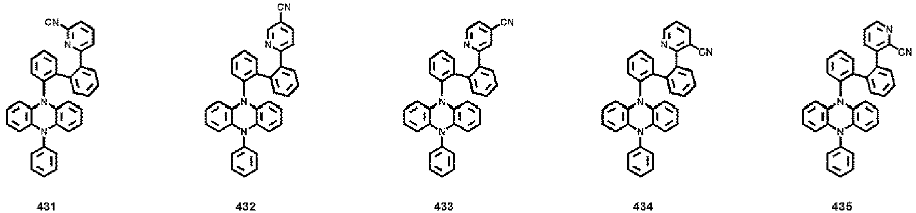

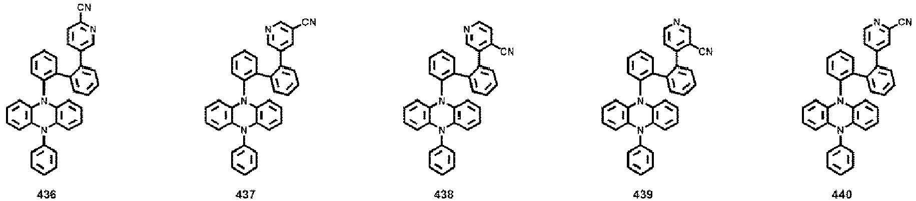

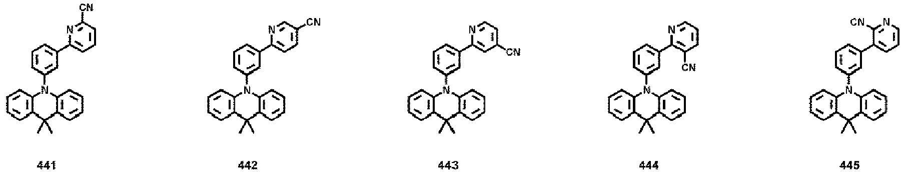

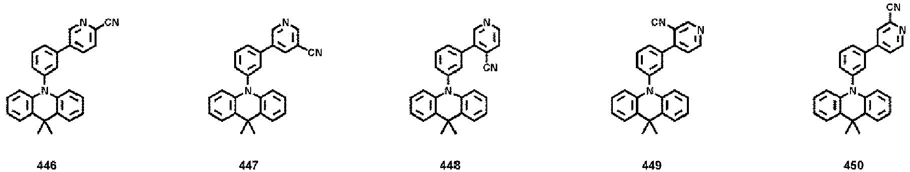

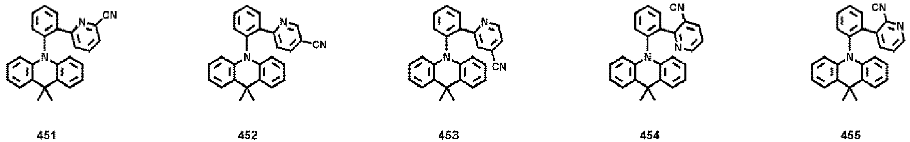

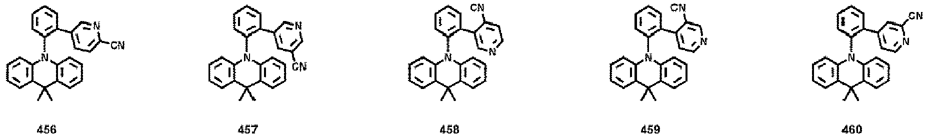

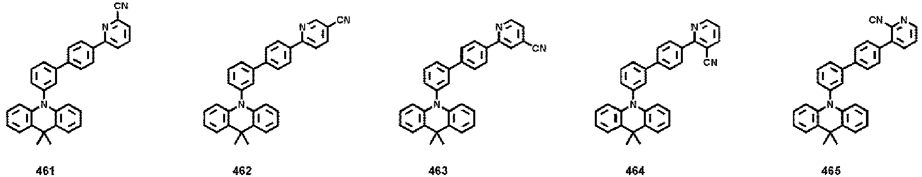

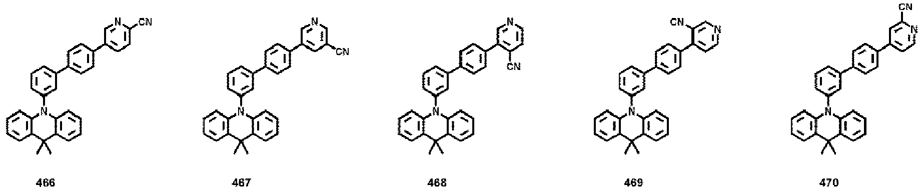

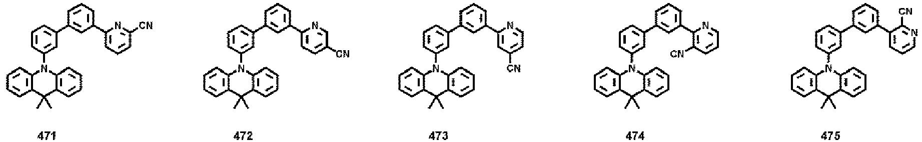

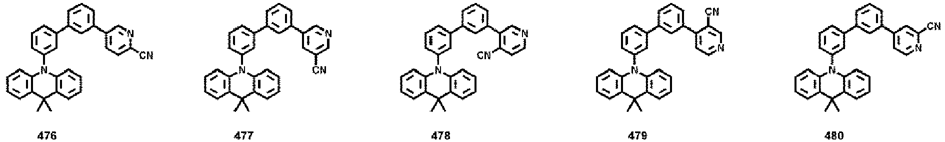

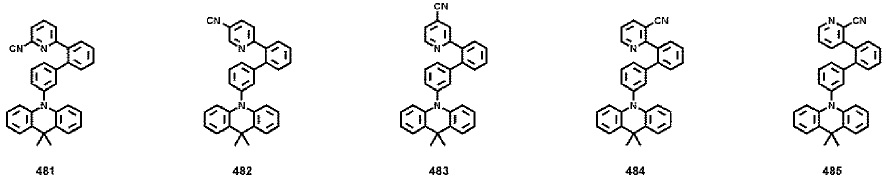

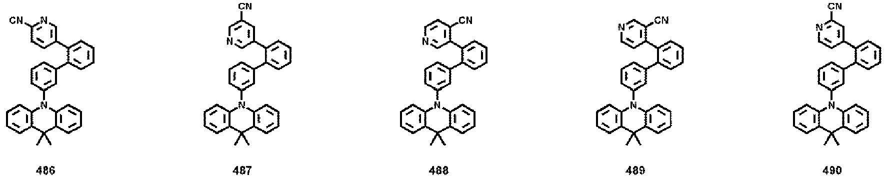

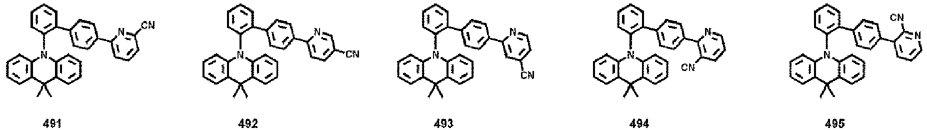

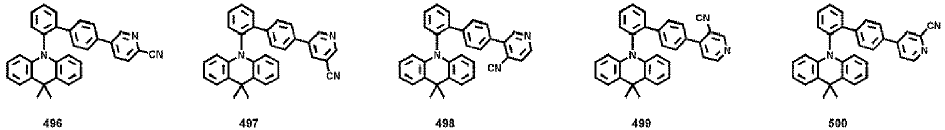

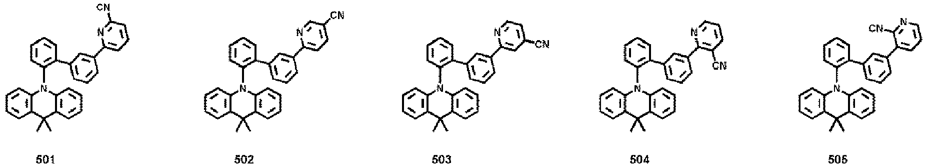

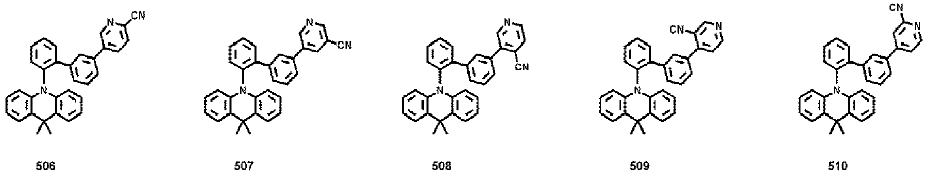

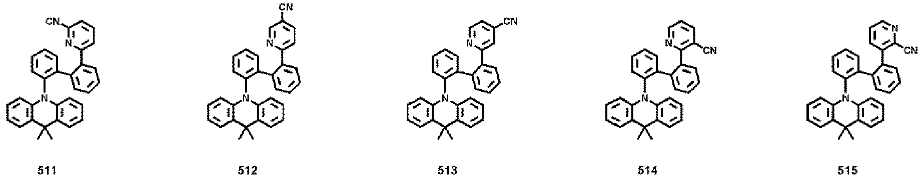

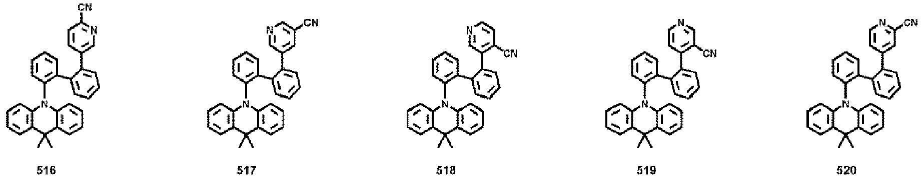

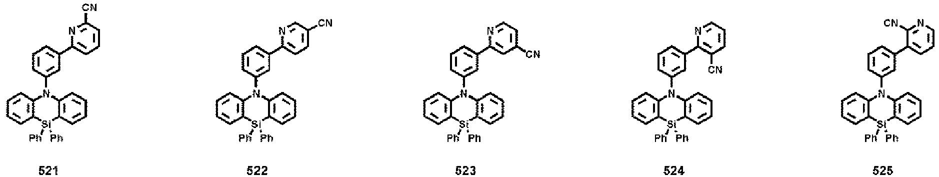

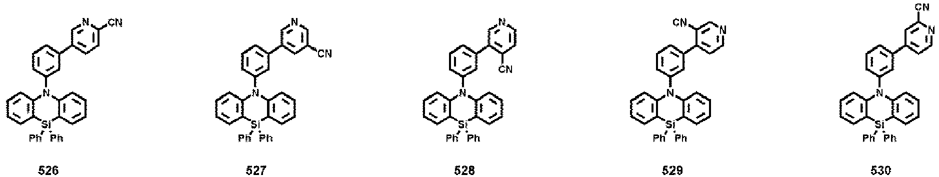

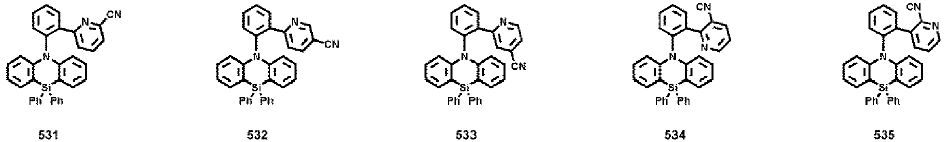

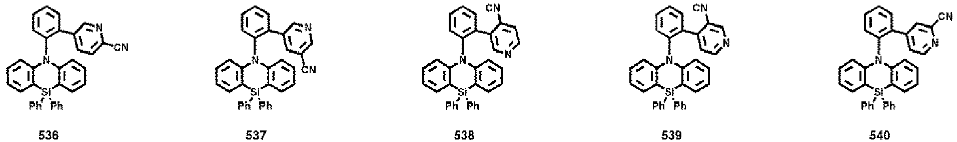

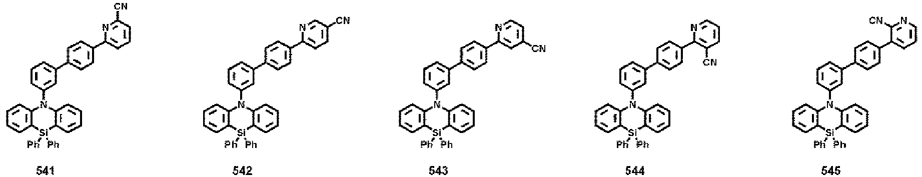

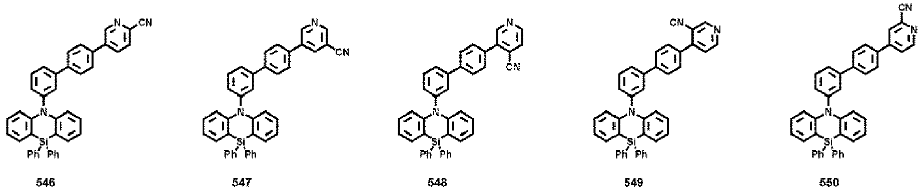

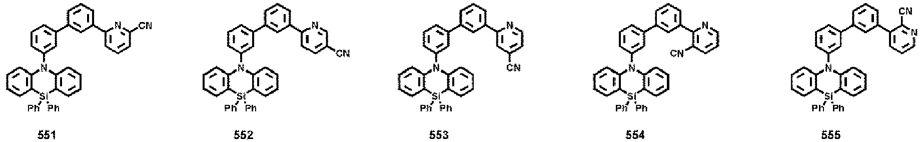

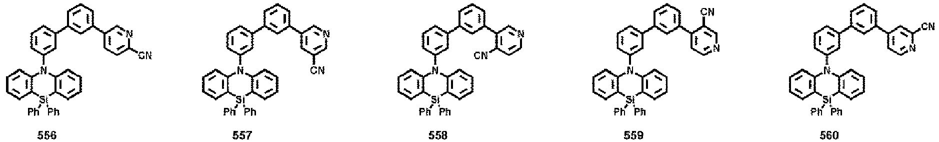

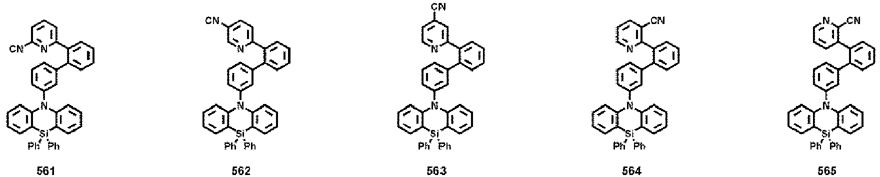

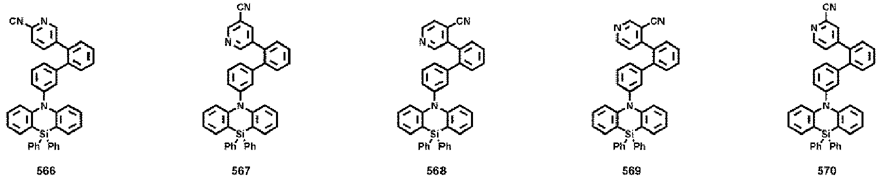

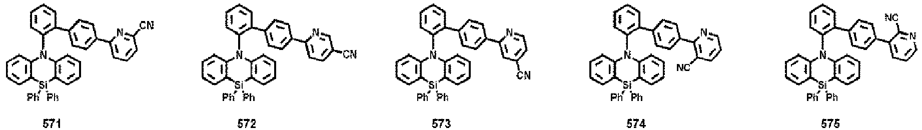

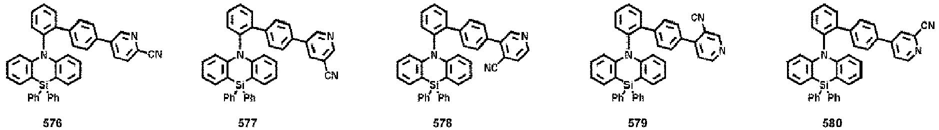

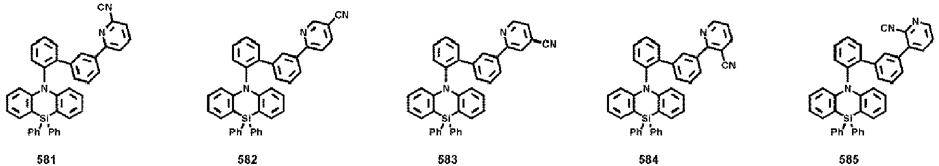

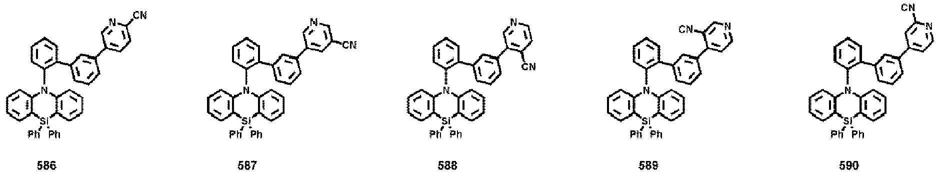

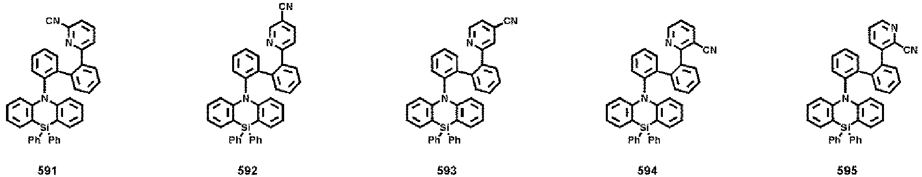

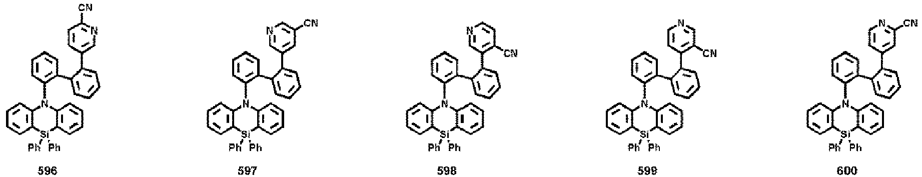

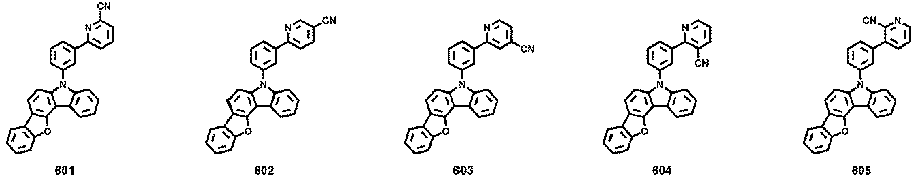

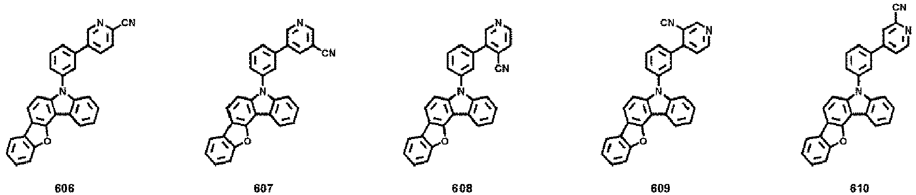

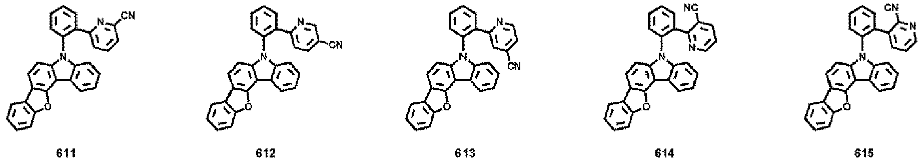

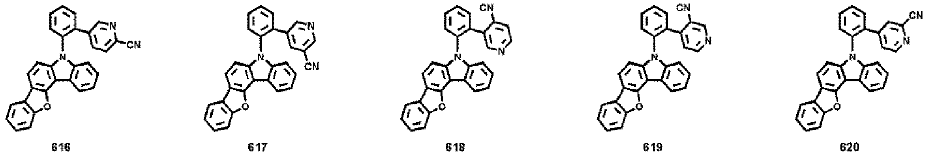

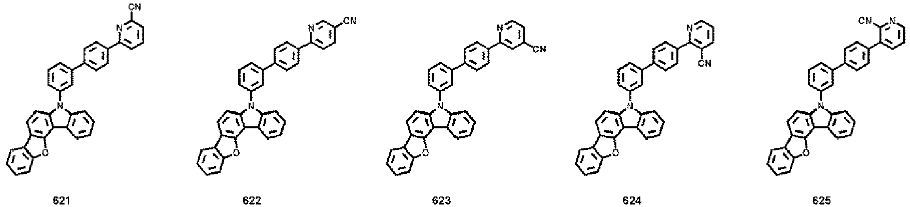

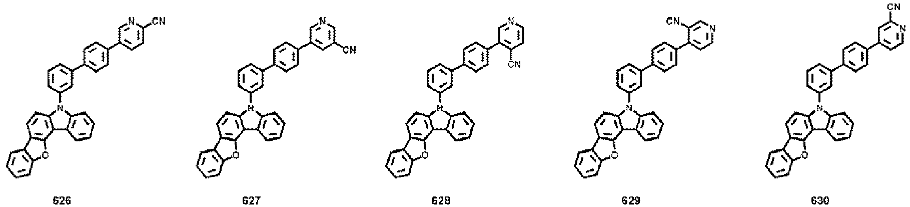

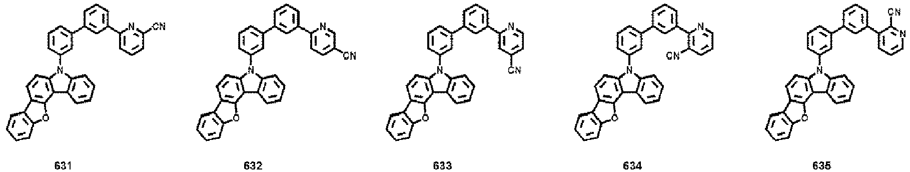

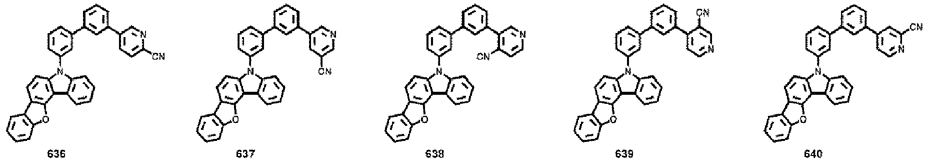

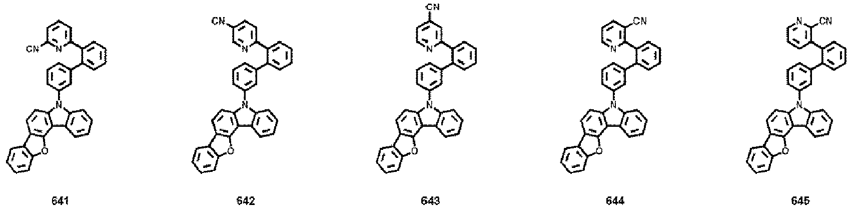

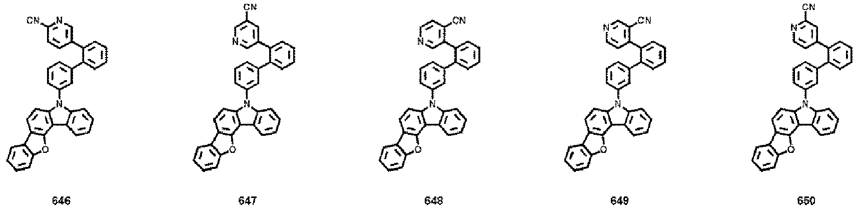

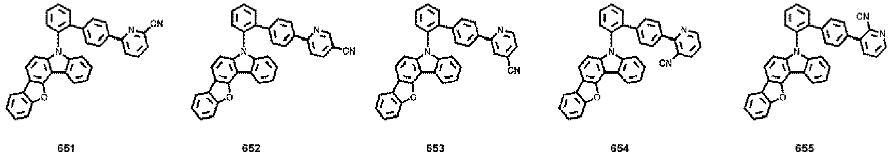

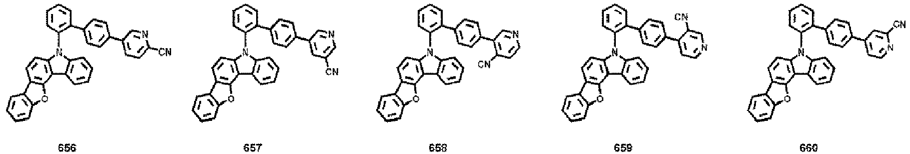

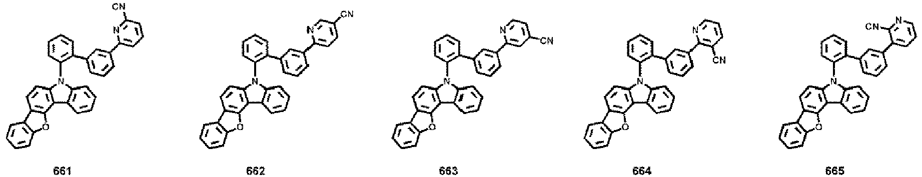

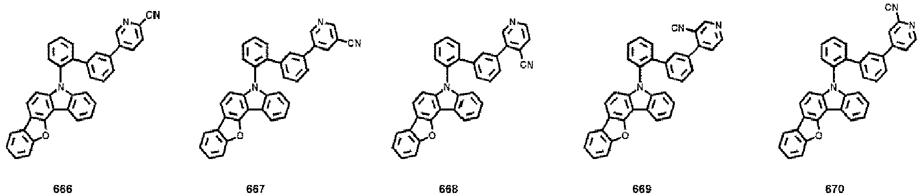

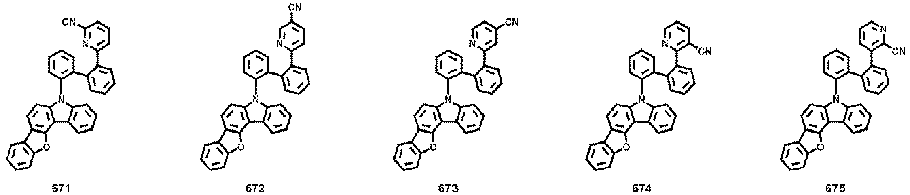

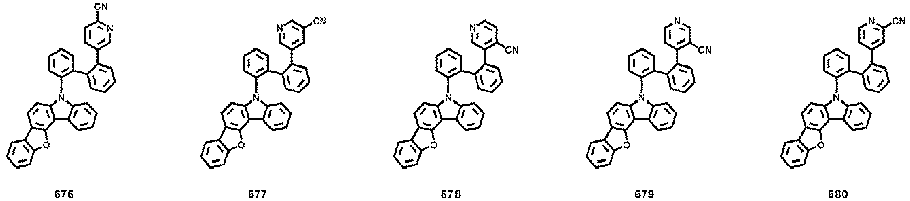

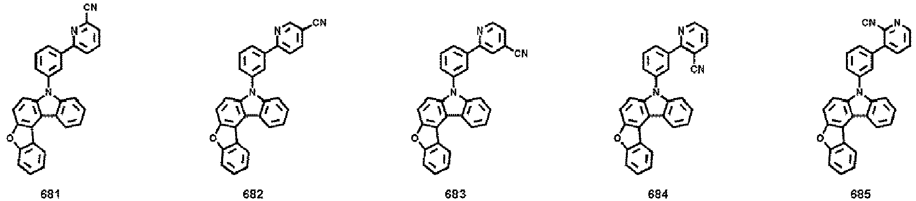

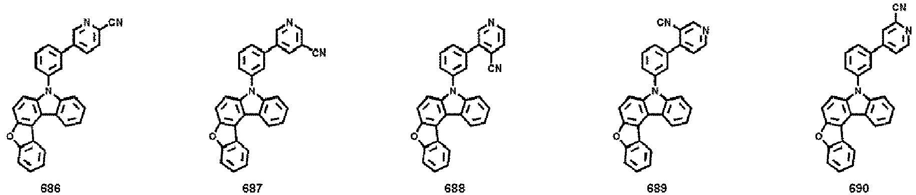

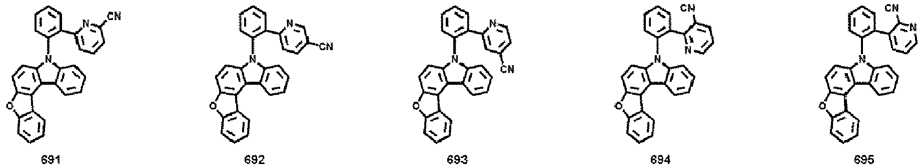

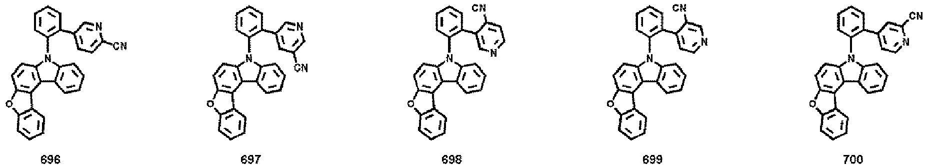

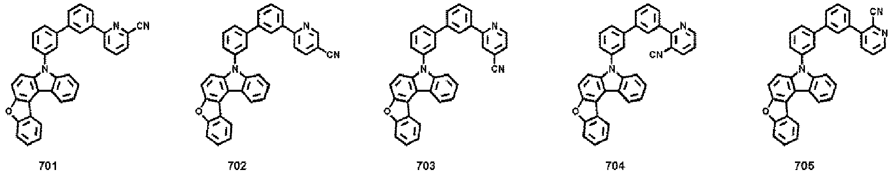

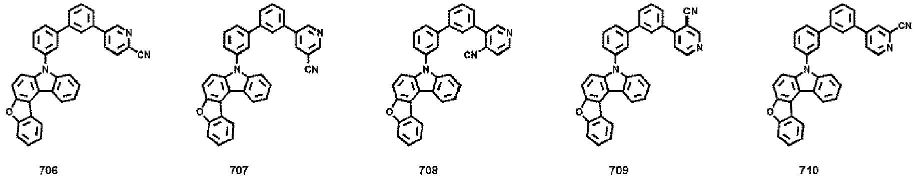

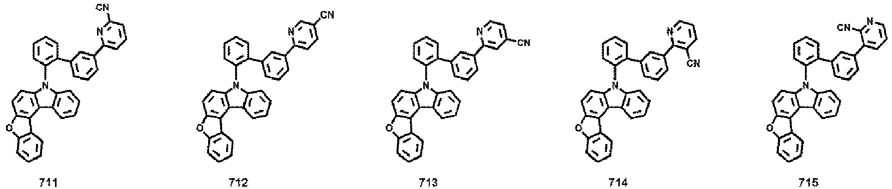

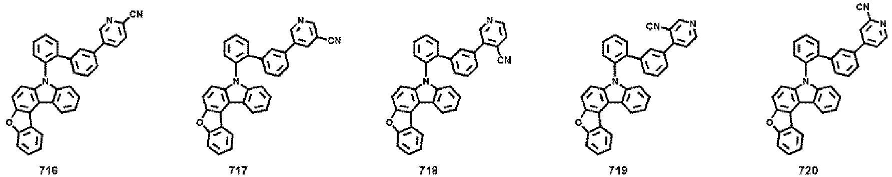

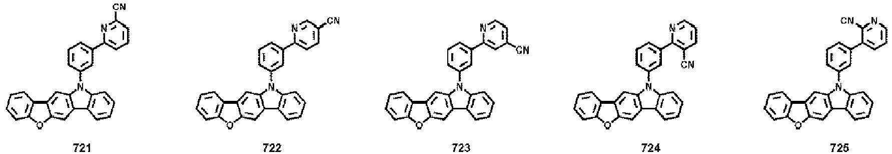

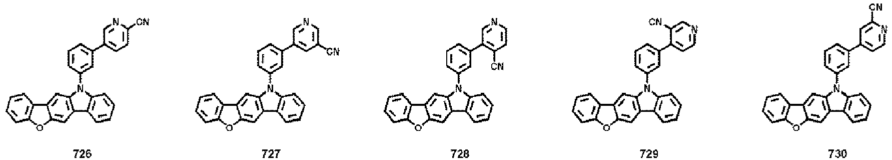

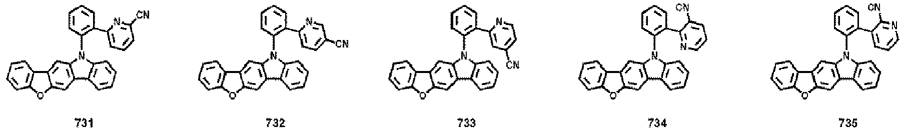

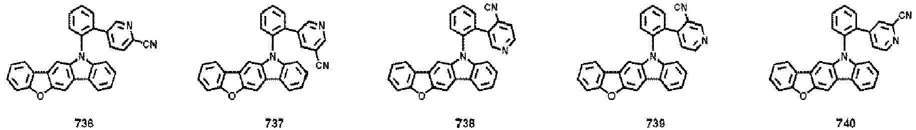

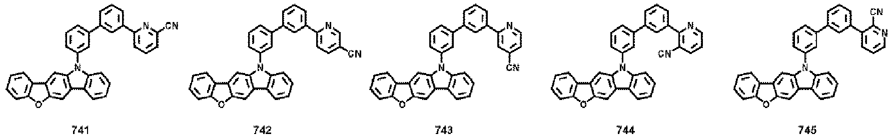

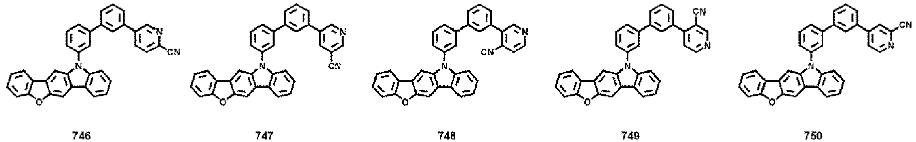

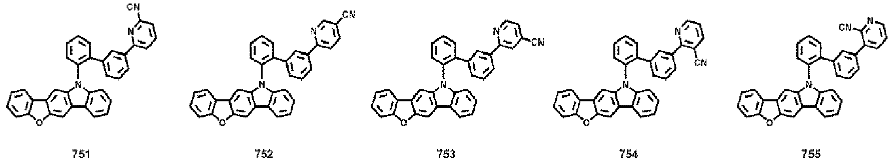

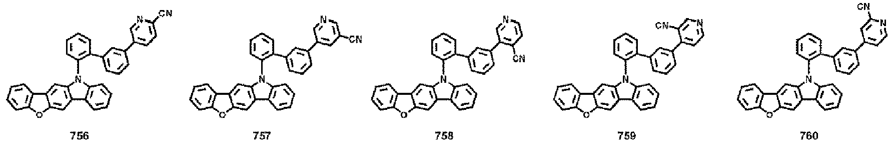

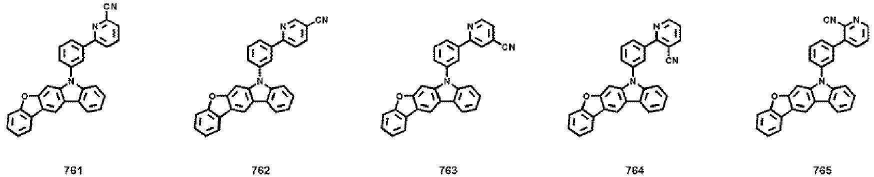

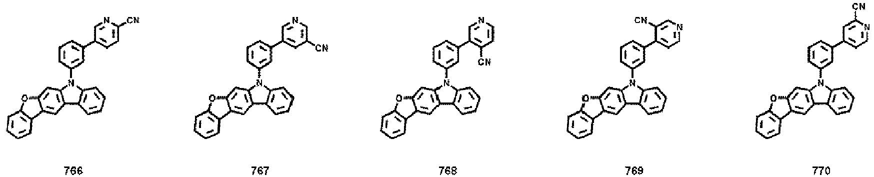

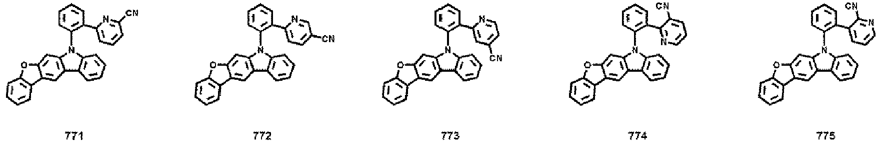

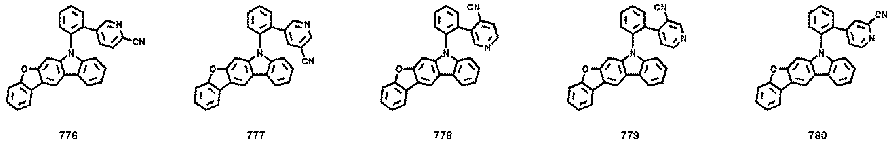

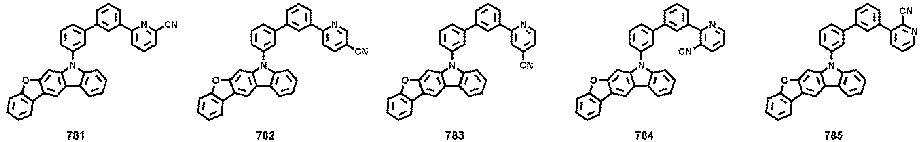

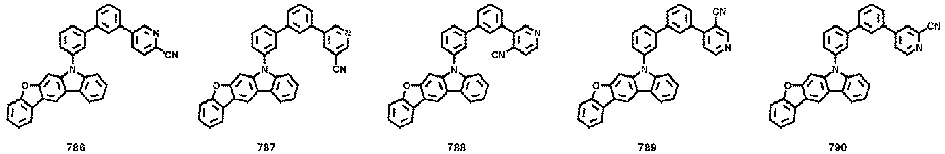

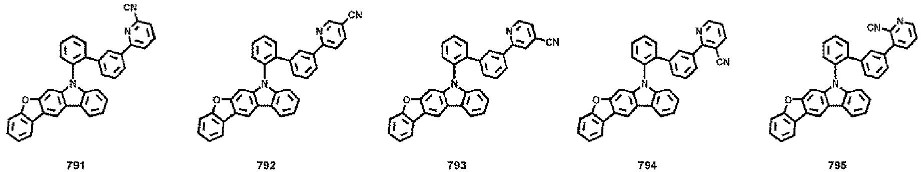

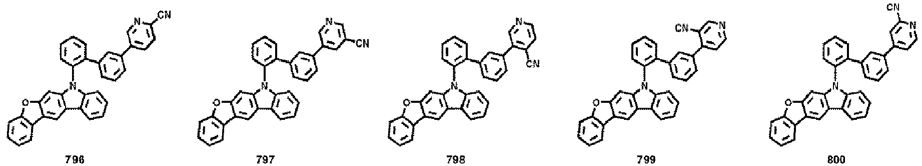

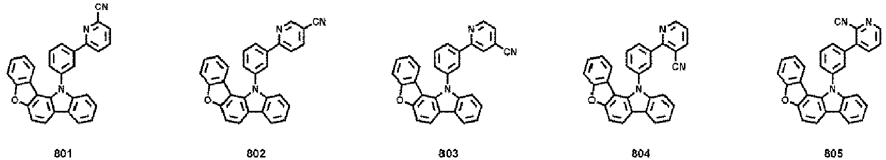

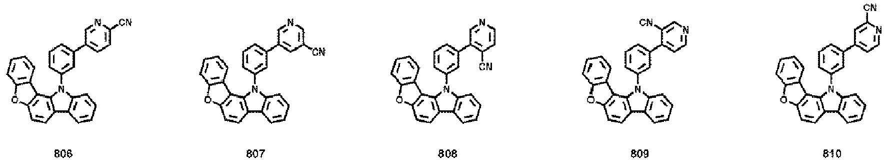

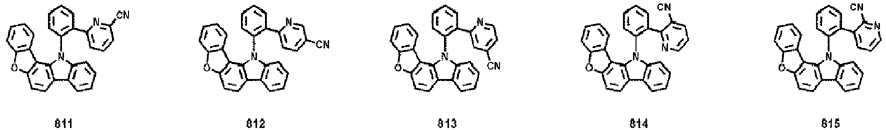

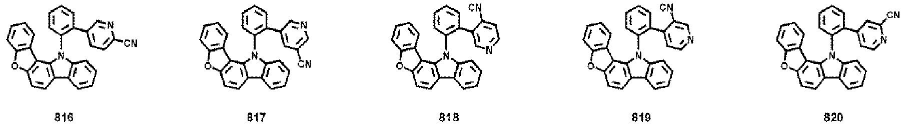

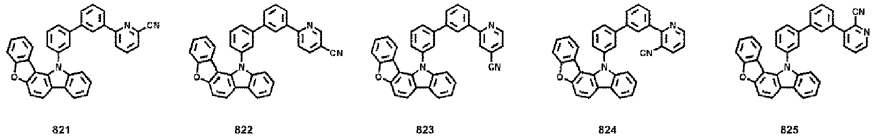

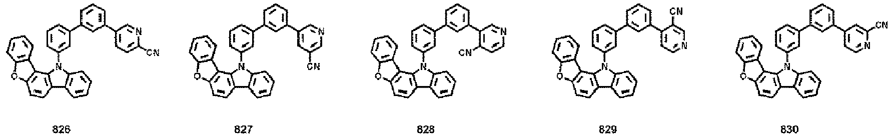

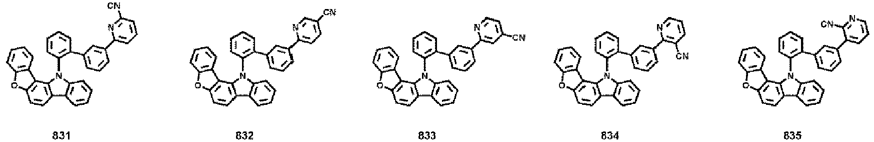

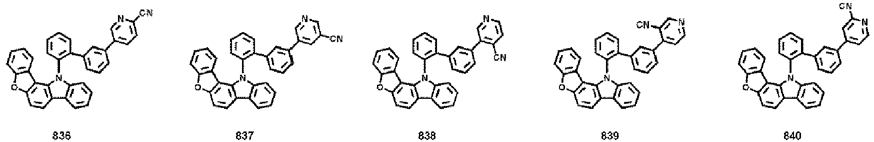

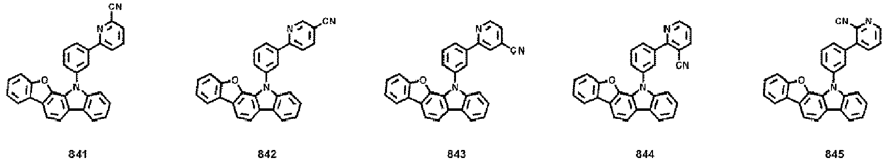

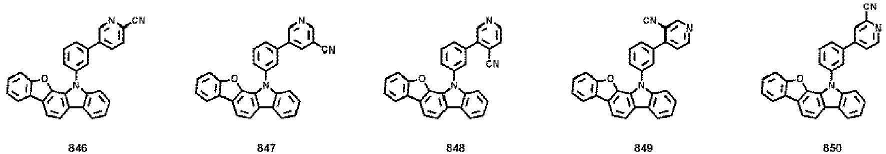

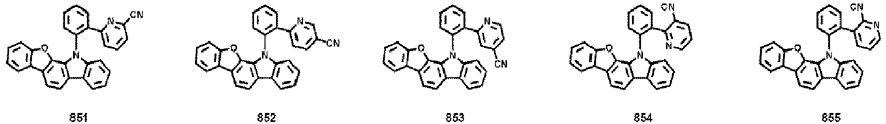

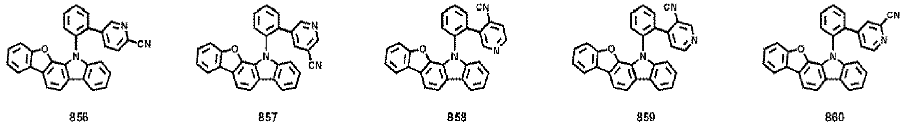

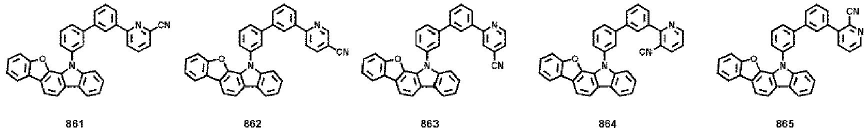

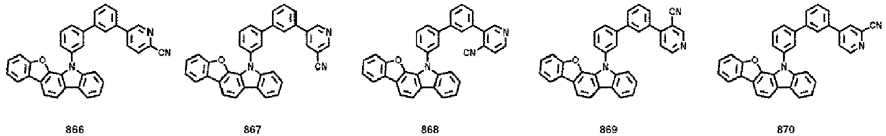

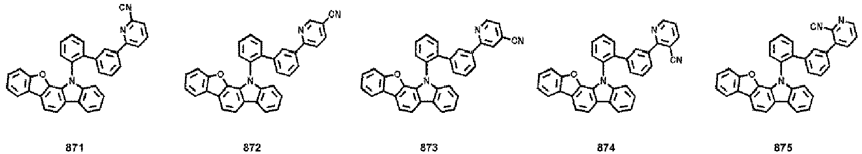

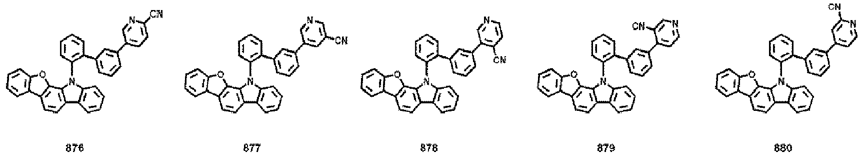

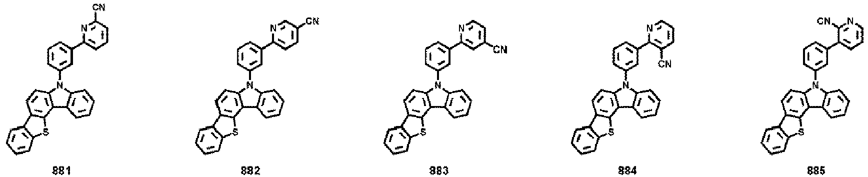

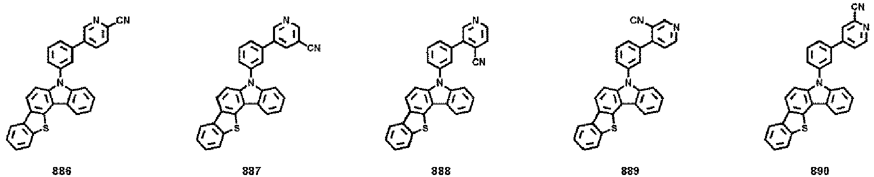

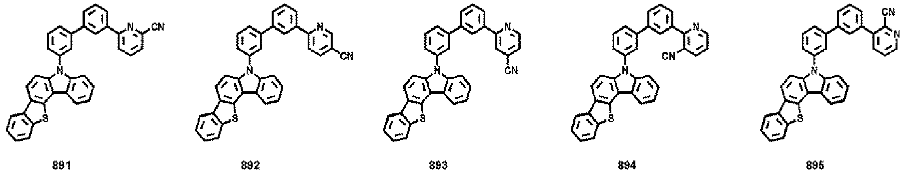

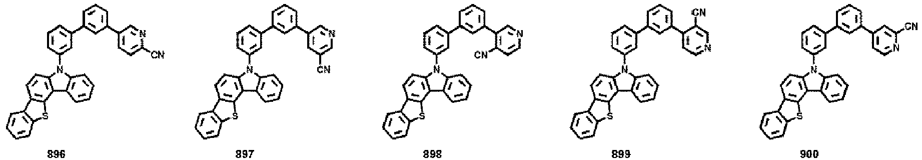

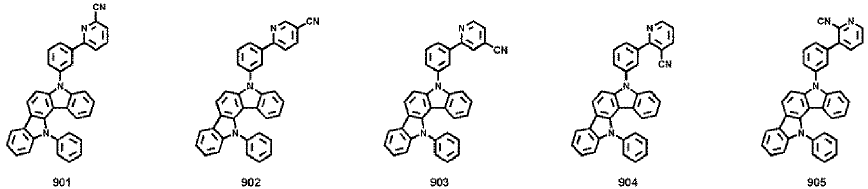

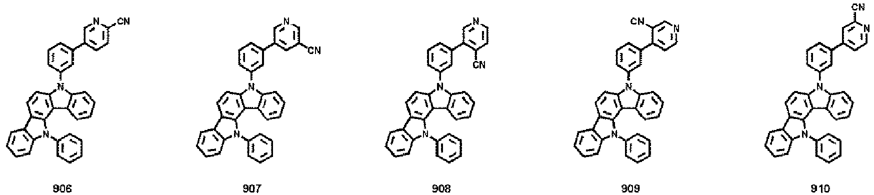

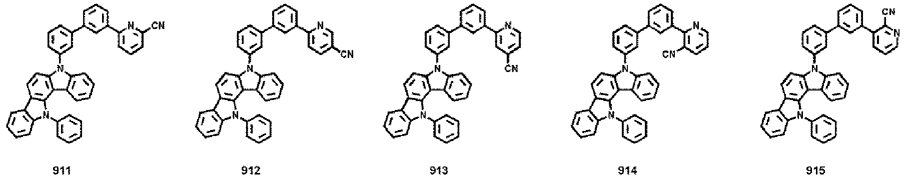

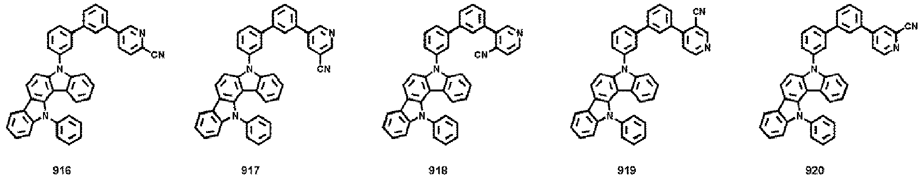

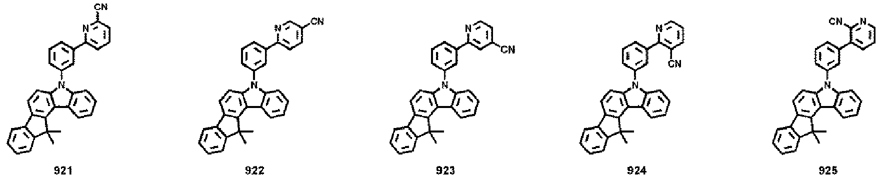

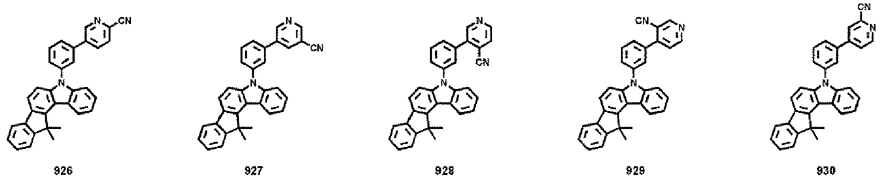

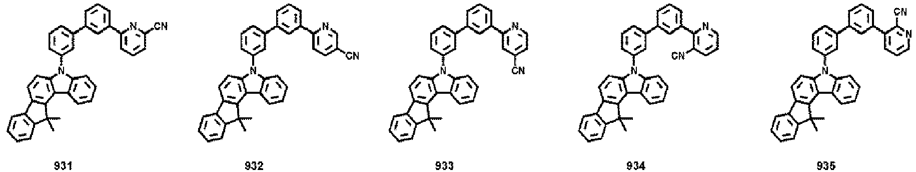

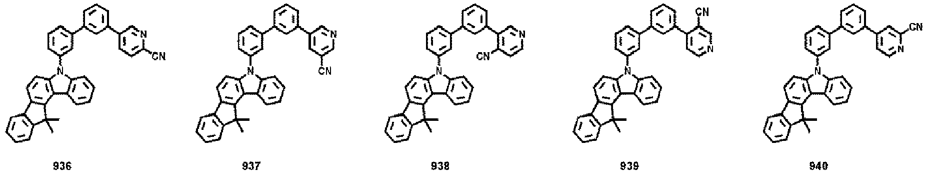

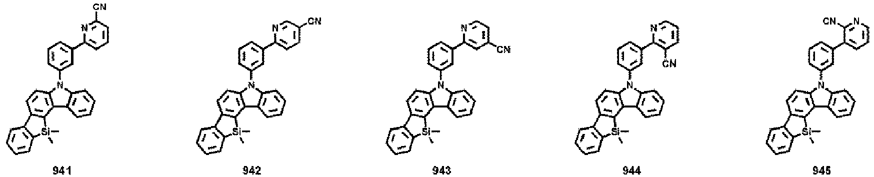

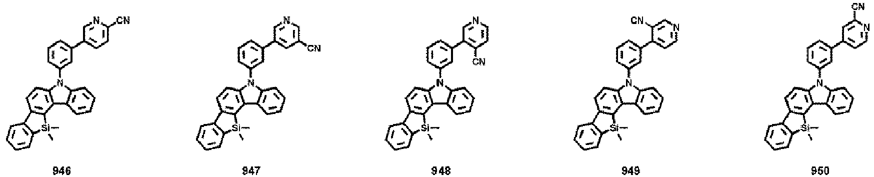

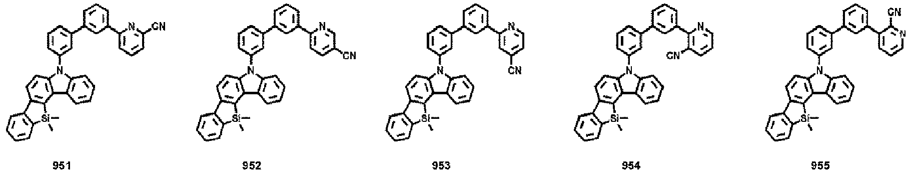

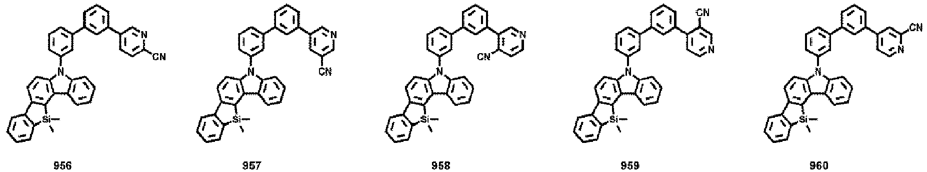

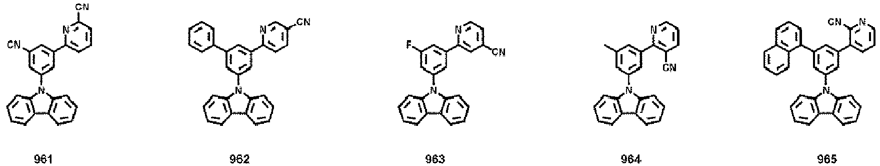

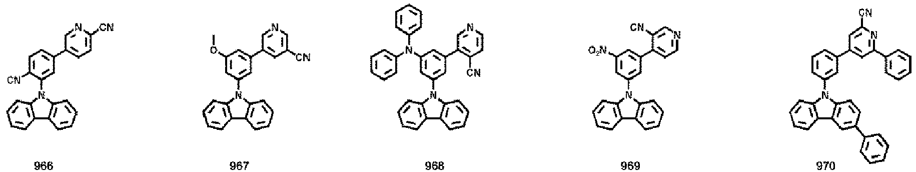

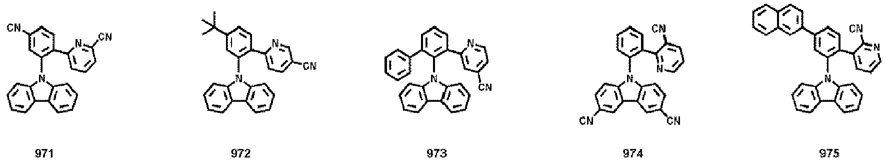

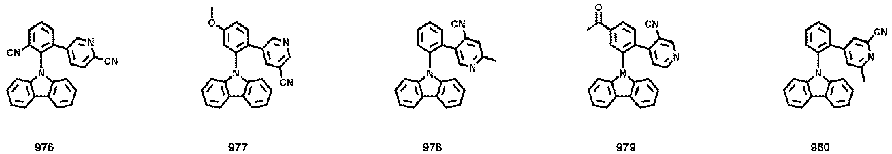

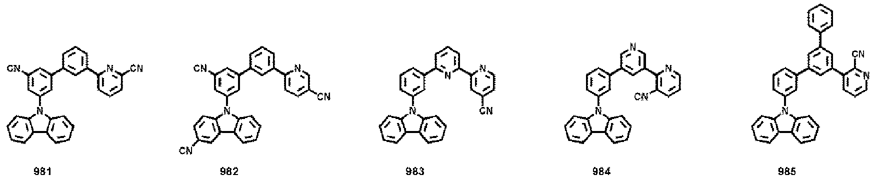

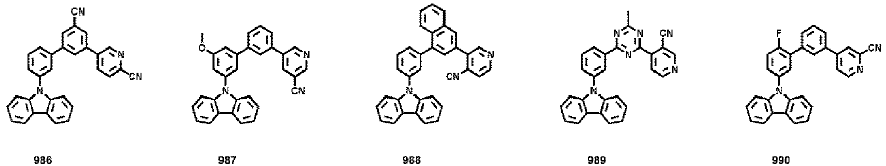

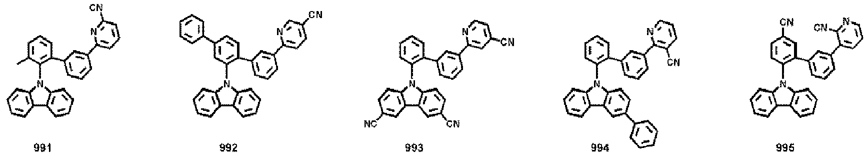

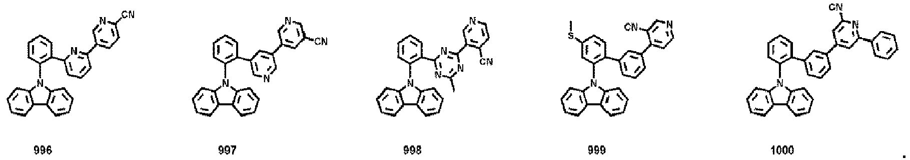

In Formulae 1 and 2, a condensed cyclic group may be linked to a benzene linker in a meta-position or ortho-position (see Formulae 1' and 2') with respect to a cyano group-containing pyridine ring, and "N" is linked to the benzene linker (see Formulae 1' and 2'). Accordingly, the condensed cyclic compound may have, due to a conjugation-bond effect control mechanism, a relatively high triplet energy (T₁) level.

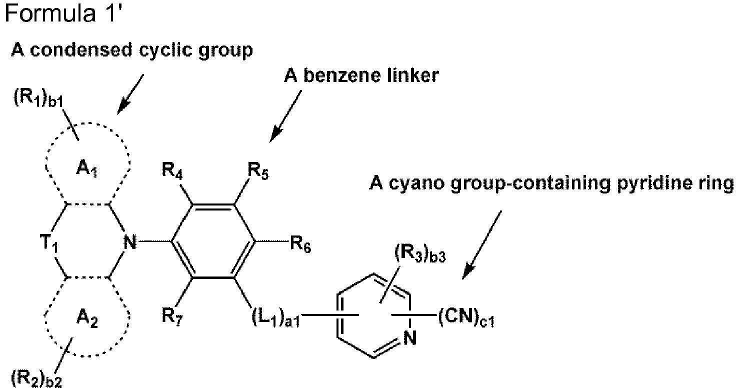

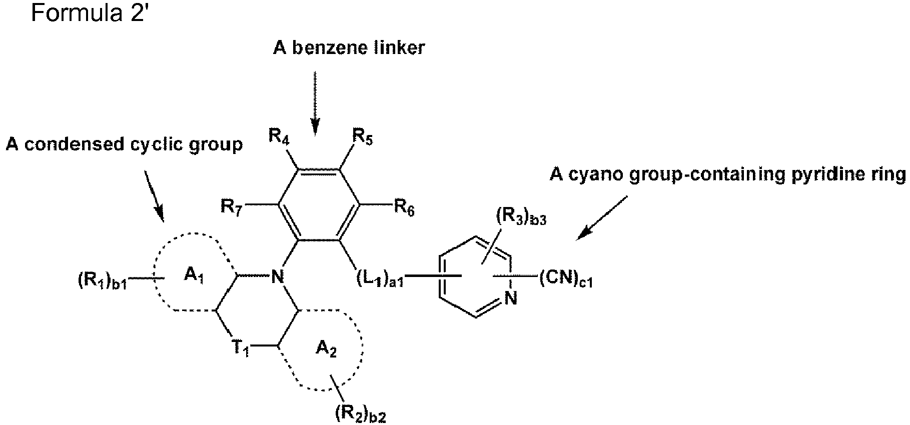

In an embodiment, because c1 in Formulae 1 and 2 may be an integer from 1 to 4, Formulae 1 and 2 may include "a cyano group-containing" pyridine ring (see a cyano group-containing a pyridine ring in Formulae 1' and 2'). Accordingly, the condensed cyclic compound may have a relatively large highest-occupied molecular orbital (HOMO) energy absolute value, a relatively large lowest unoccupied molecular orbital (LUMO) energy absolute value, and excellent electron mobility, such that the luminous efficiency of an electronic device including the condensed cyclic compound, for example, an organic light-emitting device including the condensed cyclic compound may be improved. The condensed cyclic compound has a high glass transition temperature (T_{g}) and a high thermal decomposition temperature (T_{d}), and thus, has excellent thermal stability. Accordingly, an electronic device including the condensed cyclic compound, for example, an organic light-emitting device including the condensed cyclic compound may have a long lifespan.

In one or more embodiments, each of Formulae 1 and 2 may include a cyano group-containing "pyridine ring" (see cyano group-containing pyridine ring in Formula 1' and 2'). Accordingly, the HOMO and/or LUMO energy levels of the condensed cyclic compound may be micro-controlled and electron mobility characteristics thereof may be improved while the molecular weight thereof is maintained at a relatively low level. As a result, the deposition temperature and/or sublimation temperature of the condensed cyclic compound may be decreased. The decrease in the deposition temperature and/or the sublimation temperature may contribute to the prevention of pyrolysis of compounds used in the production of an organic light-emitting device, leading to a long lifespan thereof.

As described above, the condensed cyclic compound represented by Formula 1 or Formula 2 may have electrical properties suitable for a material for an organic light-emitting device, for example, a host material in an emission layer. In an embodiment, the condensed cyclic compound may have electrical properties suitable for a material for a blue light-emitting device. Accordingly, an organic light-emitting device using the condensed cyclic compound may have high luminous efficiency and/or long lifespan.

For example, the HOMO, LUMO, T₁, and S1 energy levels of some of the compounds are structure-optimized at the (B3LYP, 6-31G(d,p)) level by using the DFT method of the Gaussian program and evaluated. Results thereof are shown in Table 1 below.

**Table 1**

| Compound No. | HOMO (eV) | LUMO (eV) | T₁ energy level (eV) | S₁ energy level (eV) |
|---|---|---|---|---|
| 1 | -5.511 | -1.900 | 2.938 | 3.156 |
| 5 | -5.427 | -1.905 | 2.979 | 3.061 |
| 6 | -5.619 | -2.137 | 2.898 | 3.028 |
| 7 | -5.582 | -1.911 | 3.092 | 3.247 |
| 9 | -5.479 | -2.038 | 2.849 | 2.956 |
| 10 | -5.606 | -2.053 | 2.945 | 3.085 |
| 15 | -5.879 | -2.097 | 3.113 | 3.323 |
| 41 | -5.312 | -1.739 | 2.966 | 2.991 |
| 44 | -5.355 | -1.796 | 2.917 | 2.971 |
| 45 | -5.582 | -1.774 | 3.136 | 3.220 |
| 46 | -5.605 | -1.968 | 2.954 | 3.043 |
| 47 | -5.612 | -1.730 | 3.163 | 3.304 |
| 48 | -5.532 | -2.017 | 2.881 | 2.935 |
| 49 | -5.606 | -1.895 | 3.020 | 3.089 |
| 50 | -5.612 | -1.883 | 3.042 | 3.117 |
| 55 | -6.030 | -2.009 | 3.110 | 3.439 |
| 85 | -5.414 | -1.900 | 2.985 | 3.179 |
| 95 | -5.867 | -2.018 | 3.010 | 3.496 |
| 105 | -5.351 | -1.828 | 3.133 | 3.176 |
| 115 | -5.809 | -1.946 | 3.119 | 3.518 |
| 165 | -5.405 | -1.697 | 3.176 | 3.228 |
| 175 | -5.863 | -1.834 | 3.124 | 3.552 |
| 605 | -5.295 | -1.939 | 2.878 | 2.932 |
| 615 | -5.435 | -1.805 | 2.978 | 3.101 |
| 635 | -5.239 | -1.850 | 3.003 | 3.062 |
| 665 | -5.285 | -1.708 | 3.012 | 3.130 |
| 915 | -5.000 | -1.827 | 2.847 | 2.852 |

From Table 1, it is confirmed that the condensed cyclic compounds represented by Formula 1 or Formula 2 have relatively high triplet energy (T₁) levels and the HOMO and/or LUMO energy levels thereof may be easily controlled depending on the type and number of substituents.

The condensed cyclic compound represented by Formula 1 or 2 may be understood by those skilled in the art with reference to the Synthesis Examples to be described later.

According to another aspect, provided is a composition including
a first compound and a second compound,
wherein the first compound is a condensed cyclic compound represented by formula 1 as described herein,
the second compound is different from the first compound,
the second compound includes at least one selected from a carbazole group, a dibenzofuran group, a dibenzothiophene group, an indenocarbazole group, an indolocarbazole group, a benzofurocarbazole group, a benzothienocarbazole group, an acridine group, a dihydroacridine group, and a triindolobenzene group, and does not include an electron withdrawing group, and
the electron withdrawing group is selected from:
-F, -CFH₂, -CF₂H, -CF₃, -CN, and -NO₂;
a C₁-C₆₀ alkyl group substituted with at least one selected from -F, -CFH₂,-CF₂H, -CF₃, -CN, and -NO₂;
a C₁-C₆₀ heteroaryl group and a monovalent non-aromatic condensed polycyclic heterocyclic group, each including *=N-*' as a ring-forming moiety; and
a C₁-C₆₀ heteroaryl group and a monovalent non-aromatic condensed polycyclic heterocyclic group, each of which includes *=N-*' as a ring-forming moiety and is substituted with at least one selected from deuterium, -F, -CFH₂, -CF₂H, -CF₃, -CI, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₇-C₆₀ arylalkyl group, a substituted or unsubstituted C₁-C₆₀ heteroaryl group, a substituted or unsubstituted C₁-C₆₀ heteroaryloxy group, a substituted or unsubstituted C₁-C₆₀ heteroarylthio group, a substituted or unsubstituted C₂-C₆₀ heteroarylalkyl group, a substituted or unsubstituted monovalent non-aromatic condensed polycyclic group, and a substituted or unsubstituted monovalent non-aromatic condensed heteropolycyclic group.

The composition may be used in the manufacture of organic layers of for example, electronic devices (for example, organic light-emitting devices).

The first compound in the composition may be an electron transport material and the second compound in the composition may be a hole transport material.

According to an embodiment, the composition may consist of the first compound and second compound, but embodiments of the present disclosure are not limited.

The description of the condensed cyclic compound represented by Formula 1, which may be the first compound of the composition, has been provided above.

For example, the second compound of the composition may be selected from the following compounds represented by Formula H-1:

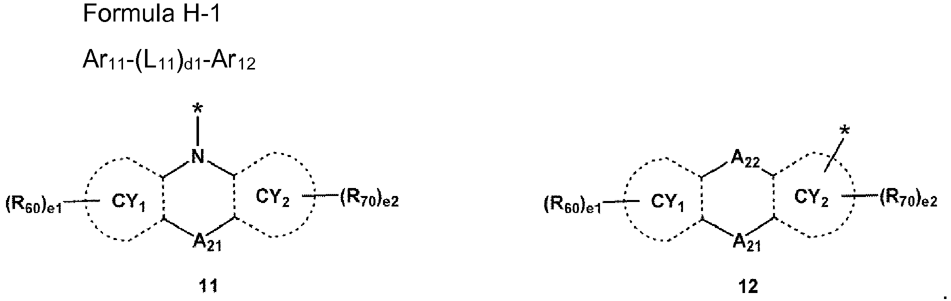

In Formulae H-1, 11, and 12,
L₁₁ may be selected from
a single bond, a phenylene group, a naphthylene group, a fluorenylene group, a carbazolylene group, a dibenzofuranylene group, and a dibenzothiophenylene group; and
a phenylene group, a naphthylene group, a fluorenylene group, a carbazolylene group, a dibenzofuranylene group, and a dibenzothiophenylene group, each substituted with at least one selected from deuterium, a C₁-C₁₀ alkyl group, a C₁-C₁₀ alkoxy group, a phenyl group, a naphthyl group, a fluorenyl group, a carbazolyl group, a dibenzofuranyl group, a dibenzothiophenyl group, a biphenyl group, and -Si(Q₁₁)(Q₁₂)(Q₁₃);
d1 is an integer from 1 to 10, and d1 is two or more, two or more groups L₁ may be identical to or different from each other,
Ar₁₁ is represented by one of Formulae 11 and 12,
Ar₁₂ may be selected from
a group represented by Formula 11 and 12, a phenyl group, and a naphthyl group; and
a phenyl group and a naphthyl group, each substituted with at least one selected from deuterium, a hydroxyl group, an amino group, an amidino group, a hydrazine group, a hydrazone group, a carboxylic acid group or a salt thereof, a sulfonic acid group or a salt thereof, a phosphoric acid group or a salt thereof, a C₁-C₂₀ alkyl group, a C₁-C₂₀ alkoxy group, a phenyl group, a naphthyl group, a fluorenyl group, a carbazolyl group, a dibenzofuranyl group, a dibenzothiophenyl group, and a biphenyl group,
ring CY₁ and ring CY₂ may each independently be selected from a benzene group, a naphthalene group, a phenanthrene group, an anthracene group, a cyclopentene group, a pyrrole group, a furan group, a thiophene group, a silole group, an indene group, an indole group, a benzofuran group, a benzothiophene group, a benzosilole group, a fluorene group, a carbazole group, a dibenzofuran group, a dibenzothiophene group, a dibenzosilole group, a benzocarbazole group, an indenocarbazole group, an indolocarbazole group, a benzofurocarbazole group, a benzothienocarbazole group, an acridine group, a dihydroacridine group, and a triindolobenzene group,
A₂₁ may be selected from a single bond, O, S, N(R₅₁), C(R₅₁)(R₅₂), and Si(R₅₁)(R₅₂),
A₂₂ may be selected from a single bond, O, S, N(R₅₃), C(R₅₃)(R₅₄), and Si(R₅₃)(R₅₄),
at least one selected from A₂₁ and A₂₂ in Formula 12 is not a single bond,
R₅₁ to R₅₄, R₆₀, and R₇₀ may each independently be selected from:
   hydrogen, deuterium, a hydroxyl group, an amino group, an amidino group, a hydrazine group, a hydrazone group, a carboxylic acid group or a salt thereof, a sulfonic acid group or a salt thereof, a phosphoric acid group or a salt thereof, a C₁-C₂₀ alkyl group, and a C₁-C₂₀ alkoxy group;
   a C₁-C₂₀ alkyl group and a C₁-C₂₀ alkoxy group, each substituted with at least one selected from deuterium, a hydroxyl group, an amino group, an amidino group, a hydrazine group, a hydrazone group, a carboxylic acid group or a salt thereof, a sulfonic acid group or a salt thereof, a phosphoric acid group or a salt thereof, a phenyl group, a naphthyl group, a fluorenyl group, a carbazolyl group, a dibenzofuranyl group, and a dibenzothiophenyl group;
   a phenyl group, a naphthyl group, a fluorenyl group, a carbazolyl group, a dibenzofuranyl group, and a dibenzothiophenyl group;
   a phenyl group, a naphthyl group, a fluorenyl group, a carbazolyl group, a dibenzofuranyl group, and a dibenzothiophenyl group, each substituted with deuterium, a hydroxyl group, an amino group, an amidino group, a hydrazine group, a hydrazone group, a carboxylic acid group or a salt thereof, a sulfonic acid group or a salt thereof, a phosphoric acid group or a salt thereof, a phenyl group, a naphthyl group, a fluorenyl group, a carbazolyl group, a dibenzofuranyl group, and a dibenzothiophenyl group; and
   -Si(Q₁)(Q₂)(Q₃),
e1 and e2 may each independently be an integer from 0 to 10,
Q₁ to Q₃ and Q₁₁ to Q₁₃ may each independently be selected from hydrogen, deuterium, a hydroxyl group, an amino group, an amidino group, a hydrazine group, a hydrazone group, a carboxylic acid group or a salt thereof, a sulfonic acid group or a salt thereof, a phosphoric acid group or a salt thereof, a phenyl group, a naphthyl group, a fluorenyl group, a carbazolyl group, a dibenzofuranyl group, a dibenzothiophenyl group, and a biphenyl group,
* indicates a binding site to a neighboring atom.

For example, at least one selected from CY₁ and CY₂ in Formulae 11 and 12 may be a benzene group, but embodiments of the present disclosure are not limited thereto.

In an embodiment, in Formula H-1,
Ar₁₁ may be a group represented by one of Formulae 11-1 to 11-8 and 12-1 to 12-8,
Ar₁₂ may be selected from:
   a group represented by one of Formulae 11-1 to 11-8 and 12-1 to 12-8, a phenyl group, and a naphthyl group; and
   a phenyl group and a naphthyl group, each substituted with at least one selected from deuterium, a hydroxyl group, an amino group, an amidino group, a hydrazine group, a hydrazone group, a carboxylic acid group or a salt thereof, a sulfonic acid group or a salt thereof, a phosphoric acid group or a salt thereof, a C₁-C₂₀ alkyl group, a C₁-C₂₀ alkoxy group, a phenyl group, a naphthyl group, a fluorenyl group, a carbazolyl group, a dibenzofuranyl group, a dibenzothiophenyl group, and a biphenyl group,
but embodiments of the present disclosure are not limited thereto:

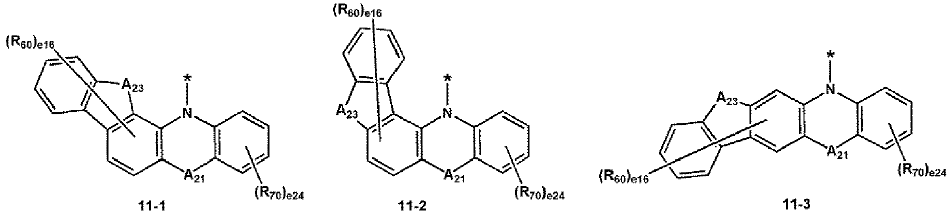

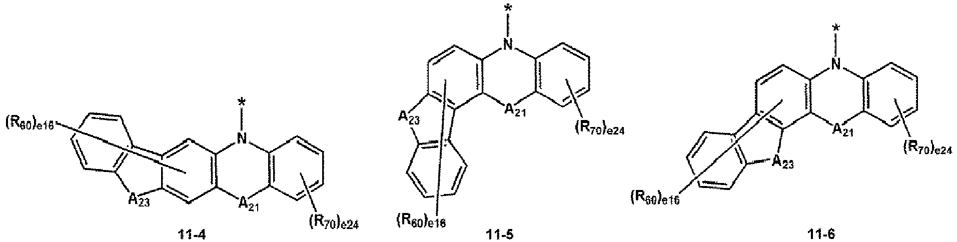

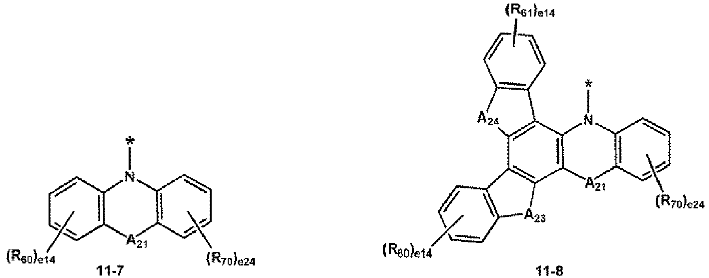

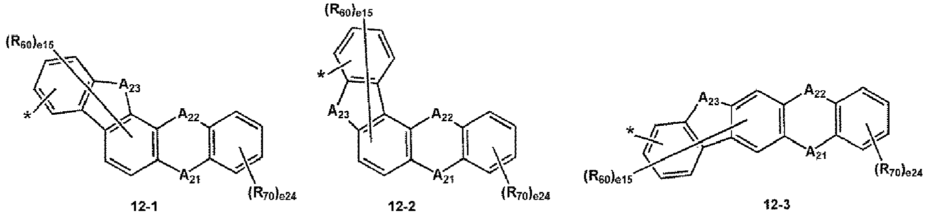

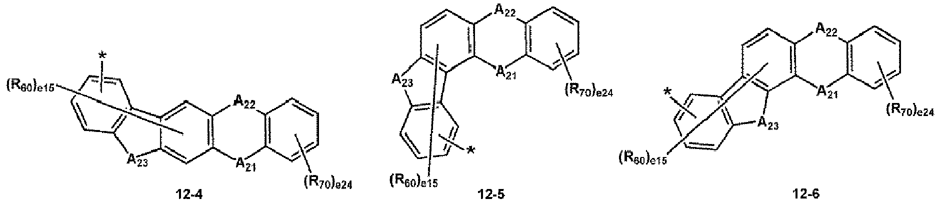

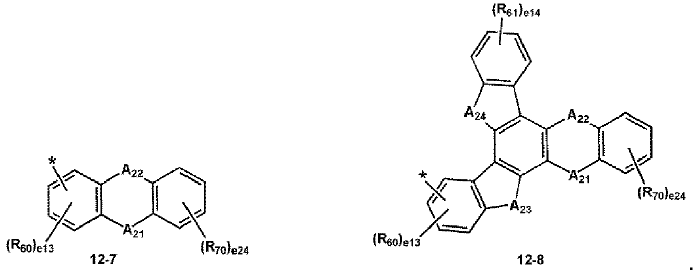

In Formulae 11-1 to 11-8 and 12-1 to 12-8,
A₂₃ may be selected from O, S, N(R₅₅), C(R₅₅)(R₅₆), and Si(R₅₅)(R₅₆),
A₂₄ may be selected from O, S, N(R₅₇), C(R₅₇)(R₅₈), and Si(R₅₇)(R₅₈),
A₂₁, A₂₂, R₆₀, and R₇₀ are the same as described above,
R₅₅ to R₅₈ are each the same as described in connection with R₅₁,
e16 may be an integer from 0 to 6,
e15 may be an integer from 0 to 5,
e14 may be an integer from 0 to 4,
e13 may be an integer from 0 to 3,
e24 may be an integer from 0 to 4,
* indicates a binding site to a neighboring atom.
In one or more embodiments,
i) the second compound in the composition may be a compound represented by Formula H-1 where L₁₁ in Formula H-1 is a single bond; or
ii) the second compound in the composition may be a compound represented by one of Formula H-1(1) to H-1(52), but embodiments of the present disclosure are not limited thereto:

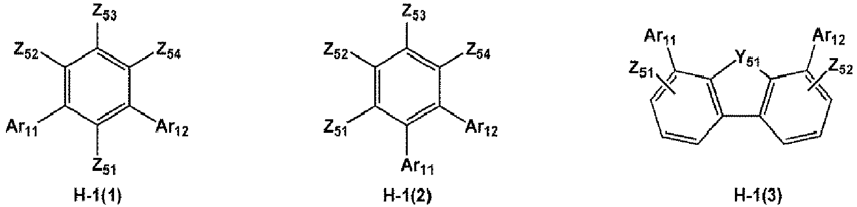

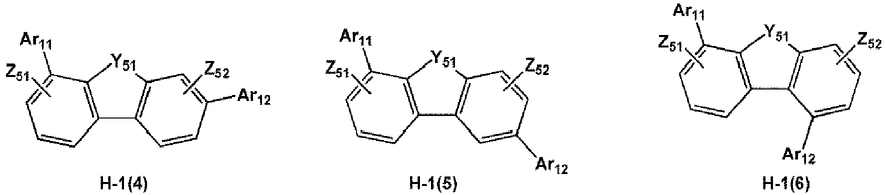

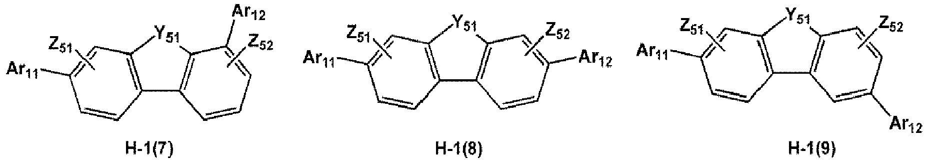

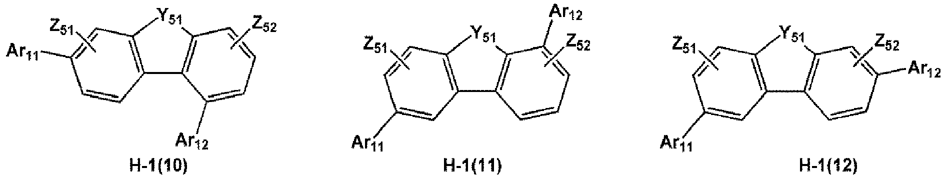

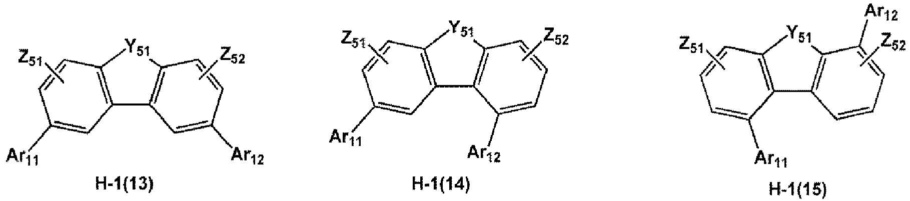

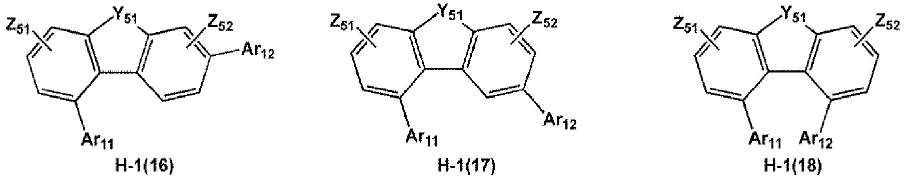

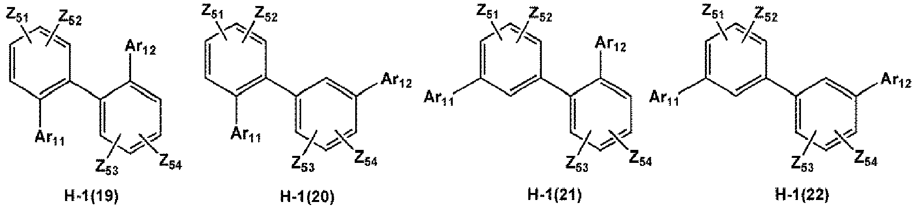

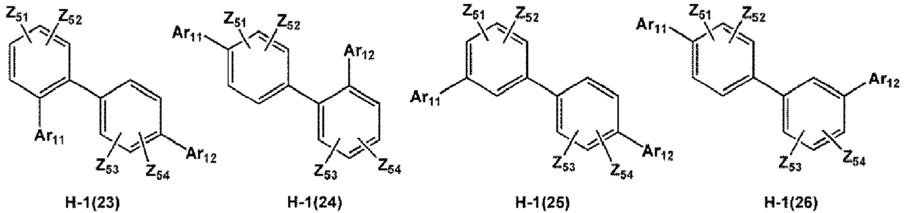

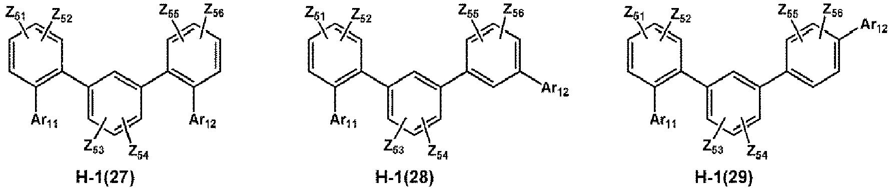

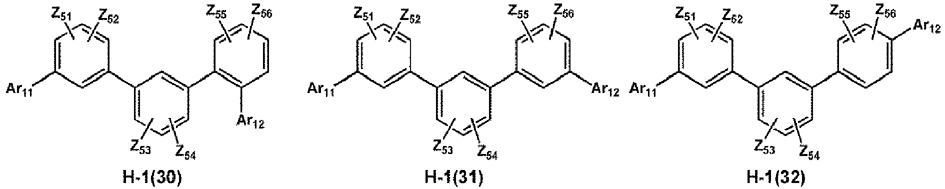

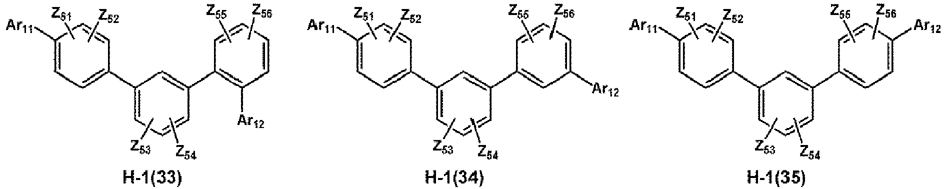

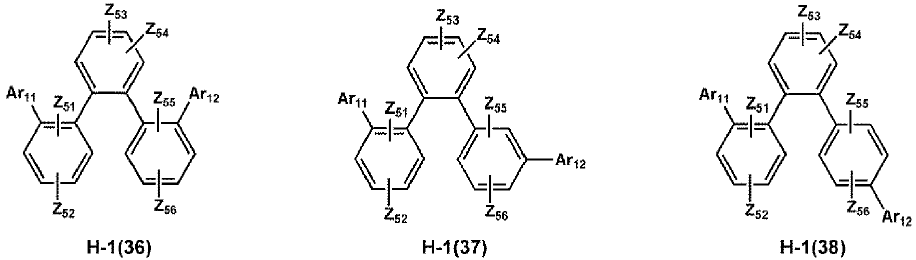

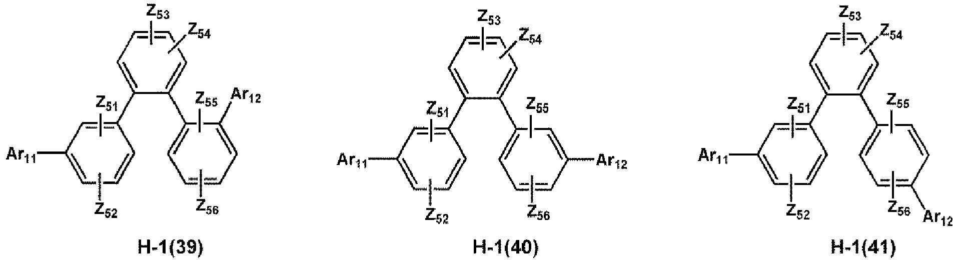

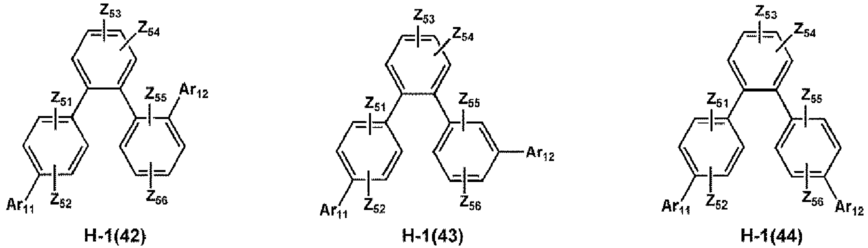

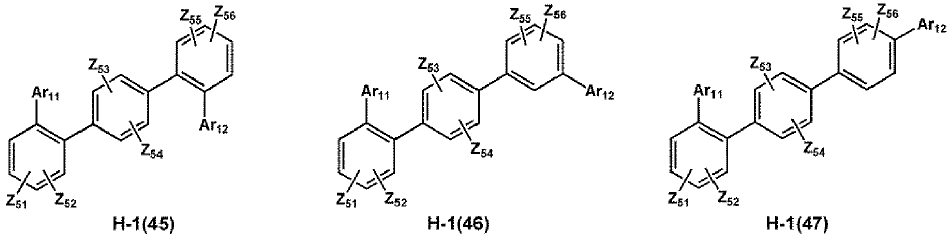

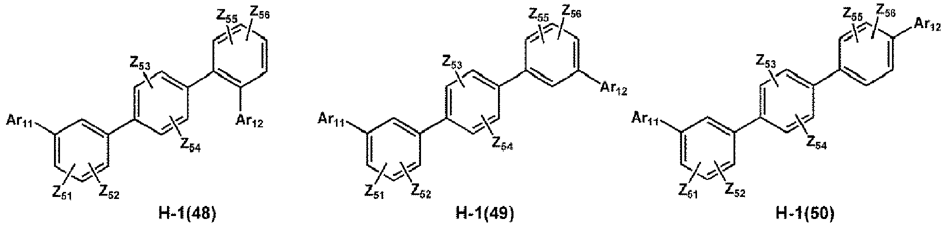

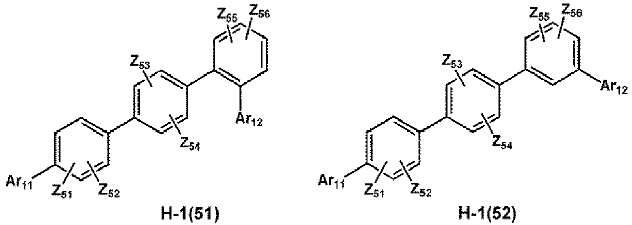

In Formulae H-1(1) to H-1(52),
Ar₁₁ and Ar₁₂ are each the same as described above,
Y₅₁ may each independently be C(Z₅₃)(Z₅₄), Si(Z₅₃)(Z₅₄), N(Z₅₅), O, or S,
Z₅₁ to Z₅₆ may each independently be selected from hydrogen, deuterium, a C₁-C₁₀ alkyl group, a C₁-C₁₀ alkoxy group, a phenyl group, a naphthyl group, a fluorenyl group, a carbazolyl group, a dibenzofuranyl group, a dibenzothiophenyl group, a biphenyl group, and -Si(Q₁₁)(Q₁₂)(Q₁₃),
Q₁₁ to Q₁₃ may each independently be selected from hydrogen, a C₁-C₁₀ alkyl group, a C₁-C₁₀ alkoxy group, a phenyl group, and a naphthyl group, but embodiments of the present disclosure are not limited thereto.

In an embodiment, the second compound in the composition may be selected from Compounds H-1 to H-41, but embodiments of the present disclosure are not limited thereto:

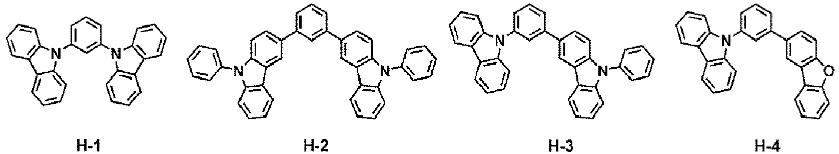

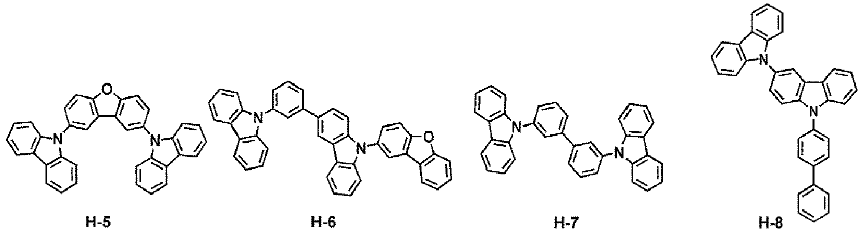

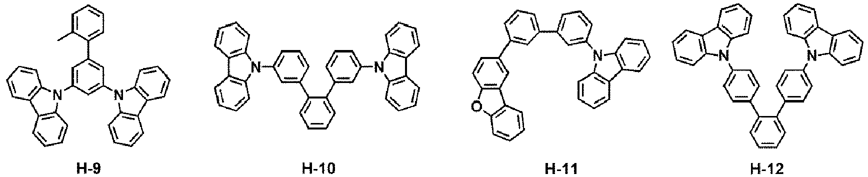

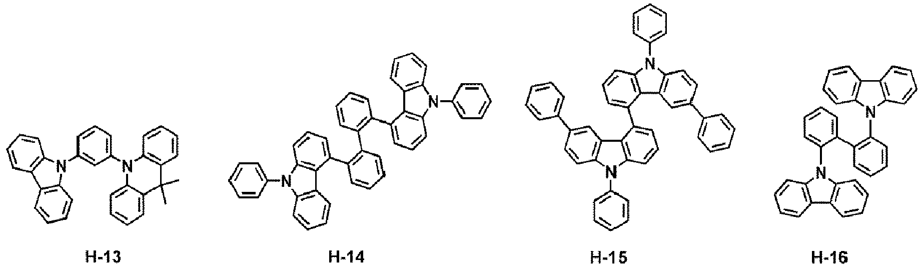

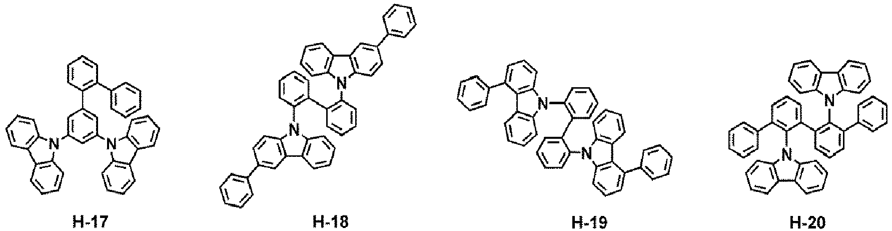

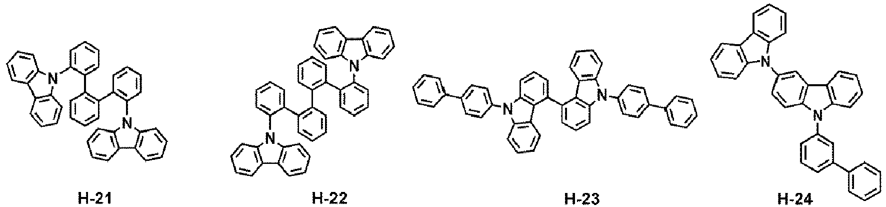

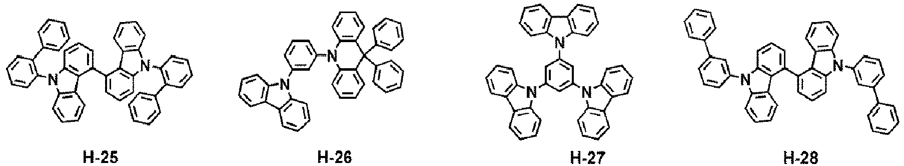

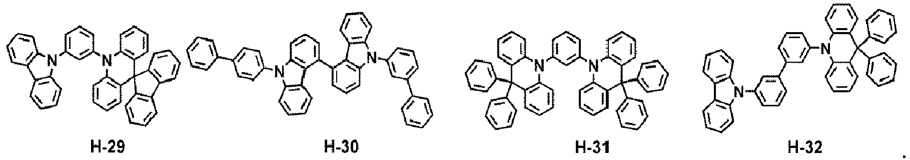

The weight ratio of the first compound to the second compound in the composition may be in the range of about 1 : 99 to about 99: 1, for example, about 70 : about 30 to about 30 : about 70. For example, the weight ratio of the first compound to the second compound in the composition may be selected from about 40 : about 60 to about 60 : about 40, but embodiments of the present disclosure are not limited thereto. While not wishing to be bound by theory, it is understood that when the weight ratio of the first compound to the second compound in the composition satisfies the ranges as described above, the composition may provide an excellent charge transport balance.

The condensed cyclic compound represented by Formula 1 or Formula 2 (or a composition including the first compound that is the condensed cyclic compound represented by Formula 1 or Formula 2 and the second compound described in the specification) may be suitable for an organic layer of an organic light-emitting device, for example, a material for an emission layer and/or electron transport region in the organic layer. Accordingly, another aspect provides an organic light-emitting device including a first electrode; a second electrode; and an organic layer disposed between the first electrode and the second electrode, wherein the organic layer includes an emission layer, and wherein the organic layer includes at least one condensed cyclic compound represented by Formula 1 or Formula 2.

While not wishing to be bound by theory, it is understood that when an organic layer including at least one condensed cyclic compound represented by Formula 1 or Formula 2 is included in an organic light-emitting device, the obtained organic light-emitting device may have low driving voltage, high luminous efficiency, high luminance, high quantum luminous efficiency, and long lifespan.

In an embodiment, in the organic light-emitting device,
the first electrode is an anode and the second electrode is a cathode,
the organic layer includes a hole transport region disposed between the first electrode and the emission layer and an electron transport region disposed between the emission layer and the second electrode,

The hole transport region may include a hole injection layer, a hole transport layer, an electron blocking layer, a buffer layer, or any combination thereof.

The electron transport region may include a hole blocking layer, an electron transport layer, an electron injection layer, or any combination thereof, but embodiments of the present disclosure are not limited thereto.

For example, the emission layer in the organic light-emitting device may include at least one condensed cyclic compound represented by Formula 1 or Formula 2.

In an embodiment, the emission layer in the organic light-emitting device may include a host and a dopant, and the host includes at least one condensed cyclic compound represented by Formula 1 or Formula 2, and the dopant may include a phosphorescent dopant or a fluorescent dopant. For example, the dopant may include a phosphorescent dopant (for example, a transition metal-containing organometallic compound or an organometallic compound represented by Formula 81). The amount of the host in the emission layer may be greater than the amount of the dopant in the emission layer. The host may further include any host in addition to the condensed cyclic compound represented by Formula 1 or Formula 2.

The emission layer may emit red light, green light, or blue light. For example, the emission layer may emit blue light.

In an embodiment, the emission layer may include a blue emission layer including a phosphorescent dopant, but embodiments of the present disclosure are not limited thereto.

In an embodiment, the organic layer may include an electron transport region disposed between the emission layer and the second electrode, and at least one condensed cyclic compound represented by Formula 1 or Formula 2 may be included in the electron transport region of the organic light-emitting device.

For example, the electron transport region of the organic light-emitting device may include at least one selected from a hole blocking layer and an electron transport layer, and the at least one selected from the hole blocking layer and the electron transport layer may include at least one condensed cyclic compound represented by Formula 1 or Formula 2.

In an embodiment, the electron transport region of the organic light-emitting device may include a hole blocking layer, and at least one condensed cyclic compound represented by Formula 1 or Formula 2 may be included in the hole blocking layer. The hole blocking layer directly contacts the emission layer.

In one or more embodiments, the electron transport region may include a hole blocking layer and an electron transport layer, the hole blocking layer is located between the emission layer and the electron transport layer, and the hole blocking layer may include at least one condensed cyclic compound represented by Formula 1 or Formula 2.

In one or more embodiments, the organic layer of the organic light-emitting device may further include, in addition to the condensed cyclic compound represented by Formula 1 or Formula 2,
i) the second compound described herein;
ii) a transition metal-containing organometallic compound (for example, the organometallic compound represented by Formula 81); or
iii) any combination thereof:

   Formula 81 M(L₈₁)ₙ₈₁(L₈₂)ₙ₈₂

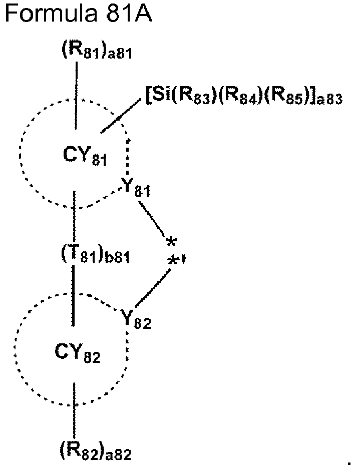

In Formulae 81 and 81A,
M may be a transition metal (for example, iridium (Ir), platinum (Pt), palladium (Pd), gold (Au), osmium (Os), titanium (Ti), zirconium (Zr), hafnium (Hf), europium (Eu), terbium (Tb), thulium (Tm), rhodium (Rh), or the like),
L₈₁ is a ligand represented by Formula 81A, n81 may be an integer from 1 to 3, and when n81 is two or more, two or more of groups L₈₁ may be identical to or different from each other,
L₈₂ may be an organic ligand, n82 may be an integer from 0 to 4, and when n82 is two or more, two or more of groups L₈₂ may be identical to or different from each other,
Y₈₁ and Y₈₂ may each independently be carbon (C) or nitrogen (N),
ring CY₈₁ and ring CY₈₂ may each independently be selected from a C₅-C₃₀ carbocyclic group and a C₁-C₃₀ heterocyclic group,
ring CY₈₁ and ring CY₈₂ may optionally be linked to each other via an organic linking group,
T₈₁ may be a single bond, a double bond, *-N(R₈₆)-*', *-B(R₈₆)-*', *-P(R₈₆)-*', *-C(R₈₆)(R₈₇)-*', *-Si(R₈₆)(R₈₇)-*', *-Ge(R₈₆)(R₈₇)-*', *-S-*', *-Se-*', *-O-*', *-C(=O)-*', *-S(=O)-*', *-S(=O)₂-*', *-C(R₈₆)=*', *=C(R₈₆)-*', *-C(R₈₆)=C(R₈₇)-*', *-C(=S)-*', or *-C≡C-*', and * and *' each indicate a binding site to a neighboring atom,
b81 may be 1, 2, or 3,
R₈₁ to R₈₇ may each independently be selected from hydrogen, deuterium,-F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, -SF₅, a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₇-C₆₀ arylalkyl group, a substituted or unsubstituted C₁-C₆₀ heteroaryl group, a substituted or unsubstituted C₁-C₆₀ heteroaryloxy group, a substituted or unsubstituted C₁-C₆₀ heteroarylthio group, a substituted or unsubstituted C₂-C₆₀ heteroarylalkyl group, a substituted or unsubstituted monovalent non-aromatic condensed polycyclic group, a substituted or unsubstituted monovalent non-aromatic condensed heteropolycyclic group, -Si(Q₈₁)(Q₈₂)(Q₈₃), -N(Q₈₄)(Q₈₅),-B(Q₈₆)(Q₈₇), and -P(=O)(Q₈₈)(Q₈₉),
a81 to a83 may each independently be an integer from 0 to 5,
when a81 is two or more, two or more groups R₈₁ may be identical to or different from each other,
when a82 is two or more, two or more groups R₈₂ may be identical to or different from each other,
when a81 is two or more, adjacent two groups R₈₁ may optionally be linked each other to form a C₅-C₃₀ carbocyclic group and a C₁-C₃₀ heterocyclic group, each substituted with at least one R₈₈ (for example, a benzene group, a cyclopentane group, a cyclohexane group, a cyclopentene group, a cyclohexene group, a norbornane group, a bicyclo[2.2.1]heptane group, a naphthalene group, a benzoindene group, a benzoindole group, a benzofuran group, a benzothiophene group, a pyridine group, a pyrimidine group, or a pyrazine group, each substituted with at least one R₈₈),
when a82 is two or more, adjacent two groups R₈₂ may optionally be linked each other to form a C₅-C₃₀ carbocyclic group and a C₁-C₃₀ heterocyclic group, each substituted with at least one R₈₉ (for example, a benzene group, a cyclopentane group, a cyclohexane group, a cyclopentene group, a cyclohexene group, a norbornane group, a bicyclo[2.2.1]heptane group, a naphthalene group, a benzoindene group, a benzoindole group, a benzofuran group, a benzothiophene group, a pyridine group, a pyrimidine group, or a pyrazine group, each substituted with at least one R₈₉),
R₈₈ may be the same as explained in connection with R₈₁,
R₈₉ may be the same as explained in connection with R₈₂,
* and *' in Formula 81A each indicates a binding site to M in Formula 81,
at least one substituent selected from the substituted C₁-C₆₀ alkyl group, the substituted C₂-C₆₀ alkenyl group, the substituted C₂-C₆₀ alkynyl group, the substituted C₁-C₆₀ alkoxy group, the substituted C₃-C₁₀ cycloalkyl group, the substituted C₁-C₁₀ heterocycloalkyl group, the substituted C₃-C₁₀ cycloalkenyl group, the substituted C₁-C₁₀ heterocycloalkenyl group, the substituted C₆-C₆₀ aryl group, the substituted C₆-C₆₀ aryloxy group, the substituted C₆-C₆₀ arylthio group, the substituted C₇-C₆₀ arylalkyl group, the substituted C₁-C₆₀ heteroaryl group, the substituted C₁-C₆₀ heteroaryloxy group, the substituted C₁-C₆₀ heteroarylthio group, the substituted C₂-C₆₀ heteroarylalkyl group, the substituted monovalent non-aromatic condensed polycyclic group, and substituted monovalent non-aromatic condensed heteropolycyclic group may be selected from deuterium, -F, -CI, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₇-C₆₀ arylalkyl group, a C₁-C₆₀ heteroaryl group, a C₁-C₆₀ heteroaryloxy group, a C₁-C₆₀ heteroarylthio group, a C₂-C₆₀ heteroarylalkyl group, a monovalent non-aromatic condensed polycyclic group, a monovalent non-aromatic condensed heteropolycyclic group, and -Si(Q₉₁)(Q₉₂)(Q₉₃), and
Q₈₁ to Q₈₉ and Q₉₁ to Q₉₃ may each independently be selected from hydrogen, deuterium, a C₁-C₆₀ alkyl group, a C₁-C₆₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₁-C₆₀ heteroaryl group, a monovalent non-aromatic condensed polycyclic group, and a monovalent non-aromatic condensed heteropolycyclic group.

In an embodiment, in Formula 81A,
a83 may be 1 or 2,
R₈₃ to R₈₅ may each independently be selected from:
   -CH₃, -CD₃, -CD₂H, -CDH₂, -CH₂CH₃, -CH₂CD₃, -CH₂CD₂H, -CH₂CDH₂,-CHDCH₃, -CHDCD₂H, -CHDCDH₂, -CHDCD₃, -CD₂CH₃, -CD₂CD₃, -CD₂CD₂H, and -CD₂CDH₂;
   an n-propyl group, an iso-propyl group, an n-butyl group, an iso-butyl group, a sec-butyl group, a tert-butyl group, an n-pentyl group, an iso-pentyl group, a sec-pentyl group, a tert-pentyl group, a phenyl group, and a naphthyl group; and
   an n-propyl group, an iso-propyl group, an n-butyl group, an iso-butyl group, a sec-butyl group, a tert-butyl group, an n-pentyl group, an iso-pentyl group, a sec-pentyl group, a tert-pentyl group, a phenyl group, and a naphthyl group, each substituted with at least one selected from deuterium, a C₁-C₁₀ alkyl group, and a phenyl group,
but embodiments of the present disclosure are not limited thereto.

In one or more embodiments, in Formula 81A,
Y₈₁ is nitrogen, and Y₈₄ is nitrogen or carbon,
ring CY₈₁ and ring CY₈₂ may each independently be selected from a cyclopentadiene group, a benzene group, a heptalene group, an indene group, a naphthalene group, an azulene group, an indacene group, acenaphthylene group, a fluorene group, a spiro-bifluorene group, a benzofluorene group, a dibenzofluorene group, a phenalene group, a phenanthrene group, an anthracene group, a fluoranthene group, a triphenylene group, a pyrene group, a chrysene group, a naphthacene group, a picene group, a perylene group, a pentacene group, a hexacene group, a pentacene group, a rubicene group, a coronene group, an ovalene group, a pyrrole group, an isoindole group, an indole group, an indazole group, a pyrazole group, an imidazole group, a triazole group, an oxazole group, an isoxazole group, an oxadiazole group, a thiazole group, an isothiazole group, a thiadiazole group, a purine group, a furan group, a thiophene group, a pyridine group, a pyrimidine group, a quinoline group, an isoquinoline group, a benzoquinoline group, a phthalazine group, a naphthyridine group, a quinoxaline group, a quinazoline group, a cinnoline group, a phenanthrididine group, an acridine group, a phenanthroline group, a phenazine group, a benzimidazole group, a benzofuran group, a benzothiophene group, an isobenzothiazole group, a benzoxazole group, an isobenzoxazole group, a benzocarbazole group, a dibenzocarbazole group, an imidazopyridine group, an imidazopyrimidine group, a dibenzofuran group, a dibenzothiophene group, a dibenzothiophene sulfone group, a carbazole group, a dibenzosilole group, and a 2,3-dihydro-1H-imidazole group.

In one or more embodiments, in Formula 81A, Y₈₁ is nitrogen, Y₈₄ is carbon, ring CY₈₁ is a 5-membered ring containing two nitrogen atoms as a ring-forming atom, and ring CY₈₂ is selected from a benzene group, a naphthalene group, a fluorene group, a dibenzofuran group, and a dibenzothiophene group, but embodiments of the present disclosure are not limited thereto.

In one or more embodiments, in Formula 81A , Y₈₁ is nitrogen, Y₈₄ is carbon, ring CY₈₁ is an imidazole group or a 2,3-dihydro-1H-imidazole group, ring CY₈₂ is selected from a benzene group, a naphthalene group, a fluorene group, a dibenzofuran group, and a dibenzothiophene group, but embodiments of the present disclosure are not limited thereto.

In one or more embodiments, in Formula 81A,
Y₈₁ may be nitrogen and Y₈₄ may be carbon,
ring CY₈₁ may be selected from a pyrrole group, a pyrazole group, an imidazole group, a triazole group, an oxazole group, an isoxazole group, an oxadiazole group, a thiazole group, an isothiazole group, a thiadiazole group, a pyridine group, a pyrimidine group, a quinoline group, an isoquinoline group, a benzoquinoline group, a phthalazine group, a naphthyridine group, a quinoxaline group, a quinazoline group, a cinnoline group, a benzimidazole group, an isobenzothiazole group, a benzoxazole group, and an isobenzoxazole group, and
ring CY₈₂ may be selected from a cyclopentadiene group, a benzene group, a naphthalene group, a fluorene group, a benzofluorene group, a dibenzofluorene group, a phenanthrene group, an anthracene group, a triphenylene group, a pyrene group, a chrysene group, a perylene group, a benzofuran group, a benzothiophene group, a benzocarbazole group, a dibenzocarbazole group, a dibenzofuran group, a dibenzothiophene group, a dibenzothiophene sulfone group, a carbazole group, and a dibenzosilole group.

In one or more embodiments, R₈₁ and R₈₂ in Formula 81A may each independently be selected from:
hydrogen, deuterium, -F, -CI, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, -SF₅, C₁-C₂₀ alkyl group, and a C₁-C₂₀ alkoxy group;
a C₁-C₂₀ alkyl group and a C₁-C₂₀ alkoxy group, each substituted with at least one selected from deuterium, -F, -CI, -Br, -I, -CD₃, -CD₂H, -CDH₂, -CF₃, -CF₂H,-CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₁₀ alkyl group, a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a naphthyl group, a pyridinyl group, and a pyrimidinyl group;
a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a naphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyridinyl group, and an imidazopyrimidinyl group;
a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a naphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyridinyl group, and an imidazopyrimidinyl group, each substituted with at least one selected from deuterium, -F, -CI, -Br, -I, -CD₃, -CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₂₀ alkyl group, a C₁-C₂₀ alkoxy group, a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a naphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyridinyl group, and an imidazopyrimidinyl group; and
-B(Q₈₆)(Q₈₇) and -P(=O)(Q₈₈)(Q₈₉),
Q₈₆ to Q₈₉ may each independently be selected from
   -CH₃, -CD₃, -CD₂H, -CDH₂, -CH₂CH₃, -CH₂CD₃, -CH₂CD₂H, -CH₂CDH₂,-CHDCH₃, -CHDCD₂H, -CHDCDH₂, -CHDCD₃, -CD₂CH₃, -CD₂CD₃, -CD₂CD₂H, and -CD₂CDH₂;
an n-propyl group, an iso-propyl group, an n-butyl group, an iso-butyl group, a sec-butyl group, a tert-butyl group, an n-pentyl group, an iso-pentyl group, a sec-pentyl group, a tert-pentyl group, a phenyl group, and a naphthyl group; and
an n-propyl group, an iso-propyl group, an n-butyl group, an iso-butyl group, a sec-butyl group, a tert-butyl group, an n-pentyl group, an iso-pentyl group, a sec-pentyl group, a tert-pentyl group, a phenyl group, and a naphthyl group, each substituted with at least one selected from deuterium, a C₁ to C₁₀ alkyl group, and a phenyl group.

In one or more embodiments, in Formula 81A, at least one selected from groups R₈₁ in the number of a81 and groups R₈₂ in the number of a82 may be a cyano group.

In one or more embodiments, in Formula 81A, at least one selected from groups R₈₂ in the number of a82 may be a cyano group.

In one or more embodiments, in Formula 81A, at least one selected from groups R₈₁ in the number of a81 and groups R₈₂ in the number of a82 may be a deuterium.

In one or more embodiments, L₈₂ in Formula 81 may be a ligand represented by one of Formulae 3-1(1) to 3-1(60), 3-1(61) to 3-1(69), 3-1(71) to 3-1(79), 3-1(81) to 3-1(88), 3-1(91) to 3-1(98), and 3-1(101) to 3-1(114):

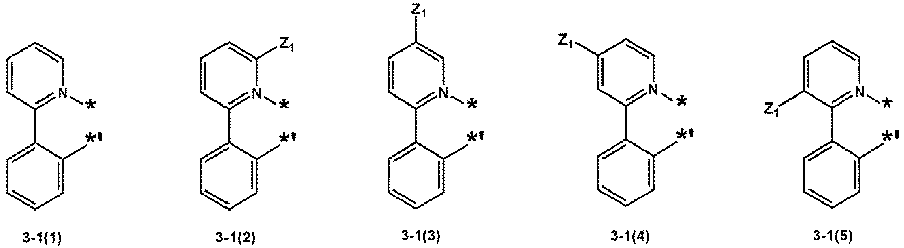

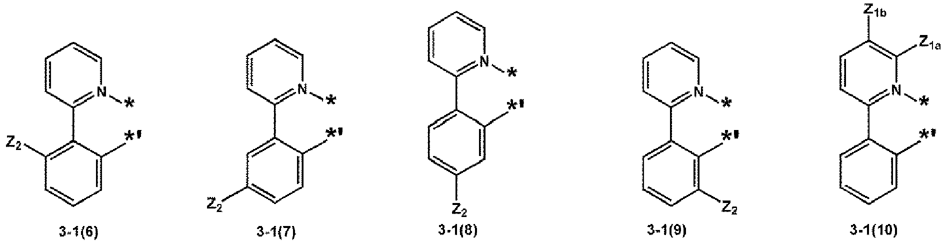

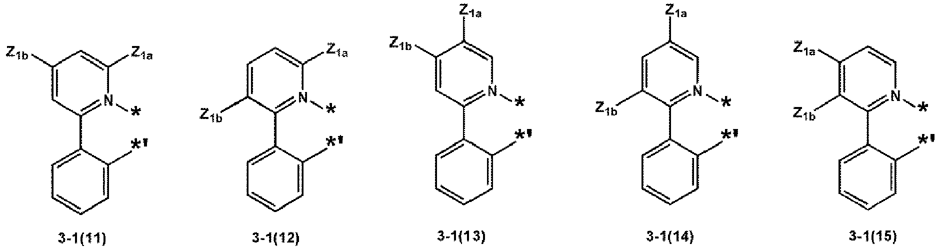

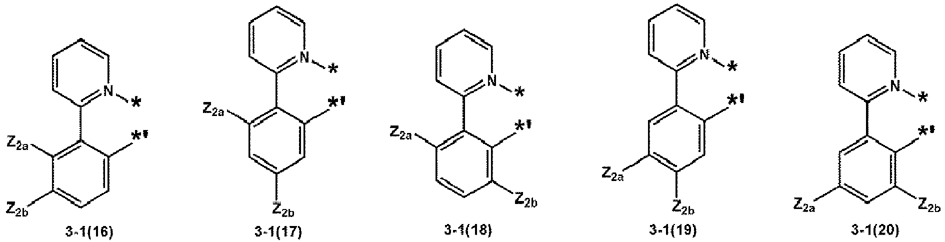

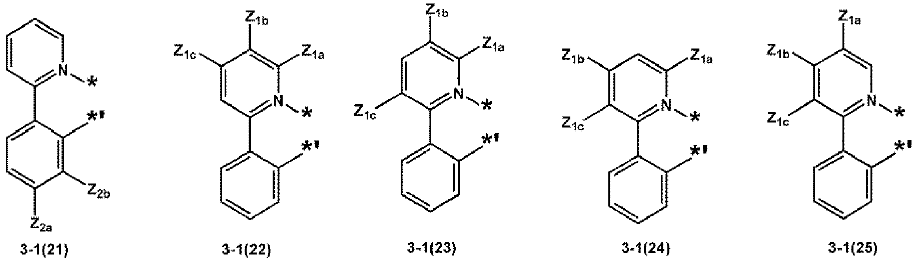

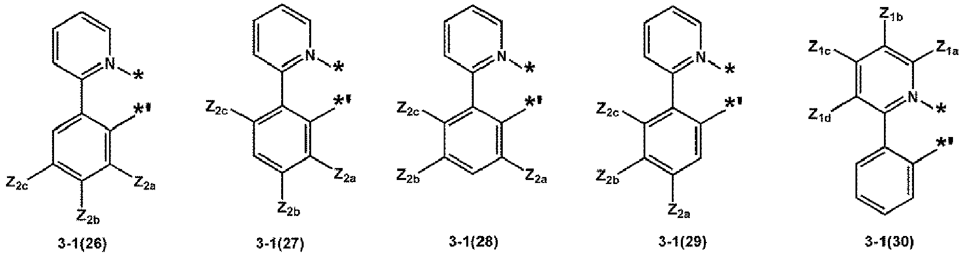

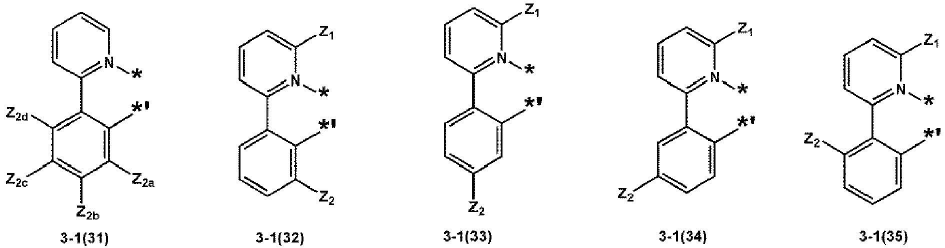

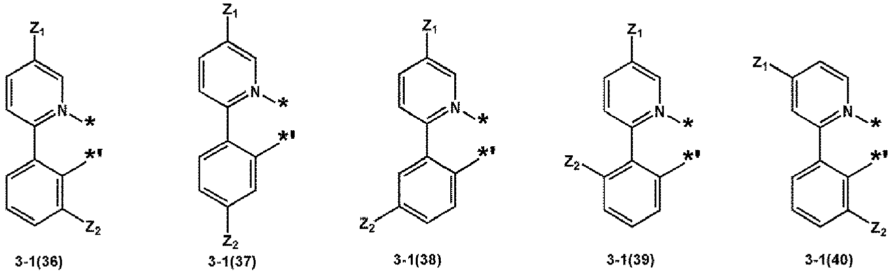

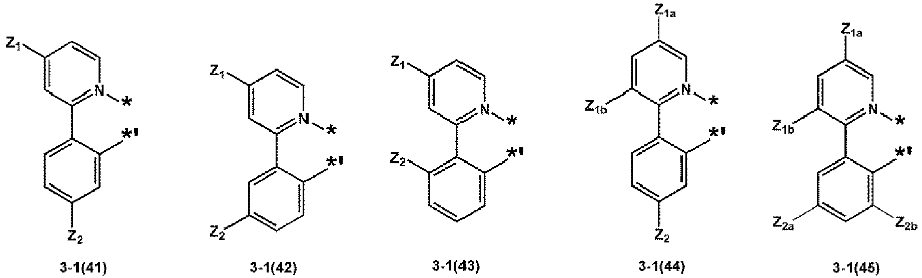

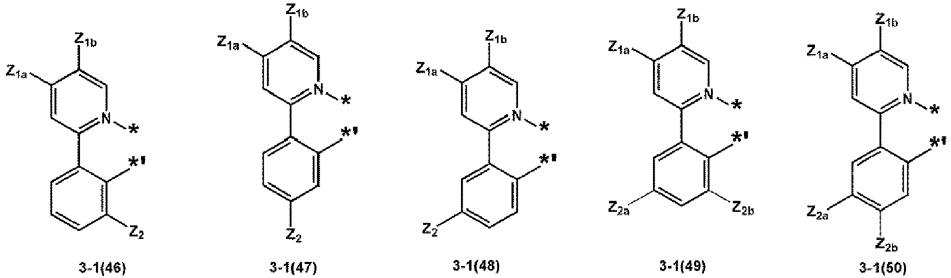

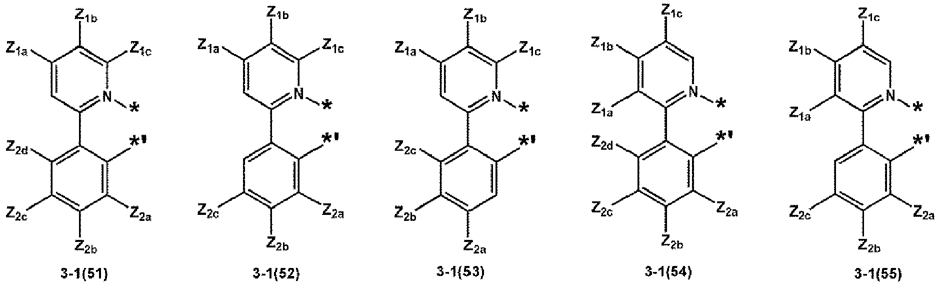

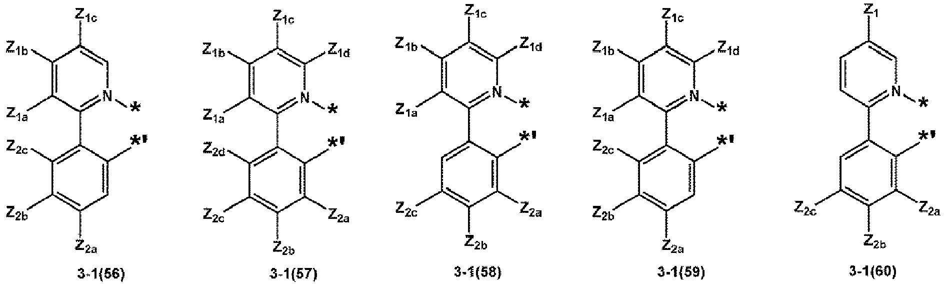

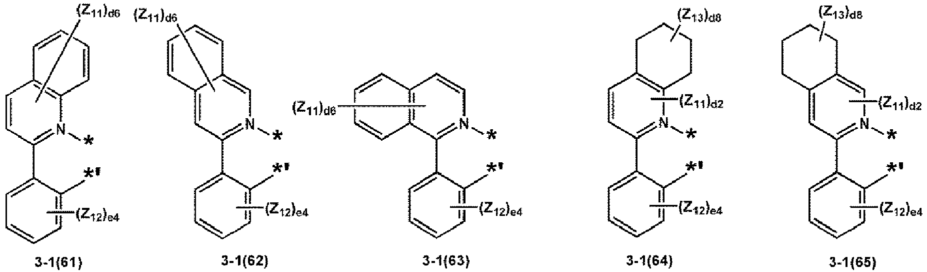

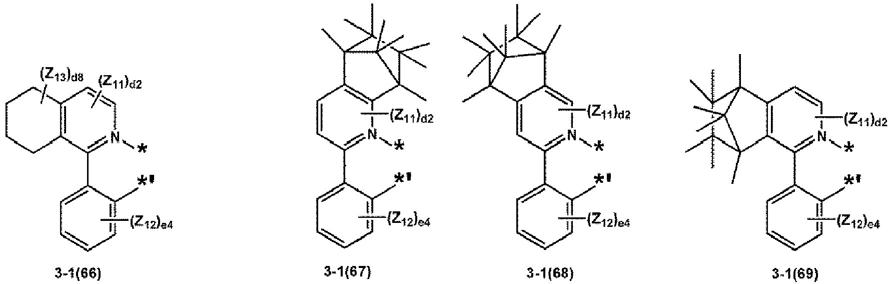

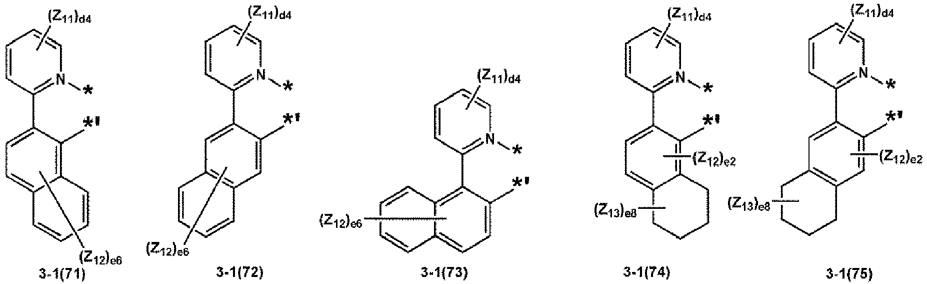

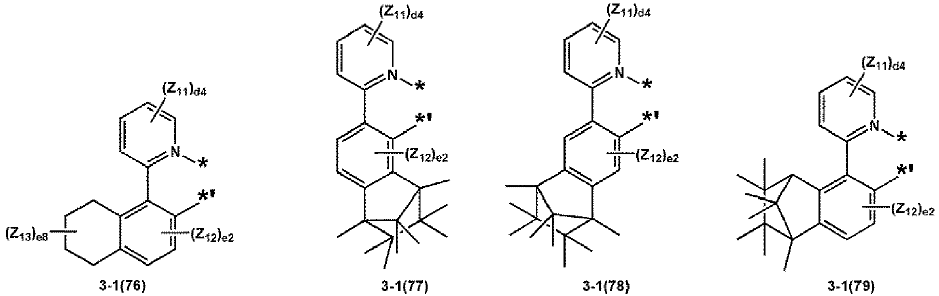

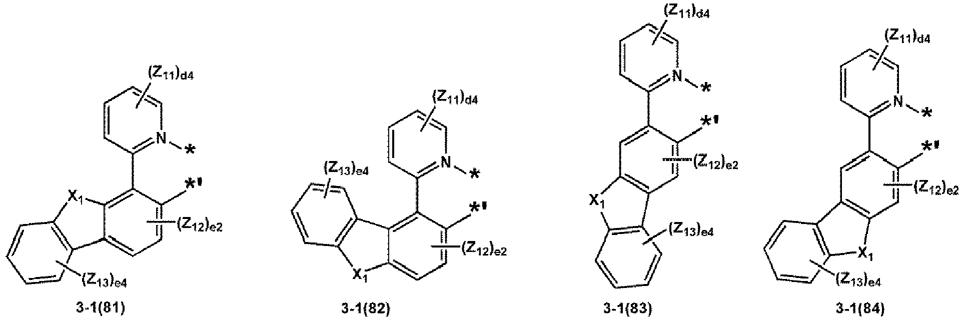

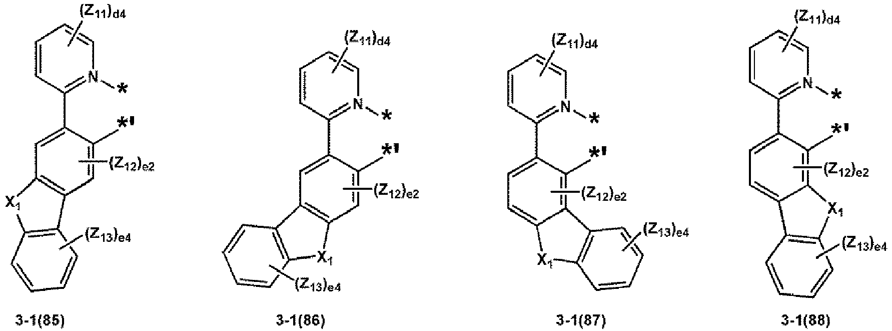

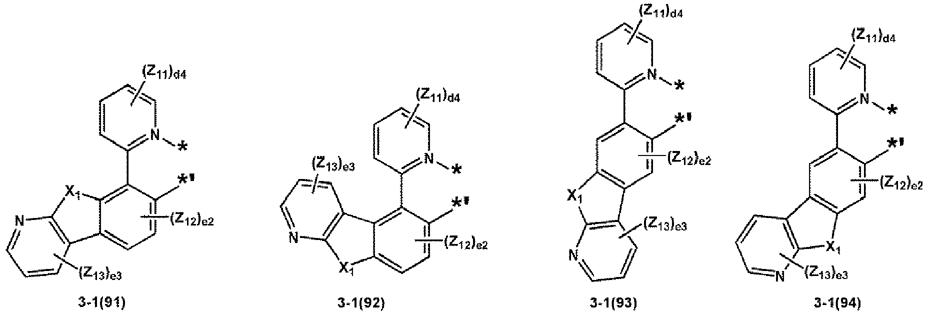

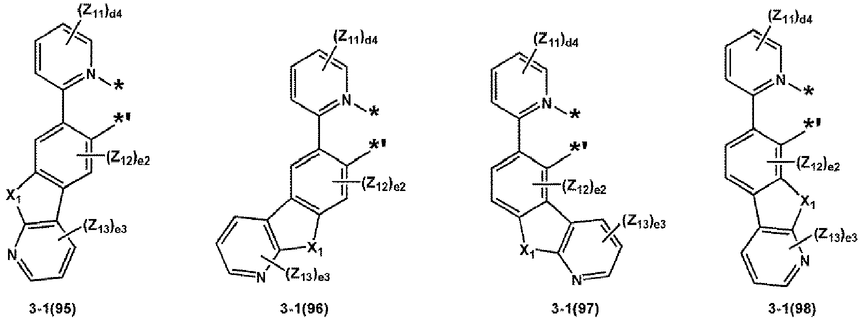

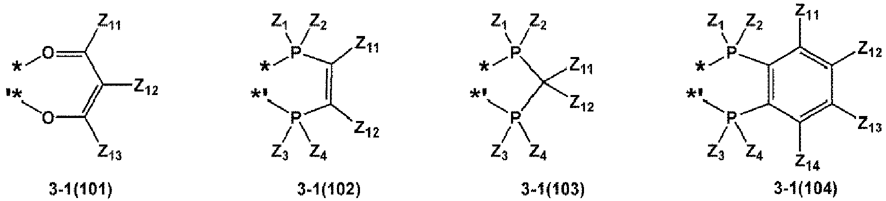

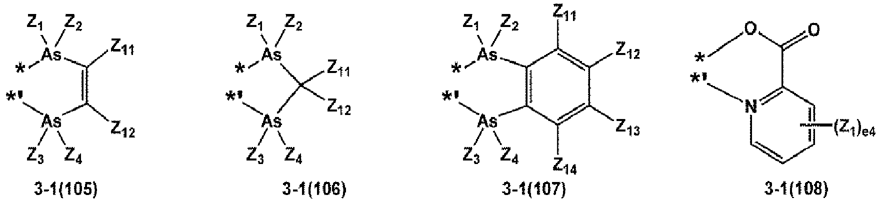

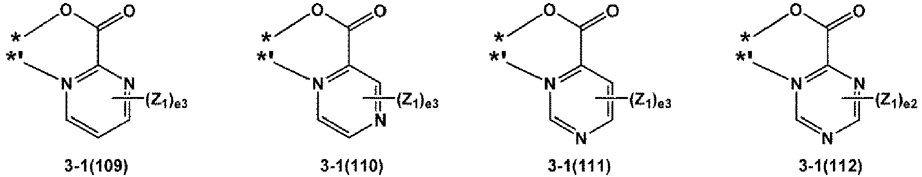

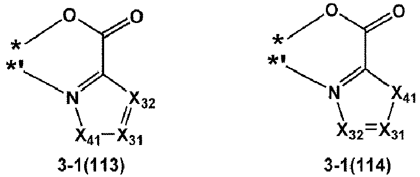

In Formulae 3-1(1) to 3-1(60), 3-1(61) to 3-1(69), 3-1(71) to 3-1(79), 3-1(81) to 3-1(88), 3-1(91) to 3-1(98), and 3-1(101) to 3-1(114),
X₁ may be O, S, C(Z₂₁)(Z₂₂), or N(Z₂₃),
X₃₁ may be N or C(Z₁ₐ) and X₃₂ may be N or C(Z_{1b}),
X₄₁ may be O, S, N(Z₁ₐ), or C(Z₁ₐ)(Z_{1b}),
Z₁ to Z₄, Z₁ₐ, Z_{1b}, Z_{1c}, Z_{1d}, Z₂ₐ, Z_{2b}, Z_{2c}, Z_{2d}, Z₁₁ to Z₁₄ and Z₂₁ to Z₂₃ may each independently be selected from:
   hydrogen, deuterium, -F, -CI, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, -SF₅, C₁-C₂₀ alkyl group, and a C₁-C₂₀ alkoxy group;
   a C₁-C₂₀ alkyl group and a C₁-C₂₀ alkoxy group, each substituted with at least one selected from deuterium, -F, -CI, -Br, -I, -CD₃, -CD₂H, -CDH₂, -CF₃, -CF₂H,-CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₁₀ alkyl group, a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a naphthyl group, a pyridinyl group, and a pyrimidinyl group;
   a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a naphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyridinyl group, and an imidazopyrimidinyl group;
   a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a naphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyridinyl group, and an imidazopyrimidinyl group, each substituted with at least one selected from deuterium, -F, -CI, -Br, -I, -CD₃, -CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₂₀ alkyl group, a C₁-C₂₀ alkoxy group, a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a naphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyridinyl group, and an imidazopyrimidinyl group; and
   -B(Q₈₆)(Q₈₇) and -P(=O)(Q₈₈)(Q₈₉),
Q₈₆ to Q₈₉ may each independently be selected from:
   -CH₃, -CD₃, -CD₂H, -CDH₂, -CH₂CH₃, -CH₂CD₃, -CH₂CD₂H, -CH₂CDH₂,-CHDCH₃, -CHDCD₂H, -CHDCDH₂, -CHDCD₃, -CD₂CH₃, -CD₂CD₃, -CD₂CD₂H, and -CD₂CDH₂;
   an n-propyl group, an iso-propyl group, an n-butyl group, an iso-butyl group, a sec-butyl group, a tert-butyl group, an n-pentyl group, an iso-pentyl group, a sec-pentyl group, a tert-pentyl group, a phenyl group, and a naphthyl group; and
   an n-propyl group, an iso-propyl group, an n-butyl group, an iso-butyl group, a sec-butyl group, a tert-butyl group, an n-pentyl group, an iso-pentyl group, a sec-pentyl group, a tert-pentyl group, a phenyl group, and a naphthyl group, each substituted with at least one selected from deuterium, a C₁ to C₁₀ alkyl group, and a phenyl group,
d2 and e2 may each independently be 0 or 2,
e3 may be an integer from 0 to 3,
d4 and e4 may each independently be an integer from 0 to 4,
d6 and e6 may each independently be an integer from 0 to 6,
d8 and e8 may each independently be an integer from 0 to 8,
* and *' each indicate a binding site to M in Formula 1.

In one or more embodiments, in Formula 81, M may be Ir and the sum of n81 and n82 may be 3; or M may be Pt and the sum of n81 and n82 may be 2.

In one or more embodiments, the organometallic compound represented by Formula 81 may be neutral rather than a salt consisting of the pair of a cation and an anion.

In one or more embodiments, the organometallic compound represented by Formula 81 may include at least one selected from compounds PD1 to PD78 and FIr₆, but embodiments of the present disclosure are not limited thereto.

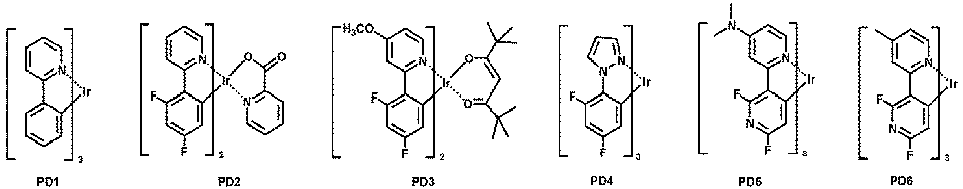

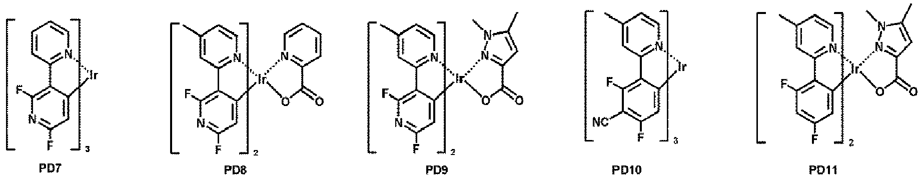

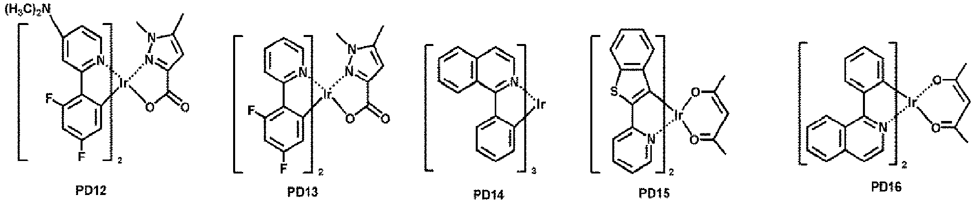

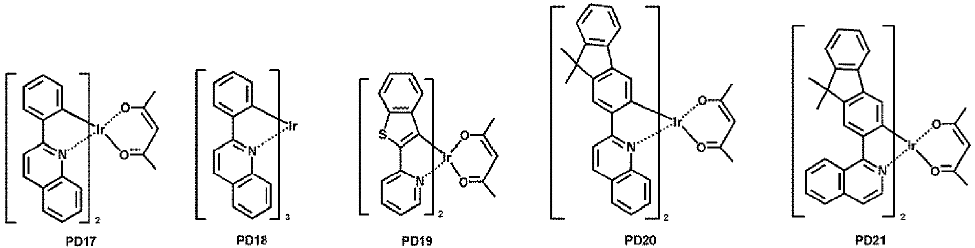

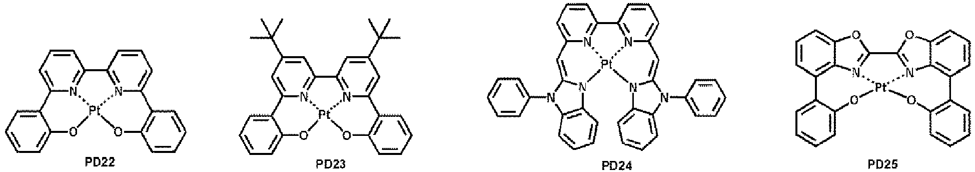

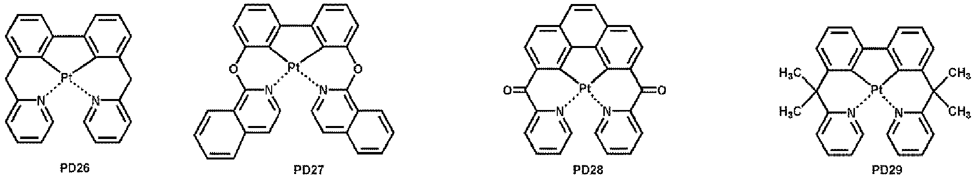

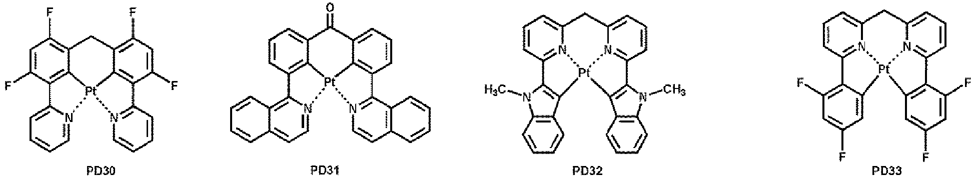

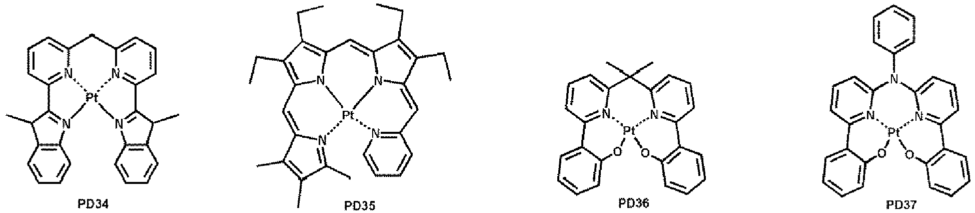

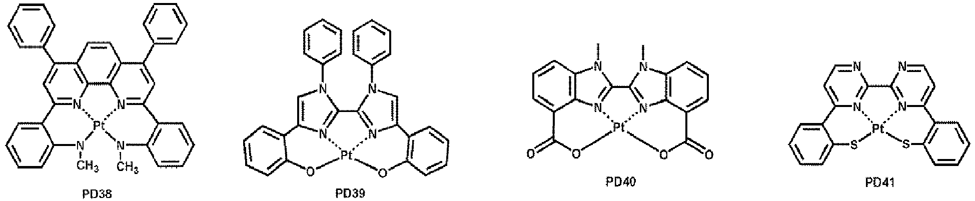

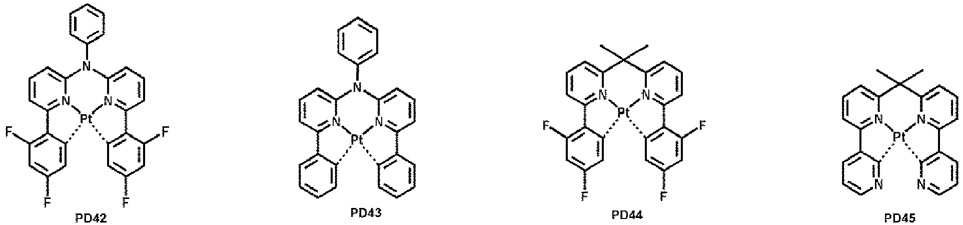

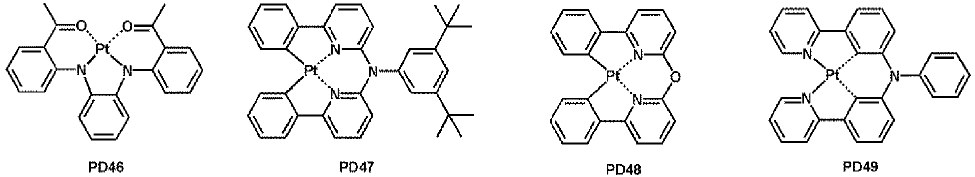

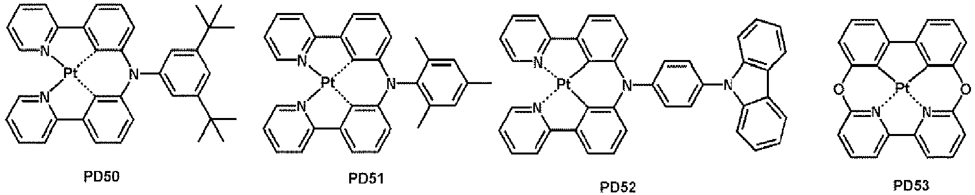

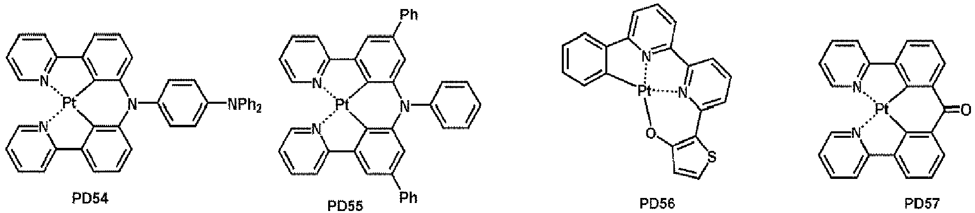

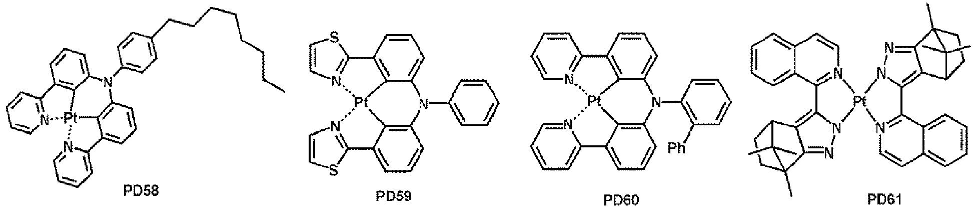

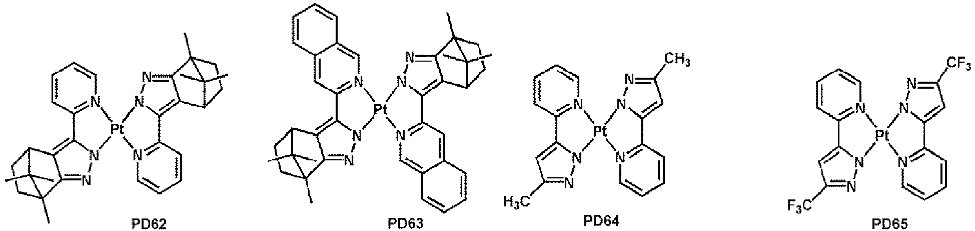

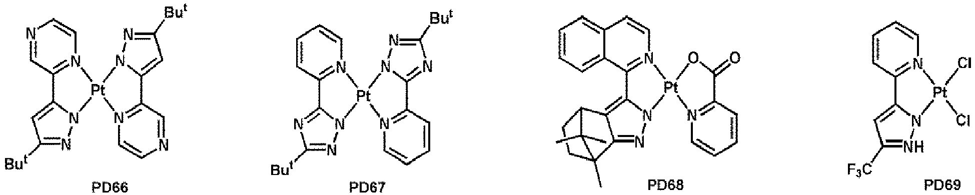

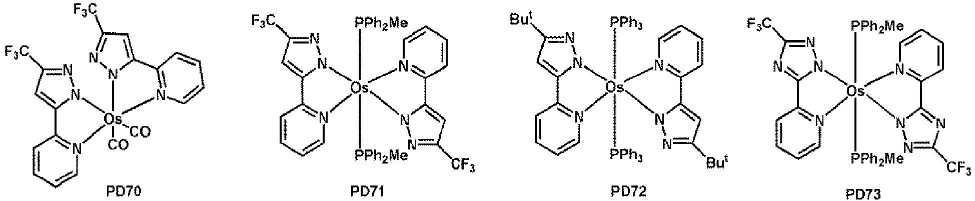

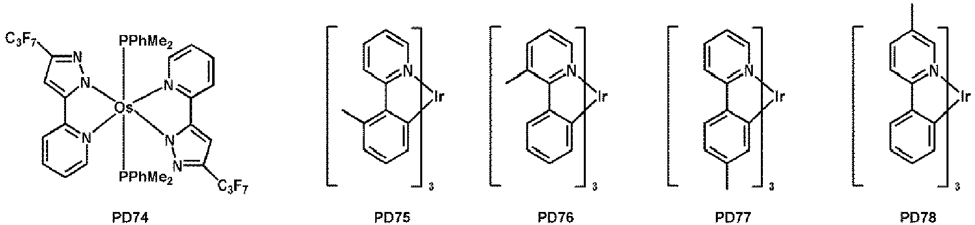

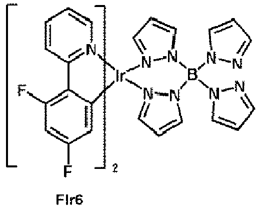

The expression "(an organic layer) includes at least one of condensed cyclic compound" as used herein may include an embodiment in which "(an organic layer) may include identical condensed cyclic compounds represented by Formula 1 or Formula 2 or an embodiment in which "(an organic layer) may include two or more different condensed cyclic compounds represented by Formula 1 or Formula 2."

For example, the organic layer may include, as the condensed cyclic compound, only Compound 1. In this embodiment, Compound 1 may be present in an emission layer of the organic light-emitting device. In one or more embodiments, the organic layer may include, as the condensed cyclic compound, Compound 1 and Compound 2. In this embodiment, Compound 1 and Compound 2 may be present in an identical layer (for example, Compound 1 and Compound 2 may both be present in an emission layer), or different layers (for example, Compound 1 may be present in an emission layer and Compound 2 may be present in a hole blocking layer).

The first electrode may be an anode, which is a hole injection electrode, and the second electrode may be a cathode, which is an electron injection electrode; or the first electrode may be a cathode, which is an electron injection electrode, and the second electrode may be an anode, which is a hole injection electrode.

The term "organic layer" as used herein refers to a single layer and/or a plurality of layers disposed between the first electrode and the second electrode of the organic light-emitting device. The "organic layer" may include, in addition to an organic compound, an organometallic complex including metal.

The FIGURE is a schematic view of an organic light-emitting device 10 according to an embodiment. Hereinafter, the structure of an organic light-emitting device according to an embodiment and a method of manufacturing an organic light-emitting device according to an embodiment will be described in connection with the FIGURE. The organic light-emitting device 10 includes a first electrode 11, an organic layer 15, and a second electrode 19, which are sequentially stacked.

A substrate may be additionally located under the first electrode 11 or above the second electrode 19. For use as the substrate, any substrate that is used in organic light-emitting devices available in the art may be used, and the substrate may be a glass substrate or a transparent plastic substrate, each having excellent mechanical strength, thermal stability, transparency, surface smoothness, ease of handling, and water resistance.

In one or more embodiments, the first electrode 11 may be formed by depositing or sputtering a material for forming the first electrode11 on the substrate. The first electrode 11 may be an anode. The material for forming the first electrode 11 may be selected from materials with a high work function to facilitate hole injection. The first electrode 11 may be a reflective electrode, a semi-transmissive electrode, or a transmissive electrode. The material for forming the first electrode 11 may be indium tin oxide (ITO), indium zinc oxide (IZO), tin oxide (SnO₂), or zinc oxide (ZnO). In one or more embodiments, the material for forming the first electrode 11 may be metal, such as magnesium (Mg), aluminum (Al), aluminum-lithium (Al-Li), calcium (Ca), magnesium-indium (Mg-In), or magnesium-silver (Mg-Ag).

The first electrode 11 may have a single-layered structure or a multi-layered structure including two or more layers. For example, the first electrode 11 may have a three-layered structure of ITO/Ag/ITO, but the structure of the first electrode 11 is not limited thereto.

The organic layer 15 is located on the first electrode 11.

The organic layer 15 may include a hole transport region, an emission layer, and an electron transport region.

The hole transport region may be disposed between the first electrode 11 and the emission layer.

The hole transport region may include at least one selected from a hole injection layer, a hole transport layer, an electron blocking layer, and a buffer layer.

The hole transport region may include only either a hole injection layer or a hole transport layer. In one or more embodiments, the hole transport region may have a hole injection layer/hole transport layer structure or a hole injection layer/hole transport layer/electron blocking layer structure, which are sequentially stacked in this stated order from the first electrode 11.

When the hole transport region includes a hole injection layer (HIL), the hole injection layer may be formed on the first electrode 11 by using one or more suitable methods, for example, vacuum deposition, spin coating, casting, and/or Langmuir-Blodgett (LB) deposition.

When a hole injection layer is formed by vacuum deposition, the deposition conditions may vary according to a material that is used to form the hole injection layer, and the structure and thermal characteristics of the hole injection layer. For example, the deposition conditions may include a deposition temperature of about 100 to about 500°C, a vacuum pressure of about 10⁻⁸ torr to about 10⁻³ torr, and a deposition rate of about 0.01 Angstroms per second (Å/sec) to about 100 Å/sec. However, the deposition conditions are not limited thereto.

When the hole injection layer is formed using spin coating, coating conditions may vary according to the material used to form the hole injection layer, and the structure and thermal properties of the hole injection layer. For example, a coating speed may be from about 2,000 revolutions per minute (rpm) to about 5,000 rpm, and a temperature at which a heat treatment is performed to remove a solvent after coating may be from about 80°C to about 200°C. However, the coating conditions are not limited thereto.

Conditions for forming a hole transport layer and an electron blocking layer may be understood by referring to conditions for forming the hole injection layer.

The hole transport region may include at least one selected from m-MTDATA, TDATA, 2-TNATA, NPB, β-NPB, TPD, Spiro-TPD, Spiro-NPB, methylated-NPB, TAPC, HMTPD, 4,4',4"-tris(N-carbazolyl)triphenylamine (TCTA), polyaniline/dodecylbenzene sulfonic acid (PANI/DBSA), poly(3,4-ethylenedioxythiophene)/poly(4-styrene sulfonate) (PEDOT/PSS), polyaniline/camphor sulfonic acid (PANI/CSA), polyaniline/poly(4-styrene sulfonate) (PANI/PSS), a compound represented by Formula 201 below, and a compound represented by Formula 202 below:

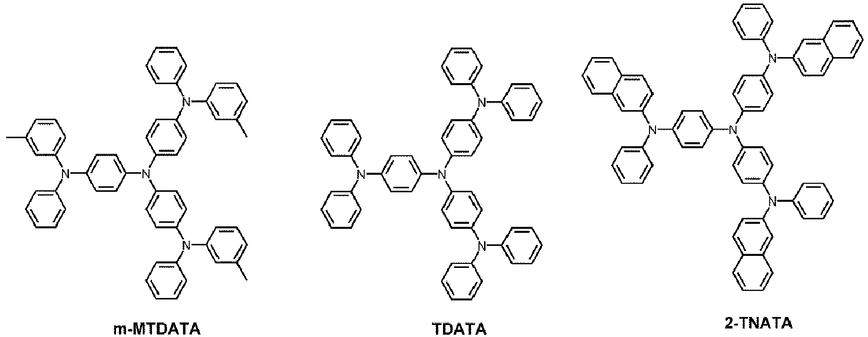

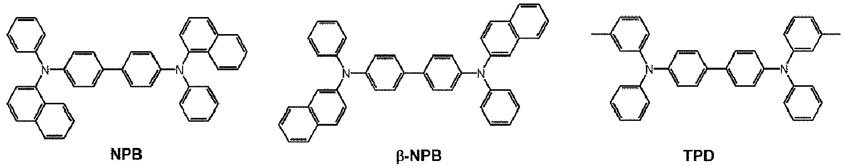

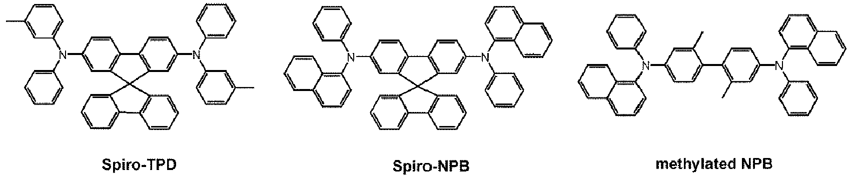

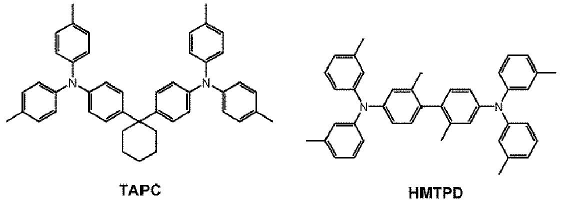

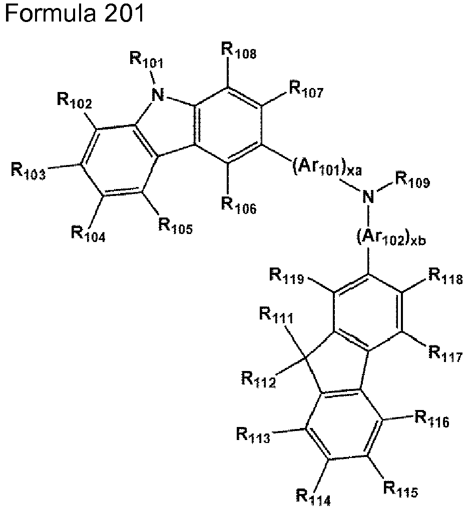

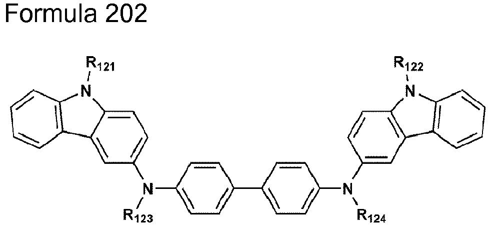

Ar₁₀₁ to Ar₁₀₂ in Formula 201 may each independently be selected from:
a phenylene group, a pentalenylene group, an indenylene group, a naphthylene group, an azulenylene group, a heptalenylene group, an acenaphthylene group, a fluorenylene group, a phenalenylene group, a phenanthrenylene group, an anthracenylene group, a fluoranthenylene group, a triphenylenylene group, a pyrenylene group, a chrysenylenylene group, a naphthacenylene group, a picenylene group, a perylenylene group, and a pentacenylene group; and
a phenylene group, a pentalenylene group, an indenylene group, a naphthylene group, an azulenylene group, a heptalenylene group, an acenaphthylene group, a fluorenylene group, a phenalenylene group, a phenanthrenylene group, an anthracenylene group, a fluoranthenylene group, a triphenylenylene group, a pyrenylene group, a chrysenylenylene group, a naphthacenylene group, a picenylene group, a perylenylene group, and a pentacenylene group, each substituted with at least one selected from deuterium, - F, -CI, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₃-C₁₀ cycloalkenyl group, a C₁-C₁₀ heterocycloalkyl group, a C₁-C₁₀ heterocycloalkenyl group, a C₆-C₆₀ aryl group, a C₆-C₆₀ aryloxy group, a C₆-C₆₀ arylthio group, a C₇-C₆₀ arylalkyl group, a C₁-C₆₀ heteroaryl group, a C₁-C₆₀ heteroaryloxy group, a C₁-C₆₀ heteroarylthio group, a C2-C₆₀ heteroarylalkyl group, a monovalent non-aromatic condensed polycyclic group, and a monovalent non-aromatic condensed heteropolycyclic group.

xa and xb in Formula 201 may each independently be an integer from 0 to 5, or 0, 1, or 2. For example, xa may be 1 and xb may be 0, but xa and xb are not limited thereto.

R₁₀₁ to R₁₀₈, R₁₁₁ to R₁₁₉ and R₁₂₁ to R₁₂₄ in Formulae 201 and 202 may each independently be selected from:
hydrogen, deuterium, -F, -CI, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₁₀ alkyl group (for example, a methyl group, an ethyl group, a propyl group, a butyl group, pentyl group, a hexyl group, etc.) and a C₁-C₁₀ alkoxy group (for example, a methoxy group, an ethoxy group, a propoxy group, a butoxy group, a pentoxy group, etc.);
a C₁-C₁₀ alkyl group and a C₁-C₁₀ alkoxy group, each substituted with at least one selected from deuterium, -F, -CI, -Br, -I, a hydroxyl group, a cyano group, a nitro group, an amino group, an amidino group, a hydrazine group, a hydrazone group, a carboxylic acid group or a salt thereof, a sulfonic acid group or a salt thereof and a phosphoric acid group or a salt thereof;
a phenyl group, a naphthyl group, an anthracenyl group, a fluorenyl group, and a pyrenyl group; and
a phenyl group, a naphthyl group, an anthracenyl group, a fluorenyl group, and a pyrenyl group, each substituted with at least one selected from deuterium, -F, -CI, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₁₀ alkyl group, and a C₁-C₁₀ alkoxy group,
but embodiments of the present disclosure are not limited thereto.

R₁₀₉ in Formula 201 may be selected from:
a phenyl group, a naphthyl group, an anthracenyl group, and a pyridinyl group; and
a phenyl group, a naphthyl group, an anthracenyl group, and a pyridinyl group, each substituted with at least one selected from deuterium, -F, -CI, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₂₀ alkyl group, a C₁-C₂₀ alkoxy group, a phenyl group, a naphthyl group, an anthracenyl group, and a pyridinyl group.

According to an embodiment, the compound represented by Formula 201 may be represented by Formula 201A below, but embodiments of the present disclosure are not limited thereto:

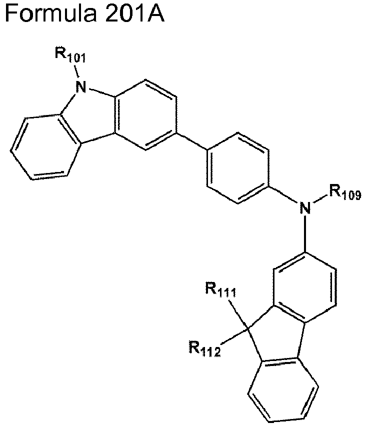

R₁₀₁, R₁₁₁, R₁₁₂, and R₁₀₉ in Formula 201A may be understood by referring to the description provided herein.

For example, the compound represented by Formula 201, and the compound represented by Formula 202 may include compounds HT1 to HT20 illustrated below, but are not limited thereto:

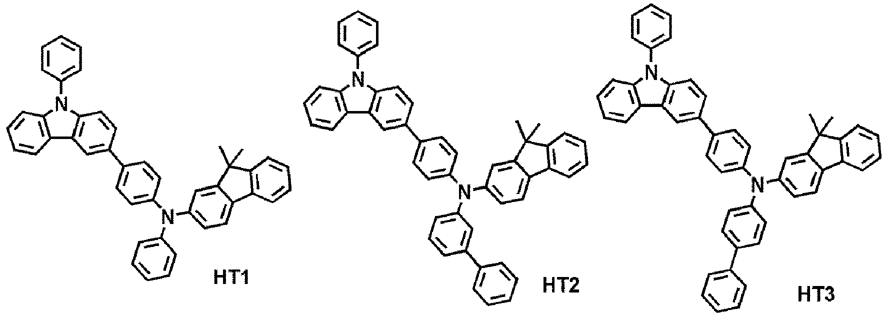

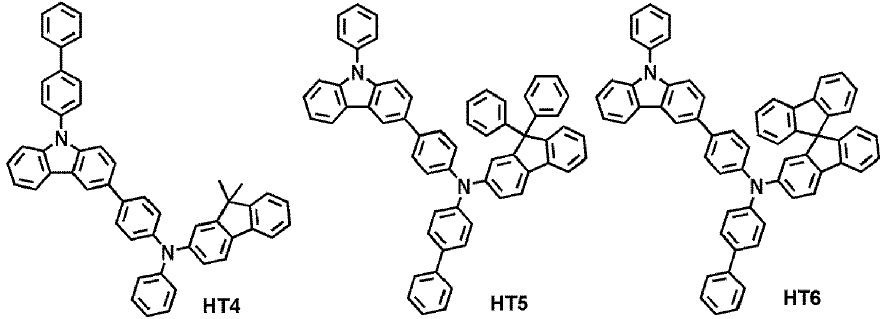

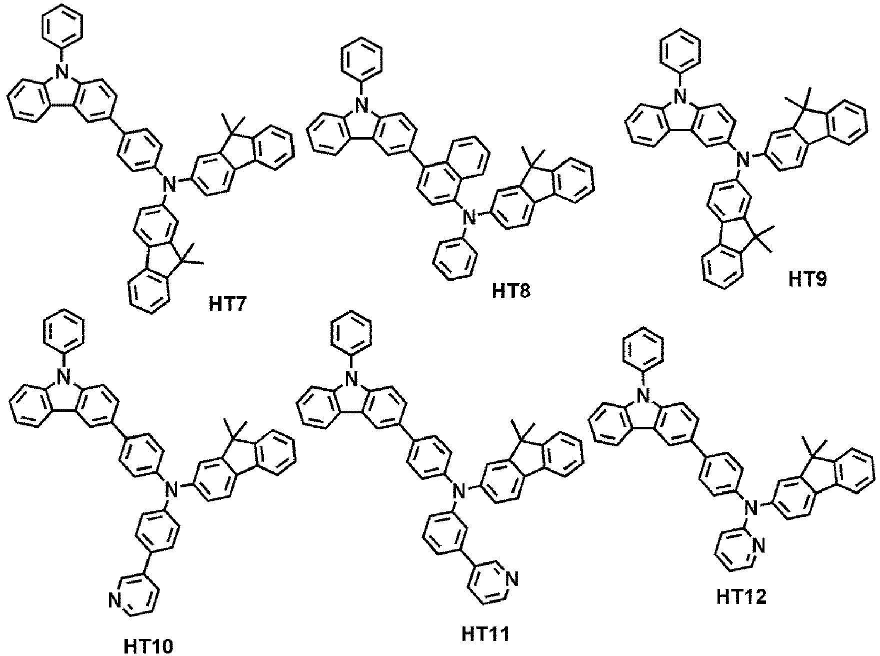

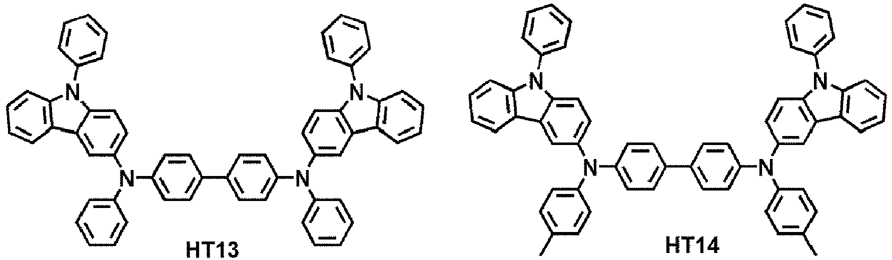

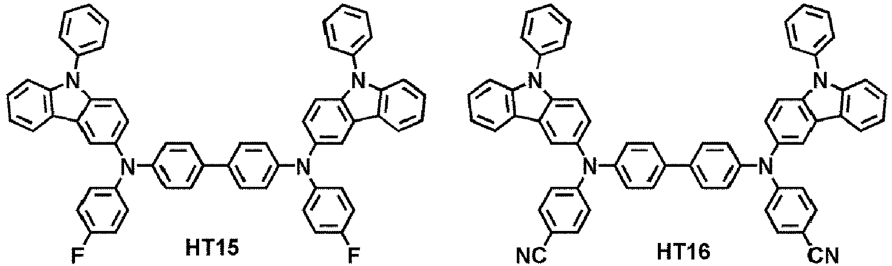

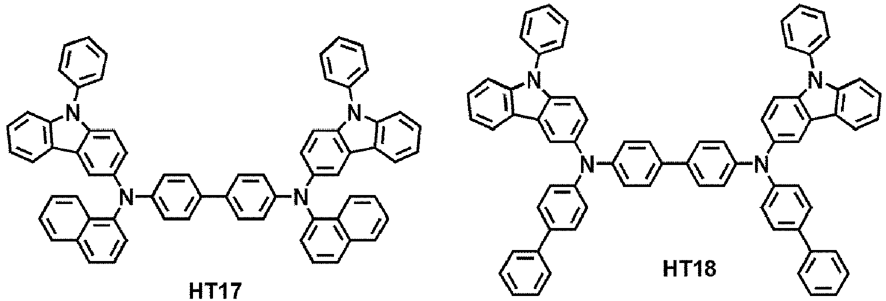

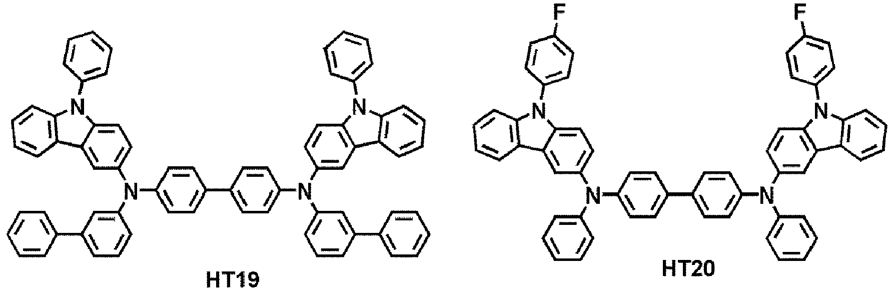

A thickness of the hole transport region may be in a range of about 100 Angstroms (Å) to about 10,000 Å, for example, about 100 Å to about 1,000 Å. When the hole transport region includes at least one of a hole injection layer and a hole transport layer, a thickness of the hole injection layer may be in a range of about 100 Å to about 10,000 Å, for example, about 100 Å to about 1,000 Å, and a thickness of the hole transport layer may be in a range of about 50 Å to about 2,000 Å, for example, about 100 Å to about 1,500 Å. While not wishing to be bound by theory, it is understood that when the thicknesses of the hole transport region, the hole injection layer and the hole transport layer are within these ranges, satisfactory hole transporting characteristics may be obtained without a substantial increase in driving voltage.

The hole transport region may further include, in addition to these materials, a charge-generation material for the improvement of conductive properties. The charge-generation material may be homogeneously or non-homogeneously dispersed in the hole transport region.

The charge-generation material may be, for example, a p-dopant. The p-dopant may be one selected from a quinone derivative, a metal oxide, and a cyano group-containing compound, but embodiments of the present disclosure are not limited thereto. Non-limiting examples of the p-dopant are a quinone derivative, such as tetracyanoquinonedimethane (TCNQ) or 2,3,5,6-tetrafluoro-tetracyano-1,4-benzoquinonedimethane (F4-TCNQ); a metal oxide, such as a tungsten oxide or a molybdenum oxide; and a cyano group-containing compound, such as Compound HT-D1 or Compound HT-D2 below, but are not limited thereto.

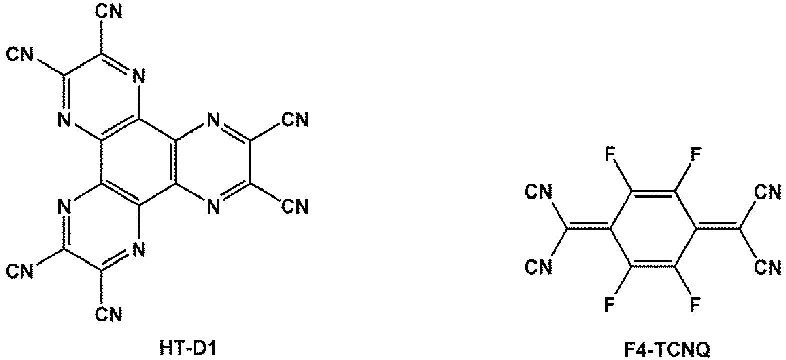

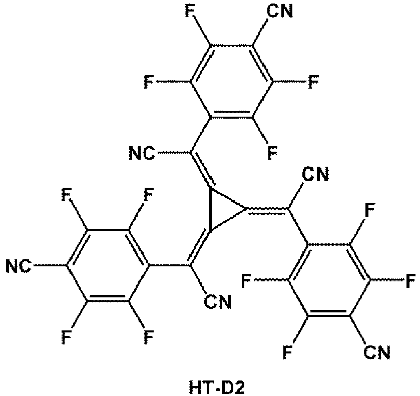

The hole transport region may include a buffer layer.

Also, the buffer layer may compensate for an optical resonance distance according to a wavelength of light emitted from the emission layer, and thus, efficiency of a formed organic light-emitting device may be improved.

Then, an emission layer (EML) may be formed on the hole transport region by vacuum deposition, spin coating, casting, LB deposition, or the like. When the emission layer is formed by vacuum deposition or spin coating, the deposition or coating conditions may be similar to those applied in forming the hole injection layer although the deposition or coating conditions may vary according to a material that is used to form the hole transport layer.

The hole transport region may further include an electron blocking layer. The electron blocking layer may include a material available in the art, for example, mCP, but embodiments of the present disclosure are not limited.

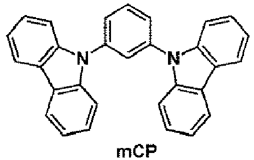

The thickness of the electron blocking layer may be about 50 Å to about 1,000 Å, for example about 70 Å to about 500 Å. While not wishing to be bound by theory, it is understood that when the thickness of the electron blocking layer is within the range described above, the electron blocking layer may have satisfactory electron blocking characteristics without a substantial increase in driving voltage.

When the organic light-emitting device is a full-color organic light-emitting device, the emission layer may be patterned into a red emission layer, a green emission layer, and a blue emission layer. In one or more embodiments, due to a stacked structure including a red emission layer, a green emission layer, and/or a blue emission layer, the emission layer may emit white light.

The emission layer may include the condensed cyclic compound represented by Formula 1.

The emission layer may include the condensed cyclic compound represented by Formula 1 alone.

In one or more embodiments, the emission layer may include:
the condensed cyclic compound represented by Formula 1,
i) the second compound (for example, a compound represented by Formula H-1);
ii) an organometallic compound represented by Formula 81; or
iii) any combination thereof.

The condensed cyclic compound represented by Formula 1, the second compound, and the organometallic compound represented by Formula 81 may each be the same as described above.

When the emission layer includes a host and a dopant, the amount of the dopant may be in the range of about 0.01 to about 20 parts by weight based on 100 parts by weight of the emission layer. However, the amount of the dopant included in the emission layer is not limited thereto. While not wishing to be bound by theory, it is understood that when the amount of the dopant satisfies the range, it may be possible to realize emission without extinction phenomenon.

When the emission layer includes the condensed cyclic compound represented by Formula 1 and the second compound, the weight ratio of the condensed cyclic compound represented by formula 1 to the second compound may be in the range of about 1: 99 to about 99: 1, for example, about 70: 30 to about 30: 70. In one or more embodiments, the weight ratio of the condensed cyclic compound represented by Formula 1 and the second compound may be in the range of about 60:40 to about 40:60. While not wishing to be bound by theory, it is understood that when the weight ratio of the condensed cyclic compound represented by Formula 1 to the second compound in the emission layer is within this range, the charge transport balance in the emission layer may be effectively performed.

A thickness of the emission layer may be in a range of about 100 Å to about 1,000 Å, for example, about 200 A to about 600 Å. While not wishing to be bound by theory, it is understood that when the thickness of the emission layer is within this range, excellent light-emission characteristics may be obtained without a substantial increase in driving voltage.

Then, an electron transport region may be located on the emission layer.

The electron transport region may include at least one selected from a hole blocking layer, an electron transport layer and an electron injection layer.

For example, the electron transport region may have a hole blocking layer/electron transport layer/electron injection layer structure or an electron transport layer/electron injection layer structure, and the structure of the electron transport region is not limited thereto. The electron transport layer may have a single-layered structure or a multi-layered structure including two or more different materials.

Conditions for forming the hole blocking layer, the electron transport layer, and the electron injection layer which constitute the electron transport region may be understood by referring to the conditions for forming the hole injection layer.

When the electron transport region includes a hole blocking layer, the hole blocking layer may include, for example, at least one of BCP and BPhen, but embodiments of the present disclosure are not limited thereto.

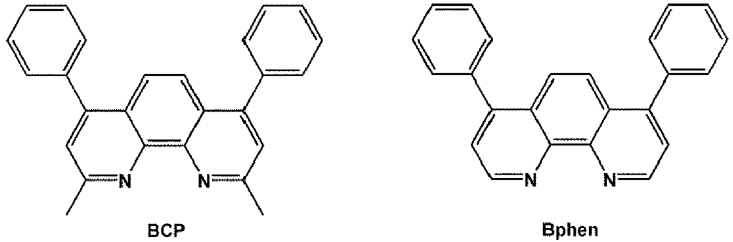

In one or more embodiments, the hole blocking layer may include the condensed cyclic compound represented by Formula 1.

A thickness of the hole blocking layer may be in a range of about 20 Å to about 1,000 Å, for example, about 30 Å to about 300 Å. While not wishing to be bound by theory, it is understood that when the thickness of the hole blocking layer is within these ranges, the hole blocking layer may have excellent hole blocking characteristics without a substantial increase in driving voltage.

The electron transport layer may further include at least one selected from BCP, BPhen, Alq₃, BAlq, TAZ, and NTAZ.

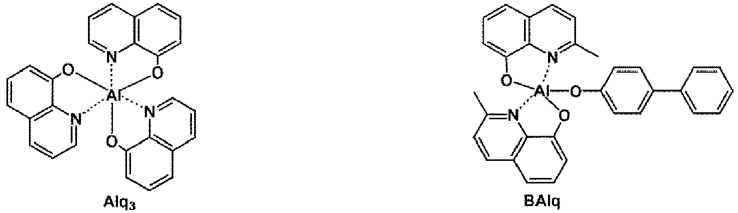

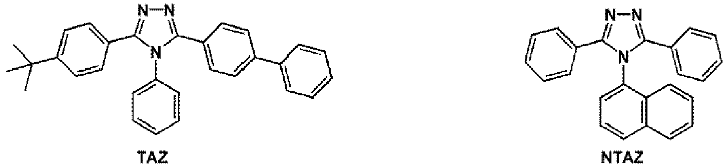

In one or more embodiments, the electron transport layer may include at least one of ET1, ET2, and ET3, but are not limited thereto:

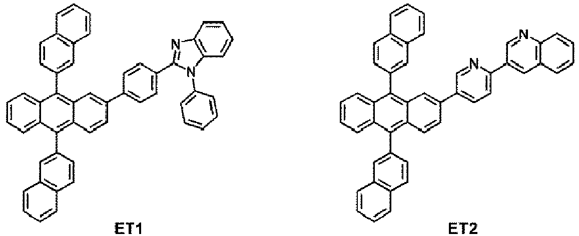

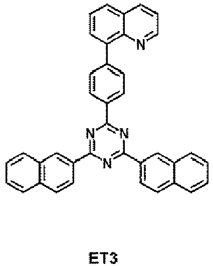

A thickness of the electron transport layer may be in a range of about 100 Å to about 1,000 Å, for example, about 150 A to about 500 Å. While not wishing to be bound by theory, it is understood that when the thickness of the electron transport layer is within the range described above, the electron transport layer may have satisfactory electron transport characteristics without a substantial increase in driving voltage.

Also, the electron transport layer may further include, in addition to the materials described above, a metal-containing material.

The metal-containing material may include a Li complex. The Li complex may include, for example, Compound ET-D1 (lithium 8-hydroxyquinolate, LiQ) or ET-D2.

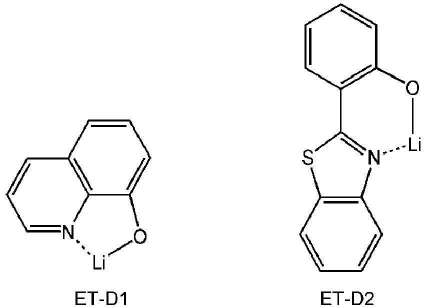

The electron transport region may include an electron injection layer (EIL) that promotes flow of electrons from the second electrode 19 thereinto.

The electron injection layer may include at least one selected from LiQ, LiF, NaCl, CsF, Li₂O, and BaO.

A thickness of the electron injection layer may be in a range of about 1 Å to about 100 Å, and, for example, about 3 Å to about 90 Å. While not wishing to be bound by theory, it is understood that when the thickness of the electron injection layer is within the range described above, the electron injection layer may have satisfactory electron injection characteristics without a substantial increase in driving voltage.

The second electrode 19 is located on the organic layer 15. The second electrode 19 may be a cathode. A material for forming the second electrode 19 may be metal, an alloy, an electrically conductive compound, or a combination thereof, which have a relatively low work function. For example, lithium (Li), magnesium (Mg), aluminum (Al), aluminum-lithium (Al-Li), calcium (Ca), magnesium-indium (Mg-In), or magnesium-silver (Mg-Ag) may be formed as the material for forming the second electrode 19. To manufacture a top-emission type light-emitting device, a transmissive electrode formed using ITO or IZO may be used as the second electrode 19.

Hereinbefore, the organic light-emitting device has been described with reference to the FIGURE, but embodiments of the present disclosure are not limited thereto.

The term "C₁-C₆₀ alkyl group" as used herein refers to a linear or branched saturated aliphatic hydrocarbon monovalent group having 1 to 60 carbon atoms, and non-limiting examples thereof include a methyl group, an ethyl group, a propyl group, an iso-butyl group, a sec-butyl group, a tert-butyl group, a pentyl group, an iso-amyl group, and a hexyl group. The term "C₁-C₆₀ alkylene group" as used herein refers to a divalent group having the same structure as that of the C₁-C₆₀ alkyl group.

The term "C₁-C₆₀ alkoxy group" as used herein refers to a monovalent group represented by -OA₁₀₁ (wherein A₁₀₁ is the C₁-C₆₀ alkyl group), and examples thereof include a methoxy group, an ethoxy group, and an iso-propyloxy group.

The term "C₂-C₆₀ alkenyl group" as used herein refers to a hydrocarbon group formed by including at least one carbon-carbon double bond in the middle or at the terminus of the C₂-C₆₀ alkyl group, and examples thereof include an ethenyl group, a propenyl group, and a butenyl group. The term "C₂-C₆₀ alkenylene group" as used herein refers to a divalent group having the same structure as that of the C₂-C₆₀ alkenyl group.

The term "C₂-C₆₀ alkynyl group" as used herein refers to a hydrocarbon group formed by including at least one carbon-carbon triple bond in the middle or at the terminus of the C₂-C₆₀ alkyl group, and examples thereof include an ethynyl group, and a propynyl group. The term "C₂-C₆₀ alkynylene group" as used herein refers to a divalent group having the same structure as that of the C₂-C₆₀ alkynyl group.

The term "C₃-C₁₀ cycloalkyl group" as used herein refers to a monovalent saturated hydrocarbon monocyclic group having 3 to 10 carbon atoms, and examples thereof include a cyclopropyl group, a cyclobutyl group, a cyclopentyl group, a cyclohexyl group, and a cycloheptyl group. The term "C₃-C₁₀ cycloalkylene group" as used herein refers to a divalent group having the same structure as that of the C₃-C₁₀ cycloalkyl group.

The term "C₁-C₁₀ heterocycloalkyl group" as used herein refers to a monovalent saturated monocyclic group having at least one heteroatom selected from N, O, P, Si, and S as a ring-forming atom and 1 to 10 carbon atoms, and non-limiting examples thereof include a tetrahydrofuranyl group, and a tetrahydrothiophenyl group. The term "C₁-C₁₀ heterocycloalkylene group" as used herein refers to a divalent group having the same structure as the C₁-C₁₀ heterocycloalkyl group.

The term "C₃-C₁₀ cycloalkenyl group" as used herein refers to a monovalent monocyclic group that has 3 to 10 carbon atoms and at least one carbon-carbon double bond in the ring thereof and no aromaticity, and non-limiting examples thereof include a cyclopentenyl group, a cyclohexenyl group, and a cycloheptenyl group. The term "C₃-C₁₀ cycloalkenylene group" as used herein refers to a divalent group having the same structure as the C₃-C₁₀ cycloalkenyl group.

The term "C₁-C₁₀ heterocycloalkenyl group" as used herein refers to a monovalent monocyclic group that has at least one heteroatom selected from N, O, P, Si, and S as a ring-forming atom, 1 to 10 carbon atoms, and at least one carbon-carbon double bond in its ring. Examples of the C₂-C₁₀ heterocycloalkenyl group are a 2,3-dihydrofuranyl group, and a 2,3-dihydrothiophenyl group. The term "C₁-C₁₀ heterocycloalkenylene group" as used herein refers to a divalent group having the same structure as the C₁-C₁₀ heterocycloalkenyl group.

The term "C₆-C₆₀ aryl group" as used herein refers to a monovalent group having a carbocyclic aromatic system having 6 to 60 carbon atoms, and the term "C₆-C₆₀ arylene group" as used herein refers to a divalent group having a carbocyclic aromatic system having 6 to 60 carbon atoms. Non-limiting examples of the C₆-C₆₀ aryl group include a phenyl group, a naphthyl group, an anthracenyl group, a phenanthrenyl group, a pyrenyl group, and a chrysenyl group. When the C₆-C₆₀ aryl group and the C₆-C₆₀ arylene group each include two or more rings, the rings may be fused to each other.

The term "C₁-C₆₀ heteroaryl group" as used herein refers to a monovalent group having a heteroaromatic system that has at least one heteroatom selected from N, O, P, Si, and S as a ring-forming atom, and 1 to 60 carbon atoms. The term "C₁-C₆₀ heteroarylene group" as used herein refers to a divalent group having a heteroaromatic system that has at least one heteroatom selected from N, O, P, Si and S as a ring-forming atom, and 1 to 60 carbon atoms. Examples of the C₁-C₆₀ heteroaryl group include a pyridinyl group, a pyrimidinyl group, a pyrazinyl group, a pyridazinyl group, a triazinyl group, a quinolinyl group, and an isoquinolinyl group. When the C₆-C₆₀ heteroaryl group and the C₆-C₆₀ heteroarylene group each include two or more rings, the rings may be fused to each other.

The term "C₆-C₆₀ aryloxy group" as used herein indicates -OA₁₀₂ (wherein A₁₀₂ is the C₆-C₆₀ aryl group), and the term "C₆-C₆₀ arylthio group" as used herein indicates -SA₁₀₃ (wherein A₁₀₃ is the C6-C60 aryl group).

The term "monovalent non-aromatic condensed polycyclic group" as used herein refers to a monovalent group in which two or more rings are condensed with each other, only carbon is used as a ring-forming atom (for example, the number of carbon atoms may be 8 to 60) and the whole molecule is a non-aromaticity group. Examples of the monovalent non-aromatic condensed polycyclic group include a fluorenyl group. The term "divalent non-aromatic condensed polycyclic group" as used herein refers to a divalent group having the same structure as the monovalent non-aromatic condensed polycyclic group.

The term "monovalent non-aromatic condensed heteropolycyclic group" as used herein refers to a monovalent group having two or more rings condensed to each other, a heteroatom selected from N, O, P, Si, and S, other than carbon atoms(for example, having 1 to 60 carbon atoms), as a ring-forming atom, and no aromaticity in its entire molecular structure. Non-limiting examples of the monovalent non-aromatic condensed heteropolycyclic group include a carbazolyl group. The term "divalent non-aromatic condensed heteropolycyclic group" as used herein refers to a divalent group having the same structure as the monovalent non-aromatic condensed heteropolycyclic group.

Regarding Formula 1, at least one selected from the substituted C₁-C₆₀ alkyl group, the substituted C₂-C₆₀ alkenyl group, the substituted C₂-C₆₀ alkynyl group, the substituted C₁-C₆₀ alkoxy group, the substituted C₃-C₁₀ cycloalkyl group, the substituted C₁-C₁₀ heterocycloalkyl group, the substituted C₃-C₁₀ cycloalkenyl group, the substituted C₁-C₁₀ heterocycloalkenyl group, the substituted C₆-C₆₀ aryl group, the substituted C₆-C₆₀ aryloxy group, the substituted C₆-C₆₀ arylthio group, the substituted C₇-C₆₀ arylalkyl group, the substituted C₁-C₆₀ heteroaryl group, the substituted C₁-C₆₀ heteroaryloxy group, the substituted C₁-C₆₀ heteroarylthio group, the substituted C₂-C₆₀ heteroarylalkyl group, the substituted monovalent non-aromatic condensed polycyclic group, and the substituted monovalent non-aromatic condensed heteropolycyclic group may be selected from:
deuterium, -CD₃, -CD₂H, -CDH₂, -F, -CI, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, and a C₁-C₆₀ alkoxy group;
a C₁-C₆₀ alkyl group, a C₂-C₆₀ alkenyl group, a C₂-C₆₀ alkynyl group, and a C₁-C₆₀ alkoxy group, each substituted with at least one selected from deuterium, - CD₃, -CD₂H, -CDH₂, -F, -CI, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₇-C₆₀ arylalkyl group, a C₁-C₆₀ heteroaryl group, a C₁-C₆₀ heteroaryloxy group, a C₁-C₆₀ heteroarylthio group, a C₂-C₆₀ heteroarylalkyl group, a monovalent non-aromatic condensed polycyclic group, a monovalent non-aromatic condensed heteropolycyclic group, -Si(Q₁₁)(Q₁₂)(Q₁₃), -N(Q₁₄)(Q₁₅), and -B(Q₁₆)(Q₁₇);
a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₇-C₆₀ arylalkyl group, a C₁-C₆₀ heteroaryl group, a C₁-C₆₀ heteroaryloxy group, a C₁-C₆₀ heteroarylthio group, a C₂-C₆₀ heteroarylalkyl group, a monovalent non-aromatic condensed polycyclic group, and a monovalent non-aromatic condensed heteropolycyclic group;
a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₇-C₆₀ arylalkyl group, a C₁-C₆₀ heteroaryl group, a C₁-C₆₀ heteroaryloxy group, a C₁-C₆₀ heteroarylthio group, a C₂-C₆₀ heteroarylalkyl group, a monovalent non-aromatic condensed polycyclic group, and a monovalent non-aromatic condensed heteropolycyclic group, each substituted with at least one selected from deuterium, -CD₃, -CD₂H, -CDH₂, -F, -CI, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₇-C₆₀ arylalkyl group, a C₁-C₆₀ heteroaryl group, a C₁-C₆₀ heteroaryloxy group, a C₁-C₆₀ heteroarylthio group, a C₂-C₆₀ heteroarylalkyl group, a monovalent non-aromatic condensed polycyclic group, a monovalent non-aromatic condensed heteropolycyclic group, -Si(Q₂₁)(Q₂₂)(Q₂₃), -N(Q24)(Q25), and - B(Q26)(Q27); and
-Si(Q₃₁)(Q₃₂)(Q₃₃), -N(Q34)(Q35), and -B(Q36)(Q37).

Q1 to Q7, Q11 to Q17, Q21 to Q27 and Q31 to Q37, which are used herein, may each independently be selected from hydrogen, deuterium, -F, -CI, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a substituted or unsubstituted C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₁-C₆₀ heteroaryl group, a monovalent non-aromatic condensed polycyclic group, and a monovalent non-aromatic condensed heteropolycyclic group.

The expressions * and *' as used herein each indicate a binding site to a neighboring atom, unless otherwise stated.

Hereinafter, a compound and an organic light-emitting device according to embodiments are described in detail with reference to Synthesis Example and Examples. However, the organic light-emitting device is not limited thereto. The wording "B was used instead of A" used in describing Synthesis Examples means that an amount of A used was identical to an amount of B used, in terms of a molar equivalent.

### EXAMPLES

### Synthesis Example 1 (Compound 1)

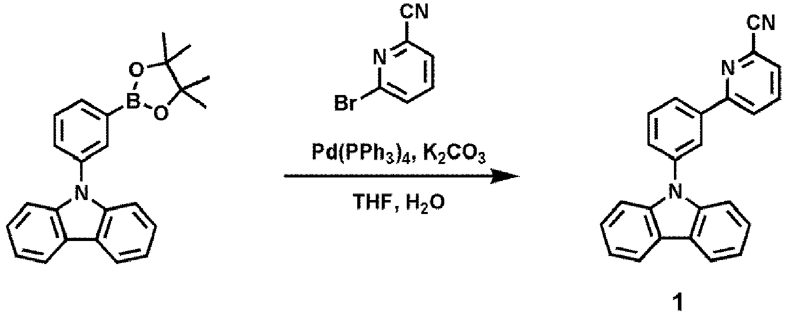

5.00 grams (g) (13.5 millimoles, mmol) of 9-(3-(4,4,5,5-tetramethyl-1,3,2-dioxaborolan-2-yl)phenyl)-9H-carbazole, 2.73g (14.9 mmol) of 6-bromopicolinonitrile, 0.782 g (0.680 mmol) of tetrakistriphenylphosphine palladium (0) (Pd(PPh₃)₄, and 4.68 g (33.9 mmol) of potassium carbonate were added to a mixture including 30 mL of THF and 15 mL of water, and the resulting mixture was stirred under reflux for 18 hours. After completion of the reaction, the reaction mixture was cooled to room temperature and the aqueous layer was removed therefrom through extraction. The organic layer was filtered under reduced pressure through a silica gel, and the filtrate was concentrated under reduced pressure. The product was filtered to obtain Compound 1 (3.37 g, the yield of 72%) by silica gel column chromatography.
LC-Mass (calculated: 345.13 g/mol, found: M⁺¹ = 346 g/mol)

### Synthesis Example 2 (Compound 5)

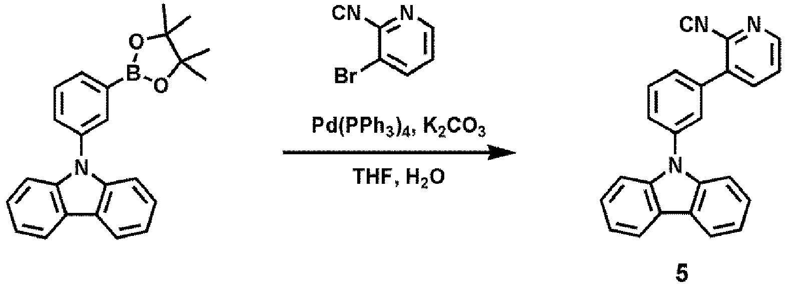

Compound 5 (3.23 g, the yield of 69%) was obtained in the same manner as in Synthesis Example 1, except that 2.73 g (14.9 mmol) of 3-bromopicolinonitrile was used instead of 6-bromopicolinonitrile.
LC-Mass (calculated : 345.13 g/mol, found : M⁺¹ = 346 g/mol)

### Synthesis Example 3 (Compound 6)

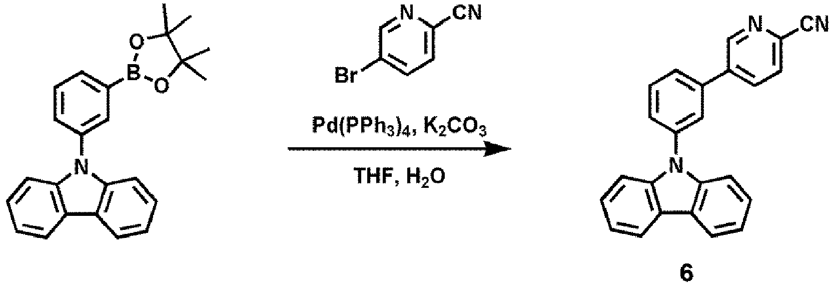

Compound 6 (3.60 g, the yield of 77%) was obtained in the same manner as in Synthesis Example 1, except that 2.73 g (14.9 mmol) of 5-bromopicolinonitrile was used instead of 6-bromopicolinonitrile.
LC-Mass (calculated : 345.13 g/mol, found : M⁺¹ = 346 g/mol)

### Synthesis Example 4 (Compound 7)

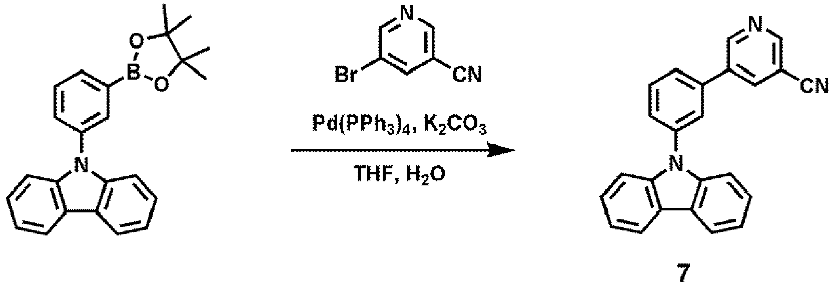

Compound 7 (3.46 g, the yield of 74%) was obtained in the same manner as in Synthesis Example 1, except that 2.73 g (14.9 mmol) of 5-bromonicotinonitrile was used instead of 6-bromopicolinonitrile.
LC-Mass (calculated : 345.13 g/mol, found : M⁺¹ = 346 g/mol)

### Synthesis Example 5 (Compound 9)

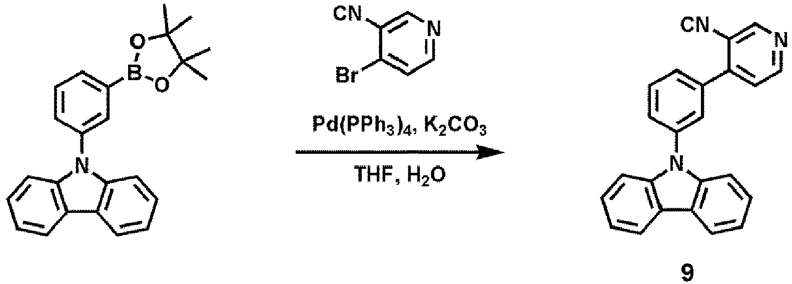

Compound 9 (2.24 g, the yield of 48%) was obtained in the same manner as in Synthesis Example 1, except that 2.73 g (14.9 mmol) of 4-bromonicotinonitrile was used instead of 6-bromopicolinonitrile.
LC-Mass (calculated : 345.13 g/mol, found : M⁺¹ = 346 g/mol)

### Synthesis Example 6 (Compound 10)

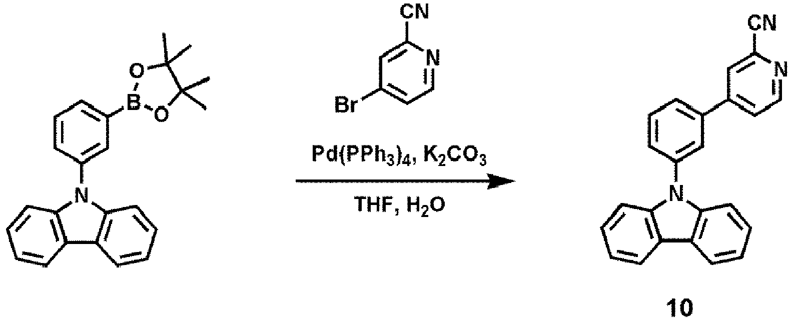

Compound 10 (2.57 g, the yield of 55%) was obtained in the same manner as in Synthesis Example 1, except that 2.73 g (14.9 mmol) of 4-bromopicolinonitrile was used instead of 6-bromopicolinonitrile.
LC-Mass (calculated: 345.13 g/mol, found: M⁺¹ = 346 g/mol)

### Synthesis Example 7 (Compound 15)

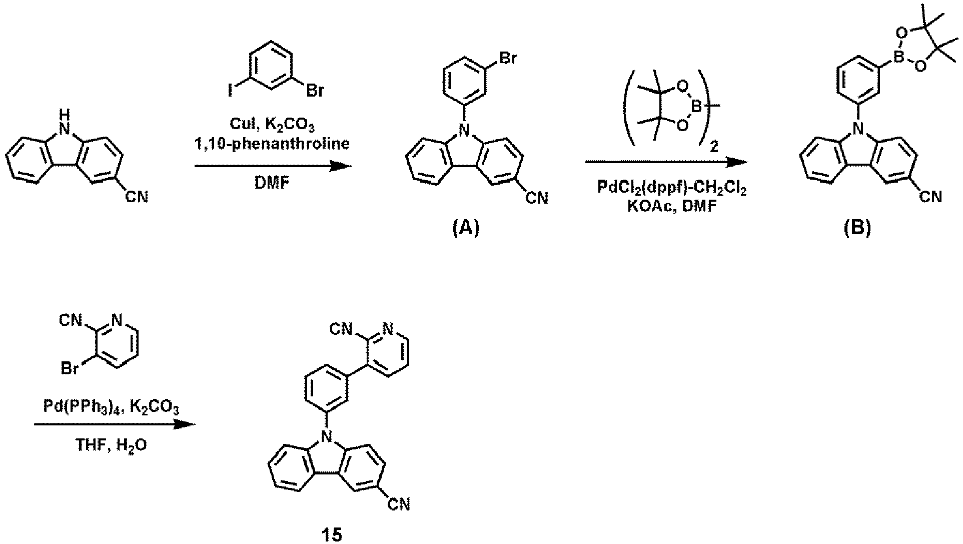

### Synthesis of Intermediate (A)

20.0 g (104 mmol) of 9H-carbazole-3-carbonitrile, 44.2 g (156 mmol) of 1-bromo-3-iodobenzene, 3.96 g (20.8 mmol) of copper iodide (Cul), 57.5 g (416 mmol) of potassium carbonate (K₂CO₃), and 7.50 g (41.6 mmol) of 1,10-phenanthroline were mixed with 350 mL of DMF, and the resulting mixture was stirred under reflux for 24 hours. After completion of the reaction, the reaction mixture was cooled to room temperature, filtered through a silica gel filter, and the filtrate was concentrated under reduced pressure. The product was separated by silica gel column chromatography to obtain Intermediate (A) (25.3 g, the yield of 70%).
LC-Mass (calculated : 346.01 g/mol, found : M⁺¹ = 347 g/mol)

### Synthesis of Intermediate (B)

25.0 g (72.0 mmol) of Intermediate (A), 21. 9 g (86.4 mmol) of bis(pinacolato)diboron, 2.94 g (3.60 mmol) of PdCl₂(dppf)·CH₂Cl₂, and 21. 2 g (216 mmol) of potassium acetate were mixed with 240 mL of DMF, and the mixture was stirred for 24 hours at a temperature of 100°C. After the completion of the reaction, the reaction mixture was cooled to room temperature, passed through silica gel under reduced pressure, and the filtrate was concentrated under reduced pressure. The product was filtered by silica gel column chromatography. The resulting product was recrystallized under the condition of dichloromethane (DCM)/n-hexane to yield Intermediate (B) (18.7 g, the yield of 66%).
LC-Mass (calculated : 394.19 g/mol, found : M⁺¹ = 395 g/mol)

### Synthesis of Compound 15

5.00 g (12.7 mmol) of Intermediate (B), 2.55 g (14.0 mmol) of 3-bromopicolinonitrile, 0.733 g (0.634 mmol) of tetrakistriphenylphosphine palladium (0) (Pd(PPh₃)₄), and 4.38 g (31.7 mmol) of potassium carbonate were added to a mixture including 30 mL of THF and 15 mL of water, and the mixture was stirred for 18 hours under reflux. After completion of the reaction, the reaction mixture was cooled to room temperature and the aqueous layer was removed therefrom through extraction. The organic layer was filtered under reduced pressure through a silica gel, and the filtrate was concentrated under reduced pressure. The product was filtered by silica gel column chromatography, thereby obtaining Compound 15 (2.72 g, the yield of 58%).
LC-Mass (calculated : 370.12 g/mol, found : M+1 = 371 g/mol)

### Synthesis Example 8 (Compound 41)

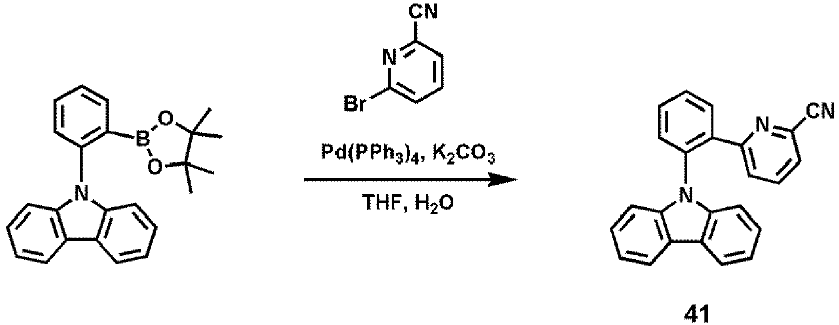

Compound 41 (2.01 g, the yield of 43%) was obtained in the same manner as in Synthesis Example 1, except that 5.00 g (13.5 mmol) of 9-(2-(4,4,5,5-tetramethyl-1,3,2-dioxaborolan-2-yl)phenyl)-9H-carbazole was used instead of 9-(3-(4,4,5,5-tetramethyl-1,3,2-dioxaborolan-2-yl)phenyl)-9H-carbazole).
LC-Mass (calculated : 345.13 g/mol, found : M⁺¹ = 346 g/mol)

### Synthesis Example 9 (Compound 45)

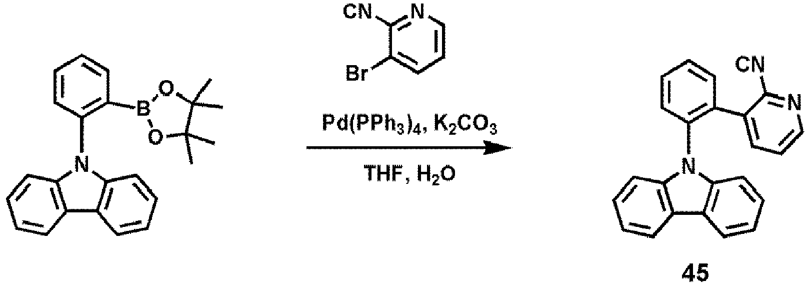

Compound 45 (1.50 g, the yield of 32%) was obtained in the same manner as in Synthesis Example 8, except that 2.73 g (14.9 mmol) of 3-bromopicolinonitrile was used instead of 6-bromopicolinonitrile.
LC-Mass (calculated : 345.13 g/mol, found : M⁺¹ = 346 g/mol)

### Synthesis Example 10 (Compound 55)

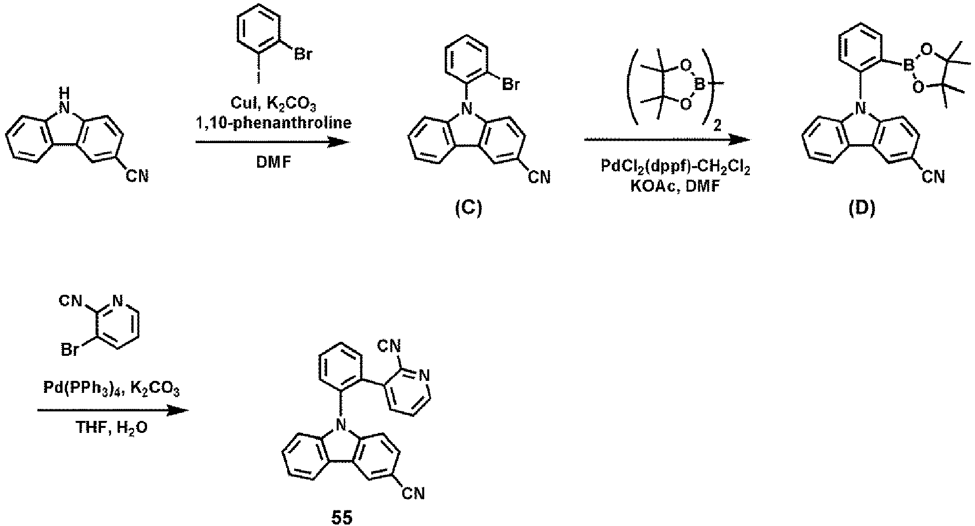

### Synthesis of Intermediate (C)

Intermediate (C) (23.5 g, the yield of 65%) was obtained in the same manner as used to prepare Intermediate (A) in Synthesis Example 7, except that 44.2 g (156 mmol) of 1-bromo-2-iodobenzene was used instead of 1-bromo-3-iodobenzene.
LC-Mass (calculated : 346.01 g/mol, found : M+1 = 347 g/mol)

### Synthesis of Intermediate (D)

Intermediate (D) (13.3 g, the yield of 51%) was obtained in the same manner as used to prepare Intermediate (B) in Synthesis Example 7, except that 23.0 g (66.2 mmol) of Intermediate (C) was used instead of Intermediate (A)
LC-Mass (calculated : 394.19 g/mol, found : M⁺¹ = 395 g/mol)

### Synthesis of Compound 55

Compound 55 (1.74 g, the yield of 37%) was obtained in the same manner as used to prepare Compound 19 in Synthesis Example 7, except that 5.00 g (12.7 mmol) of Intermediate (D) was used instead of Intermediate (B) .
LC-Mass (calculated : 370.12 g/mol, found : M⁺¹ = 371 g/mol)

### Synthesis Example 11 (Compound 105)

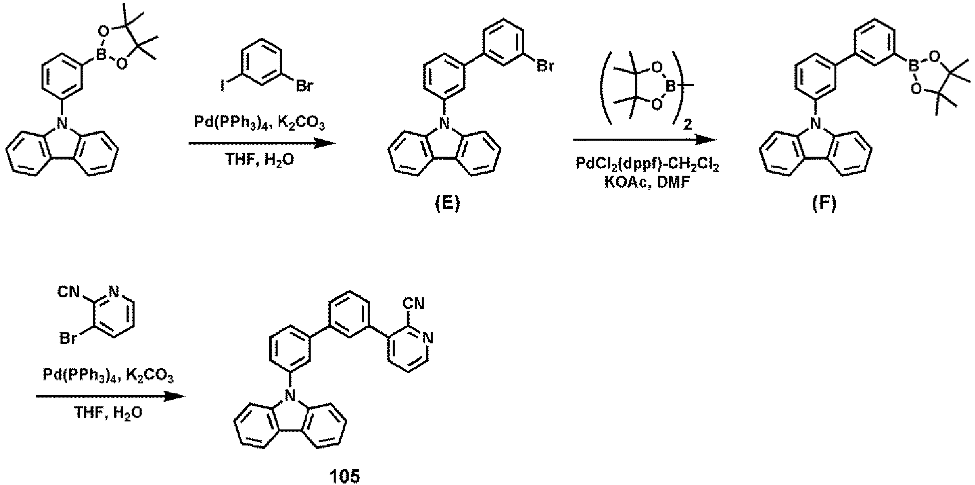

### Synthesis of Intermediate (E)

20.0 g (54.2 mmol) of 9-(3-(4,4,5,5-tetramethyl-1,3,2-dioxaborolan-2-yl)phenyl)-9H-carbazole, 23.0 g (81.2 mmol) of 1-bromo-3-iodobenzene, 3.13 g (2.71 mmol) of tetrakistriphenylphosphine palladium (0) (Pd(PPh₃)₄), and 18.7 g (135 mmol) of potassium carbonate were added to a mixture including 120 mL of THF and 60 mL of water, and the mixture was stirred for 18 hours under reflux. After completion of the reaction, the reaction mixture was cooled to room temperature and the aqueous layer was removed therefrom through extraction. The organic layer was filtered under reduced pressure through a silica gel, and the filtrate was concentrated under reduced pressure. The product was filtered by silica gel column chromatography, thereby obtaining Intermediate (E) (18.3 g, the yield of 85%)
LC-Mass (calculated : 397.05 g/mol, found : M+1 = 398 g/mol)

### Synthesis of Intermediate (F)

18.0 g (45.2 mmol) of Intermediate (E), 13.8 g (54.2 mmol) of bis(pinacolato)diboron, 1.85 g (2.26 mmol) of PdCl₂(dppf)·CH₂Cl₂, and 13.3 g (136 mmol) of potassium acetate were mixed in 150 mL of DMF, and the mixture was stirred for 24 hours at the temperature of 100°C. After the completion of the reaction, the reaction mixture was cooled to room temperature, passed through silica gel under reduced pressure, and the filtrate was concentrated under reduced pressure. The resulting product was separated by silica gel column chromatography and recrystallized under the condition of dichloromethane (DCM)/n-hexane to obtain Intermediate (F) (15.3 g, the yield of 76%).
LC-Mass (calculated : 445.22 g/mol, found : M⁺¹ = 446 g/mol)

### Synthesis of Compound 105

5.00 g (11.2 mmol) of Intermediate (F), 2.26 g (12.4 mmol) of 3-bromopicolinonitrile, 0.649 g (0.561 mmol) of tetrakistriphenylphosphine palladium(0) (Pd(PPh₃)₄), and 3.88 g (28.1 mmol) of potassium carbonate were added to a mixture including 24 mL of THF and 12 mL of water, and the resulting mixture was stirred for 18 hours during refluxing. After completion of the reaction, the reaction mixture was cooled to room temperature and the aqueous layer was removed therefrom through extraction. The organic layer was filtered under reduced pressure through a silica gel, and the filtrate was concentrated under reduced pressure. The product was filtered by silica gel column chromatography, thereby obtaining Compound 105 (3.36 g, the yield of 71%).
LC-Mass (calculated : 421.16 g/mol, found : M⁺¹ = 422 g/mol)

### Synthesis Example 12 (Compound 115)

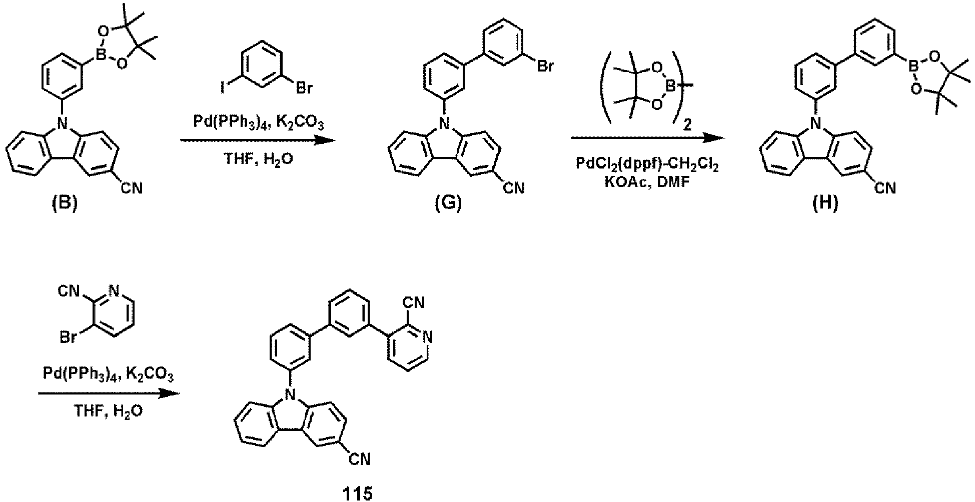

### Synthesis of Intermediate (G)

Intermediate (G) (16.5 g, the yield of 67%) was obtained in the same manner as used to prepare intermediate (E) in Synthesis Example 11, except that 20.0 g (50.7 mmol) of Intermediate (B) was used instead of 9-(3-(4,4,5,5-tetramethyl-1,3,2-dioxaborolan-2-yl)phenyl)-9H-carbazole.
LC-Mass (calculated : 422.04 g/mol, found : M⁺¹ = 423 g/mol)

### Synthesis of Intermediate (H)

Intermediate (H) (11.7 g, the yield of 66%) was obtained in the same manner as used to prepare Intermediate (F) in Synthesis Example 11, except that 16.0 g (37.8 mmol) of Intermediate (G) was used instead of Intermediate (E).
LC-Mass (calculated : 470.22 g/mol, found : M⁺¹ = 471 g/mol)

### Synthesis of Compound 115

Compound 115 (2.80 g, the yield of 59%) was obtained in the same manner as used to prepare Compound 105 in Synthesis Example 11, except that 5.00 g (10.6 mmol) of Intermediate (H) was used instead of Intermediate (F).
LC-Mass (calculated : 446.15 g/mol, found : M⁺¹ = 447 g/mol)

### Synthesis Example 13 (Compound 165)

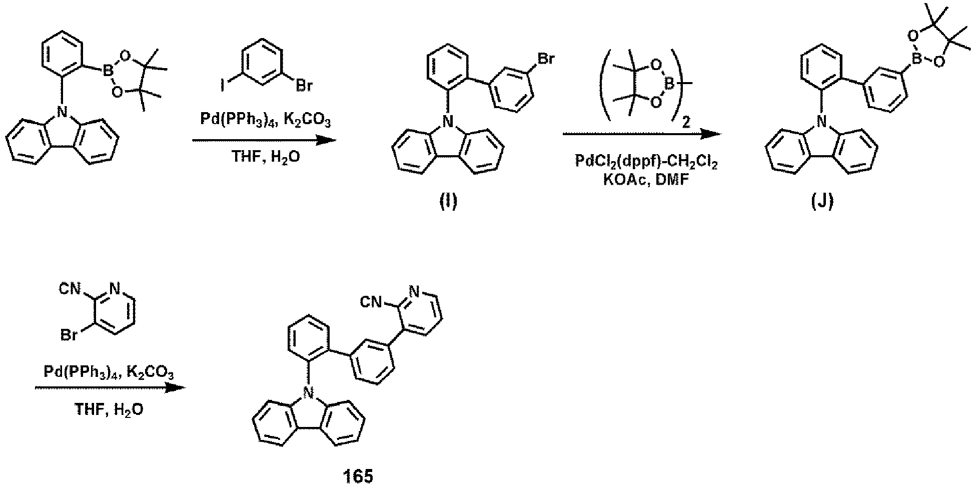

### Synthesis of Intermediate (I)

Intermediate (I) (14.2 g, the yield of 65%) was obtained in the same manner as used to prepare Intermediate (E) in Synthesis Example 11, except that 20.0 g (54.2 mmol) of 9-(2-(4,4,5,5-tetramethyl-1,3,2-dioxaborolan-2-yl)phenyl)-9H-carbazole was used instead of 9-(3-(4,4,5,5-tetramethyl-1,3,2-dioxaborolan-2-yl)phenyl)-9H-carbazole.
LC-Mass (calculated : 397.05 g/mol, found : M⁺¹ = 398 g/mol)

### Synthesis of Intermediate (J)

Intermediate (J) (9.08 g, the yield of 58%) was obtained in the same manner as used to prepare Intermediate (F) in Synthesis Example 11, except that 14.0 g (35.2 mmol) of Intermediate (I) was used instead of Intermediate (E).
LC-Mass (calculated : 445.22 g/mol, found : M⁺¹ = 446 g/mol)

### Synthesis of Compound 165

Compound 165 (3.41 g, the yield of 72%) was obtained in the same manner as used to prepare Compound 105 of Synthesis Example, except that 5.00 g (11.2 mmol) of Intermediate (J) was used instead of Intermediate (F).
LC-Mass (calculated : 421.16 g/mol, found : M⁺¹ = 422 g/mol)

### Synthesis Example 14 (Compound 175)

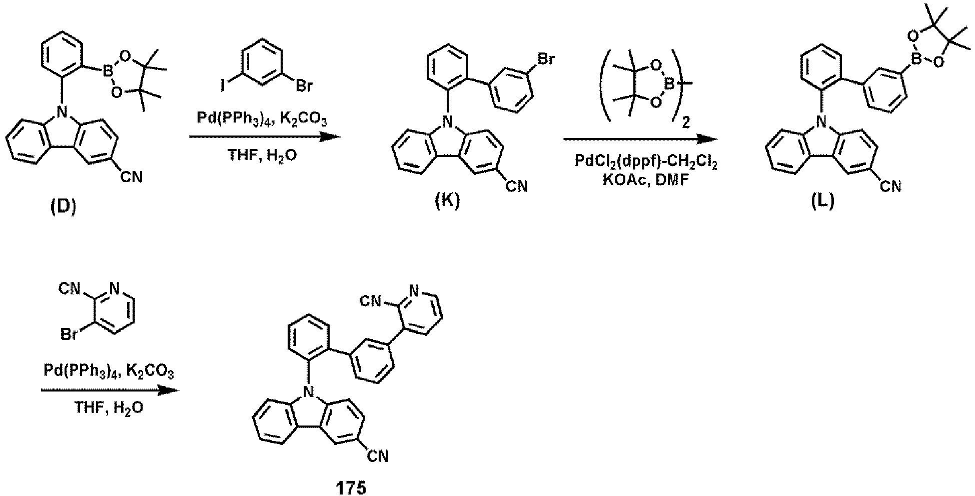

### Synthesis of Intermediate (K)

Intermediate (K) (13.5 g, the yield of 55%) was obtained in the same manner as used to prepare Intermediate (E) in Synthesis Example 11, except that 20.0 g (50.7 mmol) of Intermediate (D) was used instead of 9-(3-(4,4,5,5-tetramethyl-1,3,2-dioxaborolan-2-yl)phenyl)-9H-carbazole.
LC-Mass (calculated : 422.04 g/mol, found : M⁺¹ = 423 g/mol)

### Synthesis of Intermediate (L)

Intermediate (L) (8.67 g, the yield of 60%) was obtained in the same manner as used prepare Intermediate (F) in Synthesis Example 11, except that 13.0 g (30.7 mmol) of Intermediate (K) was used instead of Intermediate (E).
LC-Mass (calculated : 470.22 g/mol, found : M⁺¹ = 471 g/mol)

### Synthesis of Compound 175

Compound 175 (2.28 g, the yield of 48%) was obtained in the same manner as used to prepare Compound 105 in Synthesis Example 11, except that 5.00 g (10.6 mmol) of Intermediate (L) was used instead of Intermediate (F).
LC-Mass (calculated : 446.15 g/mol, found : M⁺¹ = 447 g/mol)

### Synthesis Example 15 (Compound 635)

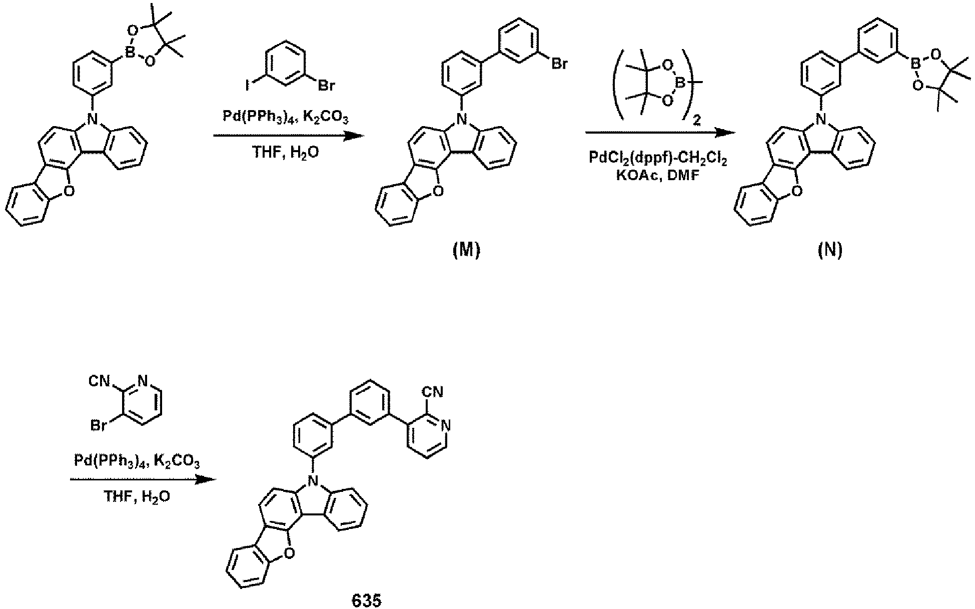

### Synthesis of Intermediate (M)

Intermediate (M) (18.6 g, the yield of 70%) was obtained in the same manner as used to prepare Intermediate (E) in Synthesis Example 11, except that 25.0 g (54.2 mmol) of 5-(3-(4,4,5,5-tetramethyl-1,3,2-dioxaborolan-2-yl)phenyl)-5H-benzofuro[3,2-c]carbazole was used instead of 9-(3-(4,4,5,5-tetramethyl-1,3,2-dioxaborolan-2-yl)phenyl)-9H-carbazole.
LC-Mass (calculated : 487.06 g/mol, found : M⁺¹ = 488 g/mol)

### Synthesis of Intermediate (N)

Intermediate (N) (10.7 g, the yield of 54%) was obtained in the same manner as used to prepare Intermediate (F) in Synthesis Example 11, except that 18.0 g (36.9 mmol) of Intermediate (M) was used instead of Intermediate (E).
LC-Mass (calculated : 535.23 g/mol, found : M⁺¹ = 536 g/mol)

### Synthesis of Compound 635

Compound 635 (3.15 g, the yield of 66%) was obtained in the same manner as used to prepare Compound 105 in Synthesis Example 11, except that 5.00 g (9.34 mmol) of Intermediate (N) was used instead of Intermediate (F).
LC-Mass (calculated : 511.17 g/mol, found : M⁺¹ = 512 g/mol)

### Comparative Synthesis Example A (Compound A)

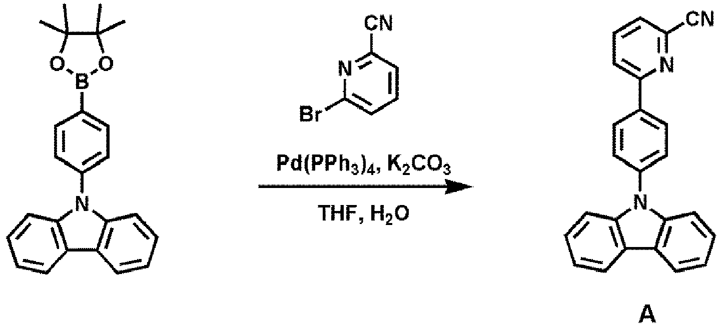

Compound A (3.65 g, the yield of 78%) was obtained in the same manner as in Synthesis Example 1, except that 5.00 g (13.5 mmol) of 9-(4-(4,4,5,5-tetramethyl-1,3,2-dioxaborolan-2-yl)phenyl)-9H-carbazole was used instead of 9-(3-(4,4,5,5-tetramethyl-1,3,2-dioxaborolan-2-yl)phenyl)-9H-carbazole).
LC-Mass (calculated : 345.13 g/mol, found : M⁺¹ = 346 g/mol)

### Comparative Synthesis Example B (Compound B)

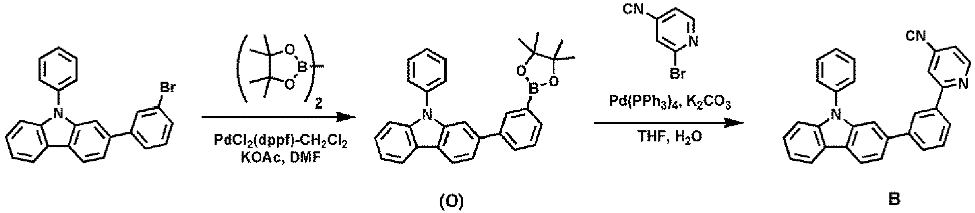

### Synthesis of Intermediate (O)

15.0 g (37.7 mmol) of 2-(3-bromophenyl)-9-phenyl-9H-carbazole, 11.5 g (45.2 mmol) of bis(pinacolato)diboron, 1.54 g (1.88 mmol) of PdCl₂(dppf)·CH₂Cl₂, and 11.1 g (113 mmol) of potassium acetate were mixed with 125 mL of DMF, and the mixture was stirred for 24 hours at a temperature of 100°C. After the completion of the reaction, the reaction mixture was cooled to room temperature, passed through silica gel under reduced pressure, and the filtrate was concentrated under reduced pressure. The resulting product was separated by silica gel column chromatography and recrystallized under the condition of dichloromethane (DCM)/n-hexane to obtain Intermediate (O) (15.3 g, the yield of 91%).
LC-Mass (calculated : 445.22 g/mol, found : M⁺¹ = 446 g/mol)

### Synthesis of Compound B

5.00 g (11.2 mmol) of Intermediate (O), 2.26 g (12.4 mmol) of 2-bromoisonicotinonitrile, 0.649 g (0.561 mmol) of tetrakistriphenylphosphine palladium (0) (Pd(PPh₃)₄), and 3.88 g (28.1 mmol) of potassium carbonate were added to a mixture including 24 mL of THF and 12 mL of water, and the resulting mixture was stirred for 18 hours under reflux. After completion of the reaction, the reaction mixture was cooled to room temperature and the aqueous layer was removed therefrom through extraction. The organic layer was filtered under reduced pressure through a silica gel, and the filtrate was concentrated under reduced pressure. The product was filtered by silica gel column chromatography, thereby obtaining Compound B (3.41 g, the yield of 72%).
LC-Mass (calculated : 421.16 g/mol, found : M⁺¹ = 422 g/mol)

### Comparative Synthesis Example C (Compound C)

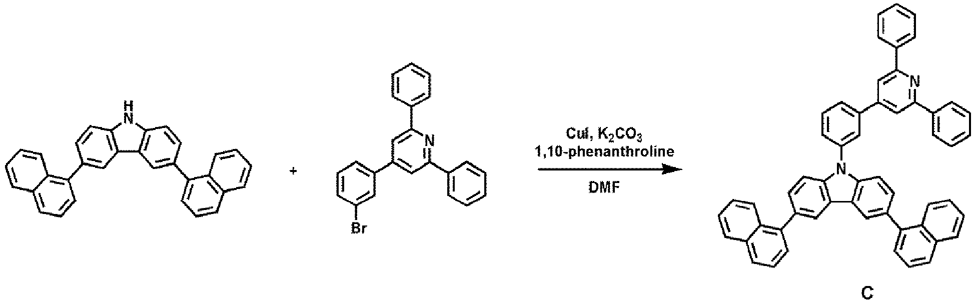

5.00 g (11.9 mmol) of 3,6-di(naphthalen-1-yl)-9H-carbazole, 5.06 g (13.1 mmol) of 4-(3-bromophenyl)-2,6-diphenylpyridine, 0.454 g (2.38 mmol) of copper iodide (Cul), 6.59 g (47.7 mmol) of potassium carbonate (K₂CO₃), and 0.859 g (4.77 mmol) of 1,10-phenanthroline were dissolved in 40 mL of DMF, and the mixture was stirred for 24 hours under reflux. After completion of the reaction, the reaction mixture was cooled to room temperature, filtered through a silica gel filter, and the filtrate was concentrated under reduced pressure. The product was separated by silica gel column chromatography to obtain Intermediate C (5.87 g, the yield of 68%).
LC-Mass (calculated : 724.29 g/mol, found : M⁺¹ = 725 g/mol)

### Comparative Synthesis Example D (Compound D)

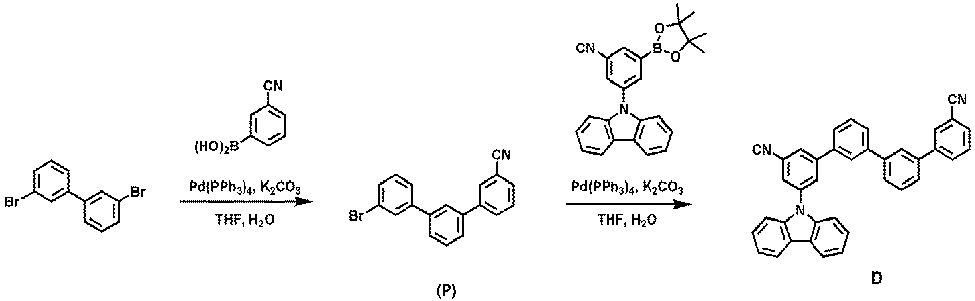

### Synthesis of Intermediate (P)

9.56 g (30.6 mmol) of 3,3'-dibromo-1,1'-biphenyl), 3.00 g (20.4 mmol) of (3-cyanophenyl)boronic acid, 1.18 g (1.02 mmol) of tetrakistriphenylphosphine palladium (0) (Pd(PPh₃)₄), and 7.05 g (51.0 mmol) of potassium carbonate were added to a mixture including 50 mL of THF and 25 mL of water, and the resulting mixture was stirred for 18 hours under reflux. After completion of the reaction, the reaction mixture was cooled to room temperature, and the aqueous layer was removed therefrom by extraction. The resultant product was filtered through silica gel, concentrated under reduced pressure, and purified by silica gel column chromatography to obtain Intermediate (P) (5.12 g, the yield of 75%).
LC-Mass (calculated : 333.02 g/mol, found : M⁺¹ = 334 g/mol)

### Synthesis of Compound D

4.00 g (12.0 mmol) of Intermediate (P), 5.19 g (13.2 mmol) of 3-(9H-carbazol-9-yl)-5-(4,4,5,5-tetramethyl-1,3,2-dioxaborolan-2-yl)benzonitrile, 0.692 g (0.598 mmol) of tetrakistriphenylphosphine palladium (0) (Pd(PPh₃)₄), and 4.14 g (29.9 mmol) of potassium carbonate were added to a mixture including 30 mL of THF and 15 mL of water, and the mixture was stirred for 18 hours under reflux. After completion of the reaction, the reaction mixture was cooled to room temperature and the aqueous layer was removed therefrom through extraction. The organic layer was filtered under reduced pressure through a silica gel, and the filtrate was concentrated under reduced pressure. The product was filtered by silica gel column chromatography, thereby obtaining Compound D (5.24 g, the yield of 84%).
LC-Mass (calculated : 521.19 g/mol, found : M⁺¹ = 522 g/mol)

### Example 1

A glass substrate, on which a 1,500 Angstrom-thick ITO (indium tin oxide) electrode (first electrode, anode) was formed, was cleaned by distilled water ultrasonication. After the distilled water ultrasonication, ultrasonic cleaning was performed with a solvent such as iso-propyl alcohol, acetone, and methanol, and the glass substrate was dried and transferred to a plasma cleaner. The glass substrate was cleaned by using oxygen plasma for 5 minutes, and then transferred to a vacuum laminator.

Compound HT3 and Compound HT-D2 were co-deposited on the ITO electrode on the glass substrate to form a hole injection layer having a thickness of 100 Å, and Compound HT3 was deposited on the hole injection layer to form a hole transport layer having a thickness of 1,300 Å, and mCP was deposited on the hole transport layer to form an electron blocking layer having a thickness of 100 Å to form a hole transport region.

Compound 1 (host) and Flr6 (dopant, 10 weight%) were co-deposited on the hole transport region to form an emission layer having a thickness of 400 Å.

BCP was vacuum-deposited on the emission layer to form a hole blocking layer having a thickness of 100 Å, and Compound ET3 and LiQ were co-deposited on the hole blocking layer to form an electron transport layer having a thickness of 300 Å, and LiQ was deposited on the electron transport layer to form an electron injection layer having a thickness of 10 Å, and an Al second electrode (cathode) having a thickness of 1,200 Å was formed on the electron injection layer, thereby completing the manufacture of an organic light-emitting device.

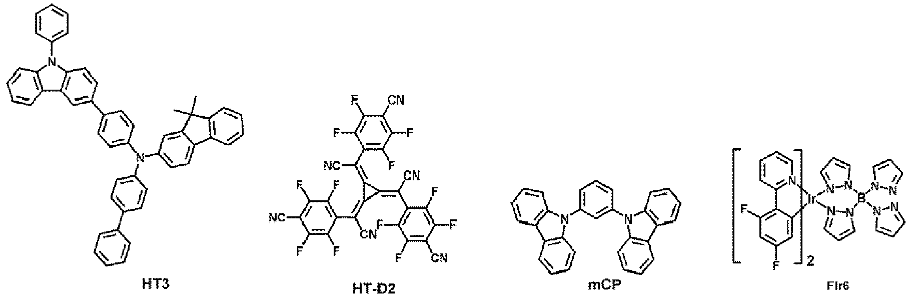

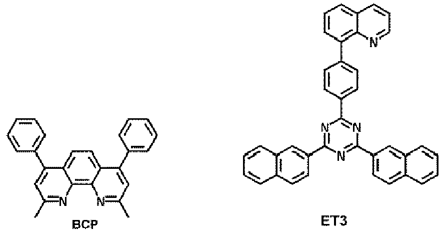

### Examples 2 to 12 and Comparative Examples A to D

Organic light-emitting devices were manufactured in the same manner as in Example 1, except that in forming an emission layer, for use as a host, corresponding compounds shown in Table 2 were used instead of Compound 1.

### Evaluation Example 1: Characterization of organic light-emitting device

The driving voltage (volts, V), current efficiency (candelas per ampere, cd/A), and durability (T₉₅ at 1,000 nit, hours, hr) of the organic light-emitting devices manufactured according to Examples 1 to 12 and Comparative Examples A to D were evaluated by using a Current-Voltmeter (Keithley 2400) and luminance meter (Minolta Cs-1000A). The results are shown in Table 2. Durability (T₉₅) refers to a time that is taken for the brightness to become 95% compared to the initial luminance of 100%. Regarding Table 2, the driving voltage, current efficiency, and durability of each of Examples 1 to 12 and Comparative Examples B to D were evaluated as a relative value (%) with respect to the driving voltage, current efficiency and durability of Comparative Example A.

**Table 2**

| | host Compound | Driving voltage (Relative value, %) | Current efficiency (Relative value, %) | Durability (Relative value, %) | Color |
|---|---|---|---|---|---|
| Example 1 | 1 | 83 | 145 | 191 | Blue |
| Example 2 | 5 | 86 | 127 | 203 | Blue |
| Example 3 | 6 | 84 | 155 | 180 | Blue |
| Example 4 | 7 | 79 | 157 | 220 | Blue |
| Example 5 | 9 | 75 | 171 | 243 | Blue |
| Example 6 | 15 | 62 | 189 | 289 | Blue |
| Example 7 | 55 | 66 | 198 | 275 | Blue |
| Example 8 | 105 | 87 | 158 | 190 | Blue |
| Example 9 | 115 | 54 | 245 | 332 | Blue |
| Example 10 | 165 | 77 | 160 | 235 | Blue |
| Example 11 | 175 | 59 | 206 | 304 | Blue |
| Example 12 | 635 | 71 | 199 | 273 | Blue |
| Comparative Example A | A | 100 | 100 | 100 | Blue |
| Comparative Example B | B | 98 | 85 | 105 | Blue |
| Comparative Example C | C | 174 | 54 | 32 | Blue |
| Comparative Example D | D | 79 | 125 | 152 | Blue |

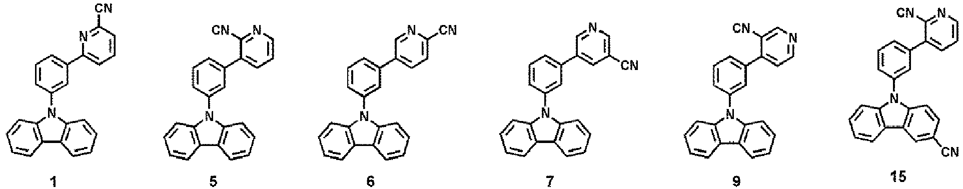

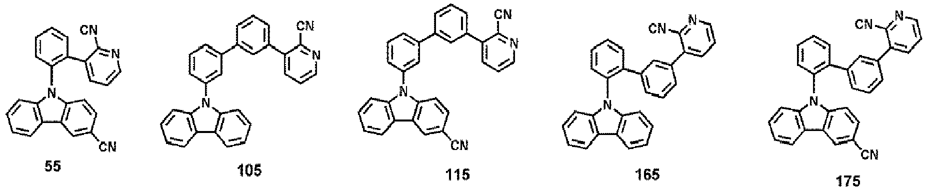

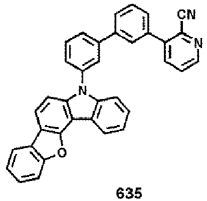

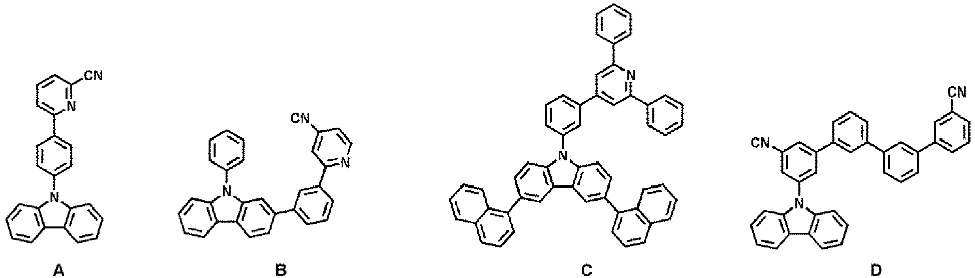

From Table 2, it was confirmed that the organic light-emitting devices of Examples 1 to 12 had low driving voltage, high current efficiency, and high durability compared to the organic light-emitting devices of Comparative Examples A to D, while emitting blue light.

The condensed cyclic compounds have excellent electric characteristics and high thermal stability. Accordingly, an organic light-emitting device including the condensed cyclic compound may have low driving voltage, high luminous efficiency, high power, high quantum efficiency, and a long lifespan.

It should be understood that embodiments described herein should be considered in a descriptive sense only and not for purposes of limitation. Descriptions of features or aspects within each embodiment should typically be considered as available for other similar features or aspects in other embodiments.

While one or more embodiments have been described with reference to the figures, it will be understood by those of ordinary skill in the art that various changes in form and details may be made therein without departing from the scope of the present disclosure as defined by the following claims.

## Claims

1. A condensed cyclic compound represented by Formula 1 or Formula 2: 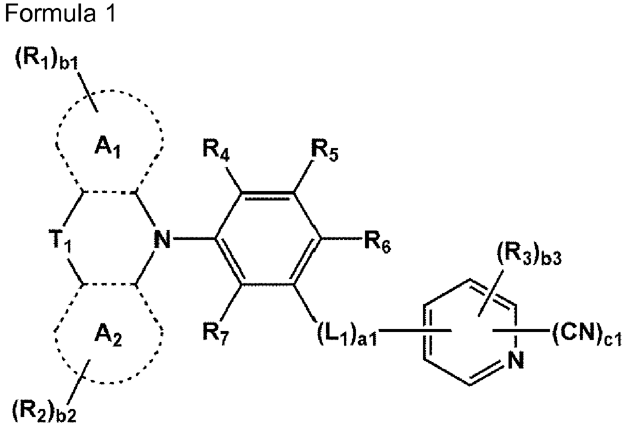 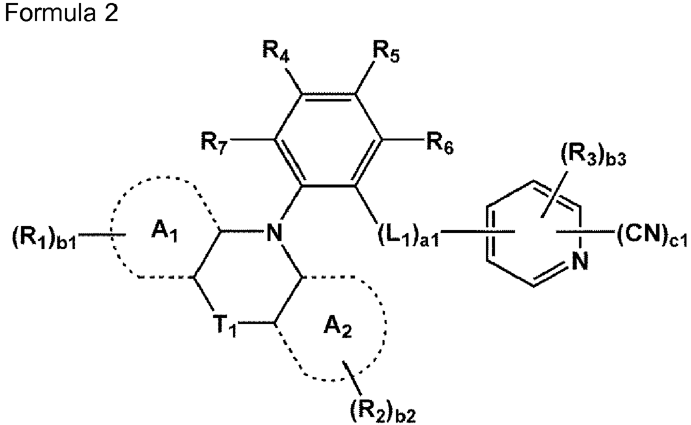 wherein, in Formulae 1 and 2,
ring A₁ and ring A₂ are each independently a C₅-C₃₀ carbocyclic group or a C₁-C₃₀ heterocyclic group,
T₁ is a single bond, O, S, N(R₈), C(R₈)(R₉), or Si(R₈)(R₉),
L₁ is a single bond, a C₆-C₆₀ arylene group unsubstituted or substituted with at least one R₁₀ₐ, a C₁-C₆₀ heteroarylene group unsubstituted or substituted with at least one R₁₀ₐ, a divalent non-aromatic condensed polycyclic group unsubstituted or substituted with at least one R₁₀ₐ, or a divalent non-aromatic condensed heteropolycyclic group unsubstituted or substituted with at least one R₁₀ₐ,
a1 is an integer from 1 to 5, wherein when a1 is two or more, two or more of groups L₁ are identical to or different from each other,
R₁ to R₉ are each independently selected from hydrogen, deuterium, -F, -CI, -Br, -I, a hydroxyl group, a cyano group (CN), a nitro group, an amino group, an amidino group, a hydrazine group, a hydrazone group, a carboxylic acid group or a salt thereof, a sulfonic acid group or a salt thereof, a phosphoric acid group or a salt thereof, a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₇-C₆₀ arylalkyl group, a substituted or unsubstituted C₁-C₆₀ heteroaryl group, a substituted or unsubstituted C₁-C₆₀ heteroaryloxy group, a substituted or unsubstituted C₁-C₆₀ heteroarylthio group, a substituted or unsubstituted C₂-C₆₀ heteroarylalkyl group, a substituted or unsubstituted monovalent non-aromatic condensed polycyclic group, a substituted or unsubstituted monovalent non-aromatic condensed heteropolycyclic group, - Si(Q₁)(Q₂)(Q₃), -N(Q4)(Q5), -B(Q6)(Q7), -S(Q₁), and -C(=O)(Q₁),
b1 and b2 are each independently an integer from 0 to 10,
b3 is an integer from 0 to 3,
c1 is an integer from 1 to 4,
R₁₀ₐ is the same as explained in connection with R₄,
at least one substituent selected from the substituted C₁-C₆₀ alkyl group, the substituted C₂-C₆₀ alkenyl group, the substituted C₂-C₆₀ alkynyl group, the substituted C₁-C₆₀ alkoxy group, the substituted C₃-C₁₀ cycloalkyl group, the substituted C₁-C₁₀ heterocycloalkyl group, the substituted C₃-C₁₀ cycloalkenyl group, the substituted C₁-C₁₀ heterocycloalkenyl group, the substituted C₆-C₆₀ aryl group, the substituted C₆-C₆₀ aryloxy group, the substituted C₆-C₆₀ arylthio group, the substituted C₇-C₆₀ arylalkyl group, the substituted C₁-C₆₀ heteroaryl group, the substituted C₁-C₆₀ heteroaryloxy group, the substituted C₁-C₆₀ heteroarylthio group, the substituted C₂-C₆₀ heteroarylalkyl group, the substituted monovalent non-aromatic condensed polycyclic group, and the substituted monovalent non-aromatic condensed heteropolycyclic group is selected from:
deuterium, -CD₃, -CD₂H, -CDH₂, -F, -CI, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, and a C₁-C₆₀ alkoxy group;
a C₁-C₆₀ alkyl group, a C₂-C₆₀ alkenyl group, a C₂-C₆₀ alkynyl group, and a C₁-C₆₀ alkoxy group, each substituted with at least one selected from deuterium, -CD₃, -CD₂H, - CDH₂, -F, -CI, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₇-C₆₀ arylalkyl group, a C₁-C₆₀ heteroaryl group, a C₁-C₆₀ heteroaryloxy group, a C₁-C₆₀ heteroarylthio group, a C₂-C₆₀ heteroarylalkyl group, a monovalent non-aromatic condensed polycyclic group, a monovalent non-aromatic condensed heteropolycyclic group, -Si(Q₁₁)(Q₁₂)(Q₁₃), -N(Q₁₄)(Q₁₅), and -B(Q₁₆)(Q₁₇);
a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₇-C₆₀ arylalkyl group, a C₁-C₆₀ heteroaryl group, a C₁-C₆₀ heteroaryloxy group, a C₁-C₆₀ heteroarylthio group, a C₂-C₆₀ heteroarylalkyl group, a monovalent non-aromatic condensed polycyclic group, and a monovalent non-aromatic condensed heteropolycyclic group;
a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₇-C₆₀ arylalkyl group, a C₁-C₆₀ heteroaryl group, a C₁-C₆₀ heteroaryloxy group, a C₁-C₆₀ heteroarylthio group, a C₂-C₆₀ heteroarylalkyl group, a monovalent non-aromatic condensed polycyclic group, and a monovalent non-aromatic condensed heteropolycyclic group, each substituted with at least one selected from deuterium, -CD₃, -CD₂H, -CDH₂, -F, -CI, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C6-C60 aryloxy group, a C₆-C₆₀ arylthio group, a C₇-C₆₀ arylalkyl group, a C₁-C₆₀ heteroaryl group, a C₁-C₆₀ heteroaryloxy group, a C₁-C₆₀ heteroarylthio group, a C₂-C₆₀ heteroarylalkyl group, a monovalent non-aromatic condensed polycyclic group, a monovalent non-aromatic condensed heteropolycyclic group, -Si(Q₂₁)(Q₂₂)(Q₂₃), -N(Q24)(Q25), and -B(Q₂₆)(Q₂₇); and
-Si(Q₃₁)(Q₃₂)(Q₃₃), -N(Q34)(Q35), and -B(Q36)(Q37), and
Q1 to Q7, Q₁₁ to Q17, Q21 to Q₂₇, and Q31 to Q37 are each independently selected from hydrogen, deuterium, -F, -CI, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₁-C₆₀ heteroaryl group, a monovalent non-aromatic condensed polycyclic group, and a monovalent non-aromatic condensed heteropolycyclic group.

2. The condensed cyclic compound of claim 1, wherein
ring A₁ and ring A₂ are each independently a benzene group, a naphthalene group, a phenanthrene group, an anthracene group, a cyclopentene group, a pyrrole group, a furan group, a thiophene group, a silole group, an indene group, an indole group, a benzofuran group, a benzothiophene group, a benzosilole group, a fluorene group, a carbazole group, a dibenzofuran group, a dibenzothiophene group, a dibenzosilole group, a benzocarbazole group, an indenocarbazole group, an indolocarbazole group, a benzofurocarbazole group, a benzothienocarbazole group, an acridine group, a dihydroacridine group, or a triindolobenzene group.

3. The condensed cyclic compound of claims 1 or 2, wherein
a group represented by 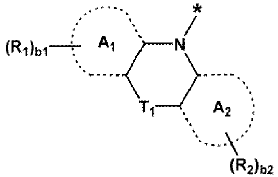 in Formulae 1 and 2 is represented by one of Formulae 3-1 to 3-7: 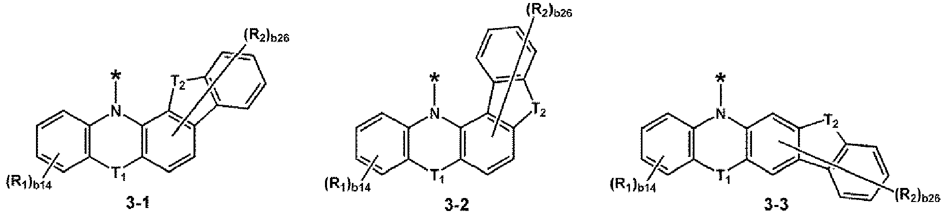 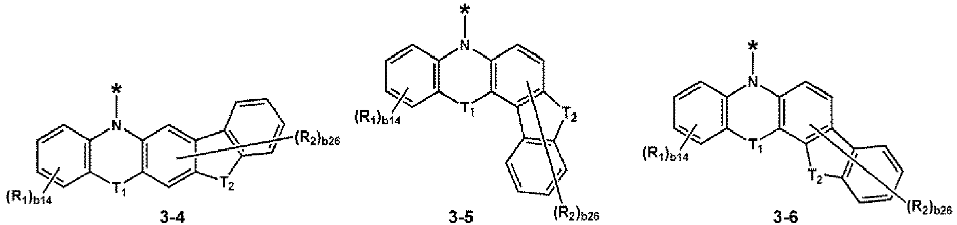 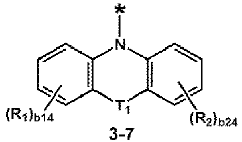 wherein, in Formulae 3-1 to 3-7,
T₁, R₁, and R₂ are the same as described in claim 1,
T₂ is C(R₁₀)(R₁₁), Si(R₁₀)(R₁₁), N(R₁₀), O, or S,
R₁₀ and R₁₁ are the same as described in connection with R₂ in claim 1,
b14 and b24 are each independently an integer from 0 to 4,
b26 is an integer from 0 to 6, and
* indicates a binding site to a neighboring atom.

4. The condensed cyclic compound of any of claims 1-3, wherein
L₁ is a single bond or a group represented by one of Formulae 4-1 to 4-38: 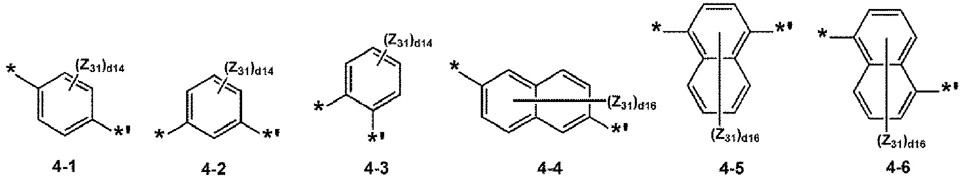 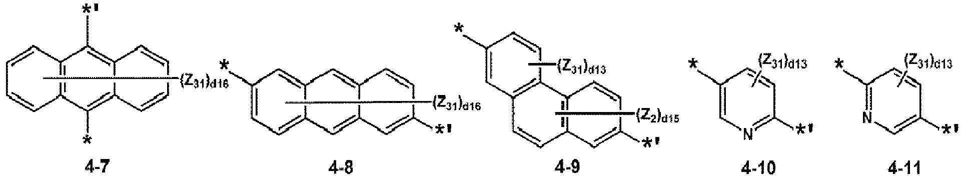 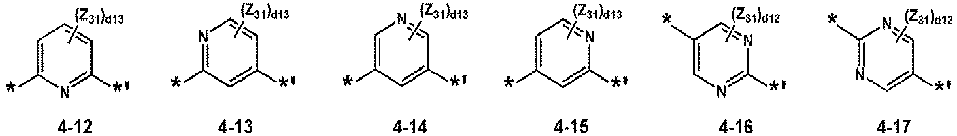 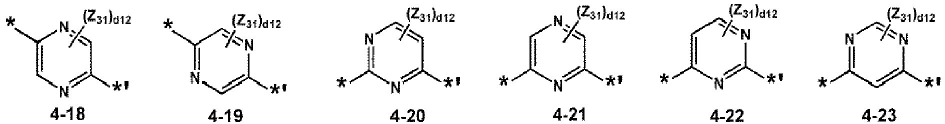 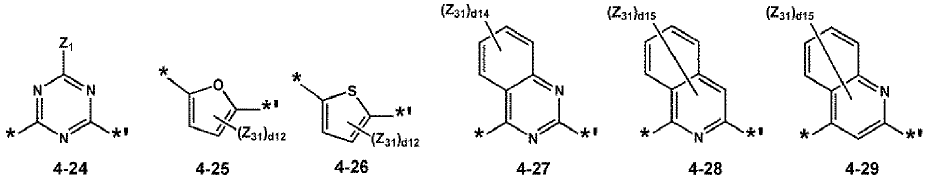 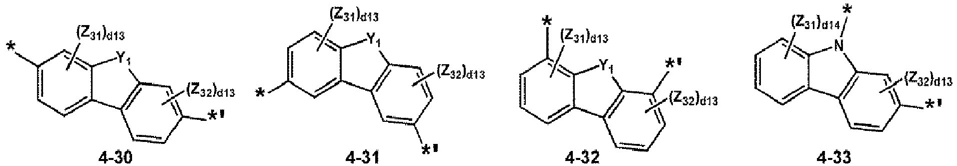 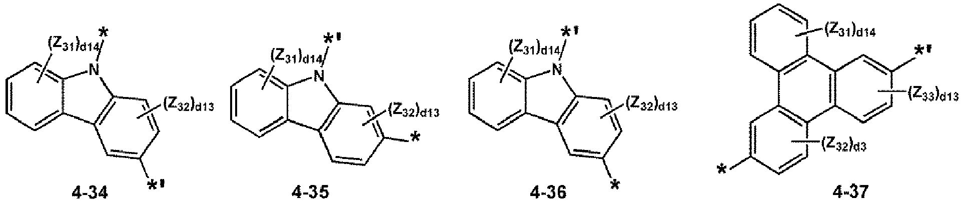 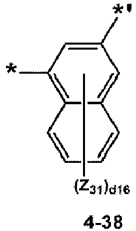 wherein, in Formulae 4-1 to 4-38,
Y₁ is O, S, C(Z₃₄)(Z₃₅), N(Z₃₄), or Si(Z₃₄)(Z₃₅),
Z₃₁ to Z₃₅ are the same as described in connection with R₄ in claim 1,
d16 is an integer from 0 to 6,
d15 is an integer from 0 to 5,
d14 is an integer from 0 to 4,
d13 is an integer from 0 to 3,
d12 is an integer from 0 to 2, and
* and *' each indicate a binding site to a neighboring atom; or
wherein
L₁ is a single bond or a group represented by one of Formulae 5-1 to 5-9: 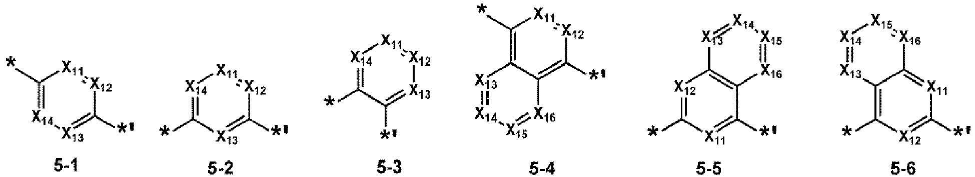 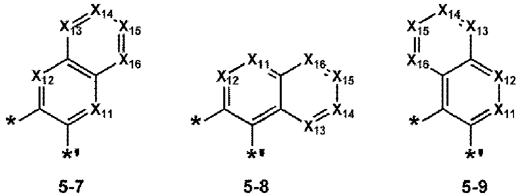 wherein, in Formulae 5-1 to 5-9,
X₁₁ is C(Z₄₁) or N, X₁₂ is C(Z₄₂) or N, X₁₃ is C(Z₄₃) or N, X₁₄ is C(Z₄₄) or N, X₁₅ is C(Z₄₅) or N, and X₁₆ is C(Z₄₆) or N,
Z₄₁ to Z₄₆ are the same as described in connection with R₄ in claim 1
* and *' each indicate a binding site to a neighboring atom.

5. The condensed cyclic compound of any of claims 1-4, wherein
i) L₁ is a single bond, or
ii) L₁ is not a single bond, and a1 is 1, 2, or 3.

6. The condensed cyclic compound of any of claims 1-5, wherein
R₁ to Rg are each independently selected from:
hydrogen, deuterium, -F, a hydroxyl group, a cyano group, and a nitro group;
a C₁-C₂₀ alkyl group and a C₁-C₂₀ alkoxy group, each unsubstituted or substituted with at least one selected from deuterium, -F, a hydroxyl group, a cyano group, and a nitro group;
a cyclopentyl group, a cyclohexyl group, a cyclopentenyl group, a cyclohexenyl group, a cycloheptenyl group, a phenyl group, a biphenyl group, a terphenyl group, a pentalenyl group, an indenyl group, a naphthyl group, an azulenyl group, a heptalenyl group, an indacenyl group, an acenaphthyl group, a fluorenyl group, a spiro-bifluorenyl group, a phenalenyl group, a phenanthrenyl group, an anthracenyl group, a fluoranthenyl group, a triphenylenyl group, a pyrenyl group, a chrysenyl group, a naphthacenyl group, a picenyl group, a perylenyl group, a pentaphenyl group, a hexacenyl group, a pyrrolyl group, an imidazolyl group, a pyrazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a carbazolyl group, a phenanthridinyl group, an acridinyl group, a phenanthrolinyl group, a phenazinyl group, a benzoxazolyl group, a benzimidazolyl group, a furanyl group, a benzofuranyl group, a thiophenyl group, a benzothiophenyl group, a thiazolyl group, an isothiazolyl group, a benzothiazolyl group, an isoxazolyl group, an oxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyridimidinyl group, an imidazopyridinyl group, a pyridoindolyl group, a benzofuropyridinyl group, a benzothienopyridinyl group, a pyrimidoindolyl group, a benzofuropyrimidinyl group, a benzothienopyrimidinyl group, a phenoxazinyl group, a pyridobenzoxazinyl group, and a pyridobenzothiazinyl group, each unsubstituted or substituted with at least one selected from deuterium, -F, a hydroxyl group, a cyano group, a nitro group, a C₁-C₂₀ alkyl group, a C₁-C₂₀ alkoxy group, a phenyl group, a biphenyl group, and a terphenyl group; and
-N(Q₄)(Q₅), -S(Q₁), and -C(=O)(Q₁),
Q₁, Q₄, and Q₅ are each independently selected from hydrogen, deuterium, a C₁-C₁₀ alkyl group, a phenyl group, a naphthyl group, a fluorenyl group, a carbazolyl group, a dibenzofuranyl group, a dibenzothiophenyl group, and a biphenyl group.

7. The condensed cyclic compound of any of claims 1-6, wherein
a group represented by 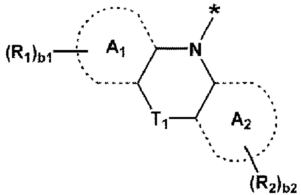 in Formulae 1 and 2 is a group represented by Formula 3(1): 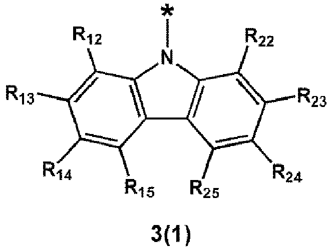
wherein, in Formula 3(1), R₁₂ to R₁₅ are the same as described in connection with R₁ in claim 1, R₂₂ to R₂₅ are the same as described in connection with R₂ in claim 1, and each of at least one selected from R₁₄ and R₂₄ is independently a cyano group, a C₁-C₁₀ alkyl group, a phenyl group, a (C₁-C₁₀ alkyl)phenyl group, a di(C₁-C₁₀ alkyl)phenyl group, a biphenyl group, a di(phenyl) phenyl group, a (cyano group)phenyl group, or a di(cyano group)phenyl group, and * indicates a binding site to a neighboring atom; and/or
wherein
c1 is 1 or 2.

8. The condensed cyclic compound of any of claims 1-7, wherein
a group represented by 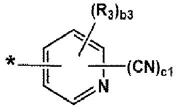 in Formulae 1 and 2 is represented by one of Formulae 6-1 to 6-3: 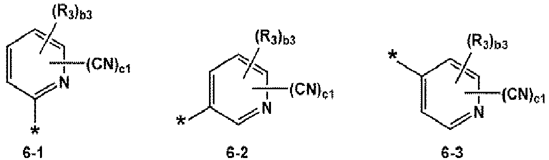
wherein R₃, b3, and c1 in Formulae 6-1 to 6-3 are the same as described in claim 1, and * indicates a binding site to a neighboring atom; or
wherein
a group represented by 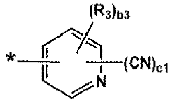 in Formulae 1 and 2 is represented by one of Formulae 7-1 to 7-39: 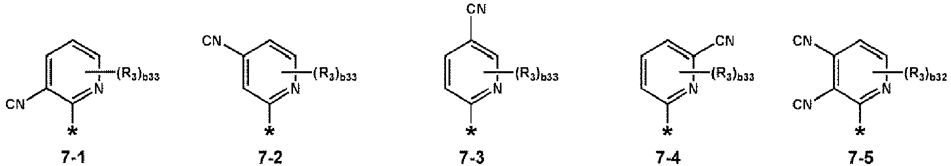 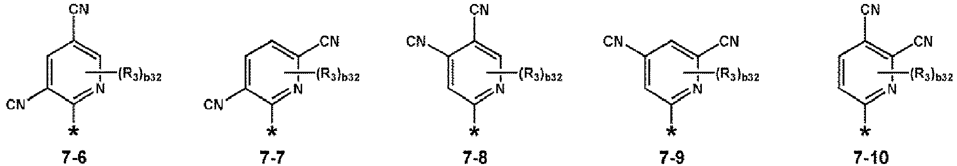 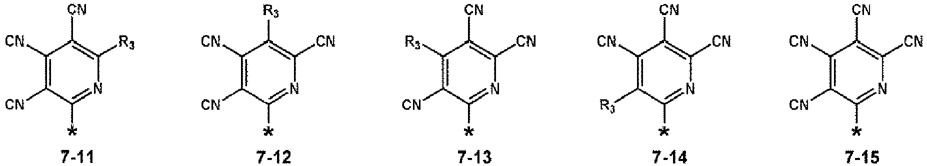 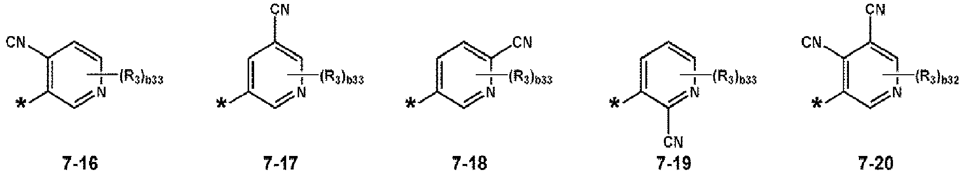 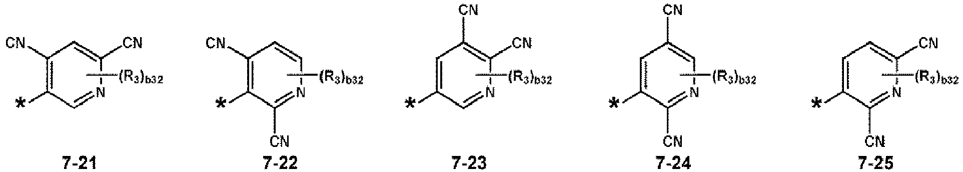 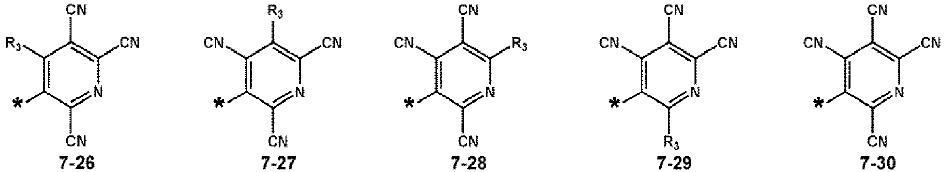 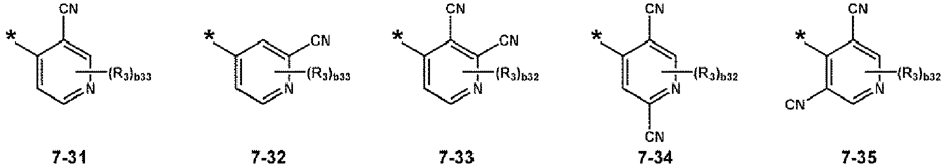 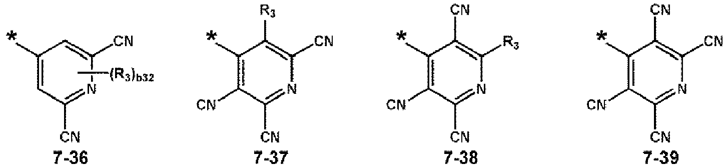 wherein, in Formulae 7-1 to 7-39,
R₃ is the same as described in claim 1, and R₃ is not a cyano group,
b33 is an integer from 0 to 3,
b32 is an integer from 0 to 2, and
* indicates a binding site to a neighboring atom.

9. The condensed cyclic compound of claim 1, wherein
the condensed cyclic compound is selected from Compounds 1 to 1000:

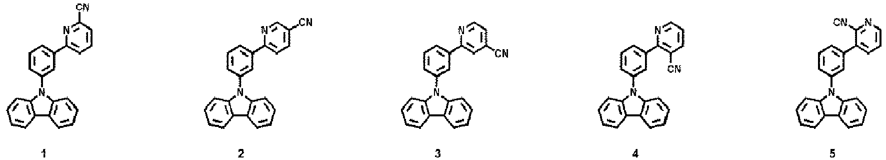

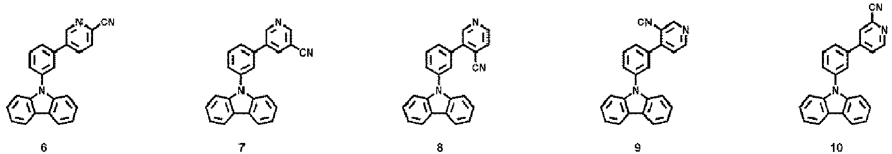

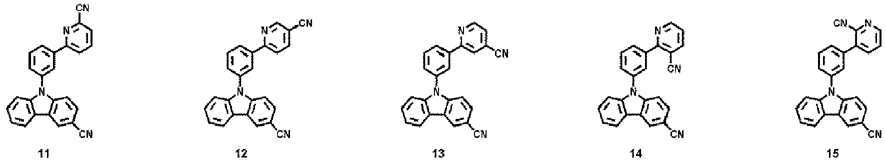

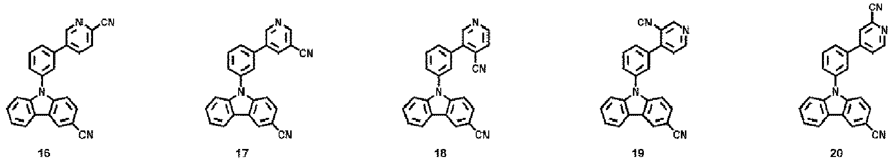

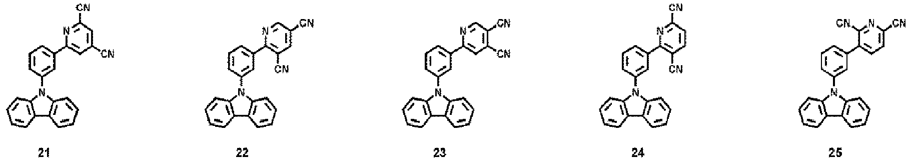

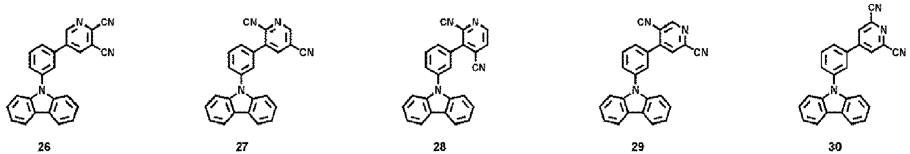

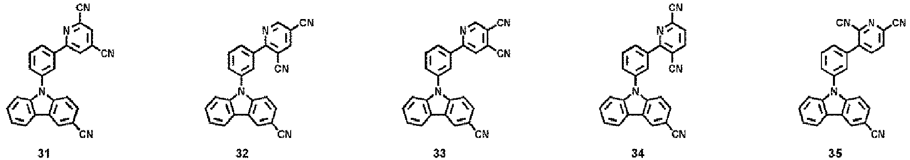

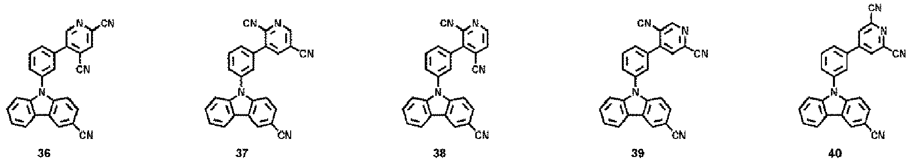

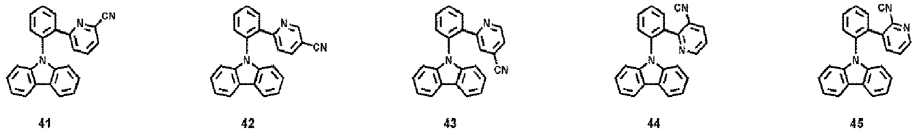

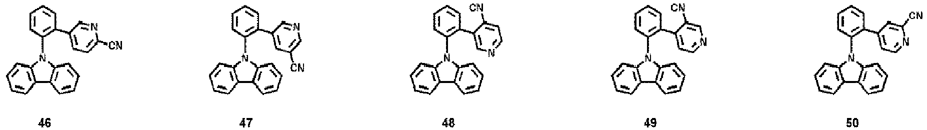

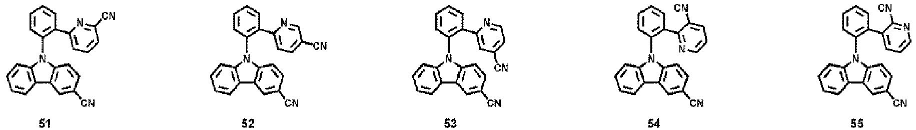

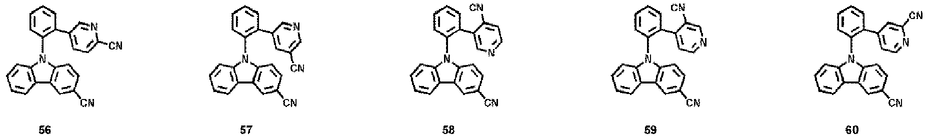

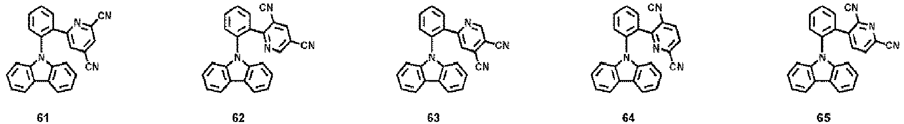

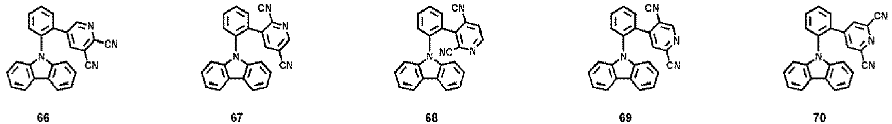

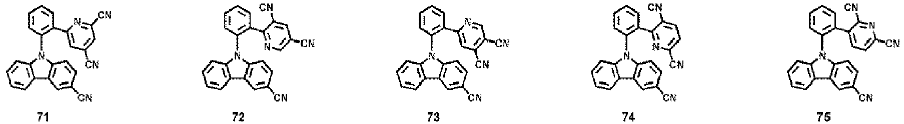

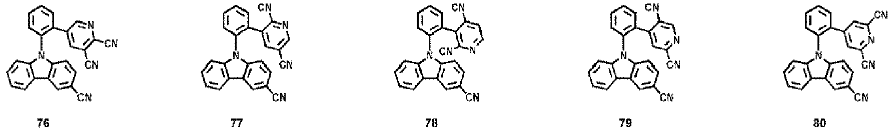

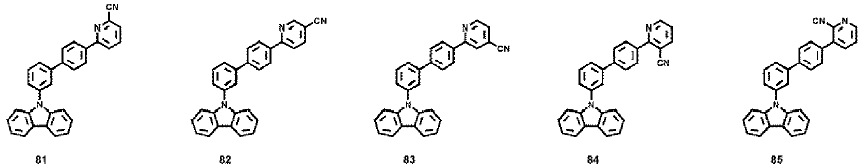

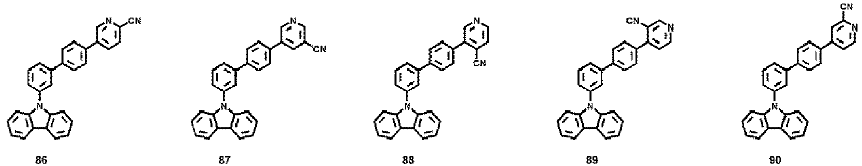

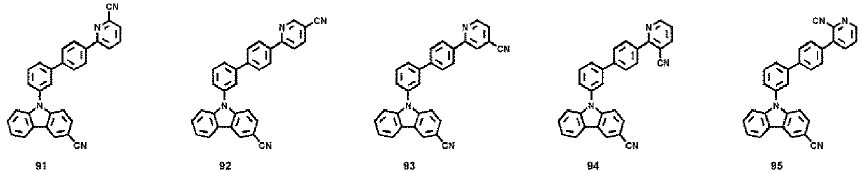

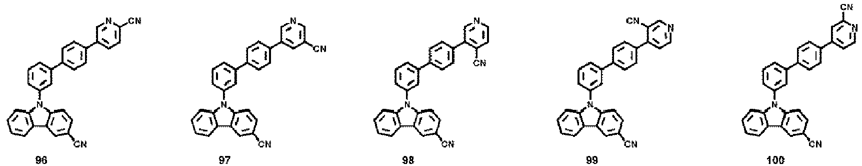

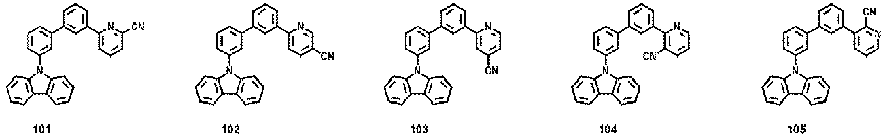

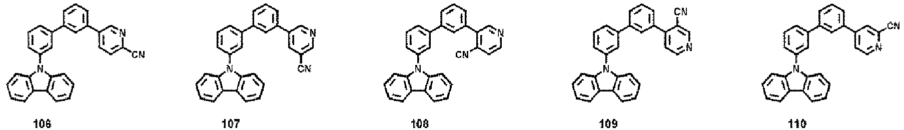

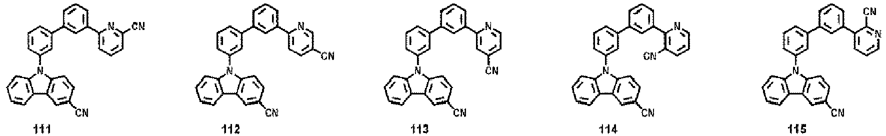

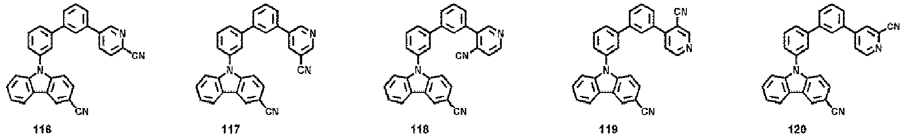

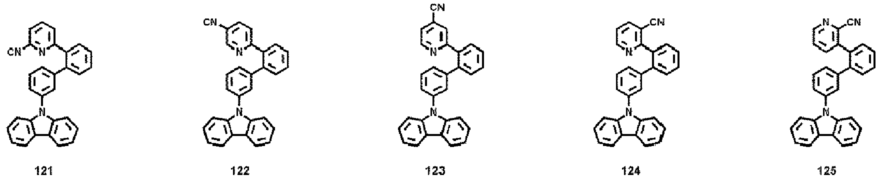

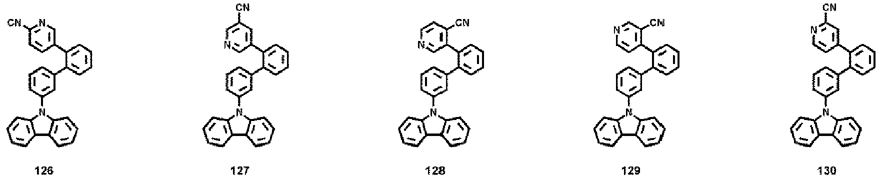

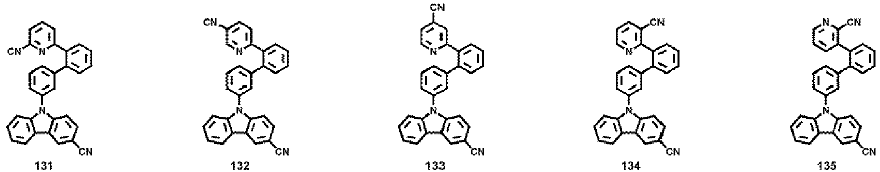

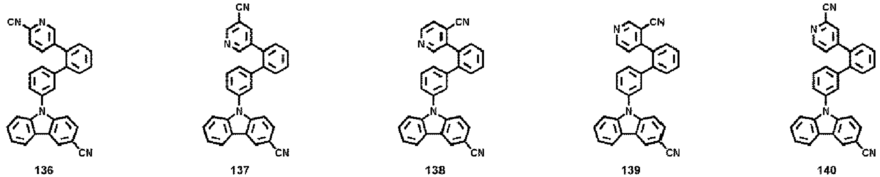

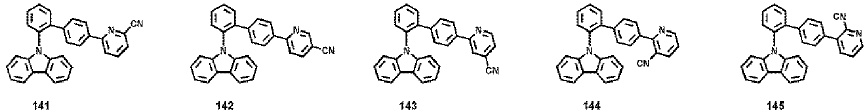

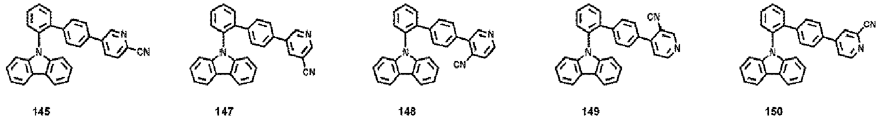

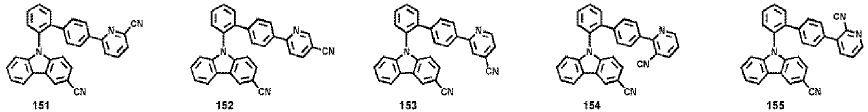

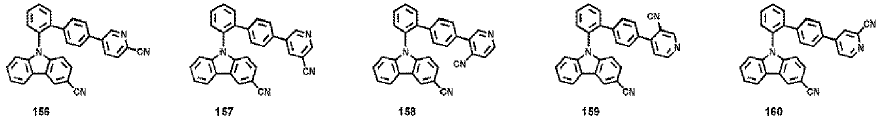

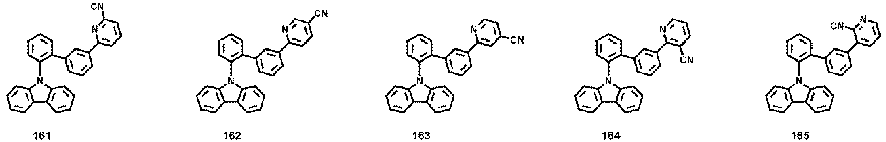

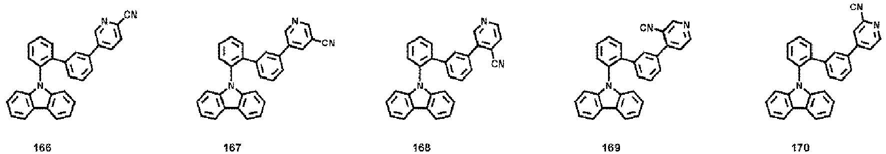

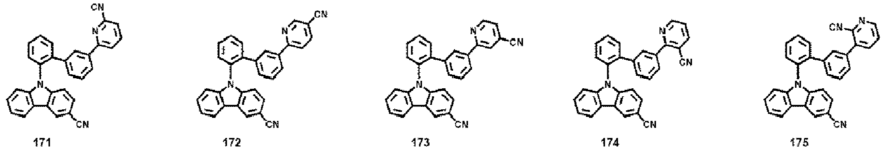

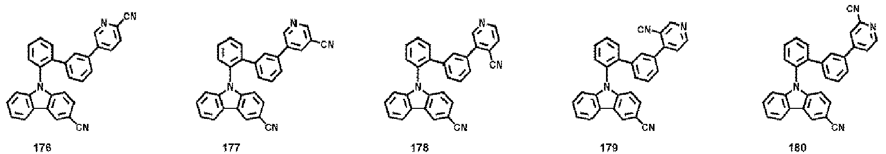

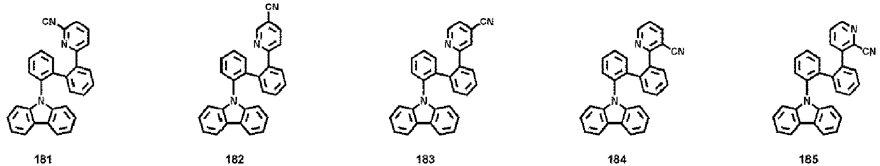

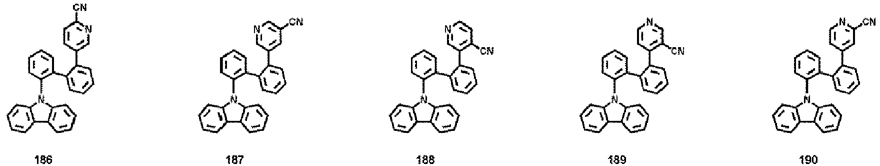

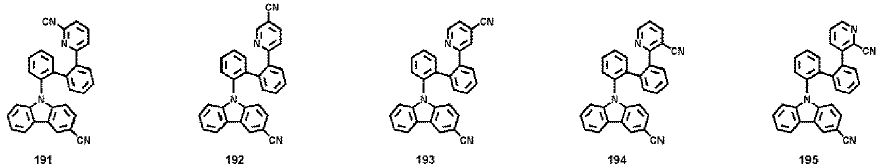

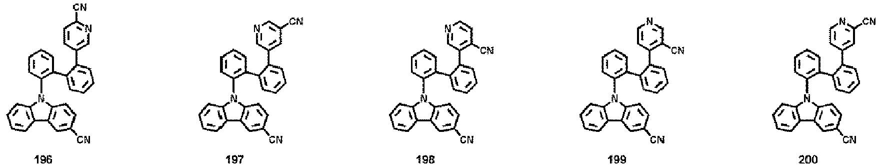

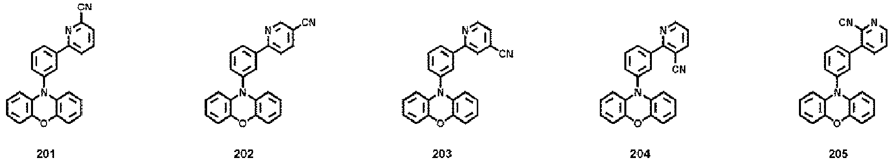

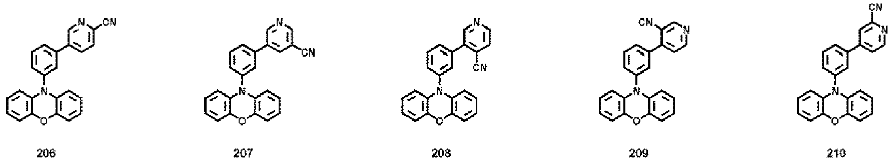

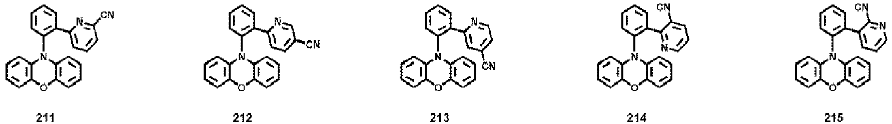

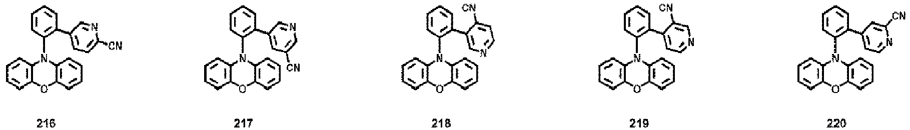

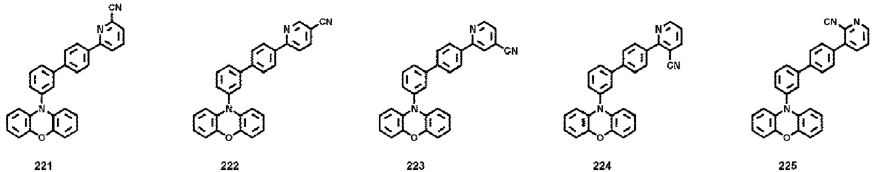

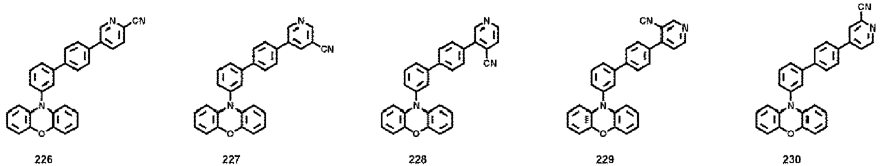

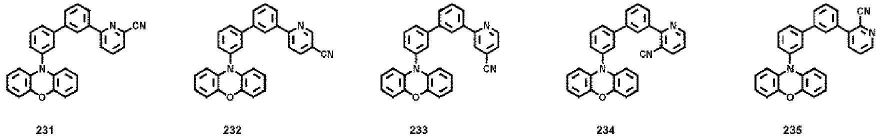

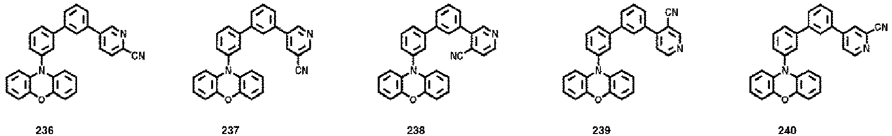

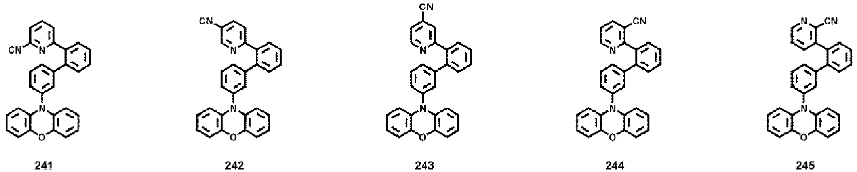

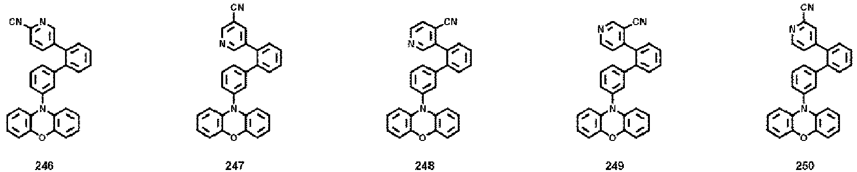

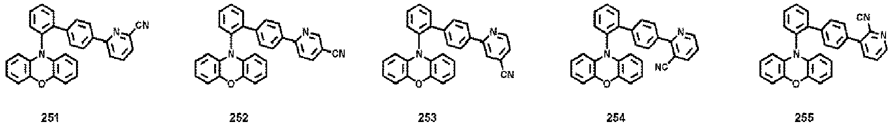

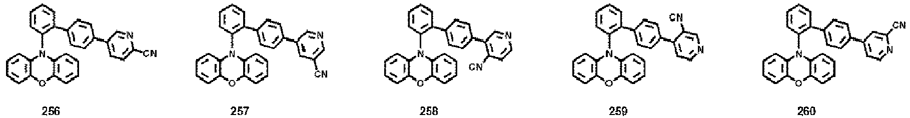

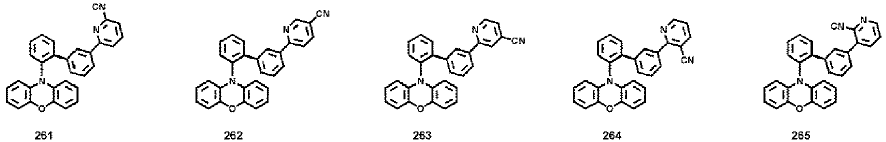

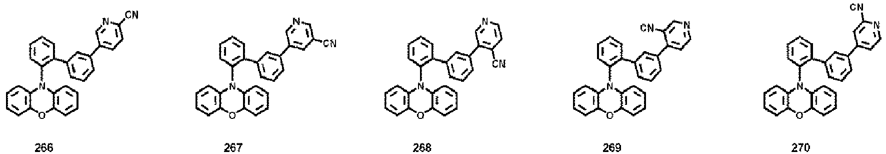

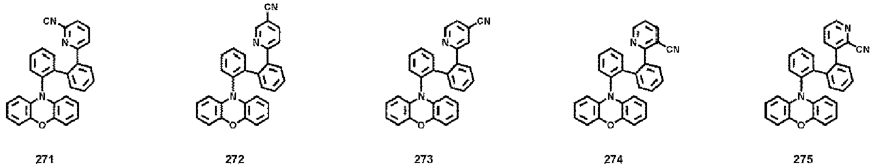

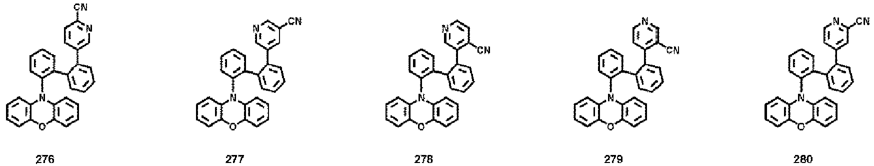

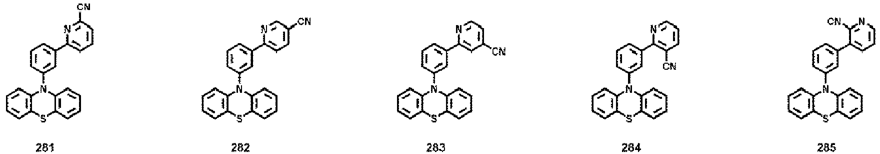

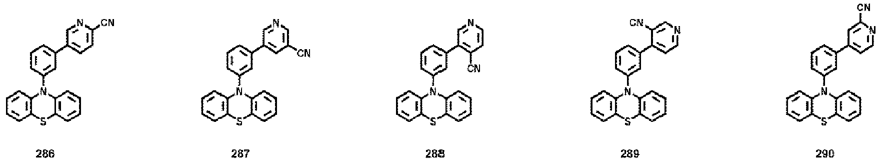

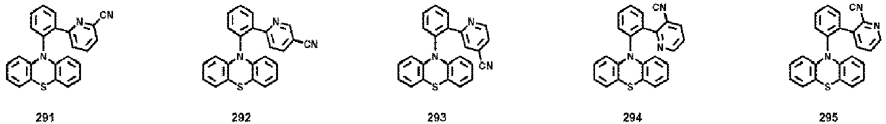

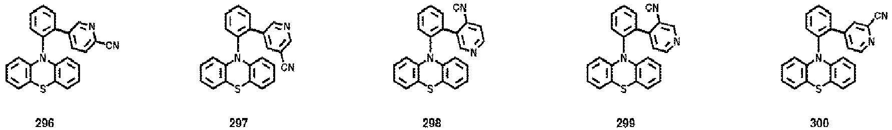

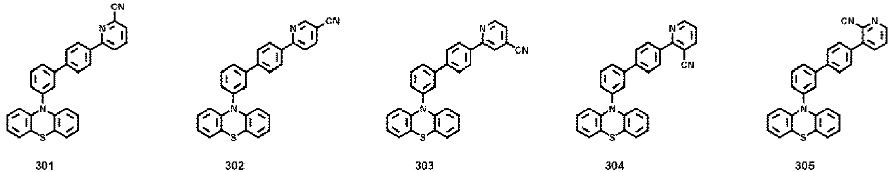

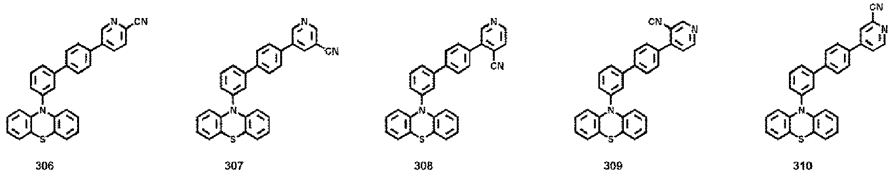

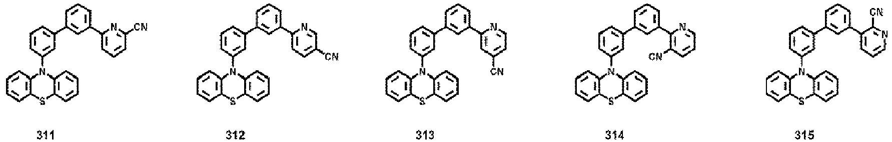

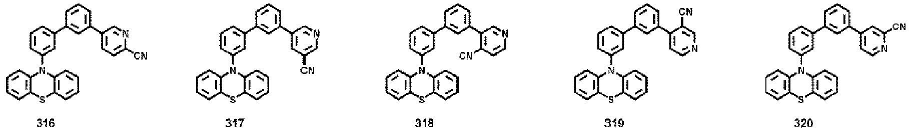

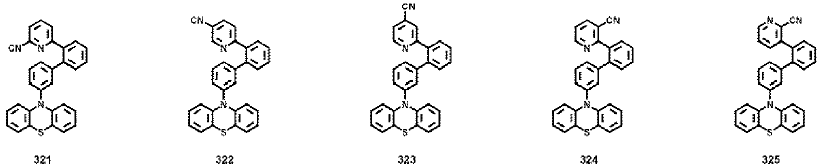

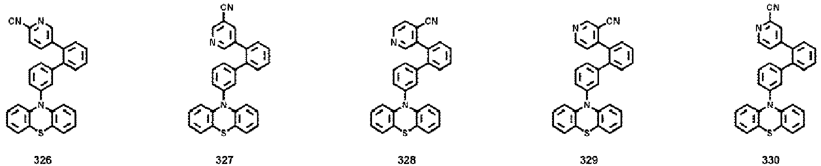

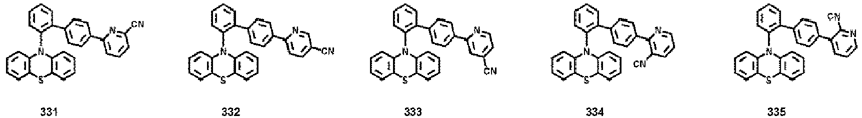

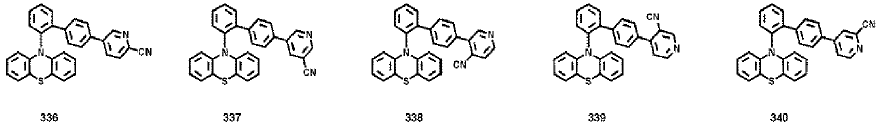

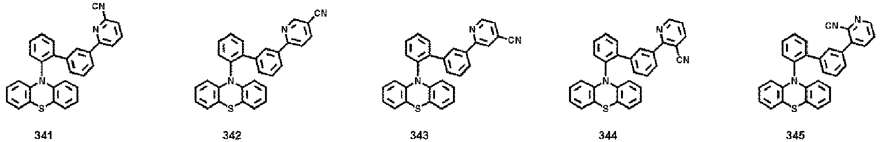

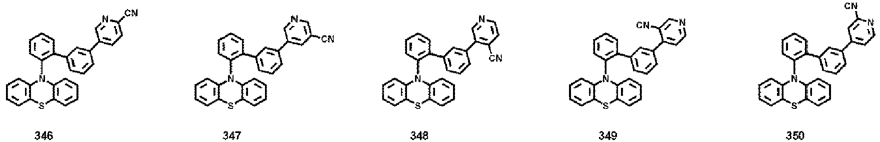

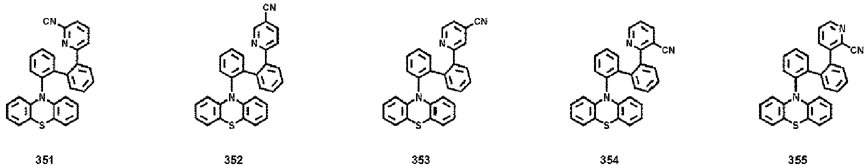

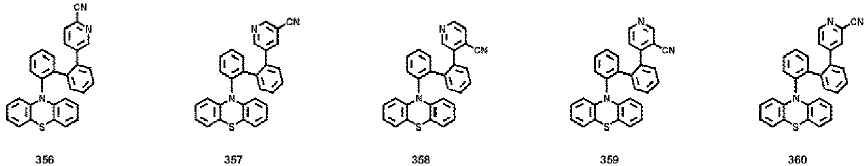

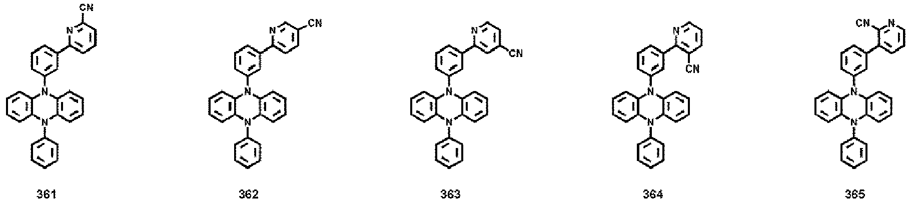

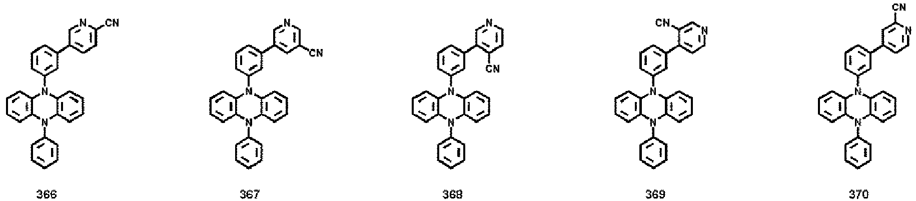

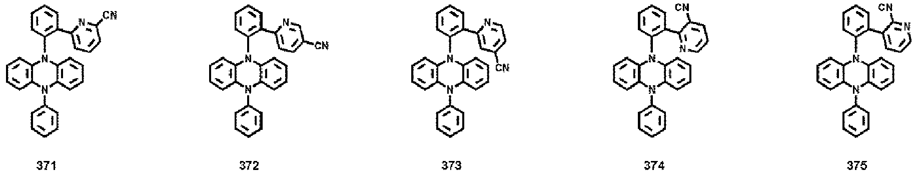

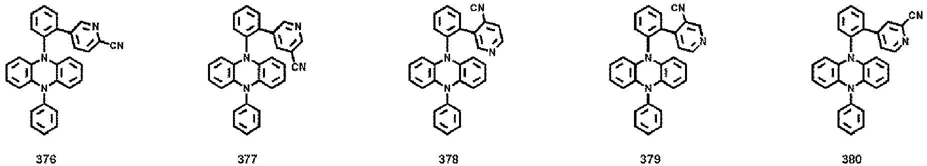

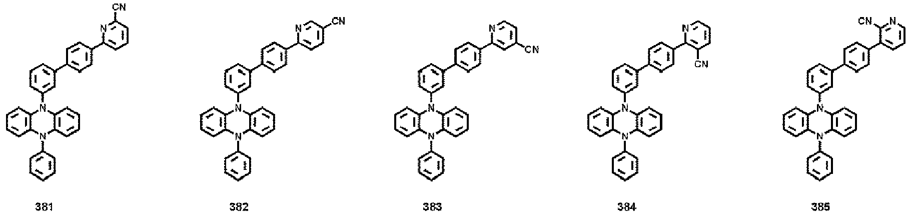

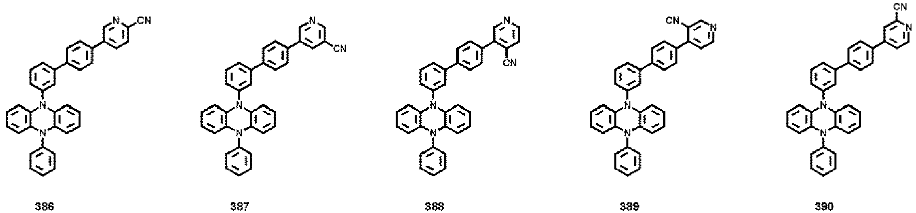

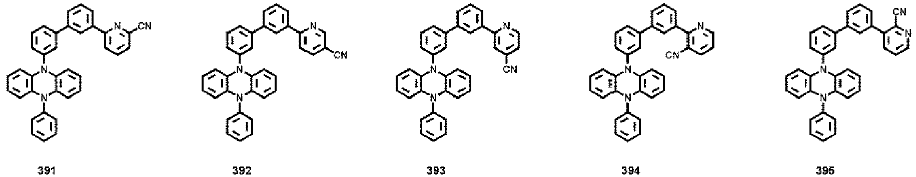

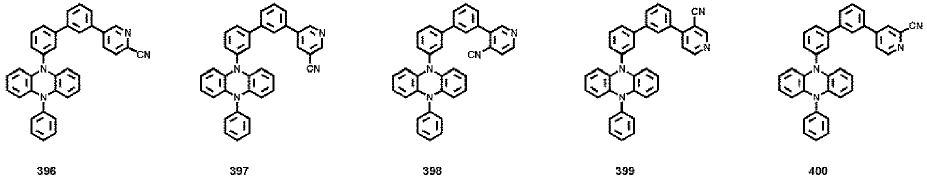

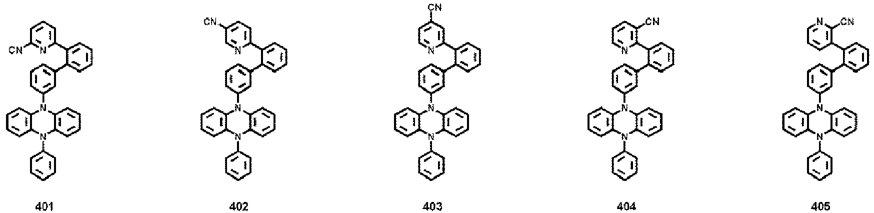

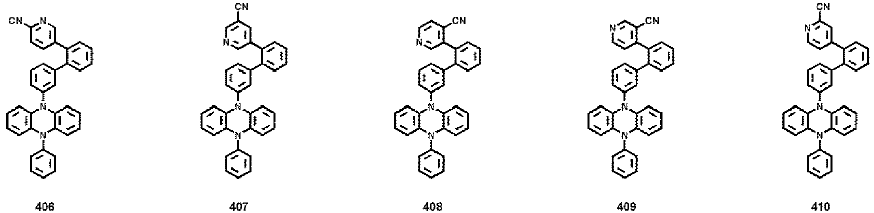

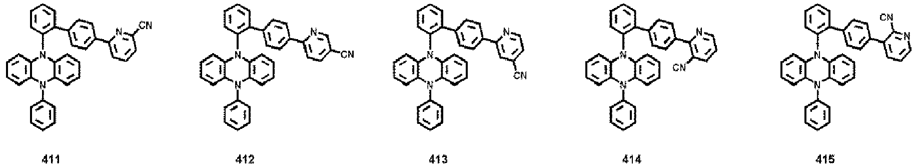

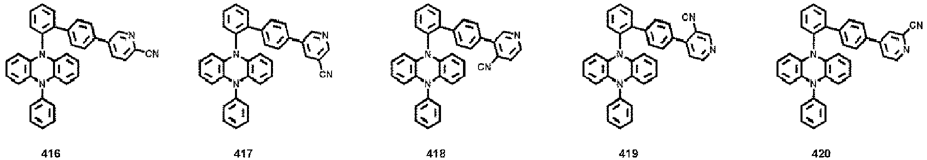

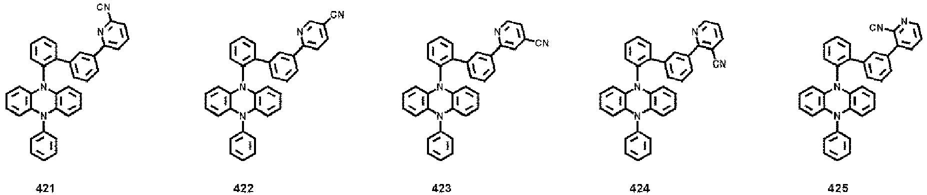

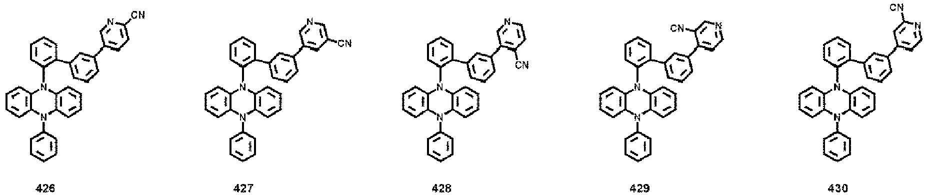

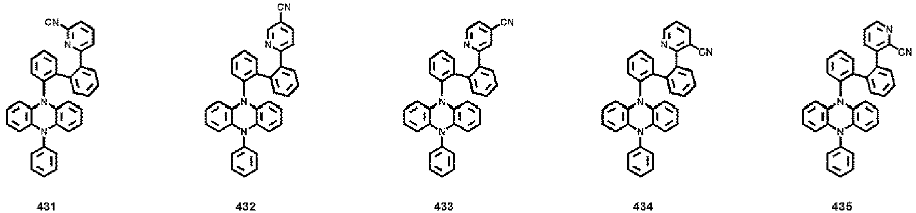

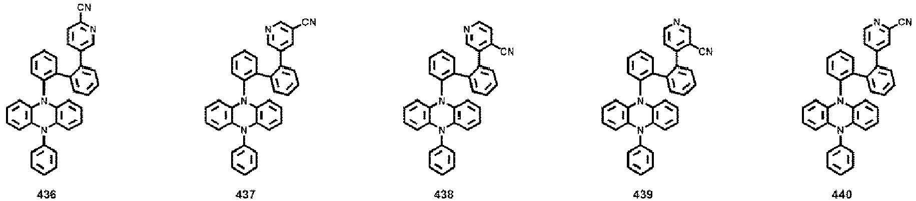

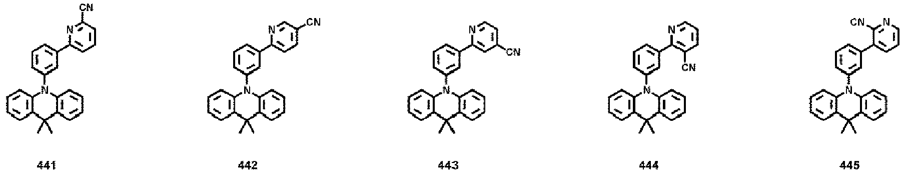

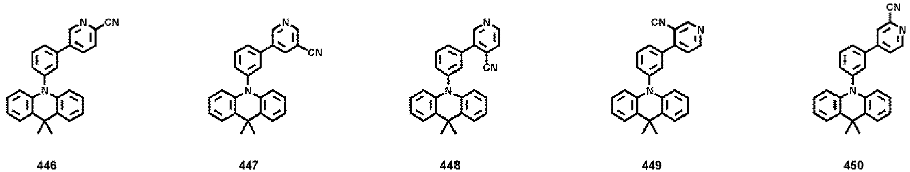

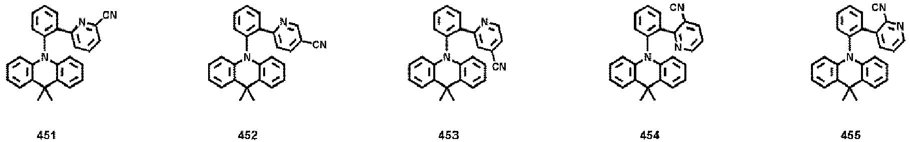

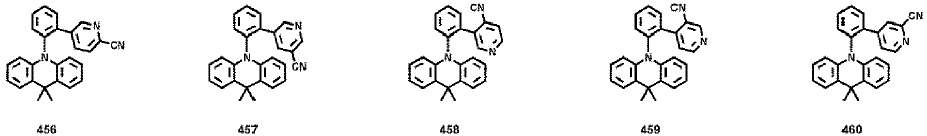

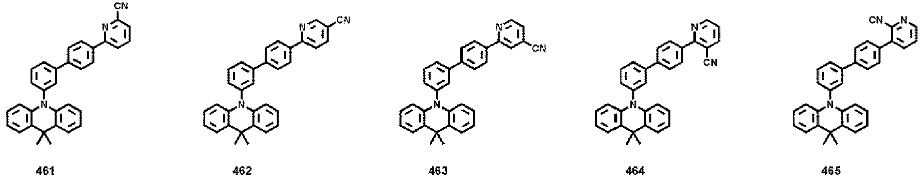

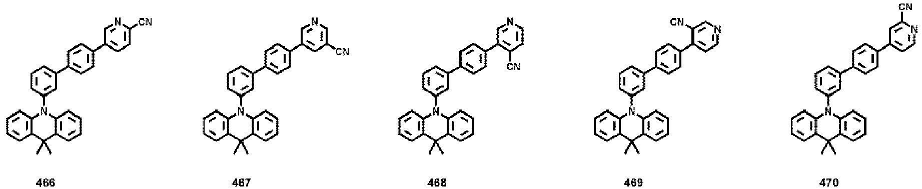

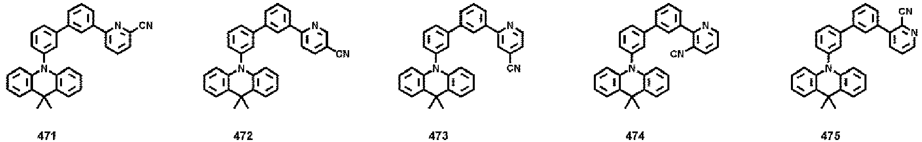

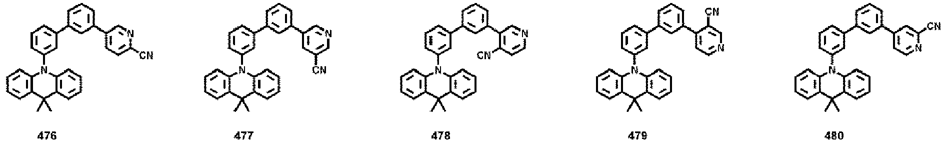

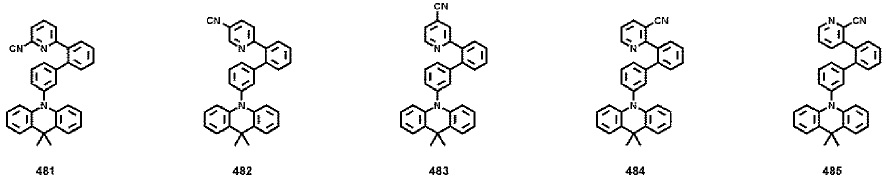

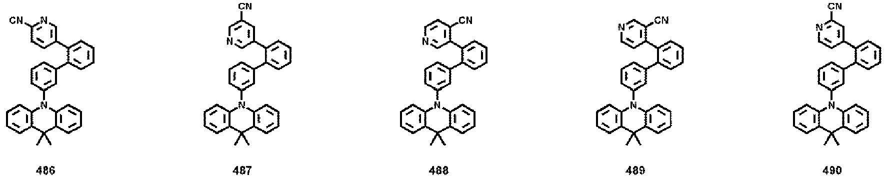

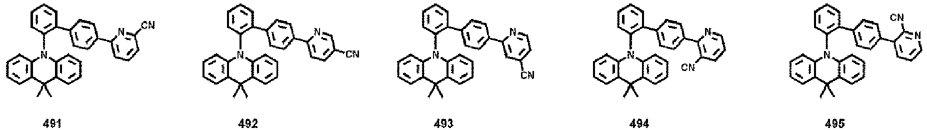

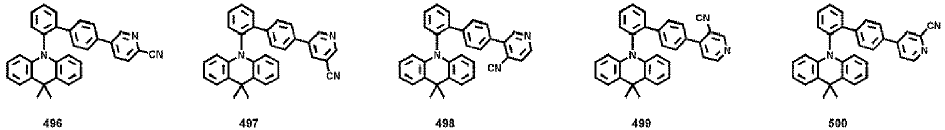

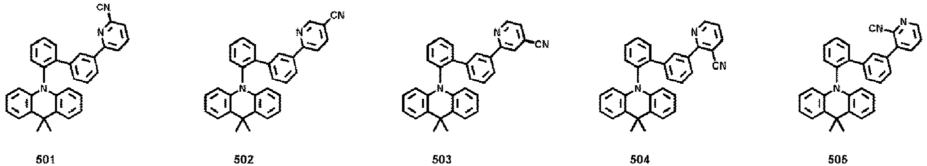

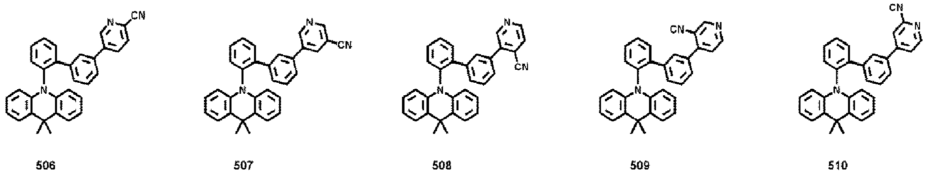

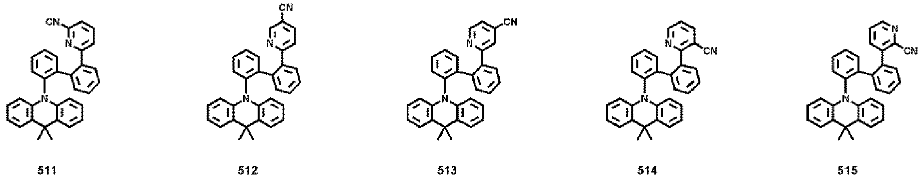

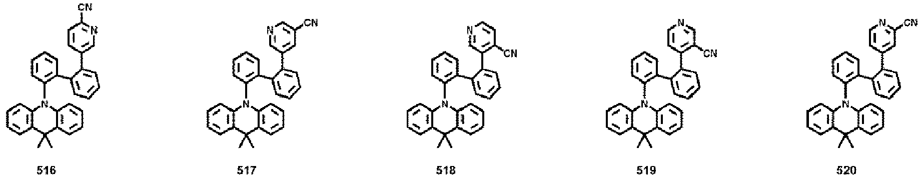

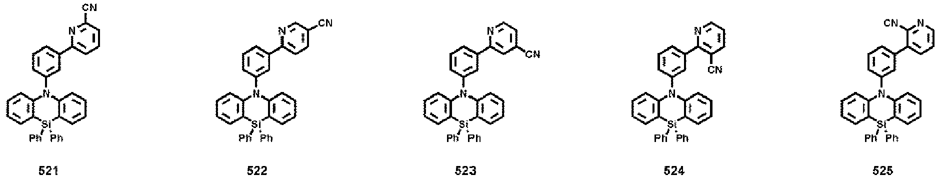

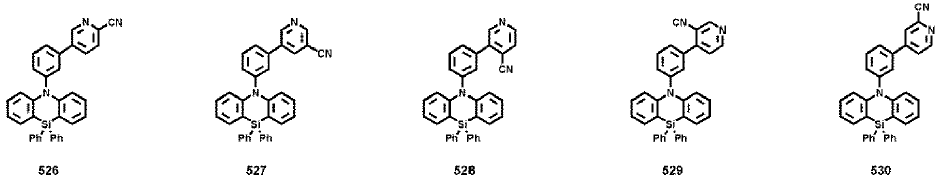

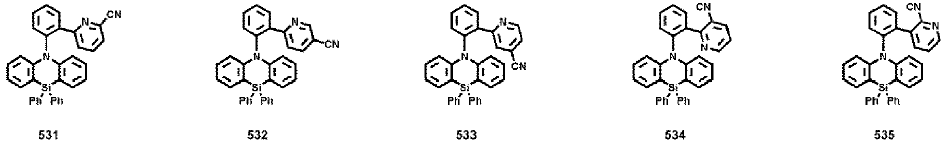

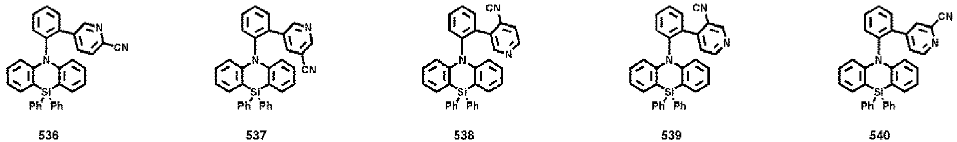

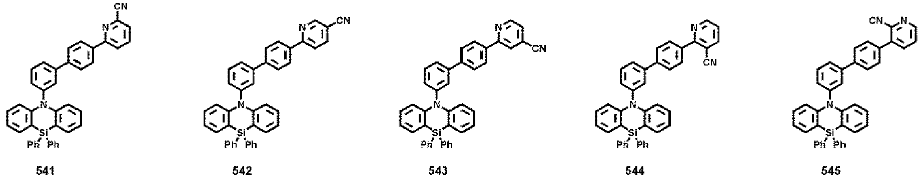

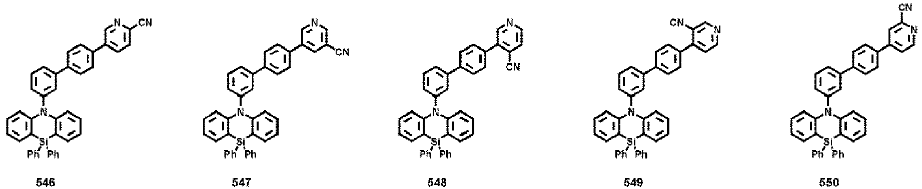

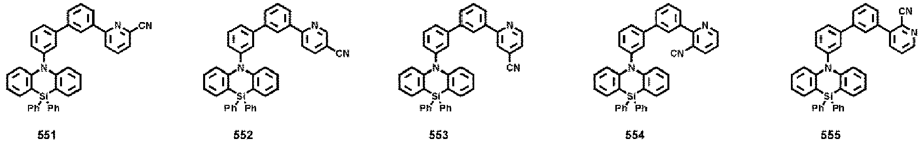

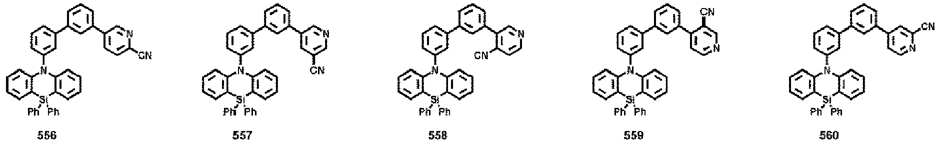

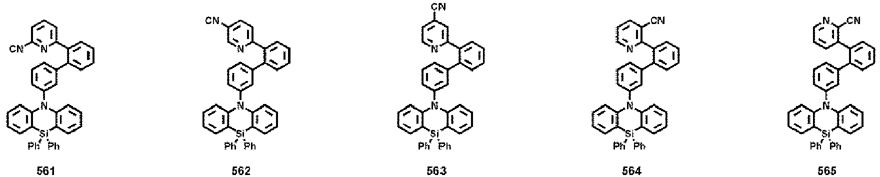

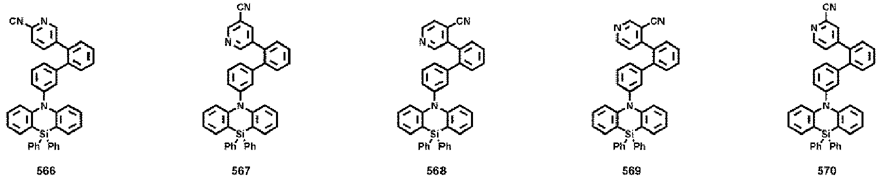

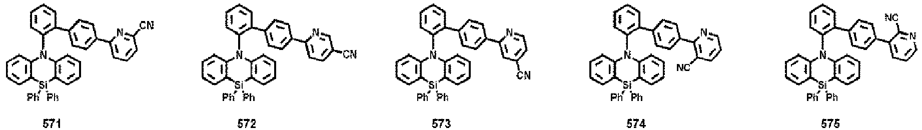

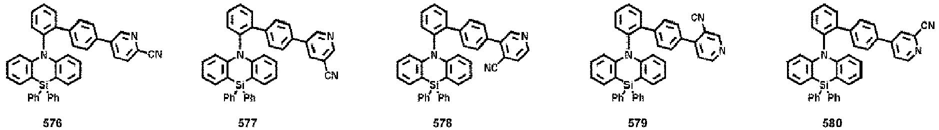

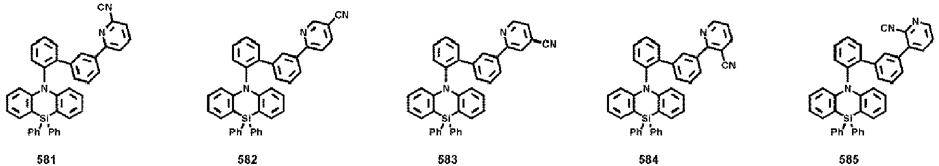

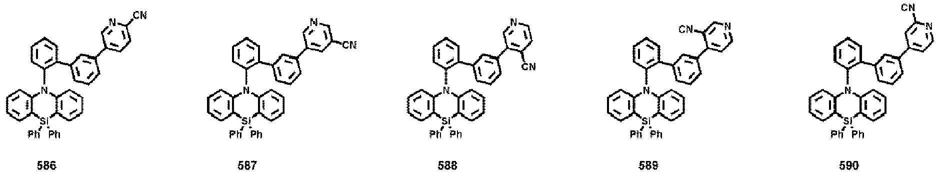

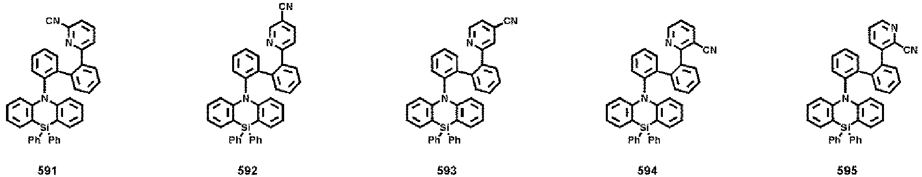

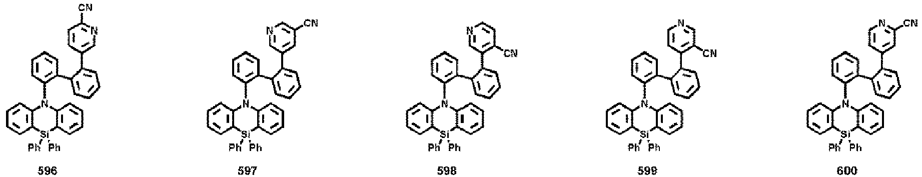

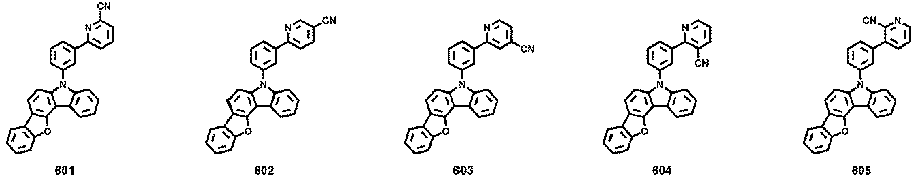

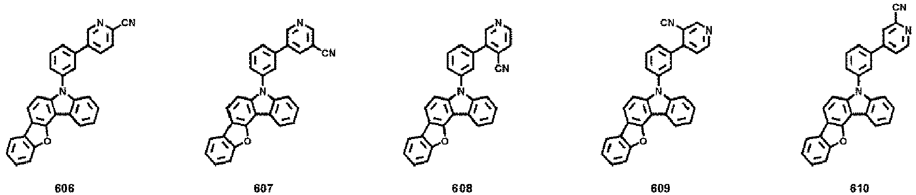

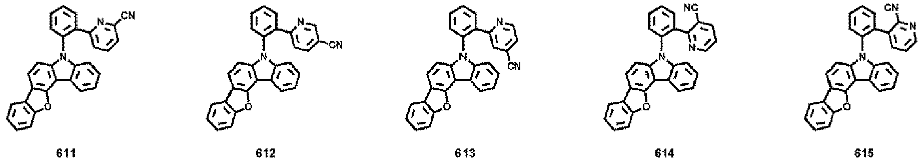

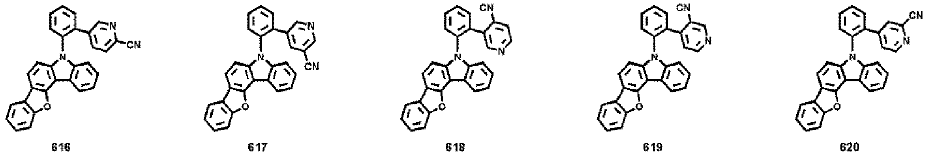

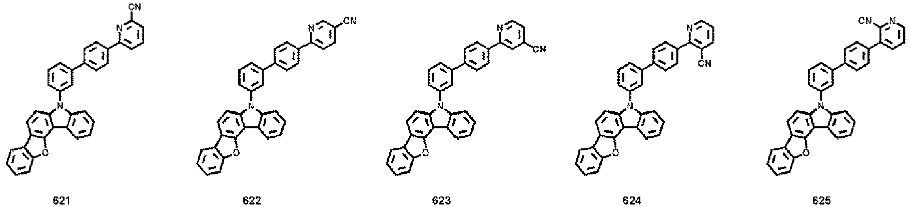

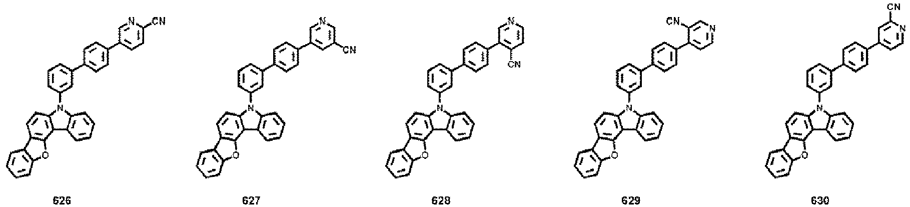

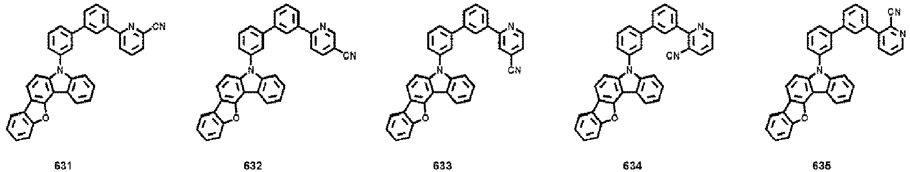

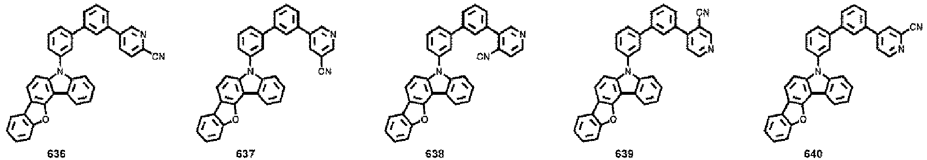

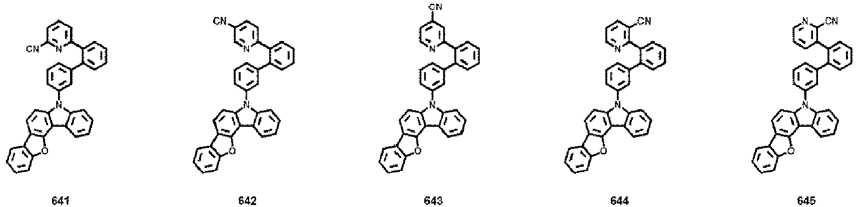

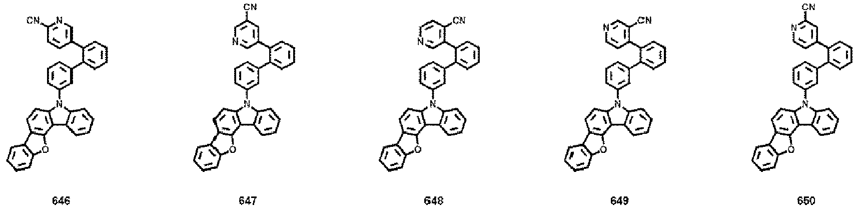

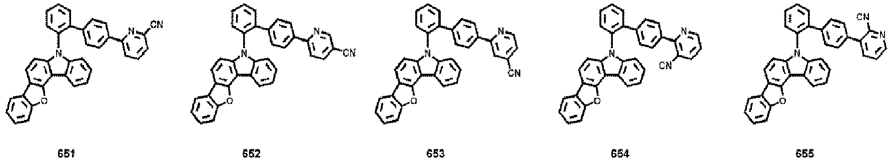

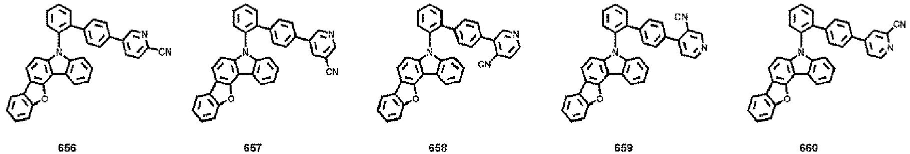

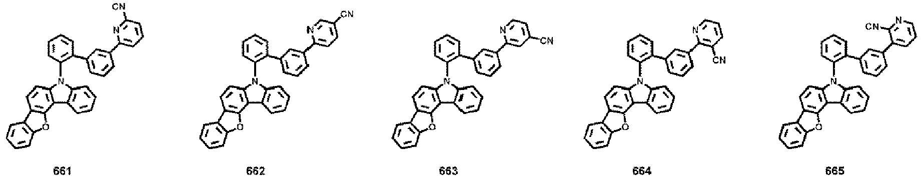

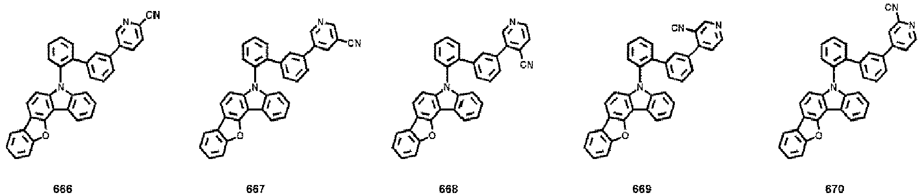

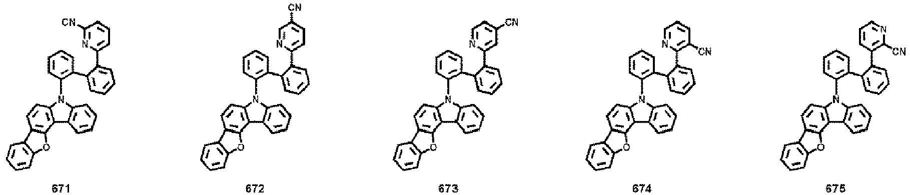

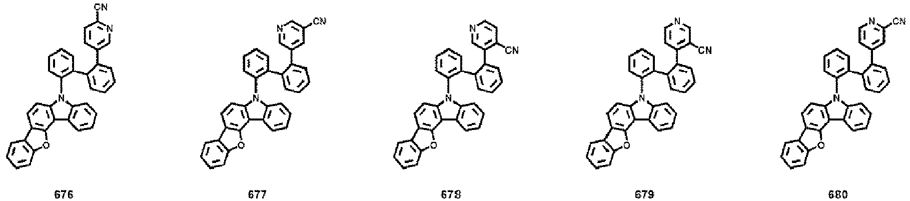

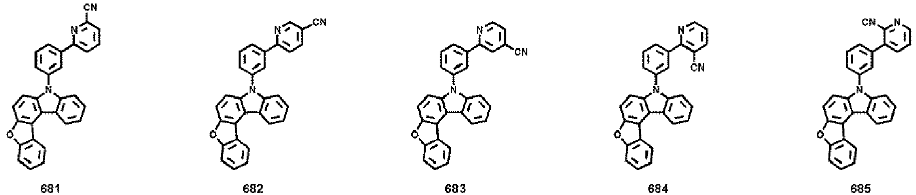

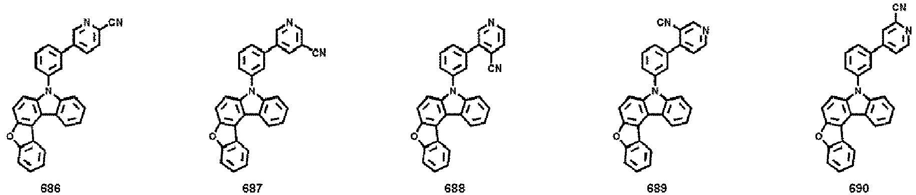

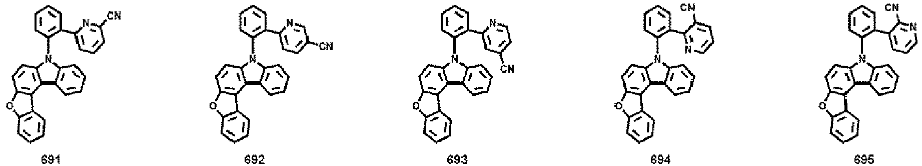

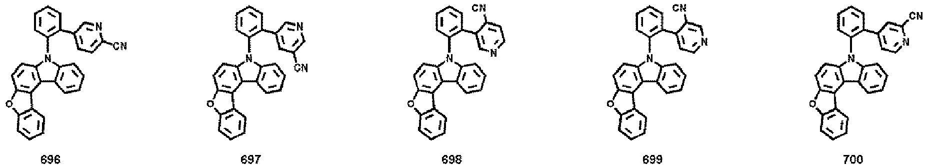

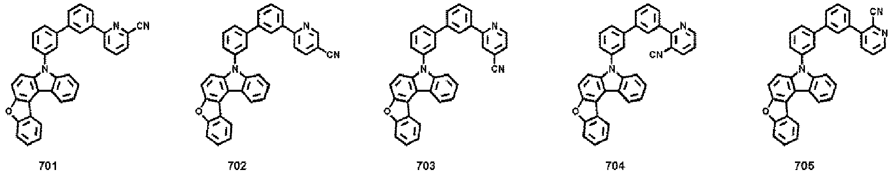

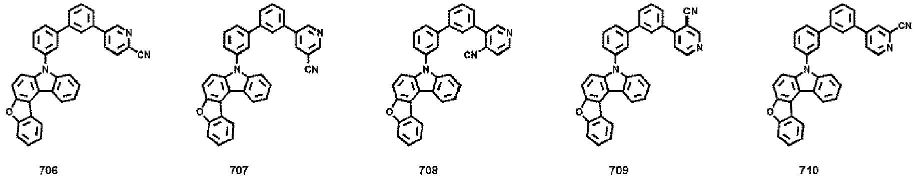

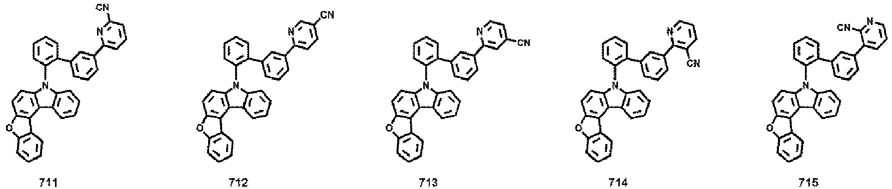

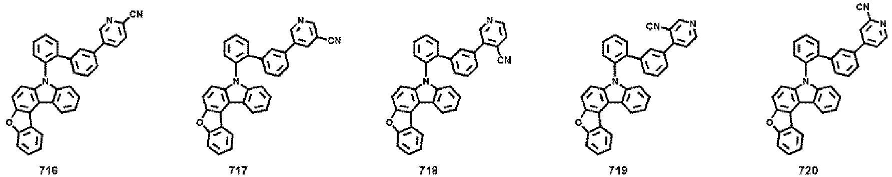

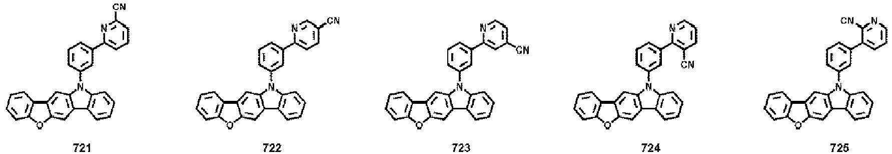

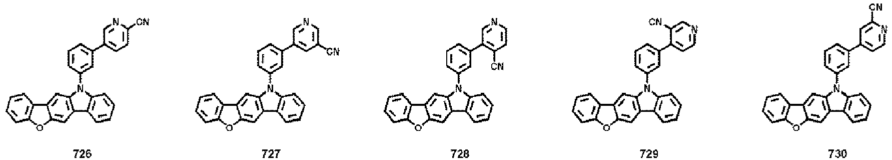

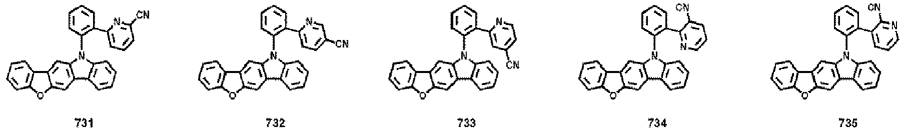

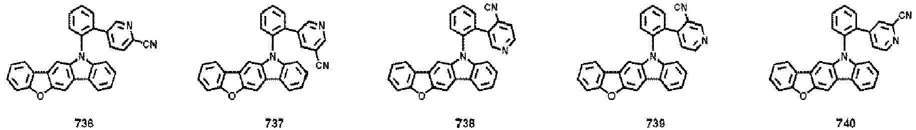

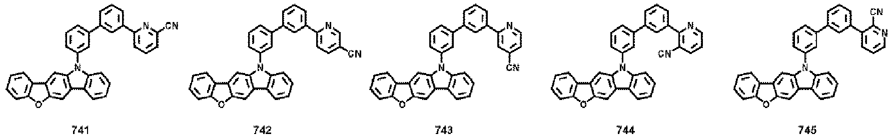

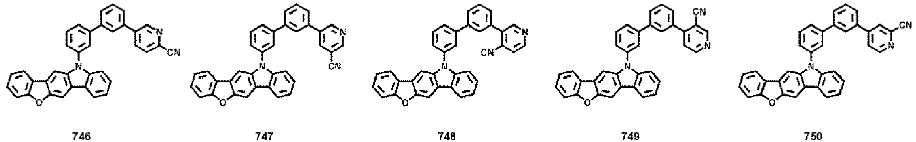

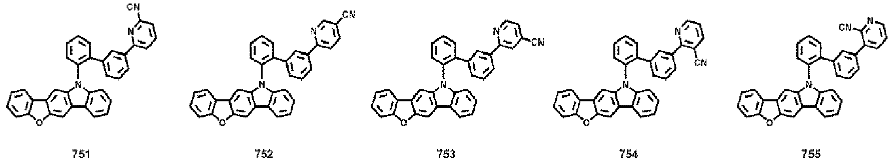

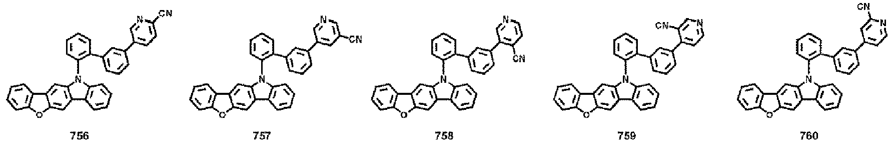

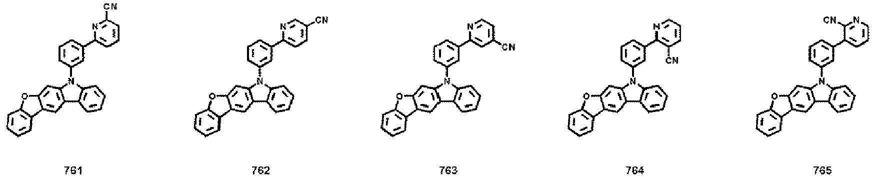

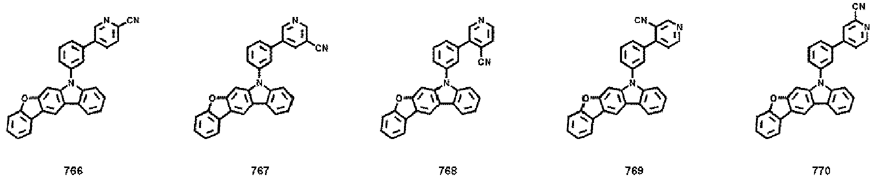

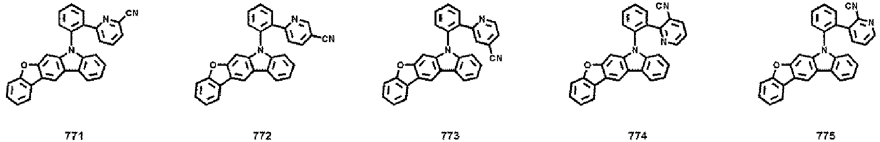

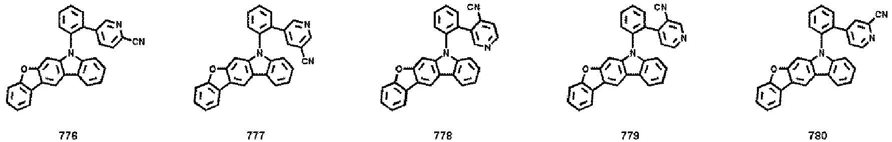

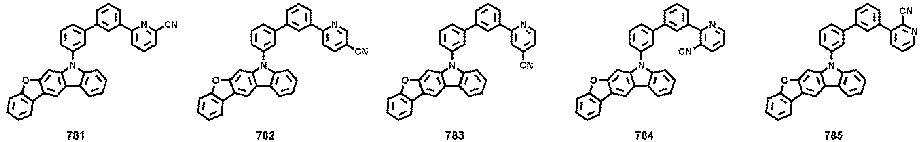

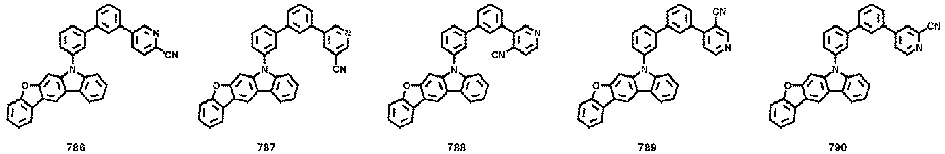

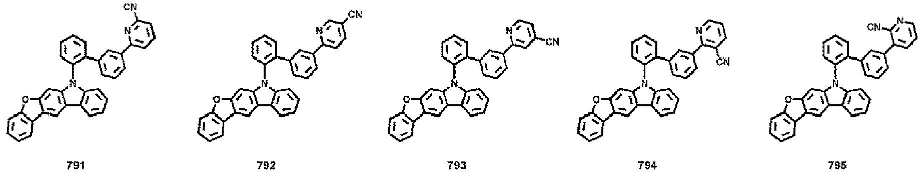

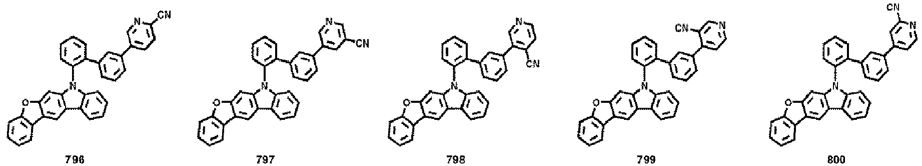

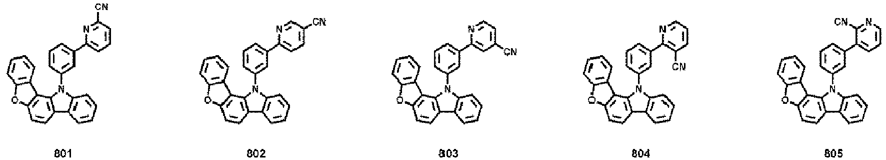

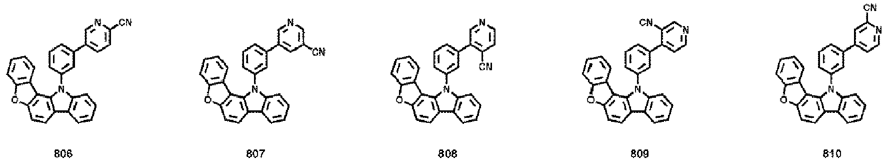

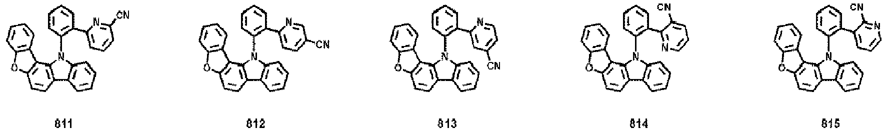

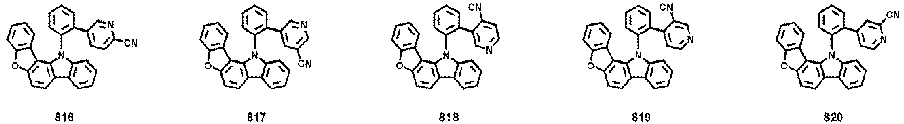

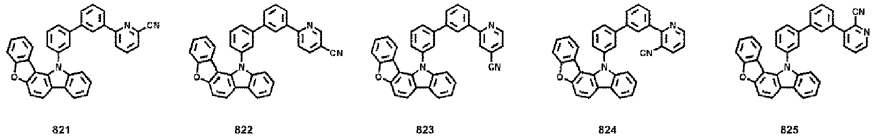

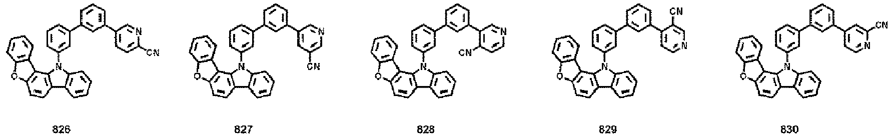

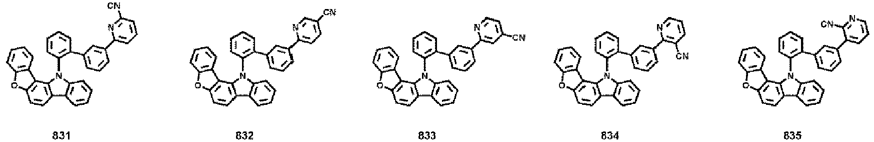

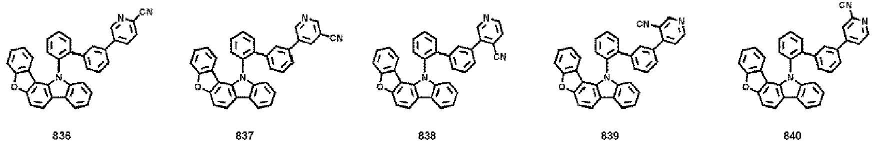

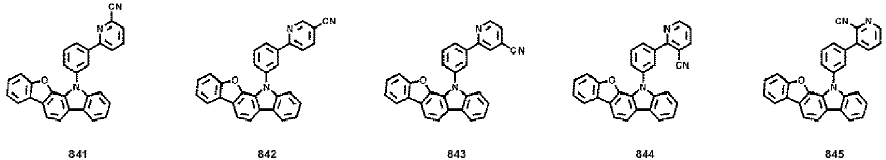

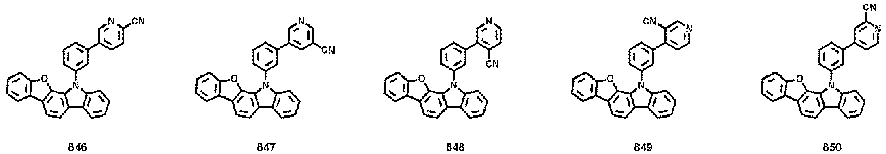

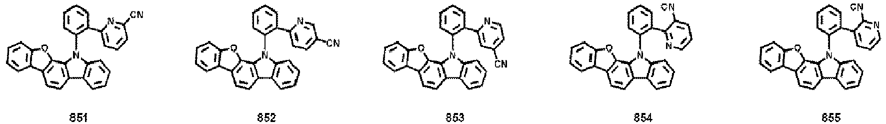

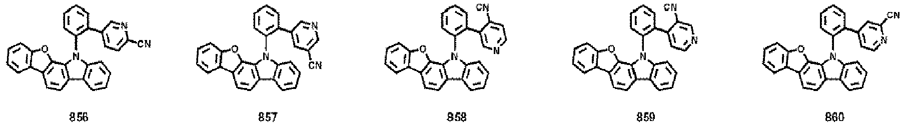

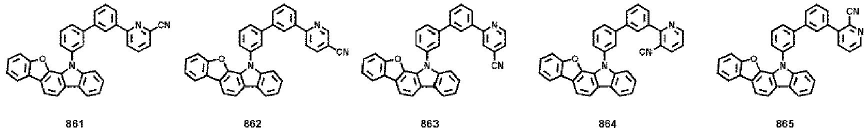

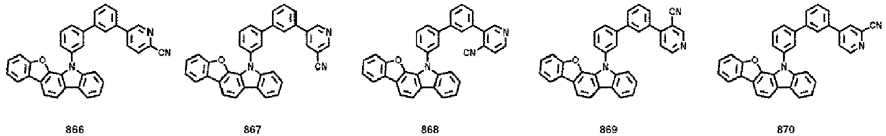

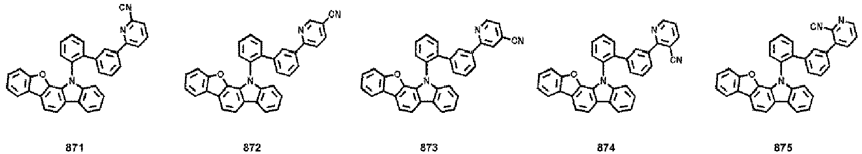

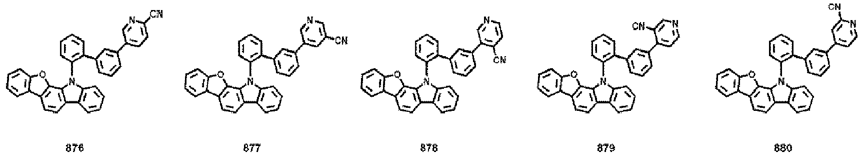

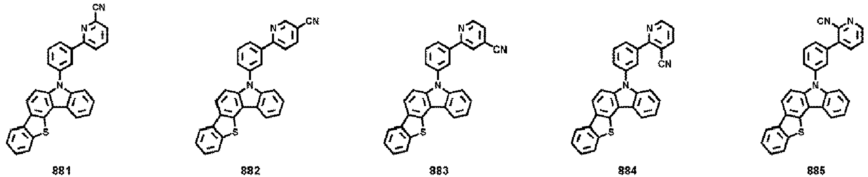

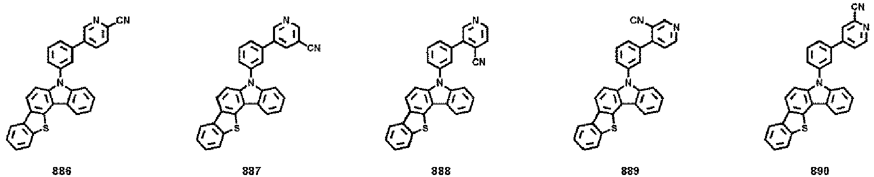

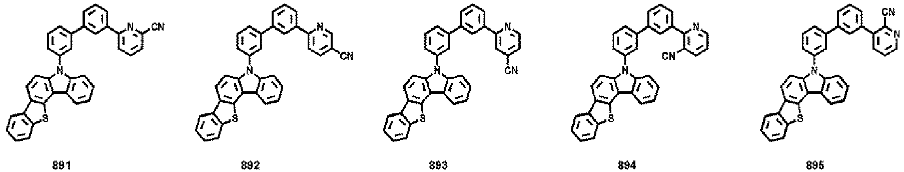

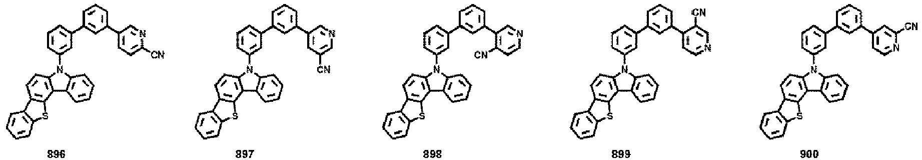

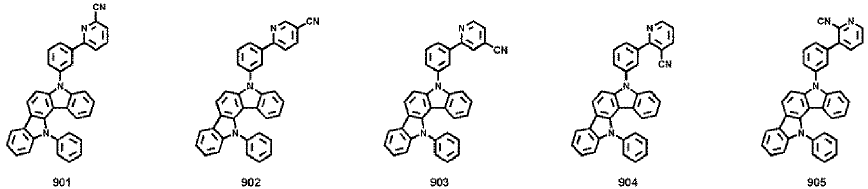

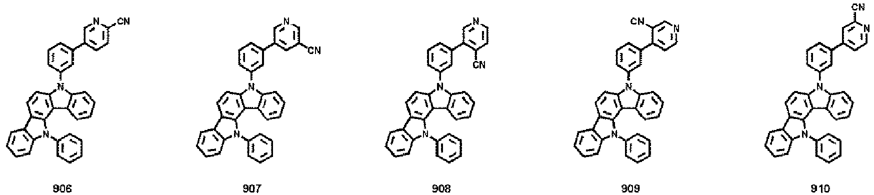

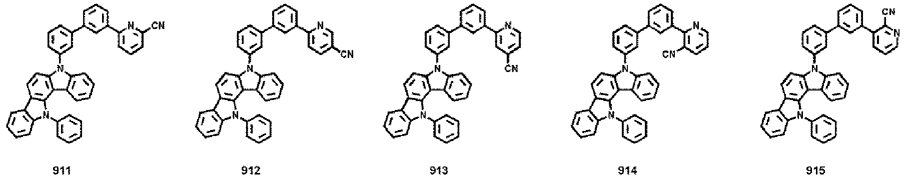

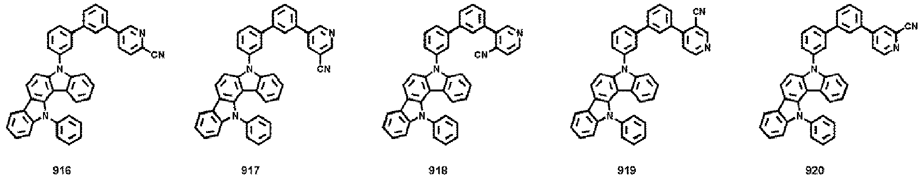

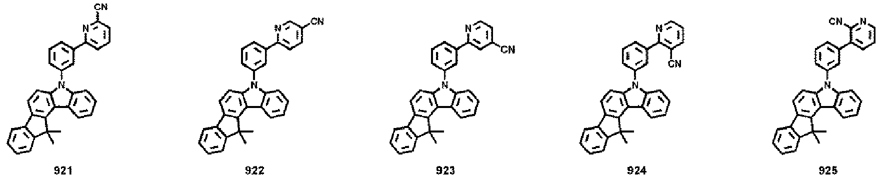

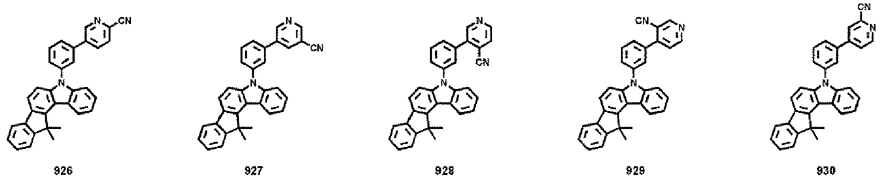

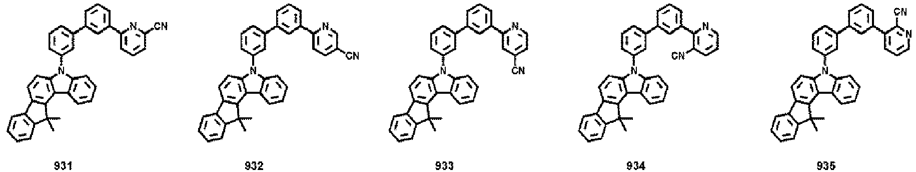

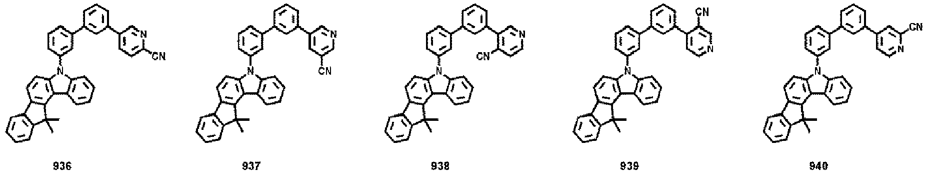

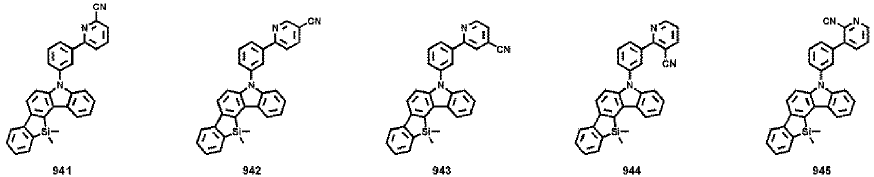

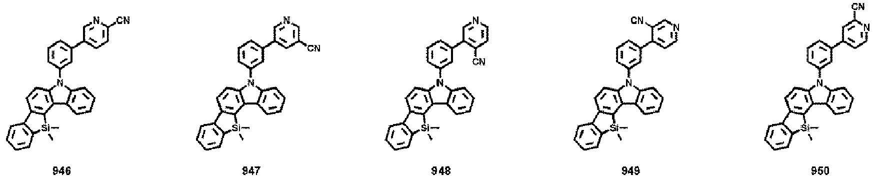

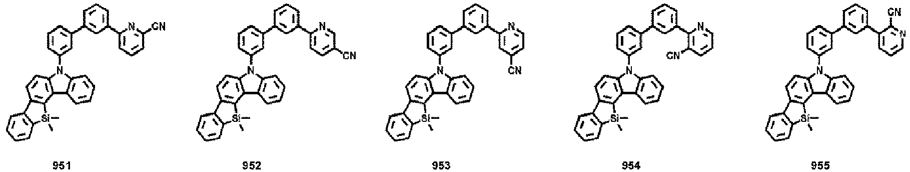

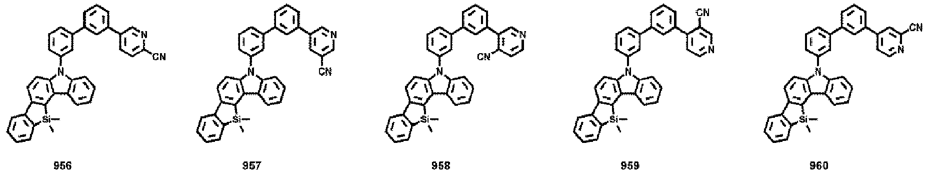

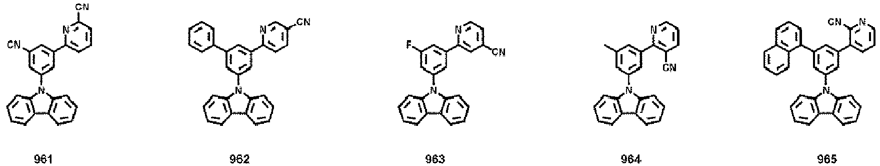

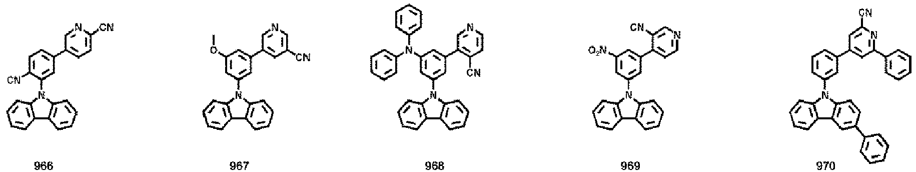

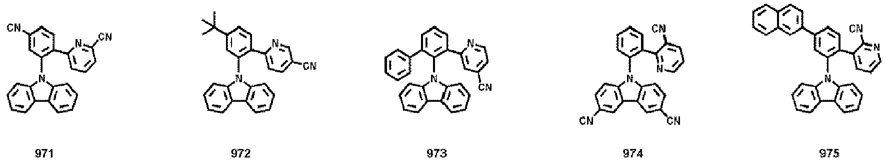

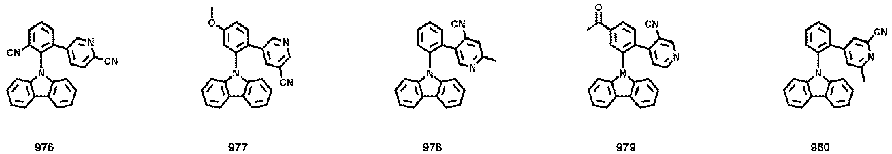

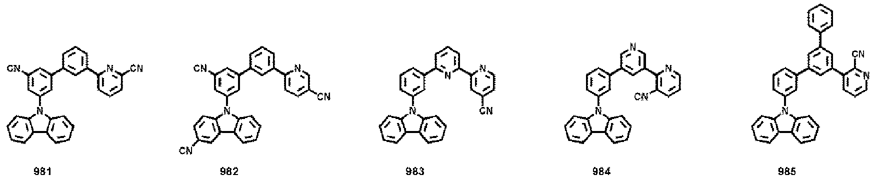

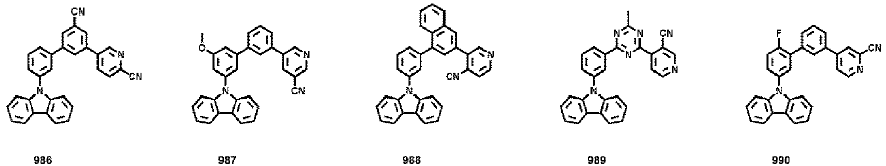

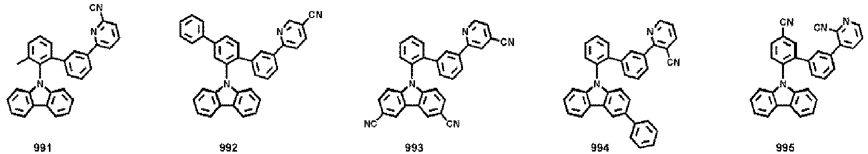

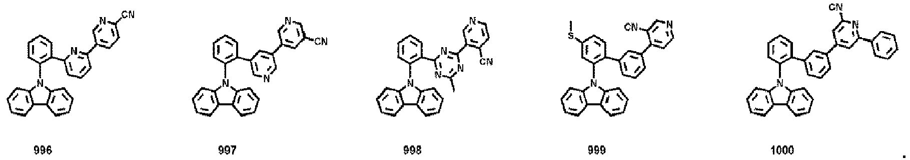

10. A composition comprising a first compound and a second compound,
wherein the first compound is the condensed cyclic compound of any of claims 1-9,
the second compound is different from the first compound,
the second compound comprises at least one selected from a carbazole group, a dibenzofuran group, a dibenzothiophene group, an indenocarbazole group, an indolocarbazole group, a benzofurocarbazole group, a benzothienocarbazole group, an acridine group, a dihydroacridine group, and a triindolobenzene group, and does not comprise an electron withdrawing group, and
the electron withdrawing group is selected from:
-F, -CFH₂, -CF₂H, -CF₃, -CN, and -NO₂;
a C₁-C₆₀ alkyl group substituted with at least one selected from -F, -CFH₂, -CF₂H, - CF₃, -CN, and -NO₂;
a C₁-C₆₀ heteroaryl group and a monovalent non-aromatic condensed polycyclic heterocyclic group, each comprising *=N-*' as a ring-forming moiety; and
a C₁-C₆₀ heteroaryl group and a monovalent non-aromatic condensed polycyclic heterocyclic group, each of which comprises *=N-*' as a ring-forming moiety and is substituted with at least one selected from deuterium, -F, -CFH₂, -CF₂H, -CF₃, -CI, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₇-C₆₀ arylalkyl group, a substituted or unsubstituted C₁-C₆₀ heteroaryl group, a substituted or unsubstituted C₁-C₆₀ heteroaryloxy group, a substituted or unsubstituted C₁-C₆₀ heteroarylthio group, a substituted or unsubstituted C₂-C₆₀ heteroarylalkyl group, a substituted or unsubstituted monovalent non-aromatic condensed polycyclic group, and a substituted or unsubstituted monovalent non-aromatic condensed heteropolycyclic group.

11. The composition of claim 10, wherein
the second compound is selected from compounds represented by Formula H-1: 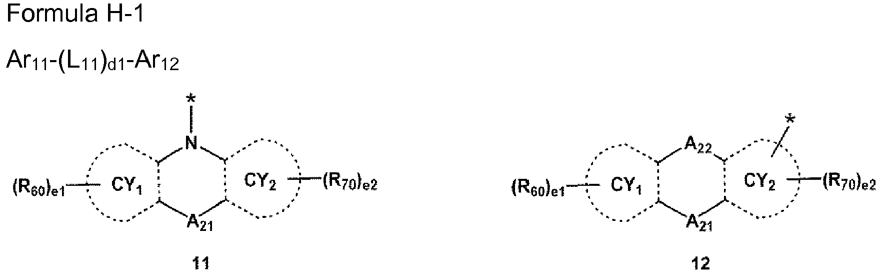 wherein, in Formulae H-1, 11, and 12,
L₁₁ is selected from:
a single bond, a phenylene group, a naphthylene group, a fluorenylene group, a carbazolylene group, a dibenzofuranylene group, and a dibenzothiophenylene group; and
a phenylene group, a naphthylene group, a fluorenylene group, a carbazolylene group, a dibenzofuranylene group, and a dibenzothiophenylene group, each substituted with at least one selected from deuterium, a C₁-C₁₀ alkyl group, a C₁-C₁₀ alkoxy group, a phenyl group, a naphthyl group, a fluorenyl group, a carbazolyl group, a dibenzofuranyl group, a dibenzothiophenyl group, a biphenyl group, and -Si(Q₁₁)(Q₁₂)(Q₁₃),
d1 is an integer from 1 to 10, and when d1 is two or more, two or more groups L₁₁ are identical to or different from each other,
Ar₁₁ is represented by one of Formulae 11 and 12,
Ar₁₂ is selected from
groups represented by Formulae 11 and 12, a phenyl group, and a naphthyl group; and
a phenyl group and a naphthyl group, each substituted with at least one selected from deuterium, a hydroxyl group, an amino group, an amidino group, a hydrazine group, a hydrazone group, a carboxylic acid group or a salt thereof, a sulfonic acid group or a salt thereof, a phosphoric acid group or a salt thereof, a C₁-C₂₀ alkyl group, a C₁-C₂₀ alkoxy group, a phenyl group, a naphthyl group, a fluorenyl group, a carbazolyl group, a dibenzofuranyl group, a dibenzothiophenyl group, and a biphenyl group,
ring CY₁ and ring CY₂ are each independently a benzene group, a naphthalene group, a phenanthrene group, an anthracene group, a cyclopentene group, a pyrrole group, a furan group, a thiophene group, a silole group, an indene group, an indole group, a benzofuran group, a benzothiophene group, a benzosilole group, a fluorene group, a carbazole group, a dibenzofuran group, a dibenzothiophene group, a dibenzosilole group, a benzocarbazole group, an indenocarbazole group, an indolocarbazole group, a benzofurocarbazole group, a benzothienocarbazole group, an acridine group, a dihydroacridine group, and a triindolobenzene group,
A₂₁ is selected from a single bond, O, S, N(R₅₁), C(R₅₁)(R₅₂), and Si(R₅₁)(R₅₂),
A22 is selected from a single bond, O, S, N(R53), C(R53)(R54), and Si(R53)(R54),
at least one selected from A₂₁ and A₂₂ in Formula 12 is not a single bond,
R₅₁ to R₅₄, R₆₀, and R₇₀ are each independently selected from:
hydrogen, deuterium, a hydroxyl group, an amino group, an amidino group, a hydrazine group, a hydrazone group, a carboxylic acid group or a salt thereof, a sulfonic acid group or a salt thereof, a phosphoric acid group or a salt thereof, a C₁-C₂₀ alkyl group, and a C₁-C₂₀ alkoxy group;
a C₁-C₂₀ alkyl group and a C₁-C₂₀ alkoxy group, each substituted with at least one selected from deuterium, a hydroxyl group, an amino group, an amidino group, a hydrazine group, a hydrazone group, a carboxylic acid group or a salt thereof, a sulfonic acid group or a salt thereof, a phosphoric acid group or a salt thereof, a phenyl group, a naphthyl group, a fluorenyl group, a carbazolyl group, a dibenzofuranyl group, and a dibenzothiophenyl group;
a phenyl group, a naphthyl group, a fluorenyl group, a carbazolyl group, a dibenzofuranyl group, and a dibenzothiophenyl group;
a phenyl group, a naphthyl group, a fluorenyl group, a carbazolyl group, a dibenzofuranyl group, and a dibenzothiophenyl group, each substituted with deuterium, a hydroxyl group, an amino group, an amidino group, a hydrazine group, a hydrazone group, a carboxylic acid group or a salt thereof, a sulfonic acid group or a salt thereof, a phosphoric acid group or a salt thereof, a phenyl group, a naphthyl group, a fluorenyl group, a carbazolyl group, a dibenzofuranyl group, and a dibenzothiophenyl group; and
-Si(Q₁)(Q2)(Q3);
e1 and e2 are each independently an integer from 0 to 10,
Q₁ to Q₃ and Q₁₁ to Q₁₃ are each independently selected from hydrogen, deuterium, a hydroxyl group, an amino group, an amidino group, a hydrazine group, a hydrazone group, a carboxylic acid group or a salt thereof, a sulfonic acid group or a salt thereof, a phosphoric acid group or a salt thereof, a phenyl group, a naphthyl group, a fluorenyl group, a carbazolyl group, a dibenzofuranyl group, a dibenzothiophenyl group, and a biphenyl group, and
* indicates a binding site to a neighboring atom.

12. The composition of claim 11, wherein
Ar₁₁ is a group represented by one of Formulae 11-1 to 11-8 and 12-1 to 12-8,
Ar₁₂ is selected from
a group represented by one of Formulae 11-1 to 11-8 and 12-1 to 12-8, a phenyl group, and a naphthyl group; and
a phenyl group and a naphthyl group, each substituted with at least one selected from deuterium, a hydroxyl group, an amino group, an amidino group, a hydrazine group, a hydrazone group, a carboxylic acid group or a salt thereof, a sulfonic acid group or a salt thereof, a phosphoric acid group or a salt thereof, a C₁-C₂₀ alkyl group, a C₁-C₂₀ alkoxy group, a phenyl group, a naphthyl group, a fluorenyl group, a carbazolyl group, a dibenzofuranyl group, a dibenzothiophenyl group, and a biphenyl group: 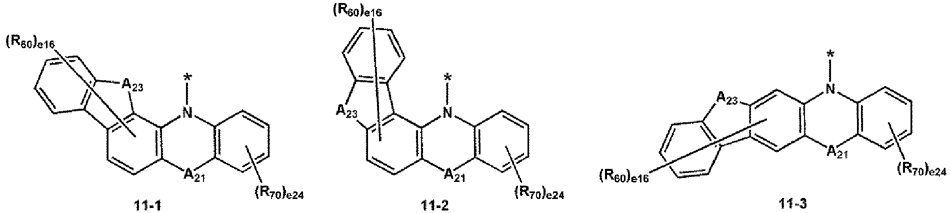 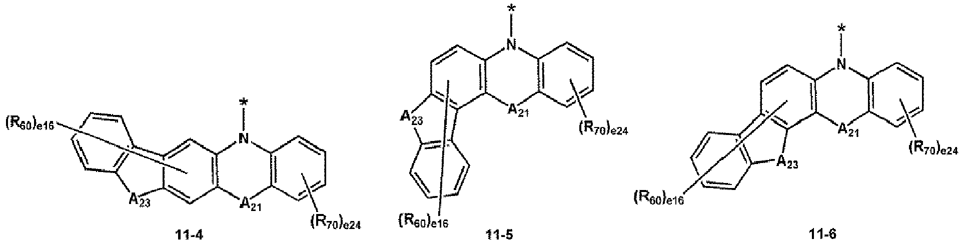 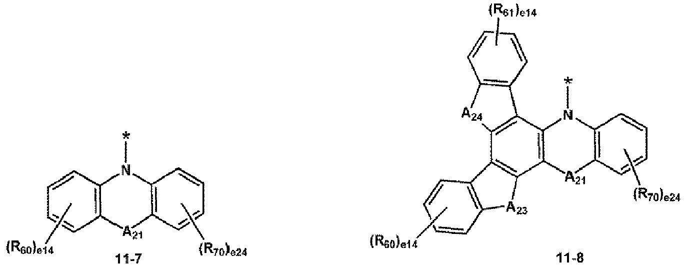 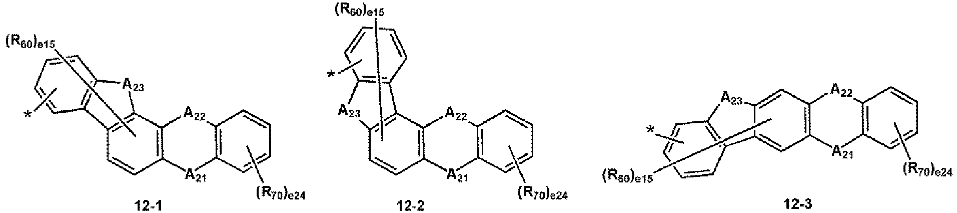 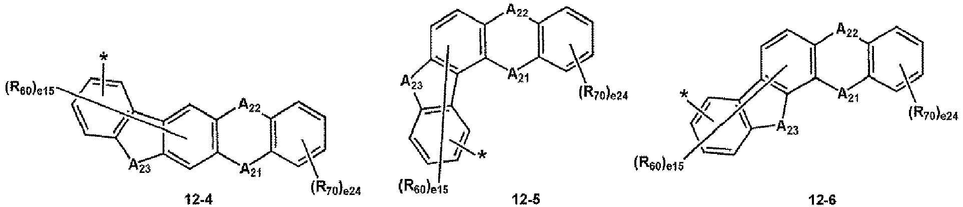 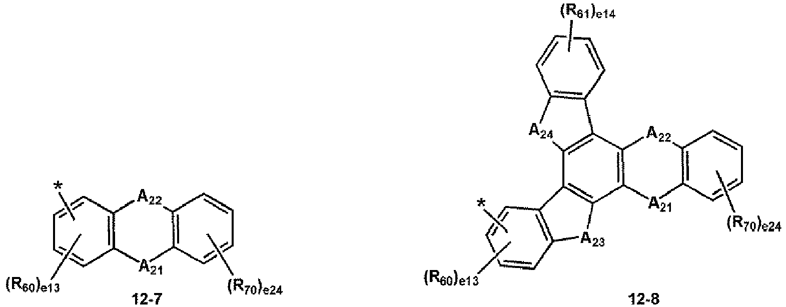 wherein, in Formulae 11-1 to 11-8 and 12-1 to 12-8,
A₂₃ is selected from O, S, N(R₅₅), C(R₅₅)(R₅₆), and Si(R₅₅)(R₅₆),
A₂₄ is selected from O, S, N(R₅₇), C(R₅₇)(R₅₈), and Si(R₅₇)(R₅₈),
A₂₁, A₂₂, R₆₀, and R₇₀ are the same as described in claim 11,
R₅₅ to R₅₈ are each the same as described in connection with R₅₁ in claim 11,
e16 is an integer from 0 to 6,
e15 is an integer from 0 to 5,
e14 is an integer from 0 to 4,
e13 is an integer from 0 to 3,
e24 is an integer from 0 to 4, and
* indicates a binding site to a neighboring atom.

13. An organic light-emitting device comprising:
a first electrode;
a second electrode; and
an organic layer disposed between the first electrode and the second electrode,
wherein the organic layer comprises at least one condensed cyclic compound represented by Formula 1 of any of claims 1-9.

14. The organic light-emitting device of claim 13, wherein
the emission layer comprises the at least one condensed cyclic compound;
preferably wherein the emission layer emits blue light.

15. The organic light-emitting device of claims 13 or 14, wherein
the emission layer comprises a host and a dopant,
the host comprises the at least one condensed cyclic compound, and
the dopant comprises a phosphorescent dopant; and/or
wherein
the organic layer comprises an electron transport region disposed between the emission layer and the second electrode,
the electron transport region comprises a hole blocking layer and an electron transport layer,
the hole blocking layer is disposed between the emission layer and the electron transport layer, and
the hole blocking layer comprises the at least one condensed cyclic compound.
